# EUROPEAN PATENT APPLICATION

(11) **EP 2 579 174 A1**
(43) Date of publication of application: **10.04.2013**
(21) Application number: 12196231.0
(22) Date of filing: 03.11.2007
(51) Int. Cl.: G06F 19/00

(54) **Diagnosis of metastatic melanoma and monitoring indicators of immunosuppression through blood leukocyte microarray analysis**

(30) Priority: 03.11.2006 US 856406 P
(62) Divisional of application: 07871360.9
(71) Applicant: Baylor Research Institute, Dallas, TX 75204 (US)
(72) Inventor: Palucka, Anna Karolina, Dallas, TX 75204 (US); Banchereau, Jacques F., Montclair, NJ 07042 (US); Chaussabel, Damien, Bainbridge Island, WA 98110 (US)
(74) Representative: Sonn & Partner Patentanwälte

(57) **Abstract**

The present invention includes compositions, systems and methods for the early detection and consistent determination of metastatic melanoma and/or immunosuppression using microarrays by calculating one or more expression vectors from the expression of one or more genes.

## Description

### TECHNICAL FIELD OF THE INVENTION

The present invention relates in general to the field of diagnostic for monitoring indicators of metastatic melanoma and/or immunosuppression, and more particularly, to a system, method and apparatus for the diagnosis, prognosis and tracking of metastatic melanoma and monitoring indicators of immunosuppression associated with transplant recipients (e.g., liver).

### LENGTHY TABLE

The patent application contains a lengthy table section. A copy of the table is available in electronic form from the USPTO web site (http://seqdata.uspto.gov/). An electronic copy of the table will also be available from the USPTO upon request and payment of the fee set forth in 37 CFR 1.19(b)(3).

### BACKGROUND OF THE INVENTION

Without limiting the scope of the invention, its background is described in connection with diagnostic methods.

Pharmacological immunosuppression has been instrumental in transplantation, transforming a last resort experimental procedure into a routinely successful one. Cyclosporin and tacrolimus/FK506 currently constitute the mainstay for transplant recipients. Activated T cells have been considered the main cellular targets for immunosuppressive treatments, but recent reports have also noted a marked effect of these drugs on antigen presenting cells, probably contributing further to the establishment of a generalized state of immune unresponsiveness (Lee et al., 2005b; Woltman et al., 2003). While severe immunosuppression generated by pharmacological treatment is necessary for the maintenance of graft survival and function, it also exposes the recipient to life-threatening infections and malignancies. Skin cancer is a well-established complication in organ transplant recipients (Gerlini et al., 2005). A recent epidemiological study showed that nearly 20% of 1115 renal transplant patients developed skin malignancies in Europe (Bordea et al., 2004), with much higher incidences being observed in less temperate climates, e.g., as high as 28% in Australia (Carroll et al., 2003). In addition to occurring more often, skin malignancies tend to take a more aggressive clinical course in transplanted patients, with a higher propensity to metastasize distantly and lead to a fatal outcome (Barrett et al., 1993).

Tumors maintain their survival by compromising the immune system. Different mechanisms have been identified including secretion of immunosuppressive factors such as cytokines (e.g., IL-10, TGF-beta), hormones (e.g., Prostaglandin E2), and others (e.g., MIA: Melanoma inhibitory activity, Tenascin C)(Jachimczak et al., 2005; Puente Navazo et al., 2001). Furthermore, tumors might promote development of suppressor T cells (Liyanage et al., 2002; Viguier et al., 2004), possibly via modulating dendritic cells (Gabrilovich, 2004; Lee et al., 2005a; Monti et al., 2004).

Thus, immunosuppression, whether from tumors or pharmacological treatments, has been linked to cancer progression. Therefore, a common molecular signature could be identified by profiling genome-wide transcriptional activity in peripheral blood mononuclear cell ("PBMC") samples obtained from immunosuppressed patients with either metastatic melanoma or liver allografts. The analysis of Leukocyte transcriptional profiles generated in the context of the present study supports this notion and identifies a blood signature of immunosuppression.

### SUMMARY OF THE INVENTION

Genomic research is facing significant challenges with the analysis of transcriptional data that are notoriously noisy, difficult to interpret and do not compare well across laboratories and platforms. The present inventors have developed an analytical strategy emphasizing the selection of biologically relevant genes at an early stage of the analysis, which are consolidated into analytical modules that overcome the inconsistencies among microarray platforms. The transcriptional modules developed may be used for the analysis of large gene expression datasets. The results derived from this analysis are easily interpretable and particularly robust, as demonstrated by the high degree of reproducibility observed across commercial microarray platforms.

Applications for this analytical process are illustrated through the mining of a large set of PBMC transcriptional profiles. Twenty-eight transcriptional modules regrouping 4,742 genes were identified. Using the present invention is it possible to demonstrate that diseases are uniquely characterized by combinations of transcriptional changes in, e.g., blood leukocytes, measured at the modular level. Indeed, module-level changes in blood leukocytes transcriptional levels constitute the molecular fingerprint of a disease or sample.

This invention has a broad range of applications. It can be used to characterize modular transcriptional components of any biological system (e.g., peripheral blood mononuclear cells (PBMCs), blood cells, fecal cells, peritoneal cells, solid organ biopsies, resected tumors, primary cells, cells lines, cell clones, etc.). Modular PBMC transcriptional data generated through this approach can be used for molecular diagnostic, prognostic, assessment of disease severity, response to drug treatment, drug toxicity, etc. Other data processed using this approach can be employed for instance in mechanistic studies, or screening of drug compounds. In fact, the data analysis strategy and mining algorithm can be implemented in generic gene expression data analysis software and may even be used to discover, develop and test new, disease- or condition-specific modules. The present invention may also be used in conjunction with pharmacogenomics, molecular diagnostic, bioinformatics and the like, wherein in-depth expression data may be used to improve the results (e.g., by improving or sub-selecting from within the sample population) that mat be obtained during clinical trails.

More particularly, the present invention includes arrays, apparatuses, systems and method for diagnosing a disease or condition by obtaining the transcriptome of a patient; analyzing the transcriptome based on one or more transcriptional modules that are indicative of a disease or condition; and determining the patient's disease or condition based on the presence, absence or level of expression of genes within the transcriptome in the one or more transcriptional modules. The transcriptional modules may be obtained by: iteratively selecting gene expression values for one or more transcriptional modules by: selecting for the module the genes from each cluster that match in every disease or condition; removing the selected genes from the analysis; and repeating the process of gene expression value selection for genes that cluster in a sub-fraction of the diseases or conditions; and iteratively repeating the generation of modules for each clusters until all gene clusters are exhausted.

Examples of clusters selected for use with the present invention include, but are not limited to, expression value clusters, keyword clusters, metabolic clusters, disease clusters, infection clusters, transplantation clusters, signaling clusters, transcriptional clusters, replication clusters, cell-cycle clusters, siRNA clusters, miRNA clusters, mitochondrial clusters, T cell clusters, B cell clusters, cytokine clusters, lymphokine clusters, heat shock clusters and combinations thereof. Examples of diseases or conditions for analysis using the present invention include, e.g., autoimmune disease, a viral infection a bacterial infection, cancer and transplant rejection. More particularly, diseases for analysis may be selected from one or more of the following conditions: systemic juvenile idiopathic arthritis, systemic lupus erythematosus, type I diabetes, liver transplant recipients, melanoma patients, and patients bacterial infections such as *Escherichia coli, Staphylococcus aureus,* viral infections such as influenza A, and combinations thereof. Specific array may even be made that detect specific diseases or conditions associated with a bioterror agent.

Cells that may be analyzed using the present invention, include, e.g., peripheral blood mononuclear cells (PBMCs), blood cells, fetal cells, peritoneal cells, solid organ biopsies, resected tumors, primary cells, cells lines, cell clones and combinations thereof. The cells may be single cells, a collection of cells, tissue, cell culture, cells in bodily fluid, e.g., blood. Cells may be obtained from a tissue biopsy, one or more sorted cell populations, cell culture, cell clones, transformed cells, biopies or a single cell. The types of cells may be, e.g., brain, liver, heart, kidney, lung, spleen, retina, bone, neural, lymph node, endocrine gland, reproductive organ, blood, nerve, vascular tissue, and olfactory epithelium cells. After cells are isolated, these mRNA from these cells is obtained and individual gene expression level analysis is performed using, e.g., a probe array, PCR, quantitative PCR, bead-based assays and combinations thereof. The individual gene expression level analysis may even be performed using hybridization of nucleic acids on a solid support using cDNA made from mRNA collected from the cells as a template for reverse transcriptase.

Pharmacological immunosuppression promotes graft survival in transplant recipients. Endogenous immunosuppression promotes tumor survival in cancer-bearing patients. Leukocytes from patients with metastatic melanoma display an endogenous immunosuppression signature common with liver transplant recipients under pharmacological immunosuppression. Blood microarray analyses were carried out in 25 healthy volunteers, 35 patients with metastatic melanoma, and 39 liver transplant recipients. Disease signatures were identified, and confirmed in an independent dataset, in comparison to healthy controls. Analysis of a set of 69 transcripts over-expressed preferentially in melanoma and transplant groups in comparison to six other diseases revealed remarkable functional convergence, including several repressors of interleukin-2 transcription, powerful inhibitors of NF-kappaB and MAPK pathways as well as antiproliferative molecules. Thus, patients with metastatic melanoma display an endogenous transcriptional signature of immunosuppression. This signature may now be used to identify patients at the high risk of metastatic melanoma progression.

The present invention includes a system and a method to analyze samples for the prognosis and diagnosis of metastatic melanoma and/or monitoring indicators of immunosuppression associated with transplant recipients (e.g., liver) using multiple variable gene expression analysis. The gene expression differences that remain can be attributed with a high degree of confidence to the unmatched variation. The gene expression differences thus identified can be used, for example, to diagnose disease, identify physiological state, design drugs and monitor therapies.

The sample may be screened by quantitating the mRNA, protein or both mRNA and protein level of the expression vector. When the screening is for mRNA levels, it may be quantitated by a method selected from polymerase chain reaction, real time polymerase chain reaction, reverse transcriptase polymerase chain reaction, hybridization, probe hybridization, and gene expression array. The screening method may also include detection of polymorphisms in the biomarker. Alternatively, the screening step may be accomplished using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing. For use with the present invention the sample may be any of a number of immune cells, e.g., leukocytes or sub-components thereof.

In one embodiment, the present invention includes a method of identifying a patient with melanoma by examining the phenotype a sample for combinations of six, seven, eight, ten, fifteen, twenty, twenty-five or more genes selected from Tables 2, 8, 9, 12 and combinations thereof.

In one embodiment, the present invention includes a method of identifying gene expression response to pharmacological immunosuppression in transplant recipients by examining the phenotype a sample for combinations of six, seven, eight, ten, fifteen, twenty, twenty-five or more genes selected from Tables 10, 11, 13 and combinations thereof.

The sample may be screened by quantitating the mRNA, protein or both mRNA and protein level of the expression vector. When mRNA level is examined, it may be quantitated by a method selected from polymerase chain reaction, real time polymerase chain reaction, reverse transcriptase polymerase chain reaction, hybridization, probe hybridization, and gene expression array. The screening method may also include detection of polymorphisms in the biomarker. Alternatively, the screening step may be accomplished using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing. For use with the present invention the sample may be any of a number of immune cells, e.g., leukocytes or sub-components thereof.

The expression vector may be screened by quantitating the mRNA, protein or both mRNA and protein level of the expression vector. When the expression vector is mRNA level, it may be quantitated by a method selected from polymerase chain reaction, real time polymerase chain reaction, reverse transcriptase polymerase chain reaction, hybridization, probe hybridization, and gene expression array. The screening method may also include detection of polymorphisms in the biomarker. Alternatively, the screening step may be accomplished using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing. For use with the present invention the sample may be any of a number of immune cells, e.g., leukocytes or sub-components thereof.

For example, a method of identifying a subject with melanoma by determining a database that includes the level of expression of one or more metastatic melanoma expression vectors. Another embodiment of the present invention includes a computer implemented method for determining the genotype of a sample by obtaining a plurality of sample probe intensities. Diagnosing metastatic melanoma is based upon the sample probe intensities and calculating a linear correlation coefficient between the sample probe intensities and reference probe intensities.

The present invention also includes a computer readable medium including computer-executable instructions for performing the method of determining the genotype of a sample. The method of determining the phenotype includes obtaining a plurality of sample probe intensities and diagnosing melanoma based upon the sample probe intensities for two or more metastatic melanoma expression vectors selected from those listed in Tables 2, 3, 8, 9, 12 and combinations thereof into a dataset; and calculating a linear correlation coefficient between the sample probe intensities and a reference probe intensity. The tentative phenotype is accepted as the phenotype of the sample if the linear correlation coefficient is greater than a threshold value. In addition, the present invention includes a microarray for identifying a human subject with melanoma. The microarray includes the detection of expression of two or more metastatic melanoma genes listed in Tables 2, 8, 9, 12 and combinations thereof into a dataset. The present invention provides a method of distinguishing between metastatic melanoma and immunosuppression associated with transplants by determining the level of expression of one or more genes, and calculating one or more gene expression vectors. The melanoma-specific transcriptome-expression vectors may include values for the upregulation or downregulation of six or more genes listed in Tables 2, 3, 8, 9, 12 and combinations thereof. The present invention provides a method of identifying a subject with immunosuppression associated with transplants by determining the level of expression of one or more immunosuppression associated expression vectors. The immunosuppression-specific transcriptome-expression vectors may include values for the upregulation or downregulation of six or more genes listed in Tables 10, 11 and 13 and combinations thereof.

The present invention also includes a computer implemented method for determining the propensity for immunosuppression in a sample including obtaining a plurality of sample probe intensities and diagnosing immunosuppression based upon the sample probe intensities. A linear correlation coefficient is calculating between the plurality of sample probe intensities and a reference probe intensity. A tentative genotype is then accepted as the genotype of the sample if the linear correlation coefficient is greater than a threshold value. The melanoma-specific transcriptome-expression vectors may include values for the upregulation or downregulation of six or more genes listed in Tables 2, 8, 9, 12 and combinations thereof. The immunosuppression-specific transcriptome-expression vectors may include values for the upregulation or downregulation of six or more genes listed in Tables 10, 11 and 13 and combinations thereof.

A computer readable medium is also included that has computer-executable instructions for performing the method for determining the phenotype of a sample. The method for determining the phenotype of a sample includes obtaining a plurality of sample probe intensities and diagnosing immunosuppression based upon the sample probe intensities for two or more immunosuppression associated expression vectors. A linear correlation coefficient is calculated between the sample probe intensities and a reference probe intensity and a tentative phenotype is accepted as the phenotype of the sample if the linear correlation coefficient is greater than a threshold value. The present invention also includes a system for diagnosing immunosuppression including an expression level detector for determining the expression level of two or more immunosuppression expression vectors selected from the 1, 2, 3, 4, 5, 6, 8, 10, 15, 20, 25 or more genes. The melanoma-specific transcriptome-expression vectors that are used to generate expression data for each gene, which is saved into a dataset, may include values for the upregulation or downregulation of six or more genes listed in Tables 2, 8, 9, 12 and combinations thereof. The immunosuppression-specific transcriptome-expression vectors may include values in a dataset that includes the upregulation or downregulation of six or more genes listed in Tables 10, 11 and 13 and combinations thereof. The presence of melanoma or immunosuppression is determined based on the presence of two or more positive indicators in a gene expression vector dataset.

The arrays, methods and systems of the present invention may even be used to select patients for a clinical trial by obtaining the transcriptome of a prospective patient; comparing the transcriptome to one or more transcriptional modules that are indicative of a disease or condition that is to be treated in the clinical trial; and determining the likelihood that a patient is a good candidate for the clinical trial based on the presence, absence or level of one or more genes that are expressed in the patient's transcriptome within one or more transcriptional modules that are correlated with success in a clinical trial. Generally, for each module a vector that correlates with a sum of the proportion of transcripts in a sample may be used, e.g., when each module includes a vector and wherein one or more diseases or conditions is associated with the one or more vectors. Therefore, each module may include a vector that correlates to the expression level of one or more genes within each module.

The present invention also includes arrays, e.g., custom microarrays that include nucleic acid probes immobilized on a solid support that includes sufficient probes from one or more expression vectors to provide a sufficient proportion of differentially expressed genes to distinguish between one or more diseases. For example, an array of nucleic acid probes immobilized on a solid support, in which the array includes at least two sets of probe modules, wherein the probes in the first probe set have one or more interrogation positions respectively corresponding to one or more diseases. The array may have between 100 and 100,000 probes, and each probe may be, e.g., 9-21 nucleotides long. When separated into organized probe sets, these may be interrogated separately.

The present invention also includes one or more nucleic acid probes immobilized on a solid support to form a module array that includes at least one pair of first and second probe groups, each group having one or more probes as defined by Table 1. The probe groups are selected to provide a composite transcriptional marker vector that is consistent across microarray platforms. In fact, the probe groups may even be used to provide a composite transcriptional marker vector that is consistent across microarray platforms and displayed in a summary for regulatory approval. The skilled artisan will appreciate that using the modules of the present invention it is possible to rapidly develop one or more disease specific arrays that may be used to rapidly diagnose or distinguish between different disease and/or conditions.

The present invention also include a method for displaying transcriptome vector data from a transcriptome vector dataset by separating one or more genes into one or more modules to visually display an aggregate gene expression vector value for each of the modules; and displaying the aggregate gene expression vector value for overexpression, underexpression or equal expression of the aggregate gene expression vector value in each module. In one example, overexpression is identified with a first identifier and underexpression is identified with a second identifier. Examples of identifiers include colors, shapes, patterns, light/dark, on/off, symbols and combinations thereof. For example, overexpression is identified with a first identifier and underexpression is identified with a second identifier, wherein the first identifier is a first color and the second identifier is a second color, and wherein first and second identifiers are superimposed to provide a combined color.

### BRIEF DESCRIPTION OF THE DRAWINGS

For a more complete understanding of the features and advantages of the present invention, reference is now made to the detailed description of the invention along with the accompanying figures and in which:
FIGURES 1A to 1C show the basic microarray data mining strategy steps involved in accepted gene-level microarray data analysis (FIGURE 1A), the modular mining strategy of the present invention
FIGURE 1B and a full size representation of the module extraction algorithm FIGURE 1C to generate on or more datasets used to create the expression vectors;
FIGURE 2 is a graph representing transcriptional profiles showing levels of modular gene expression profiles across an independent group of samples;
FIGURE 3 is a distribution of keyword occurrence in the literature obtained for four sets of coordinately expressed genes;
FIGURE 4 illustrates a modular microarray analysis strategy for characterization of the transcriptional system;
FIGURE 5 is an analysis of patient blood leukocyte transcriptional profiles;
FIGURE 6 illustrates module maps of transcriptional changes caused by disease;
FIGURE 7 illustrates the identification of a blood leukocyte transcriptional signature in patients with metastatic melanoma;
FIGURE 8 illustrates the validation of microarray results in an independent set of samples;
FIGURE 9 illustrates the identification of a blood leukocyte transcriptional signature in transplant recipients under immunosuppressive drug therapy
FIGURE 10-13 illustrate detailed results of the module-level analysis;
FIGURE 14 illustrates the module-level analysis for distinctive transcriptional signatures in blood from patients with metastatic melanoma and from liver transplant recipients;
FIGURE 15 illustrates the mapping transcriptional changes in patient blood leukocytes at the module level;
FIGURE 16 illustrates the module-level analysis for common transcriptional signatures in blood from patients with metastatic melanoma and from liver transplant recipients;
FIGURE 17 illustrates the analysis of significance patterns with genes expressed at higher levels in both melanoma and liver transplant patients compared to healthy volunteers;
FIGURE 18 illustrates the modular distribution of ubiquitous and specific gene signatures common to melanoma and transplant groups;
FIGURE 19 illustrates the transcriptional signature of immunosuppression;
FIGURE 20 shows a statistical group comparison between patients and their respective controls;
FIGURE 21 shows the analysis of significance patterns for genes over-expressed in SLE patients but not in patients with acute Influenza A infection;
FIGURE 22 shows the patterns of significance for genes common to Influenza A and SLE; and
FIGURE 23 is a functional analysis of genes shared by patients with Influenza infection and Lupus grouped according to significance patterns.

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims. Unless defined otherwise, all technical and scientific terms used herein have the meaning commonly understood by a person skilled in the art to which this invention belongs. The following references provide one of skill with a general definition of many of the terms used in this invention: Singleton, et al., Dictionary Of Microbiology And Molecular Biology (2d ed. 1994); The Cambridge Dictionary Of Science And Technology (Walker ed., 1988); The Glossary Of Genetics, 5th Ed., R. Rieger et al. (eds.), Springer Verlag (1991); and Hale & Marham, The Harper Collins Dictionary Of Biology (1991).

Various biochemical and molecular biology methods are well known in the art. For example, methods of isolation and purification of nucleic acids are described in detail in WO 97/10365, WO 97/27317, Chapter 3 of Laboratory Techniques in Biochemistry and Molecular Biology: Hybridization With Nucleic Acid Probes, Part I. Theory and Nucleic Acid Preparation, (P. Tijssen, ed.) Elsevier, N.Y. (1993); Chapter 3 of Laboratory Techniques in Biochemistry and Molecular Biology: Hybridization With Nucleic Acid Probes, Part 1. Theory and Nucleic Acid Preparation, (P. Tijssen, ed.) Elsevier, N.Y. (1993); and Sambrook et al., Molecular Cloning: A Laboratory Manual, Cold Spring Harbor Press, N.Y., (1989); and Current Protocols in Molecular Biology, (Ausubel, F. M. et al., eds.) John Wiley & Sons, Inc., New York (1987-1999), including supplements such as supplement 46 (April 1999).

### BIOINFORMATICS DEFINITIONS

As used herein, an "object" refers to any item or information of interest (generally textual, including noun, verb, adjective, adverb, phrase, sentence, symbol, numeric characters, etc.). Therefore, an object is anything that can form a relationship and anything that can be obtained, identified, and/or searched from a source. "Objects" include, but are not limited to, an entity of interest such as gene, protein, disease, phenotype, mechanism, drug, etc. In some aspects, an object may be data, as further described below.

As used herein, a "relationship" refers to the co-occurrence of objects within the same unit (e.g., a phrase, sentence, two or more lines of text, a paragraph, a section of a webpage, a page, a magazine, paper, book, etc.). It may be text, symbols, numbers and combinations, thereof

As used herein, "meta data content" refers to information as to the organization of text in a data source. Meta data can comprise standard metadata such as Dublin Core metadata or can be collection-specific. Examples of metadata formats include, but are not limited to, Machine Readable Catalog (MARC) records used for library catalogs, Resource Description Format (RDF) and the Extensible Markup Language (XML). Meta objects may be generated manually or through automated information extraction algorithms.

As used herein, an "engine" refers to a program that performs a core or essential function for other programs. For example, an engine may be a central program in an operating system or application program that coordinates the overall operation of other programs. The term "engine" may also refer to a program containing an algorithm that can be changed. For example, a knowledge discovery engine may be designed so that its approach to identifying relationships can be changed to reflect new rules of identifying and ranking relationships.

As used herein, "semantic analysis" refers to the identification of relationships between words that represent similar concepts, e.g., though suffix removal or stemming or by employing a thesaurus. "Statistical analysis" refers to a technique based on counting the number of occurrences of each term (word, word root, word stem, n-gram, phrase, etc.). In collections unrestricted as to subject, the same phrase used in different contexts may represent different concepts. Statistical analysis of phrase co-occurrence can help to resolve word sense ambiguity. "Syntactic analysis" can be used to further decrease ambiguity by part-of-speech analysis. As used herein, one or more of such analyses are referred to more generally as "lexical analysis." "Artificial intelligence (AI)" refers to methods by which a non-human device, such as a computer, performs tasks that humans would deem noteworthy or "intelligent." Examples include identifying pictures, understanding spoken words or written text, and solving problems.

As used herein, the term "database" or "dataset" refer to repositories for raw or compiled data, even if various informational facets can be found within the data fields. A database is typically organized so its contents can be accessed, managed, and updated (e.g., the database is dynamic). The term "database" and "source" are also used interchangeably in the present invention, because primary sources of data and information are databases. However, a "source database" or "source data" refers in general to data, e.g., unstructured text and/or structured data, which are input into the system for identifying objects and determining relationships. A source database may or may not be a relational database. However, a system database usually includes a relational database or some equivalent type of database or dataset which stores or includes stored values relating to relationships between objects.

As used herein, a "system database" and "relational database" are used interchangeably and refer to one or more collections of data organized as a set of tables containing data fitted into predefined categories. For example, a database table may comprise one or more categories defined by columns (e.g. attributes), while rows of the database may contain a unique object for the categories defined by the columns. Thus, an object such as the identity of a gene might have columns for its presence, absence and/or level of expression of the gene. A row of a relational database may also be referred to as a "set" and is generally defined by the values of its columns. A "domain" in the context of a relational database is a range of valid values a field such as a column may include.

As used herein, a "domain of knowledge" refers to an area of study over which the system is operative, for example, all biomedical data. It should be pointed out that there is advantage to combining data from several domains, for example, biomedical data and engineering data, for this diverse data can sometimes link things that cannot be put together for a normal person that is only familiar with one area or research/study (one domain). A "distributed database" refers to a database that may be dispersed or replicated among different points in a network.

Terms such "data" and "information" are often used interchangeably, as are "information" and "knowledge." As used herein, "data" is the most fundamental unit that is an empirical measurement or set of measurements. Data is compiled to contribute to information, but it is fundamentally independent of it. Information, by contrast, is derived from interests, e.g., data (the unit) may be gathered on ethnicity, gender, height, weight and diet for the purpose of finding variables correlated with risk of cardiovascular disease. However, the same data could be used to develop a formula or to create "information" about dietary preferences, i.e., likelihood that certain products in a supermarket have a higher likelihood of selling.

As used herein, "information" refers to a data set that may include numbers, letters, sets of numbers, sets of letters, or conclusions resulting or derived from a set of data. "Data" is then a measurement or statistic and the fundamental unit of information. "Information" may also include other types of data such as words, symbols, text, such as unstructured free text, code, etc. "Knowledge" is loosely defined as a set of information that gives sufficient understanding of a system to model cause and effect. To extend the previous example, information on demographics, gender and prior purchases may be used to develop a regional marketing strategy for food sales while information on nationality could be used by buyers as a guideline for importation of products. It is important to note that there are no strict boundaries between data, information, and knowledge; the three terms are, at times, considered to be equivalent. In general, data comes from examining, information comes from correlating, and knowledge comes from modeling.

As used herein, "a program" or "computer program" refers generally to a syntactic unit that conforms to the rules of a particular programming language and that is composed of declarations and statements or instructions, divisible into, "code segments" needed to solve or execute a certain function, task, or problem. A programming language is generally an artificial language for expressing programs.

As used herein, a "system" or a "computer system" generally refers to one or more computers, peripheral equipment, and software that perform data processing. A "user" or "system operator" in general includes a person, that uses a computer network accessed through a "user device" (e.g., a computer, a wireless device, etc) for the purpose of data processing and information exchange. A "computer" is generally a functional unit that can perform substantial computations, including numerous arithmetic operations and logic operations without human intervention.

As used herein, "application software" or an "application program" refers generally to software or a program that is specific to the solution of an application problem. An "application problem" is generally a problem submitted by an end user and requiring information processing for its solution.

As used herein, a "natural language" refers to a language whose rules are based on current usage without being specifically prescribed, e.g., English, Spanish or Chinese. As used herein, an "artificial language" refers to a language whose rules are explicitly established prior to its use, e.g., computer-programming languages such as C, C++, Java, BASIC, FORTRAN, or COBOL.

As used herein, "statistical relevance" refers to using one or more of the ranking schemes (O/E ratio, strength, etc.), where a relationship is determined to be statistically relevant if it occurs significantly more frequently than would be expected by random chance.

As used herein, the terms "coordinately regulated genes" or "transcriptional modules" are used interchangeably to refer to grouped, gene expression profiles (e.g., signal values associated with a specific gene sequence) of specific genes. Each transcriptional module correlates two key pieces of data, a literature search portion and actual empirical gene expression value data obtained from a gene microarray. The set of genes that is selected into a transcriptional module based on the analysis of gene expression data (using the module extraction algorithm described above). Additional steps are taught by Chaussabel, D. & Sher, A. Mining microarray expression data by literature profiling. Genome Biol 3, RESEARCH0055 (2002), (http://genomebiology.com/2002/3/10/research/0055) relevant portions incorporated herein by reference and expression data obtained from a disease or condition of interest, e.g., Systemic Lupus erythematosus, arthritis, lymphoma, carcinoma, melanoma, acute infection, autoimmune disorders, autoinflammatory disorders, etc.).

The Table below lists examples of keywords that were used to develop the literature search portion or contribution to the transcription modules. The skilled artisan will recognize that other terms may easily be selected for other conditions, e.g., specific cancers, specific infectious disease, transplantation, etc. For example, genes and signals for those genes associated with T cell activation are described hereinbelow as Module ID "M 2.8" in which certain keywords (e.g., Lymphoma, T-cell, CD4, CD8, TCR, Thymus, Lymphoid, IL2) were used to identify key T-cell associated genes, e.g., T-cell surface markers (CD5, CD6, CD7, CD26, CD28, CD96); molecules expressed by lymphoid lineage cells (lymphotoxin beta, IL2-inducible T-cell kinase, TCF7; and T-cell differentiation protein mal, GATA3, STAT5B). Next, the complete module is developed by correlating data from a patient population for these genes (regardless of platform, presence/absence and/or up or downregulation) to generate the transcriptional module. In some cases, the gene profile does not match (at this time) any particular clustering of genes for these disease conditions and data, however, certain physiological pathways (e.g., cAMP signaling, zinc-finger proteins, cell surface markers, etc.) are found within the "Underdetermined" modules. In fact, the gene expression data set may be used to extract genes that have coordinated expression prior to matching to the keyword search, i.e., either data set may be correlated prior to cross-referencing with the second data set.

**Table 1. Examples of Genes within Distinct Modules**

| **Module I.D.** | **Number of probe sets** | **Keyword selection** | **Assessment** |
|---|---|---|---|
| M 1.1 | 76 | Ig, Immunoglobulin, Bone, Marrow, PreB, IgM,Mu. | Plasma cells. Includes genes coding for Immunoglobulin chains (e.g. IGHM, IGJ, IGLL1, IGKC, IGHD) and the plasma cell marker CD38. |
| M 1.2 | 130 | Platelet, Adhesion, Aggregation, Endothelial, Vascular | Platelets. Includes genes coding for platelet glycoproteins (ITGA2B, ITGB3, GP6, GP1A/B), and platelet-derived immune mediators such as PPPB (pro-platelet basic protein) and PF4 (platelet factor 4). |
| M 1.3 | 80 | Immunoreceptor, BCR, B-cell, IgG | B-cells. Includes genes coding for B-cell surface markers (CD72, CD79A/B, CD19, CD22) and other B-cell associated molecules: Early B-cell factor (EBF), B-cell linker (BLNK) and B lymphoid tyrosine kinase (BLK). |
| M 1.4 | 132 | Replication, Repression, Repair, CREB, Lymphoid, TNF-alpha | Undetermined. This set includes regulators and targets of cAMP signaling pathway (JUND, ATF4, CREM, PDE4, NR4A2, VIL2), as well as repressors of TNF-alpha mediated NF-KB activation (CYLD, ASK, TNFAIP3). |
| M 1.5 | 142 | Monocytes, Dendritic, MHC, Costimulatory, TLR4, MYD88 | Myeloid lineage. Includes molecules expressed by cells of the myeloid lineage (CD86, CD163, FCGR2A), some of which being involved in pathogen recognition (CD14, TLR2, MYD88). This set also includes TNF family members (TNFR2, BAFF). |
| M 1.6 | 141 | Zinc, Finger, P53, RAS | Undetermined. This set includes genes coding for signaling molecules, e.g. the zinc finger containing inhibitor of activated STAT (PIAS1 and PIAS2), or the nuclear factor of activated T-cells NFATC3. |
| M 1.7 | 129 | Ribosome, Translational, 40S, 60S, HLA | MHC/Ribosomal proteins. Almost exclusively formed by genes coding MHC class I molecules (HLA-A,B,C,G,E)+ Beta 2-microglobulin (B2M) or Ribosomal proteins (RPLs, RPSs). |
| M 1.8 | 154 | Metabolism, Biosynthesis, Replication, Helicase | Undetermined. Includes genes encoding metabolic enzymes (GLS, NSF1, NAT1) and factors involved in DNA replication (PURA, TERF2, EIF2S1). |
| M 2.1 | 95 | NK, Killer, Cytolytic, CD8, Cell-mediated, T-cell, CTL, IFN-g | Cytotoxic cells. Includes cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW). |
| M 2.2 | 49 | Granulocytes, Neutrophils, Defense, Myeloid, Marrow | Neutrophils. This set includes innate molecules that are found in neutrophil granules (Lactotransferrin: LTF, defensin: DEAF1, Bacterial Permeability Increasing protein: BPI, Cathelicidin antimicrobial protein: CAMP...). |
| M 2.3 | 148 | Erythrocytes, Red, Anemia, Globin, Hemoglobin | Erythrocytes. Includes hemoglobin genes (HGBs) and other erythrocyte-associated genes (erythrocytic alkirin:ANK1, Glycophorin C: GYPC, hydroxymethylbilane synthase: HMBS, erythroid associated factor: ERAF). |
| M 2.4 | 133 | Ribonucleoprotein, 60S, nucleolus, Assembly, Elongation | Ribosomal proteins. Including genes encoding ribosomal proteins (RPLs, RPSs), Eukaryotic Translation Elongation factor family members (EEFs) and Nucleolar proteins (NPM1, NOAL2, NAP1L1). |
| M 2.5 | 315 | Adenoma, Interstitial, Mesenchyme, Dendrite, Motor | Undetermined. This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin). |
| M 2.6 | 165 | Granulocytes, Monocytes, Myeloid, ERK, Necrosis | Myeloid lineage. Includes genes expressed in myeloid lineage cells (IGTB2/CD 18, Lymphotoxin beta receptor, Myeloid related proteins 8/14 Formyl peptide receptor 1), such as Monocytes and Neutrophils. |
| M 2.7 | 71 | No keywords extracted. | Undetermined. This module is largely composed of transcripts with no known function. Only 20 genes associated with literature, including a member of the chemokine-like factor superfamily (CKLFSF8). |
| M 2.8 | 141 | Lymphoma, T-cell, CD4, CD8, TCR, Thymus, Lymphoid, IL2 | T-cells. Includes T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B). |
| M 2.9 | 159 | ERK, Transactivation, Cytoskeletal, MAPK, JNK | Undetermined. Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1). |
| M 2.10 | 106 | Myeloid, Macrophage, Dendritic, Inflammatory, Interleukin | Undetermined. Includes genes encoding for Immune-related cell surface molecules (CD36, CD86, LILRB), cytokines (IL15) and molecules involved in signaling pathways (FYB, TICAM2-Toll-like receptor pathway). |
| M 2.11 | 176 | Replication, Repress, RAS, Autophosphorylation, Oncogenic | Undetermined. Includes kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS). |
| M 3.1 | 122 | ISRE, Influenza, Antiviral, IFN-gamma, IFN-alpha, Interferon | hiterferon-inducible. This set includes interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G). |
| M 3.2 | 322 | TGF-beta, TNF, Inflammatory, Apoptotic, Lipopolysaccharide | Inflammation I. Includes genes encoding molecules involved in inflammatory processes (e.g. IL8, ICAM1, C5R1, CD44, PLAUR, IL1A, CXCL16), and regulators of apoptosis (MCL1, FOXO3A, RARA, BCL3/6/2A1, GADD45B). |
| M 3.3 | 276 | Inflammatory, Defense, Lysosomal, Oxidative, LPS | Inflammation II. Includes molecules inducing or inducible by inflammation (IL18, ALOX5, ANPEP, AOAH, HMOX1, SERPINB1), as well as lysosomal enzymes (PPT1, CTSB/S, NEU1, ASAH1, LAMP2, CAST). |
| M 3.4 | 325 | Ligase, Kinase, KIP1, Ubiquitin, Chaperone | Undetermined. Includes protein phosphatases (PPP1R12A, PTPRC, PPP1CB, PPM1B) and phosphoinositide 3-kinase (PI3K) family members (PIK3CA, PIK32A, PIPSK3). |
| M 3.5 | 22 | No keyword extracted | Undetermined. Composed of only a small number of transcripts. Includes hemoglobin genes (HBA1, HBA2, HBB). |
| M 3.6 | 288 | Ribosomal, T-cell, Beta-catenin | Undetermined. This set includes mitochondrial ribosomal proteins (MRPLs, MRPs), mitochondrial elongations factors (GFM1/2), Sortin Nexins (SN1/6/14) as well as lysosomal ATPases (ATP6VIC/D). |
| M 3.7 | 301 | Spliceosome, Methylation, Ubiquitin | Undetermined. Includes genes encoding proteasome subunits (PSMA2/5, PSMB5/8); ubiquitin protein ligases HIP2, STUB1, as well as components of ubiqutin ligase complexes (SUGT1). |
| M 3.8 | 284 | CDC, TCR, CREB, Glycosylase | Undetermined. Includes genes encoding enzymes: aminomethyltransferase, arginyltransferase, asparagines synthetase, diacylglycerol kinase, inositol phosphatases, methyltransferases, helicases... |
| M 3.9 | 260 | Chromatin, Checkpoint, Replication, Transactivation | Undetermined. Includes genes encoding kinases (IBTK, PRKRIR, PRKDC, PRKCI) and phosphatases (e.g. PTPLB, PPP2CB/3CB, PTPRC, MTM1, MTMR2). |

As used herein, the term "array" refers to a solid support or substrate with one or more peptides or nucleic acid probes attached to the support. Arrays typically have one or more different nucleic acid or peptide probes that are coupled to a surface of a substrate in different, known locations. These arrays, also described as "microarrays", "gene-chips" or DNA chips that may have 10,000; 20,000, 30,000; or 40,000 different identifiable genes based on the known genome, e.g., the human genome. These pan-arrays are used to detect the entire "transcriptome" or transcriptional pool of genes that are expressed or found in a sample, e.g., nucleic acids that are expressed as RNA, mRNA and the like that may be subjected to RT and/or RT-PCR to made a complementary set of DNA replicons. Arrays may be produced using mechanical synthesis methods, light directed synthesis methods and the like that incorporate a combination of non-lithographic and/or photolithographic methods and solid phase synthesis methods. Bead arrays that include 50-mer oligonucleotide probes attached to 3 micrometer beads may be used that are, e.g., lodged into microwells at the surface of a glass slide or are part of a liquid phase suspension arrays (e.g., Luminex or Illumina) that are digital beadarrays in liquid phase and uses "barcoded" glass rods for detection and identification.

Various techniques for the synthesis of these nucleic acid arrays have been described, e.g., fabricated on a surface of virtually any shape or even a multiplicity of surfaces. Arrays may be peptides or nucleic acids on beads, gels, polymeric surfaces, fibers such as fiber optics, glass or any other appropriate substrate. Arrays may be packaged in such a manner as to allow for diagnostics or other manipulation of an all inclusive device, see for example, U.S. Pat. No. 6,955,788, relevant portions incorporated herein by reference.

### BIOLOGICAL DEFINITIONS

As used herein, the term "disease" refers to a physiological state of an organism with any abnormal biological state of a cell. Disease includes, but is not limited to, an interruption, cessation or disorder of cells, tissues, body functions, systems or organs that may be inherent, inherited, caused by an infection, caused by abnormal cell function, abnormal cell division and the like. A disease that leads to a "disease state" is generally detrimental to the biological system, that is, the host of the disease. With respect to the present invention, any biological state, such as an infection (e.g., viral, bacterial, fungal, helminthic, etc.), inflammation, autoinflammation, autoimmunity, anaphylaxis, allergies, premalignancy, malignancy, surgical, transplantation, physiological, and the like that is associated with a disease or disorder is considered to be a disease state. A pathological state is generally the equivalent of a disease state.

Disease states may also be categorized into different levels of disease state. As used herein, the level of a disease or disease state is an arbitrary measure reflecting the progression of a disease or disease state as well as the physiological response upon, during and after treatment. Generally, a disease or disease state will progress through levels or stages, wherein the affects of the disease become increasingly severe. The level of a disease state may be impacted by the physiological state of cells in the sample.

As used herein, the terms "therapy" or "therapeutic regimen" refer to those medical steps taken to alleviate or alter a disease state, e.g., a course of treatment intended to reduce or eliminate the affects or symptoms of a disease using pharmacological, surgical, dietary and/or other techniques. A therapeutic regimen may include a prescribed dosage of one or more drugs or surgery. Therapies will most often be beneficial and reduce the disease state but in many instances the effect of a therapy will have non-desirable or side-effects. The effect of therapy will also be impacted by the physiological state of the host, e.g., age, gender, genetics, weight, other disease conditions, etc.

As used herein, the term "pharmacological state" or "pharmacological status" refers to those samples that will be, are and/or were treated with one or more drugs, surgery and the like that may affect the pharmacological state of one or more nucleic acids in a sample, e.g., newly transcribed, stabilized and/or destabilized as a result of the pharmacological intervention. The pharmacological state of a sample relates to changes in the biological status before, during and/or after drug treatment and may serve a diagnostic or prognostic function, as taught herein. Some changes following drug treatment or surgery may be relevant to the disease state and/or may be unrelated side-effects of the therapy. Changes in the pharmacological state are the likely results of the duration of therapy, types and doses of drugs prescribed, degree of compliance with a given course of therapy, and/or un-prescribed drugs ingested.

As used herein, the term "biological state" refers to the state of the transcriptome (that is the entire collection of RNA transcripts) of the cellular sample isolated and purified for the analysis of changes in expression. The biological state reflects the physiological state of the cells in the sample by measuring the abundance and/or activity of cellular constituents, characterizing according to morphological phenotype or a combination of the methods for the detection of transcripts.

As used herein, the term "expression profile" refers to the relative abundance of RNA, DNA or protein abundances or activity levels. The expression profile can be a measurement for example of the transcriptional state or the translational state by any number of methods and using any of a number of gene-chips, gene arrays, beads, multiplex PCR, quantitiative PCR, run-on assays, Northern blot analysis, Western blot analysis, protein expression, fluorescence activated cell sorting (FACS), enzyme linked immunosorbent assays (ELISA), chemiluminescence studies, enzymatic assays, proliferation studies or any other method, apparatus and system for the determination and/or analysis of gene expression that are readily commercially available.

As used herein, the term "transcriptional state" of a sample includes the identities and relative abundances of the RNA species, especially mRNAs present in the sample. The entire transcriptional state of a sample, that is the combination of identity and abundance of RNA, is also referred to herein as the transcriptome. Generally, a substantial fraction of all the relative constituents of the entire set of RNA species in the sample are measured.

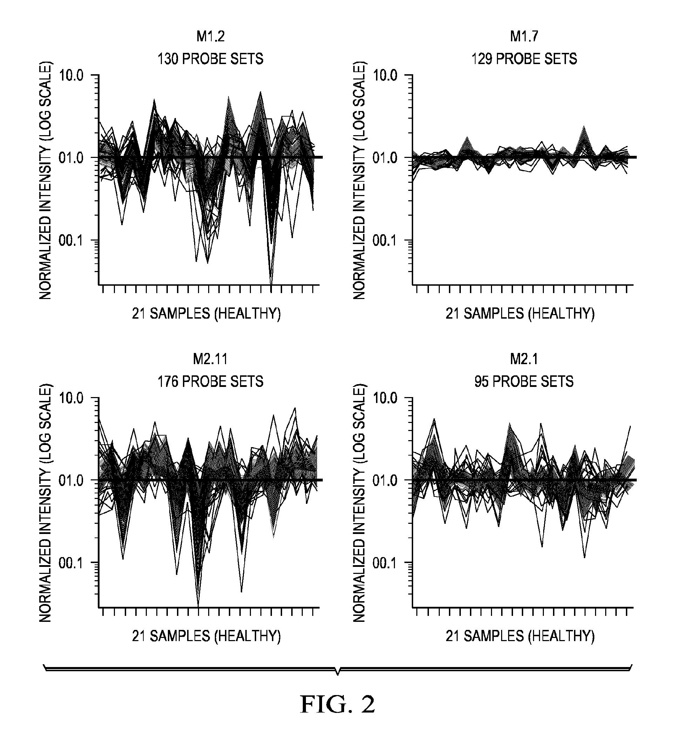

As used herein, the term "transcriptional vectors," "expression vectors," "genomic vectors" (used interchangeably) refers to transcriptional expression data that reflects the "proportion of differentially expressed genes." For example, for each module the proportion of transcripts differentially expressed between at least two groups (e.g., healthy subjects versus patients). This vector is derived from the comparison of two groups of samples. The first analytical step is used for the selection of disease-specific sets of transcripts within each module. Next, there is the "expression level." The group comparison for a given disease provides the list of differentially expressed transcripts for each module. It was found that different diseases yield different subsets of modular transcripts. With this expression level it is then possible to calculate vectors for each module(s) for a single sample by averaging expression values of disease-specific subsets of genes identified as being differentially expressed. This approach permits the generation of maps of modular expression vectors for a single sample, e.g., those described in the module maps disclosed herein. These vector module maps represent an averaged expression level for each module (instead of a proportion of differentially expressed genes) that can be derived for each sample. These composite "expression vectors" are formed through successive rounds of selection: 1) of the modules that were significantly changed across study groups and 2) of the genes within these modules which are significantly changed across study groups (Figure 2, *step II*). Expression levels are subsequently derived by averaging the values obtained for the subset of transcripts forming each vector (Figure 2, *step III*). Patient profiles can then be represented by plotting expression levels obtained for each of these vectors on a graph (e.g. on a radar plot). Therefore a set of vectors results from two round of selection, first at the module level, and then at the gene level. Vector expression values are composite by construction as they derive from the average expression values of the transcript forming the vector.

Using the present invention it is possible to identify and distinguish diseases not only at the module-level, but also at the gene-level; i.e., two diseases can have the same vector (identical proportion of differentially expressed transcripts, identical "polarity"), but the gene composition of the expression vector can still be disease-specific. This disease-specific customization permits the user to optimize the performance of a given set of markers by increasing its specificity.

Using modules as a foundation grounds expression vectors to coherent functional and transcriptional units containing minimized amounts of noise. Furthermore, the present invention takes advantage of composite transcriptional markers. As used herein, the term "composite transcriptional markers" refers to the average expression values of multiple genes (subsets of modules) as compared to using individual genes as markers (and the composition of these markers can be disease-specific). The composite transcriptional markers approach is unique because the user can develop multivariate microarray scores to assess disease severity in patients with, e.g., SLE, or to derive expression vectors disclosed herein. The fact that expression vectors are composite (i.e. formed by a combination of transcripts) further contributes to the stability of these markers. Most importantly, it has been found that using the composite modular transcriptional markers of the present invention the results found herein are reproducible across microarray platform, thereby providing greater reliability for regulatory approval. Indeed, vector expression values proved remarkably robust, as indicated by the excellent reproducibility obtained across microarray platforms; as well as the validation results obtained in an independent set of pediatric lupus patients. These results are of importance since improving the reliability of microarray data is a prerequisite for the widespread use of this technology in clinical practice.

Gene expression monitoring systems for use with the present invention may include customized gene arrays with a limited and/or basic number of genes that are specific and/or customized for the one or more target diseases. Unlike the general, pan-genome arrays that are in customary use, the present invention provides for not only the use of these general pan-arrays for retrospective gene and genome analysis without the need to use a specific platform, but more importantly, it provides for the development of customized arrays that provide an optimal gene set for analysis without the need for the thousands of other, non-relevant genes. One distinct advantage of the optimized arrays and modules of the present invention over the existing art is a reduction in the financial costs (e.g., cost per assay, materials, equipment, time, personnel, training, etc.), and more importantly, the environmental cost of manufacturing pan-arrays where the vast majority of the data is irrelevant. The modules of the present invention allow for the first time the design of simple, custom arrays that provide optimal data with the least number of probes while maximizing the signal to noise ratio. By eliminating the total number of genes for analysis, it is possible to, e.g., eliminate the need to manufacture thousands of expensive platinum masks for photolithography during the manufacture of pan-genetic chips that provide vast amounts of irrelevant data. Using the present invention it is possible to completely avoid the need for microarrays if the limited probe set(s) of the present invention are used with, e.g., digital optical chemistry arrays, ball bead arrays, beads (e.g., Luminex), multiplex PCR, quantitiative PCR, run-on assays, Northern blot analysis, or even, for protein analysis, e.g., Western blot analysis, 2-D and 3-D gel protein expression, MALDI, MALDI-TOF, fluorescence activated cell sorting (FACS) (cell surface or intracellular), enzyme linked immunosorbent assays (ELISA), chemiluminescence studies, enzymatic assays, proliferation studies or any other method, apparatus and system for the determination and/or analysis of gene expression that are readily commercially available.

The "molecular fingerprinting system" of the present invention may be used to facilitate and conduct a comparative analysis of expression in different cells or tissues, different subpopulations of the same cells or tissues, different physiological states of the same cells or tissue, different developmental stages of the same cells or tissue, or different cell populations of the same tissue against other diseases and/or normal cell controls. In some cases, the normal or wild-type expression data may be from samples analyzed at or about the same time or it may be expression data obtained or culled from existing gene array expression databases, e.g., public databases such as the NCBI Gene Expression Omnibus database.

As used herein, the term "differentially expressed" refers to the measurement of a cellular constituent (e.g., nucleic acid, protein, enzymatic activity and the like) that varies in two or more samples, e.g., between a disease sample and a normal sample. The cellular constituent may be on or off (present or absent), upregulated relative to a reference or downregulated relative to the reference. For use with gene-chips or gene-arrays, differential gene expression of nucleic acids, e.g., mRNA or other RNAs (miRNA, siRNA, hnRNA, rRNA, tRNA, etc.) may be used to distinguish between cell types or nucleic acids. Most commonly, the measurement of the transcriptional state of a cell is accomplished by quantitative reverse transcriptase (RT) and/or quantitative reverse transcriptase-polymerase chain reaction (RT-PCR), genomic expression analysis, post-translational analysis, modifications to genomic DNA, translocations, in situ hybridization and the like.

For some disease states it is possible to identify cellular or morphological differences, especially at early levels of the disease state. The present invention avoids the need to identify those specific mutations or one or more genes by looking at modules of genes of the cells themselves or, more importantly, of the cellular RNA expression of genes from immune effector cells that are acting within their regular physiologic context, that is, during immune activation, immune tolerance or even immune anergy. While a genetic mutation may result in a dramatic change in the expression levels of a group of genes, biological systems often compensate for changes by altering the expression of other genes. As a result of these internal compensation responses, many perturbations may have minimal effects on observable phenotypes of the system but profound effects to the composition of cellular constituents. Likewise, the actual copies of a gene transcript may not increase or decrease, however, the longevity or half-life of the transcript may be affected leading to greatly increases protein production. The present invention eliminates the need of detecting the actual message by, in one embodiment, looking at effector cells (e.g., leukocytes, lymphocytes and/or sub-populations thereof) rather than single messages and/or mutations.

The skilled artisan will appreciate readily that samples may be obtained from a variety of sources including, e.g., single cells, a collection of cells, tissue, cell culture and the like. In certain cases, it may even be possible to isolate sufficient RNA from cells found in, e.g., urine, blood, saliva, tissue or biopsy samples and the like. In certain circumstances, enough cells and/or RNA may be obtained from: mucosal secretion, feces, tears, blood plasma, peritoneal fluid, interstitial fluid, intradural, cerebrospinal fluid, sweat or other bodily fluids. The nucleic acid source, e.g., from tissue or cell sources, may include a tissue biopsy sample, one or more sorted cell populations, cell culture, cell clones, transformed cells, biopies or a single cell. The tissue source may include, e.g., brain, liver, heart, kidney, lung, spleen, retina, bone, neural, lymph node, endocrine gland, reproductive organ, blood, nerve, vascular tissue, and olfactory epithelium.

The present invention includes the following basic components, which may be used alone or in combination, namely, one or more data mining algorithms; one or more module-level analytical processes; the characterization of blood leukocyte transcriptional modules; the use of aggregated modular data in multivariate analyses for the molecular diagnostic/prognostic of human diseases; and/or visualization of module-level data and results. Using the present invention it is also possible to develop and analyze composite transcriptional markers, which may be further aggregated into a single multivariate score.

The present inventors have recognized that current microarray-based research is facing significant challenges with the analysis of data that are notoriously "noisy," that is, data that is difficult to interpret and does not compare well across laboratories and platforms. A widely accepted approach for the analysis of microarray data begins with the identification of subsets of genes differentially expressed between study groups. Next, the users try subsequently to "make sense" out of resulting gene lists using pattern discovery algorithms and existing scientific knowledge.

Rather than deal with the great variability across platforms, the present inventors have developed a strategy that emphasized the selection of biologically relevant genes at an early stage of the analysis. Briefly, the method includes the identification of the transcriptional components characterizing a given biological system for which an improved data mining algorithm was developed to analyze and extract groups of coordinately expressed genes, or transcriptional modules, from large collections of data.

The biomarker discovery strategy described herein is particularly well adapted for the exploitation of microarray data acquired on a global scale. Starting from ~44,000 transcripts a set of 28 modules was defined that are composed of nearly 5000 transcripts. Sets of disease-specific composite expression vectors were then derived. Vector expression values (expression vectors) proved remarkably robust, as indicated by the excellent reproducibility obtained across microarray platforms. This finding is notable, since improving the reliability of microarray data is a prerequisite for the widespread use of this technology in clinical practice. Finally, expression vectors can in turn be combined to obtain unique multivariate scores, therefore delivering results in a form that is compatible with mainstream clinical practice. Interestingly, multivariate scores recapitulate global patterns of change rather than changes in individual markers. The development of such "global biomarkers" can be used for both diagnostic and pharmacogenomics fields.

In one example, twenty-eight transcriptional modules regrouping 4742 probe sets were obtained from 239 blood leukocyte transcriptional profiles. Functional convergence among genes forming these modules was demonstrated through literature profiling. The second step consisted of studying perturbations of transcriptional systems on a modular basis. To illustrate this concept, leukocyte transcriptional profiles obtained from healthy volunteers and patients were obtained, compared and analyzed. Further validation of this gene fingerprinting strategy was obtained through the analysis of a published microarray dataset. Remarkably, the modular transcriptional apparatus, system and methods of the present invention using pre-existing data showed a high degree of reproducibility across two commercial microarray platforms.

The present invention includes the implementation of a widely applicable, two-step microarray data mining strategy designed for the modular analysis of transcriptional systems. This novel approach was used to characterize transcriptional signatures of blood leukocytes, which constitutes the most accessible source of clinically relevant information.

As demonstrated herein, it is possible to determine, differential and/or distinguish between two disease based on two vectors even if the vector is identical (+/+) for two diseases - e.g. M1.3 = 53% down for both SLE and FLU because the composition of each vector can still be used to differentiate them. For example, even though the proportion and polarity of differentially expressed transcripts is identical between the two diseases for M1.3, the gene composition can still be disease-specific. The combination of gene-level and module-level analysis considerably increases resolution. Furthermore, it is possible to use 2, 3, 4, 5, 10, 15, 20, 25, 28 or more modules to differentiate diseases.

The term "gene" refers to a nucleic acid (e.g., DNA) sequence that includes coding sequences necessary for the production of a polypeptide (e.g., ), precursor, or RNA (e.g., mRNA). The polypeptide may be encoded by a full length coding sequence or by any portion of the coding sequence so long as the desired activity or functional property (e.g., enzymatic activity, ligand binding, signal transduction, immunogenicity, etc.) of the full-length or fragment is retained. The term also encompasses the coding region of a structural gene and the sequences located adjacent to the coding region on both the 5' and 3' ends for a distance of about 2 kb or more on either end such that the gene corresponds to the length of the full-length mRNA and 5' regulatory sequences which influence the transcriptional properties of the gene. Sequences located 5' of the coding region and present on the mRNA are referred to as 5'-untranslated sequences. The 5'-untranslated sequences usually contain the regulatory sequences. Sequences located 3' or downstream of the coding region and present on the mRNA are referred to as 3'-untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "nucleic acid" refers to any nucleic acid containing molecule, including but not limited to, DNA, cDNA and RNA. In particular, the terms "a gene in Table X" refers to at least a portion or the full-length sequence listed in a particular table, as found hereinbelow. The gene may even be found or detected a genomic form, that is, it includes one or more intron(s). Genomic forms of a gene may also include sequences located on both the 5' and 3' end of the coding sequences that are present on the RNA transcript. These sequences are referred to as "flanking" sequences or regions. The 5' flanking region may contain regulatory sequences such as promoters and enhancers that control or influence the transcription of the gene. The 3' flanking region may contain sequences that influence the transcription termination, post-transcriptional cleavage, mRNA stability and polyadenylation.

As used herein, the term "wild-type" refers to a gene or gene product isolated from a naturally occurring source. A wild-type gene is that which is most frequently observed in a population and is thus arbitrarily designed the "normal" or "wild-type" form of the gene. In contrast, the term "modified" or "mutant" refers to a gene or gene product that displays modifications in sequence and/or functional properties (i.e., altered characteristics) when compared to the wild-type gene or gene product. It is noted that naturally occurring mutants can be isolated; these are identified by the fact that they have altered characteristics (including altered nucleic acid sequences) when compared to the wild-type gene or gene product.

As used herein, the term "polymorphism" refers to the regular and simultaneous occurrence in a single interbreeding population of two or more alleles of a gene, where the frequency of the rarer alleles is greater than can be explained by recurrent mutation alone (typically greater than 1%).

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide protein) chain. The DNA sequence thus codes for the amino acid sequence.

As used herein, the terms "complementary" or "complementarity" are used in reference to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

As used herein, the term "hybridization" is used in reference to the pairing of complementary nucleic acids. Hybridization and the strength of hybridization (i.e., the strength of the association between the nucleic acids) is impacted by such factors as the degree of complementarity between the nucleic acids, stringency of the conditions involved, the Tₘ of the formed hybrid, and the G:C ratio within the nucleic acids. A single molecule that contains pairing of complementary nucleic acids within its structure is said to be "self hybridized."

As used herein the term "stringency" is used in reference to the conditions of temperature, ionic strength, and the presence of other compounds such as organic solvents, under which nucleic acid hybridizations are conducted. Under "low stringency conditions" a nucleic acid sequence of interest will hybridize to its exact complement, sequences with single base mismatches, closely related sequences (e.g., sequences with 90% or greater homology), and sequences having only partial homology (e.g., sequences with 50-90% homology). Under "medium stringency conditions," a nucleic acid sequence of interest will hybridize only to its exact complement, sequences with single base mismatches, and closely related sequences (e.g., 90% or greater homology). Under "high stringency conditions," a nucleic acid sequence of interest will hybridize only to its exact complement, and (depending on conditions such a temperature) sequences with single base mismatches. In other words, under conditions of high stringency the temperature can be raised so as to exclude hybridization to sequences with single base mismatches.

As used herein, the term "probe" refers to an oligonucleotide (i.e., a sequence of nucleotides), whether occurring naturally as in a purified restriction digest or produced synthetically, recombinantly or by PCR amplification, that is capable of hybridizing to another oligonucleotide of interest. A probe may be single-stranded or double-stranded. Probes are useful in the detection, identification and isolation of particular gene sequences. Any probe used in the present invention may be labeled with any "reporter molecule," so that it is detectable in any detection system, including, but not limited to enzyme (e.g., ELISA, as well as enzyme-based histochemical assays), fluorescent, radioactive, luminescent systems and the like. It is not intended that the present invention be limited to any particular detection system or label.

As used herein, the term "target," refers to the region of nucleic acid bounded by the primers. Thus, the "target" is sought to be sorted out from other nucleic acid sequences. A "segment" is defined as a region of nucleic acid within the target sequence.

As used herein, the term "Southern blot" refers to the analysis of DNA on agarose or acrylamide gels to fractionate the DNA according to size followed by transfer of the DNA from the gel to a solid support, such as nitrocellulose or a nylon membrane. The immobilized DNA is then probed with a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (Sambrook et al., Molecular Cloning: A Laboratory Manual, Cold Spring Harbor Press, NY, pp 9.31-9.58, 1989).

As used herein, the term "Northern blot" refers to the analysis of RNA by electrophoresis of RNA on agarose gels, to fractionate the RNA according to size followed by transfer of the RNA from the gel to a solid support, such as nitrocellulose or a nylon membrane. The immobilized RNA is then probed with a labeled probe to detect RNA species complementary to the probe used. Northern blots are a standard tool of molecular biologists (Sambrook, et al., supra, pp 7.39-7.52, 1989).

As used herein, the term "Western blot" refers to the analysis of protein(s) (or polypeptides) immobilized onto a support such as nitrocellulose or a membrane. The proteins are run on acrylamide gels to separate the proteins, followed by transfer of the protein from the gel to a solid support, such as nitrocellulose or a nylon membrane. The immobilized proteins are then exposed to antibodies with reactivity against an antigen of interest. The binding of the antibodies may be detected by various methods, including the use of radiolabeled antibodies.

As used herein, the term "polymerase chain reaction" ("PCR") refers to the method of K. B. Mullis (U.S. Pat. Nos. 4,683,195 4,683,202, and 4,965,188, hereby incorporated by reference), which describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "real time PCR" as used herein, refers to various PCR applications in which amplification is measured during as opposed to after completion of the reaction. Reagents suitable for use in real time PCR embodiments of the present invention include but are not limited to TaqMan probes, molecular beacons, Scorpions primers or double-stranded DNA binding dyes.

As used herein, the term "transcriptional upregulation" as used herein refers to an increase in synthesis of RNA, by RNA polymerases using a DNA template. For example, when used in reference to the methods of the present invention, the term "transcriptional upregulation" refers to an increase of least 1 to 2 fold, 2 to 3 fold, 3 to 10 fold, and even greater than 10 fold, in the quantity of mRNA corresponding to a gene of interest detected in a sample derived from an individual predisposed to SLE as compared to that detected in a sample derived from an individual who is not predisposed to SLE. However, the system and evaluation is sufficiently specific to require less that a 2 fold change in expression to be detected. Furthermore, the change in expression may be at the cellular level (change in expression within a single cell or cell populations) or may even be evaluated at a tissue level, where there is a change in the number of cells that are expressing the gene. Particularly useful differences are those that are statistically significant.

Conversely, the term "transcriptional downregulation" refers to a decrease in synthesis of RNA, by RNA polymerases using a DNA template. For example, when used in reference to the methods of the present invention, the term "transcriptional downregulation" refers to a decrease of least 2 fold, 2 to 3 fold, 3 to 10 fold, and even greater than 10 fold, in the quantity of mRNA corresponding to a gene of interest detected in a sample derived from an individual predisposed to SLE as compared to that detected in a sample derived from an individual who is not predisposed to such a condition or to a database of information for wild-type and/or normal control, e.g., fibromyalgia. Again, the system and evaluation is sufficiently specific to require less that a 2 fold change in expression to be detected. Particularly useful differences are those that are statistically significant.

Both transcriptional "upregulation"/overexpression and transcriptional "downregulation"/underexpression may also be indirectly monitored through measurement of the translation product or protein level corresponding to the gene of interest. The present invention is not limited to any given mechanism related to upregulation or downregulation of transcription.

The term "eukaryotic cell" as used herein refers to a cell or organism with membrane-bound, structurally discrete nucleus and other well-developed subcellular compartments. Eukaryotes include all organisms except viruses, bacteria, and bluegreen algae.

As used herein, the term "in vitro transcription" refers to a transcription reaction comprising a purified DNA template containing a promoter, ribonucleotide triphosphates, a buffer system that includes a reducing agent and cations, e.g., DTT and magnesium ions, and an appropriate RNA polymerase, which is performed outside of a living cell or organism.

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

As used herein, the term "diagnosis" refers to the determination of the nature of a case of disease. In some embodiments of the present invention, methods for making a diagnosis are provided which permit determination of SLE.

The present invention may be used alone or in combination with disease therapy to monitor disease progression and/or patient management. For example, a patient may be tested one or more times to determine the best course of treatment, determine if the treatment is having the intended medical effect, if the patient is not a candidate for that particular therapy and combinations thereof. The skilled artisan will recognize that one or more of the expression vectors may be indicative of one or more diseases and may be affected by other conditions, be they acute or chronic.

As used herein, the term "pharmacogenetic test" refers to an assay intended to study interindividual variations in DNA sequence related to, e.g., drug absorption and disposition (pharmacokinetics) or drug action (pharmacodynamics), which may include polymorphic variations in one or more genes that encode the functions of, e.g., transporters, metabolizing enzymes, receptors and other proteins.

As used herein, the term "pharmacogenomic test" refers to an assay used to study interindividual variations in whole-genome or candidate genes, e.g., single-nucleotide polymorphism (SNP) maps or haplotype markers, and the alteration of gene expression or inactivation that may be correlated with pharmacological function and therapeutic response.

As used herein, an "expression profile" refers to the measurement of the relative abundance of a plurality of cellular constituents. Such measurements may include, e.g., RNA or protein abundances or activity levels. The expression profile can be a measurement for example of the transcriptional state or the translational state. See U.S. Pat. Nos. 6,040,138, 5,800,992, 6,020135, 6,033,860, relevant portions incorporated herein by reference. The gene expression monitoring system, include nucleic acid probe arrays, membrane blot (such as used in hybridization analysis such as Northern, Southern, dot, and the like), or microwells, sample tubes, gels, beads or fibers (or any solid support comprising bound nucleic acids). See, e.g., U.S. Pat. Nos. 5,770,722, 5,874,219, 5,744,305, 5,677,195 and 5,445,934, relevant portions incorporated herein by reference. The gene expression monitoring system may also comprise nucleic acid probes in solution.

The gene expression monitoring system according to the present invention may be used to facilitate a comparative analysis of expression in different cells or tissues, different subpopulations of the same cells or tissues, different physiological states of the same cells or tissue, different developmental stages of the same cells or tissue, or different cell populations of the same tissue.

As used herein, the term "differentially expressed: refers to the measurement of a cellular constituent varies in two or more samples. The cellular constituent can be either up-regulated in the test sample relative to the reference or down-regulated in the test sample relative to one or more references. Differential gene expression can also be used to distinguish between cell types or nucleic acids. See U.S. Pat. No. 5,800,992, relevant portions incorporated herein by reference.

Therapy or Therapeutic Regimen: In order to alleviate or alter a disease state, a therapy or therapeutic regimen is often undertaken. A therapy or therapeutic regimen, as used herein, refers to a course of treatment intended to reduce or eliminate the affects or symptoms of a disease. A therapeutic regimen will typically comprise, but is not limited to, a prescribed dosage of one or more drugs or surgery. Therapies, ideally, will be beneficial and reduce the disease state but in many instances the effect of a therapy will have non-desirable effects as well. The effect of therapy will also be impacted by the physiological state of the sample.

Modules display distinct "transcriptional behavior". It is widely assumed that co-expressed genes are functionally linked. This concept of "guilt by association" is particularly compelling in cases where genes follow complex expression patterns across many samples. The present inventors discovered that transcriptional modules form coherent biological units and, therefore, predicted that the co-expression properties identified in the initial dataset would be conserved in an independent set of samples. Data were obtained for PBMCs isolated from the blood of twenty-one healthy volunteers. These samples were not used in the module selection process described above.

The present invention includes the following basic components, which may be used alone or in combination, namely, one or more data mining algorithms; one or more module-level analytical processes; the characterization of blood leukocyte transcriptional modules; the use of aggregated modular data in multivariate analyses for the molecular diagnostic/prognostic of human diseases; and/or visualization of module-level data and results. Using the present invention it is also possible to develop and analyze composite transcriptional markers, which may be further aggregated into a single multivariate score.

The present inventors have recognized that current microarray-based research is facing significant challenges with the analysis of data that are notoriously "noisy," that is, data that is difficult to interpret and does not compare well across laboratories and platforms. A widely accepted approach for the analysis of microarray data begins with the identification of subsets of genes differentially expressed between study groups. Next, the users try subsequently to "make sense" out of resulting gene lists using pattern discovery algorithms and existing scientific knowledge.

Rather than deal with the great variability across platforms, the present inventors have developed a strategy that emphasized the selection of biologically relevant genes at an early stage of the analysis. Briefly, the method includes the identification of the transcriptional components characterizing a given biological system for which an improved data mining algorithm was developed to analyze and extract groups of coordinately expressed genes, or transcriptional modules, from large collections of data.

The biomarker discovery strategy that we have developed is particularly well adapted for the exploitation of microarray data acquired on a global scale. Starting from ~44,000 transcripts the inventors defined 28 modules composed of nearly 5000 transcripts. Sets of disease-specific composite expression vectors were then derived. Vector expression values proved remarkably robust, as indicated by the excellent reproducibility obtained across microarray platforms. This finding is notable, since improving the reliability of microarray data is a prerequisite for the widespread use of this technology in clinical practice. Finally, vectors can in turn be combined to obtain unique multivariate scores, therefore delivering results in a form that is compatible with mainstream clinical practice. Interestingly, multivariate scores recapitulate global patterns of change rather than changes in individual markers. The development of such "global biomarkers" constitutes therefore a promising prospect for both diagnostic and pharmacogenomics fields.

In one example, twenty-eight transcriptional modules regrouping 4742 probe sets were obtained from 239 blood leukocyte transcriptional profiles. Functional convergence among genes forming these modules was demonstrated through literature profiling. The second step consisted of studying perturbations of transcriptional systems on a modular basis. To illustrate this concept, leukocyte transcriptional profiles obtained from healthy volunteers and patients were obtained, compared and analyzed. Further validation of this gene fingerprinting strategy was obtained through the analysis of a published microarray dataset. Remarkably, the modular transcriptional apparatus, system and methods of the present invention using pre-existing data showed a high degree of reproducibility across two commercial microarray platforms.

The present invention includes the implementation of a widely applicable, two-step microarray data mining strategy designed for the modular analysis of transcriptional systems. This novel approach was used to characterize transcriptional signatures of blood leukocytes, which constitutes the most accessible source of clinically relevant information.

As demonstrated herein, it is possible to determine, differential and/or distinguish between two disease based on two vectors even if the vector is identical (+/+) for two diseases - e.g. M1.3 = 53% down for both SLE and FLU because the composition of each vector can still be used to differentiate them. For example, even though the proportion and polarity of differentially expressed transcripts is identical between the two diseases for M1.3, the gene composition can still be disease-specific. The combination of gene-level and module-level analysis considerably increases resolution. Furthermore, it is possible to use 2, 3, 4, 5, 10, 15, 20, 25, 28 or more modules to differentiate diseases.

Material and methods. Processing of blood samples. All blood samples were collected in acid citrate dextrose tubes (BD Vacutainer) and immediately delivered at room temperature to the Baylor Institute for Immunology Research, Dallas, TX, for processing. Peripheral blood mononuclear cells (PBMCs) from 3-4 ml of blood were isolated *via* Ficoll gradient and immediately lysed in RLT reagent (Qiagen, Valencia, CA) with beta-mercaptoethanol (BME) and stored at -80°C prior to the RNA extraction step.

Microarray analysis. Total RNA was isolated using the RNeasy kit (Qiagen) according to the manufacturer's instructions and RNA integrity was assessed using an Agilent 2100 Bioanalyzer (Agilent, Palo Alto, CA).

Affymetrix GeneChips: These microarrays include short oligonucleotide probe sets synthesized *in situ* on a quartz wafer. Target labeling was performed according to the manufacturer's standard protocol (Affymetrix Inc., Santa Clara, CA). Biotinylated cRNA targets were purified and subsequently hybridized to Affymetrix HG-U133A and U133B GeneChips (>44,000 probe sets). Arrays were scanned using an Affymetrix confocal laser scanner. Microarray Suite, Version 5.0 (MAS 5.0; Affymetrix) software was used to assess fluorescent hybridization signals, to normalize signals, and to evaluate signal detection calls. Normalization of signal values per chip was achieved using the MAS 5.0 global method of scaling to the target intensity value of 500 per GeneChip. A gene expression analysis software program, GeneSpring, Version 7.1 (Agilent), was used to perform statistical analysis and hierarchical clustering.

Illumina BeadChips: These microarrays include 50mer oligonucleotide probes attached to 3µm beads, which are lodged into microwells at the surface of a glass slide. Samples were processed and acquired by Illumina Inc. (San Diego, CA) on the basis of a service contract. Targets were prepared using the Illumina RNA amplification kit (Ambion, Austin, TX). cRNA targets were hybridized to Sentrix HumanRef8 BeadChips (>25,000 probes), which were scanned on an Illumina BeadStation 500. Illumina's Beadstudio software was used to assess fluorescent hybridization signals.

Literature profiling. The literature profiling algorithm employed in this study has been previously described in detail (Chaussabel, D. & Sher, A. Mining microarray expression data by literature profiling. Genome Biol 3, RESEARCH0055 (2002), relevant portions incorporated herein by reference). This approach links genes sharing similar keywords. It uses hierarchical clustering, a popular unsupervised pattern discovery algorithm, to analyze patterns of term occurrence in literature abstracts. Step 1: A gene:literature index identifying pertinent publications for each gene is created. Step 2: Term occurrence frequencies were computed by a text processor. Step 3: Stringent filter criteria are used to select relevant keywords (i.e., eliminate terms with either high or low frequency across all genes and retain the few discerning terms characterized by a pattern of high occurrence for only a few genes). Step 4: Two-way hierarchical clustering groups of genes and relevant keywords based on occurrence patterns, providing a visual representation of functional relationships existing among a group of genes.

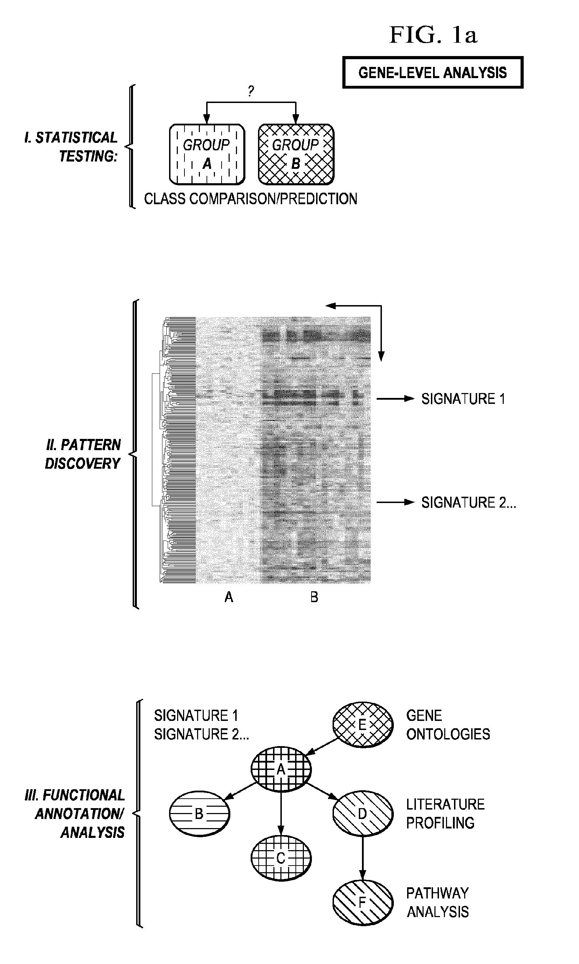

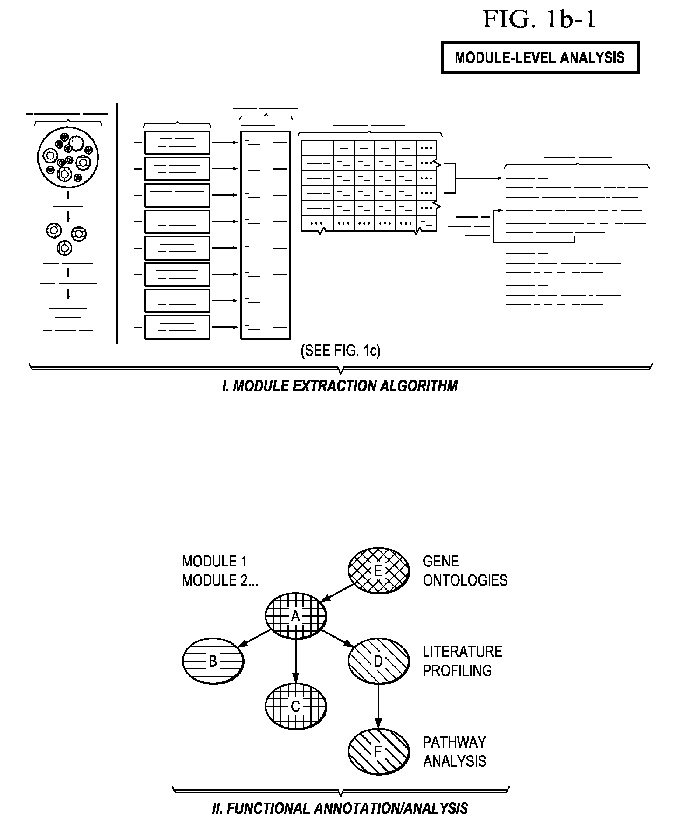

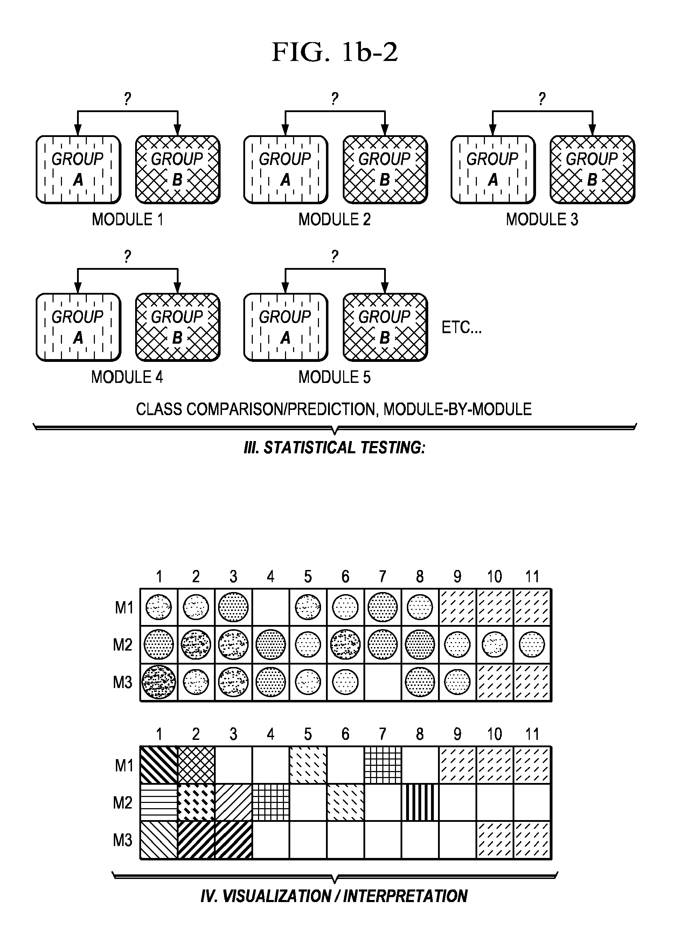

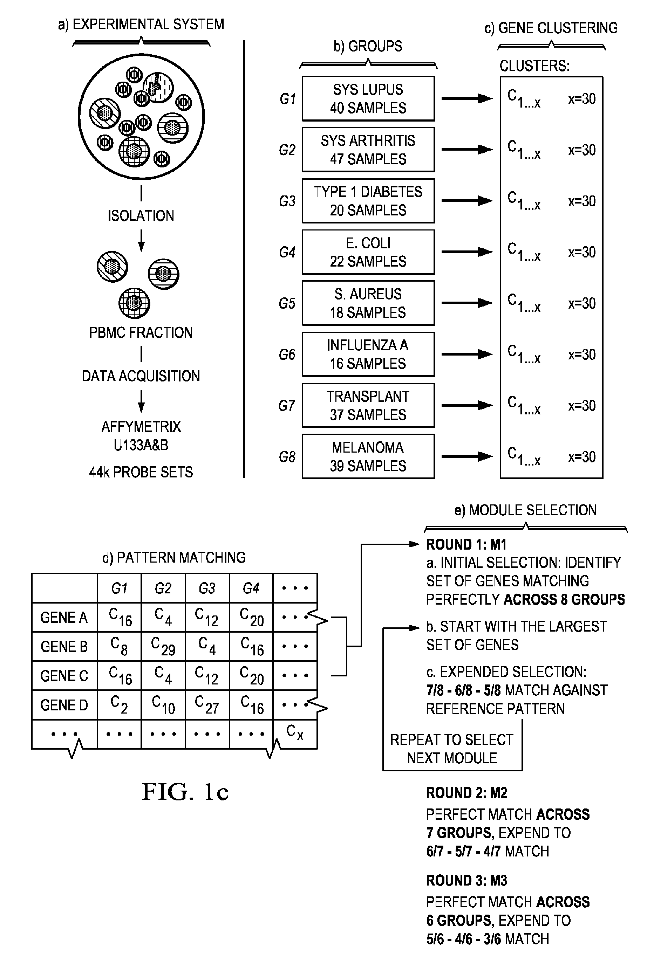

Modular data mining algorithm. First, one or more transcriptional components are identified that permit the characterization of biological systems beyond the level of single genes. Sets of coordinately regulated genes, or transcriptional modules, were extracted using a novel mining algorithm, which was applied to a large set of blood leukocyte microarray profiles (Figure 1). Gene expression profiles from a total of 239 peripheral blood mononuclear cells (PBMCs) samples were generated using Affymetrix U133A&B GeneChips (>44,000 probe sets). Transcriptional data were obtained for eight experimental groups (systemic juvenile idiopathic arthritis, systemic lupus erythematosus, type I diabetes, liver transplant recipients, melanoma patients, and patients with acute infections: *Escherichia coli, Staphylococcus aureus* and influenza A). For each group, transcripts with an absent flag call across all conditions were filtered out. The remaining genes were distributed among thirty sets by hierarchical clustering (clusters C1 through C30). The cluster assignment for each gene was recorded in a table and distribution patterns were compared among all the genes. Modules were selected using an iterative process, starting with the largest set of genes that belonged to the same cluster in all study groups (i.e. genes that were found in the same cluster in eight of the eight experimental groups). The selection was then expanded from this core reference pattern to include genes with 7/8, 6/8 and 5/8 matches. The resulting set of genes formed a transcriptional module and was withdrawn from the selection pool. The process was then repeated starting with the second largest group of genes, progressively reducing the level of stringency. This analysis led to the identification of 5348 transcripts that were distributed among twenty-eight modules (a complete list is provided as supplementary material). Each module is assigned a unique identifier indicating the round and order of selection (i.e. M3.1 was the first module identified in the third round of selection).

Analysis of "significance patterns" was performed on gene expression data generated from PBMCs obtained from patients and healthy volunteers using Affymetrix HG-U133A GeneChips that were run on the same Affymetrix system, using standard operating procedures. P values were obtained by comparing 7 groups of patients to their respective healthy control groups (Mann-Whitney rank test). The groups were composed of pediatric patients with: 1) Systemic Lupus Erythomatosus (SLE, 16 samples), 2) Influenza A (16 samples), 3) *Staphylococcus aureus* (16 samples), 4) *Escherichia coli* (16 samples) and *5) Streptococcus pneumoniae* (14 samples); as well as adult transplant recipients: 6) Liver transplant patients that have accepted the graft under immunosuppressive therapy (16 samples) and 7) bone marrow transplant recipients undergoing graft versus host disease (GVHD, 12 samples). Control groups were also formed taking into account age, sex and project (10 samples in each group). Genes significantly changed (p<0.01) in the "study group" (Influenza A and/or SLE) were divided in two sets: overexpressed versus control and under-expressed versus control. P-values of the genes forming the overexpressed set were obtained for the "reference groups" (infections with *E. coli, S. aureus, S. pneumoniae,* Liver transplant recipients and graft versus host disease). P-values of the reference groups were set to 1 when genes were under-expressed. The same procedure was used in the set of genes under-expressed in study group, only this time P-values of the reference group were set to 1 when genes were overexpressed. P-value data were processed with a gene expression analysis software program, GeneSpring, Version 7.1 (Agilent), that was used to perform hierarchical clustering and group genes based on significance patterns.

Modules display distinct "transcriptional behavior". It is widely assumed that co-expressed genes are functionally linked. This concept of "guilt by association" is particularly compelling in cases where genes follow complex expression patterns across many samples. The present inventors discovered that transcriptional modules form coherent biological units and, therefore, predicted that the co-expression properties identified in the initial dataset would be conserved in an independent set of samples. Data were obtained for PBMCs isolated from the blood of twenty-one healthy volunteers. These samples were not used in the module selection process described above.

FIGURE 2 shows gene expression profiles of four different modules are shown (Figure 2: M1.2, M1.7, M2.11 and M2.1). In the graphs of Figure 2, each line represents the expression level (y-axis) of a single gene across multiple samples (21 samples on the x-axis). Differences in gene expression in this example represent inter-individual variation between "healthy" individuals. It was found that within each module genes display a coherent "transcriptional behavior". Indeed, the variation in gene expression appeared to be consistent across all the samples (for some samples the expression of all the genes was elevated and formed a peak, while in others levels were low for all the genes which formed a dip). Importantly, inter-individual variations appeared to be module-specific as peaks and dips formed for different samples in M1.2, M2.11 and M2.1. Furthermore, the amplitude of variation was also characteristic of each module, with levels of expression being more variable for M1.2 and M2.11 than M2.1 and especially M1.7. Thus, we find that transcriptional modules constitute independent biological variables.

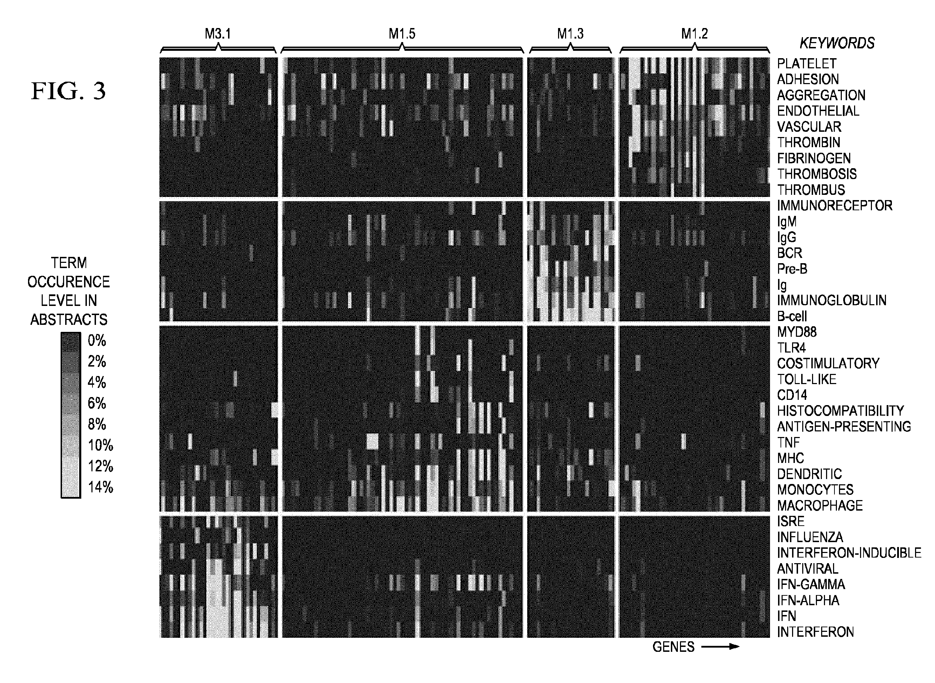

Functional characterization of transcriptional modules. Next, the modules were characterized at a functional level. A text mining approach was employed to extract keywords from the biomedical literature collected for each gene (described in ¹⁸). The distribution of keywords associated to the four modules that were analyzed is clearly distinct (Figure 3). The following is a list of keywords that may be associated with certain modules.
● Keywords highly specific for **M1.2** included *Platelet, Aggregation* or *Thrombosis,* and were associated with genes such as **ITGA2B** (Integrin alpha 2b, platelet glycoprotein IIb), **PF4** (platelet factor 4), **SELP** (Selectin P) and **GP6** (platelet glycoprotein 6).
● Keywords highly specific for **M1.3** included *B-cell, Immunoglobulin* or *IgG* and were associated with genes such as **CD19, CD22, CD72A, BLNK** (B cell linker protein), **BLK** (B lymphoid tyrosine kinase) and **PAX5** (paired box gene 5, a B-cell lineage specific activator).
● Keywords highly specific for **M1.5** included *Monocyte, Dendritic, CD14* or *Toll-like* and were associated with genes such as **MYD88** (myeloid differentiation primary response gene 88), **CD86, TLR2** (Toll-like receptor 2), **LILRB2** (leukocyte immunoglobulin-like receptor B2) and **CD163.**
● Keywords highly specific for **M3.1** included *Interferon, IFN-alpha, Antiviral,* or *ISRE* and were associated with genes such as **STAT1** (signal transducer and activator of transcription 1), **CXCL10** (CXC chemokine ligand 10, IP-10), **OAS2** (oligoadenylate synthetase 2) and **MX2** (myxovirus resistance 2).

This contrasted pattern of term occurrence denotes the remarkable functional coherence of each module. Information extracted from the literature for all the modules that have been identified permit a comprehensive functional characterization of the PBMC system at a transcriptional level. Table 2 provides an example of genes that may be used to distinguish between immune responses to, e.g., melanoma and liver transplant.

**Table 2: Genes in Module 1.4 Used to Distinguish Immune Responses**

| **moduleID** | **Entrez ID** | **Gene Symbol** | **Gene Title** |
|---|---|---|---|
| 1.4 | 55544 | RNPC1 | RNA-binding region (RNP1, RRM) containing 1 |
| 1.4 | 5930 | RBBP6 | retinoblastoma binding protein 6 |
| 1.4 | 80273 | GRPEL1 | GrpE-like 1, mitochondrial (*E*. *coli)* |
| 1.4 | 57162 | PELI1 | pellino homolog 1 (Drosophila) |
| 1.4 | 9921 | RNF10 | ring finger protein 10 /// ring finger protein 10 |
| 1.4 | 90637 | LOC90637 | hypothetical protein LOC90637 |
| 1.4 | 80314 | EPC1 | Enhancer of polycomb homolog 1 (Drosophila) |
| 1.4 | --- | --- | Full length insert cDNA clone ZB81B12 |
| 1.4 | 5756 | PTK9 | PTK9 protein tyrosine kinase 9 |
| 1.4 | 55038 | CDCA4 | cell division cycle associated 4 |
| 1.4 | 5187 | PER1 | period homolog 1 (Drosophila) |
| 1.4 | 9205 | ZNF237 | zinc finger protein 237 |
| 1.4 | 25976 | TIPARP | TCDD-inducible poly(ADP-ribose) polymerase |
| 1.4 | 57018 | CCNL1 | cycLin L1 |
| 1.4 | 64061 | TSPYL2 | TSPY-like 2 |
| 1.4 | 81488 | GRINL1A | glutamate receptor, ionotropic, N-methyl D-aspartate-like 1A |
| 1.4 | 22850 | KIAA0863 | KIAA0863 protein |
| 1.4 | 23764 | MAFF | v-maf musculoaponeurotic fibrosarcoma oncogene homolog F (avian) |
| 1.4 | 29035 | PR00149 | PRO0149 protein |
| 1.4 | 7803 | PTP4A1 | protein tyrosine phosphatase type IVA, member 1 |
| 1.4 | 11171 | STRAP | serine/threonine kinase receptor associated protein |
| 1.4 | 5814 | PURB | purine-rich element binding protein B |
| 1.4 | 5142 | PDE4B | phosphodiesterase 4B, cAMP-specific (phosphodiesterase E4 dunce homolog, Drosophila) |
| 1.4 | 30836 | ERBP | estrogen receptor binding protein |
| 1.4 | 6782 | STCH | stress 70 protein chaperone, microsome-associated, 60kDa |
| 1.4 | 10950 | BTG3 | BTG family, member 3 |
| 1.4 | 7037 | TFRC | transferrin receptor (p90, CD71) |
| 1.4 | 54934 | FLJ20436 | hypothetical protein FLJ20436 |
| 1.4 | 5144 | PDE4D | phosphodiesterase 4D, cAMP-specific (phosphodiesterase E3 dunce homolog, Drosophila) |
| 1.4 | 9929 | KIAA0063 | KIAA0063 gene product |
| 1.4 | 143187 | VTI1A | Vesicle transport through interaction with t-SNAREs homolog 1A (yeast) |
| 1.4 | 440309 | --- | LOC440309 |
| 1.4 | 150094 | SNF1LK | SNF1-like kinase /// SNF1-like kinase |
| 1.4 | 1850 | DUSP8 | dual specificity phosphatase 8 |
| 1.4 | 9584 | RNPC2 | RNA-binding region (RNP1, RRM) containing 2 |
| 1.4 | 140735 | Dlc2 | dynein light chain 2 |
| 1.4 | 54542 | MNAB | membrane associated DNA binding protein |
| 1.4 | 9262 | STK17B | serine/threonine kinase 17b (apoptosis-inducing) |
| 1.4 | 7128 | TNFAIP3 | tumor necrosis factor, alpha-induced protein 3 |
| 1.4 | 3183 | HNRPC | heterogeneous nuclear ribonucleoprotein C (C1/C2) |
| 1.4 | 5144 | PDE4D | Phosphodiesterase 4D, cAMP-specific (phosphodiesterase E3 dunce homolog, Drosophila) |
| 1.4 | 80311 | KLHL15 | kelch-like 15 (Drosophila) |
| 1.4 | 22850 | KIAA0863 | KIAA0863 protein |
| 1.4 | 5996 | RGS1 | --- |
| 1.4 | 468 | ATF4 | activating transcription factor 4 (tax-responsive enhancer element B67) |
| 1.4 | --- | --- | --- |
| 1.4 | 7430 | VIL2 | villin 2 (ezrin) |
| 1.4 | 6627 | SNRPA1 | small nuclear ribonucleoprotein polypeptide A' |
| 1.4 | 7750 | ZNF198 | zinc finger protein 198 |
| 1.4 | 1390 | CREM | cAMP responsive element modulator |
| 1.4 | 10291 | SF3A1 | splicing factor 3a, subunit 1, 120kDa |
| 1.4 | 9308 | CD83 | CD83 antigen (activated B lymphocytes, immunoglobulin superfamily) |
| 1.4 | 63935 | C20orf67 | --- |
| 1.4 | 10049 | DNAJB6 | DnaJ (Hsp40) homolog, subfamily B, member 6 |
| 1.4 | 51526 | C20orf111 | chromosome 20 open reading frame 111 |
| 1.4 | 55500 | ETNKl | ethanolamine kinase 1 /// ethanolamine kinase 1 |
| 1.4 | 79441 | C4orf15 | chromosome 4 open reading frame 15 |
| 1.4 | 11236 | RNF139 | ring finger protein 139 |
| 1.4 | 246243 | RNASEH1 | ribonuclease H1 |
| 1.4 | 3727 | JUND | jun D proto-oncogene |
| 1.4 | 6500 | SKP1A | S-phase kinase-associated protein 1A (p19A) |
| 1.4 | 4204 | MECP2 | Methyl CpG binding protein 2 (Rett syndrome) |
| 1.4 | 3189 | HNRPH3 | heterogeneous nuclear ribonucleoprotein H3 (2H9) |
| 1.4 | 222161 | DKFZp586I1420 | hypothetical protein DKFZp586I1420 |
| 1.4 | 266812 | NAP1L5 | nucleosome assembly protein 1-like 5 |
| 1.4 | 9908 | G3BP2 | Ras-GTPase activating protein SH3 domain-binding protein 2 |
| 1.4 | 10425 | ARIH2 | --- |
| 1.4 | 55422 | ZNF331 | Zinc finger protein 331 |
| 1.4 | 8454 | CUL1 | Cullin 1 |
| 1.4 | 51119 | SBDS | Shwachman-Bodian-Diamond syndrome |
| 1.4 | 6309 | SC5DL | sterol-C5-desaturase (ERG3 delta-5-desaturase homolog, fungal)-like |
| 1.4 | 5277 | PIGA | phosphatidylinositol glycan, class A (paroxysmal nocturnal hemoglobinuria) /// phosphatidylinositol glycan, class A (paroxysmal nocturnal hemoglobinuria) |
| 1.4 | 3422 | IDI1 | isopentenyl-diphosphate delta isomerase |
| 1.4 | 63935 | C20\orf67 | chromosome 20 open reading frame 67 |
| 1.4 | 7975 | MAFK | v-maf musculoaponeurotic fibrosarcoma oncogene homolog K (avian) |
| 1.4 | 7456 | WASPIP | Wiskott-Aldrich syndrome protein interacting protein |
| 1.4 | 55975 | KLHL7 | kelch-like 7 (Drosophila) |
| 1.4 | 7128 | TNFAIP3 | tumor necrosis factor, alpha-induced protein 3 |
| 1.4 | 388796 | LOC388796 | hypothetical LOC388796 |
| 1.4 | 25852 | ARMC8 | armadillo repeat containing 8 |
| 1.4 | 54542 | MNAB | Membrane associated DNA binding protein |
| 1.4 | 55422 | ZNF331 | zinc finger protein 331 |
| 1.4 | 1390 | CREM | cAMP responsive element modulator |
| 1.4 | 10209 | SUI1 | putative translation initiation factor |
| 1.4 | 10049 | DNAJB6 | DnaJ (Hsp40) homolog, subfamily B, member 6 |
| 1.4 | 4929 | NR4A2 | nuclear receptor subfamily 4, group A, member 2 |
| 1.4 | 1540 | CYLD | cylindromatosis (turban tumor syndrome) |
| 1.4 | 4929 | NR4A2 | nuclear receptor subfamily 4, group A, member 2 |
| 1.4 | 5805 | PTS | 6-pyruvoyltetrahydropterin synthase |
| 1.4 | 10926 | ASK | activator of S phase kinase |
| 1.4 | 10923 | PC4 | activated RNA polymerase II transcription cofactor 4 /// similar to Activated RNA polymerase II transcriptional coactivator p15 (Positive cofactor 4) (PC4) (p14) |
| 1.4 | 388796 | RNU71A | Hypothetical LOC388796 |
| 1.4 | 133746 | JMY | junction-mediating and regulatory protein |
| 1.4 | 90634 | CG018 | Hypothetical gene CG018 |
| 1.4 | 10209 | SUI1 | putative translation initiation factor |
| 1.4 | 1847 | DUSP5 | dual specificity phosphatase 5 |
| 1.4 | 7088 | TLE1 | Transducin-like enhancer of split 1 (E(sp1) homolog, Drosophila) |
| 1.4 | 84275 | MGC4399 | mitochondrial carrier protein |
| 1.4 | --- | --- | --- |
| 1.4 | 7803 | PTP4A1 | protein tyrosine phosphatase type IVA, member 1 |
| 1.4 | 55422 | ZNF331 | zinc finger protein 331 |
| 1.4 | --- | --- | CDNA clone IMAGE:30332316, partial cds |
| 1.4 | 3609 | ILF3 | interleukin enhancer binding factor 3, 90kDa |
| 1.4 | --- | --- | Homo sapiens, clone IMAGE:4753714, mRNA |
| 1.4 | 6651 | SON | SON DNA binding protein |
| 1.4 | 11276 | AP1GBP1 | AP1 gamma subunit binding protein 1 |
| 1.4 | 84124 | ZNF394 | zinc finger protein 394 |
| 1.4 | 63935 | C20orf67 | --- |
| 1.4 | 1983 | EIF5 | eukaryotic translation initiation factor 5 |
| 1.4 | 80063 | ATF7IP2 | Activating transcription factor 7 interacting protein 2 |
| 1.4 | 285831 | LOC285831 | hypothetical protein LOC285831 |
| 1.4 | 81873 | ARPC5L | actin related protein 2/3 complex, subunit 5-like |
| 1.4 | 144438 | LOC144438 | hypothetical protein LOC144438 |
| 1.4 | 10209 | SUI1 | putative translation initiation factor |
| 1.4 | 3021 | H3F3B | H3 histone, family 3B (H3.3B) |
| 1.4 | 25948 | KBTBD2 | kelch repeat and BTB (POZ) domain containing 2 |
| 1.4 | --- | --- | CDNA FLJ40725 fis, clone TKIDN1000001, highly similar to Translocase of inner mitochondrial membrane 23 |
| 1.4 | 1540 | CYLD | cylindromatosis (turban tumor syndrome) |
| 1.4 | 5144 | PDE4D | phosphodiesterase 4D, cAMP-specific (phosphodiesterase |
| 1.4 | 51182 | HSPA14 | E3 dunce homolog, Drosophila) heat shock 70kDa protein 14 |
| 1.4 | 29080 | HSPC128 | HSPC128 protein |
| 1.4 | 8731 | RNMT | RNA (guanine-7-) methyltransferase |
| 1.4 | 3423 | IDS | iduronate 2-sulfatase (Hunter syndrome) |
| 1.4 | 283991 | MGC29814 | hypothetical protein MGC29814 |
| 1.4 | 1454 | CSNK1E | Casein kinase 1, epsilon |
| 1.4 | 26051 | PPP1R16B | protein phosphatase 1, regulatory (inhibitor) subunit 16B |
| 1.4 | 3422 | IDI1 | isopentenyl-diphosphate delta isomerase |
| 1.4 | 5887 | RAD23B | RAD23 homolog B (S. cerevisiae) |
| 1.4 | 5144 | PDE4D | Phosphodiesterase 4D, cAMP-specific (phosphodiesterase E3 dunce homolog, Drosophila) |
| 1.4 | 49854 | ZNF295 | zinc finger protein 295 |
| 1.4 | 60493 | FLJ13149 | hypothetical protein FLJ13149 |
| 1.4 | 10950 | BTG3 | BTG family, member 3 |

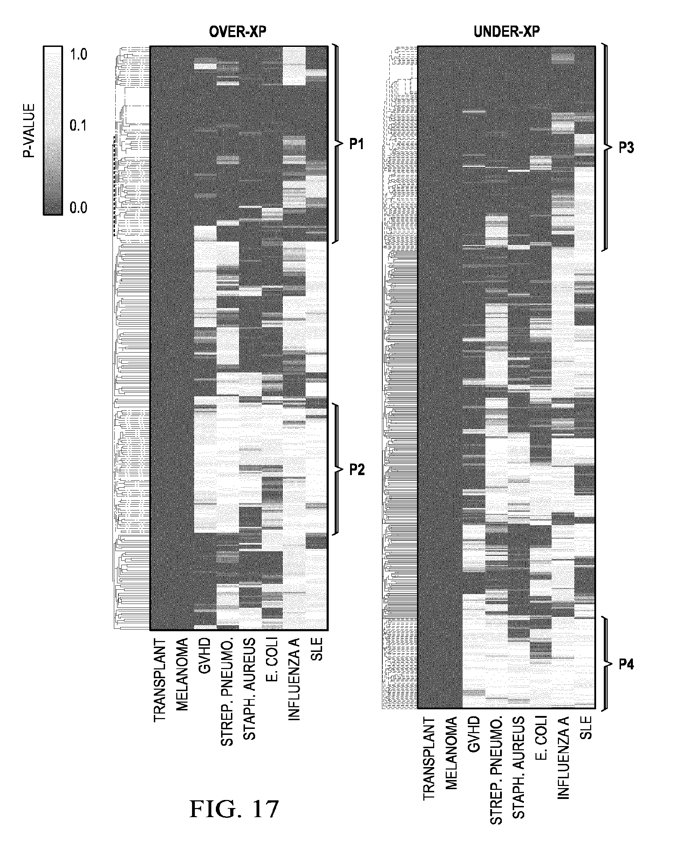

Yet another group that may be used alone or in combination with the genes listed in supplementary table that includes the data shown in Figure 17, denoted P2, and which may include one or more the following genes that are overexpressed, e.g., WARS; IFI53; IFP53; GAMMA-2; FAM46C; FLJ20202; H3F3B; H3.3B; FOXK2; ILF; ILF1; ILF-1; DUSP5; HVH3; ARF6; DKFZp762C186; BRD2; NAT; RNF3; FSRG1; RING3; D6S113E; KIAA9001; RORA; ROR1; ROR2; ROR3; RZRA; NR1F1; DKFZp762C186; DNAJB1; SUI1; CXCR4; HM89; LAP3; NPYR; WHIM; LESTR; NPY3R; HSY3RR; NPYY3R; D2S201E; GRINL1A; CTSB; TRIP-Br2; PDE4B; DPDE4; PDEIVB; PMAIP1; APR; NOXA; BTG2; PC3; TIS21; ASAHL; SON; SUI1; A121; ISO1; HERPUD1; SUP; Mif1; KIAA0025; DUSP2; PAC1; PAC-1; RNF139; RCA1; TRC8; HRCA1; MGC31961; TNFAIP3; A20; TNFA1P2; ARS2; HNRPL; hnRNP-L; P/OKc1.14; C20orf67; C20orf111; HSPC207; dJ1183I21.1; ZNF331; RITA; ZNF361; ZNF463; C20orf67; IER5; SBBI48; ; SUI1; JUN; AP1; CD69; TOB1; H3F3B; H3.3B; FOLR1; TNFAIP3; TCF8; BZP; ZEB; ZEB1; AREB6; ZFHEP; NIL-2A; ZFHX1A; NIL-2-A; DUSP10; MKP5; MKP-5; GGTLA4; MGC50550; dJ831C21.2; PMAIP1; ZC3HAV1; ZAP; FLB6421; ZC3HDC2; FLJ13288; MGC48898; DSIPI; DIP; GILZ; hDIP; TSC-22R; MCL1; TM; EAT; MCL1L; MCL1S; MGC1839; SH3TC1; FLJ20356; CIAS1; FCU; MWS; FCAS; NALP3; C1orf7; PYPAF1; AII/AVP; AGTAVPRL; SLC15A3; PHT2; PTR3; hPTR3; PTDSR; PSR; PTDSR1; KIAA0585; BHLHB2; DEC1; STRA13; Stra14; HMGE; KIAA0063; NR4A2; NOT; RNR1; HZF-3; NURR1; TINUR; NR4A2; NOT; RNR1; HZF-3; NURR1; TINUR; PTS; PTPS; HEAB; CLP1; hClp1; AREG; SDGF; CRDGF; MGC13647; EDG4; LPA2; EDG-4; LPAR2; CREM; ICER; MGC17881; MGC41893; CD83; BL11; HB15; ZNF394; FLJ12298, and combinations thereof.

Module-based microarray data mining strategy. Results from "traditional" microarray analyses are notoriously noisy and difficult to interpret. A widely accepted approach for microarray data analyses includes three basic steps: 1) Use of a statistical test to select genes differentially expressed between study groups; 2) Apply pattern discovery algorithms to identify signatures among the resulting gene lists; and 3) Interpret the data using knowledge derived from the literature or ontology databases.

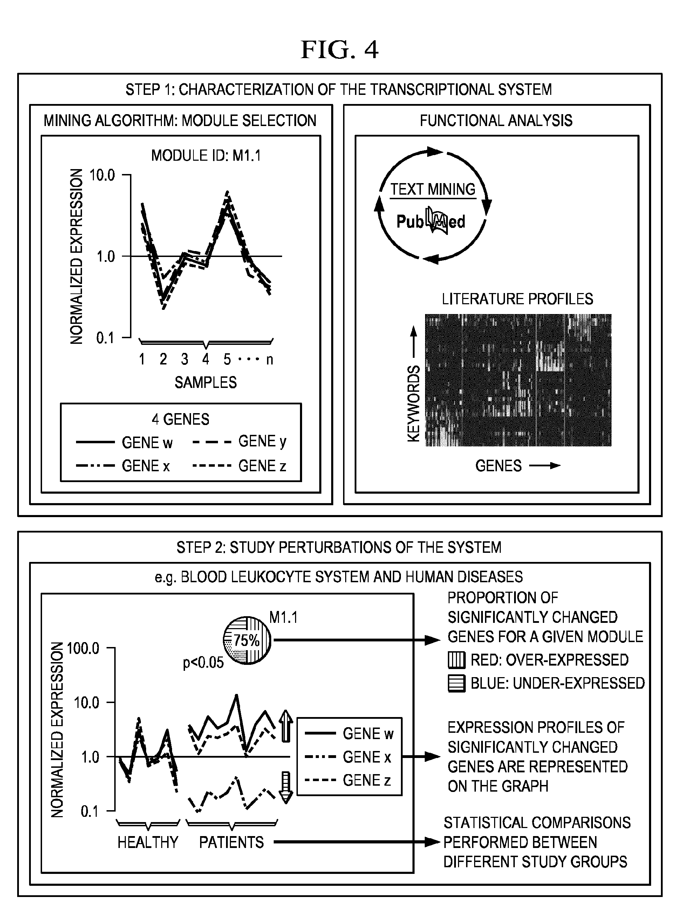

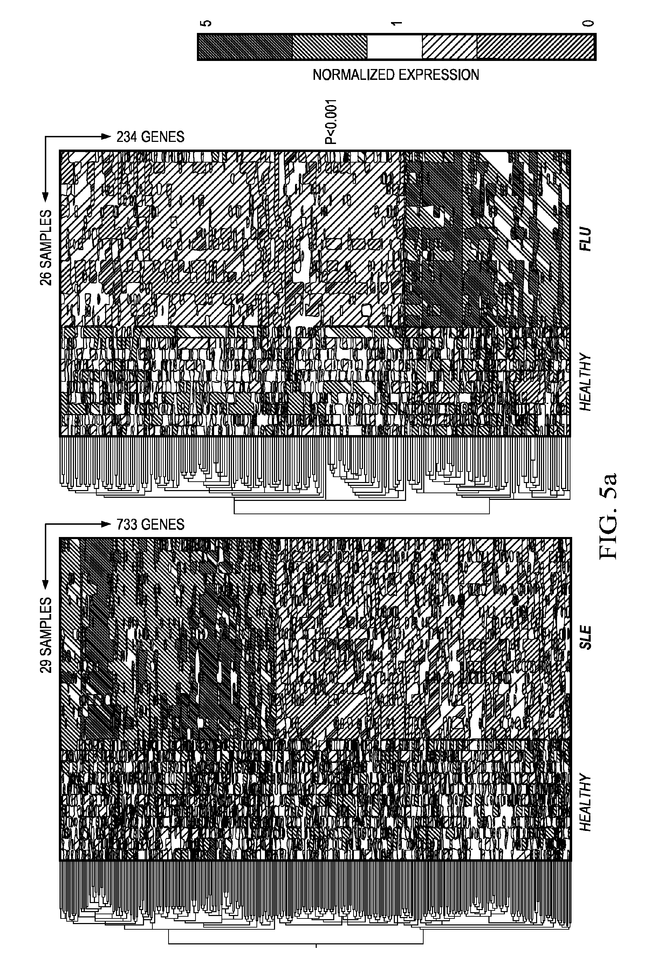

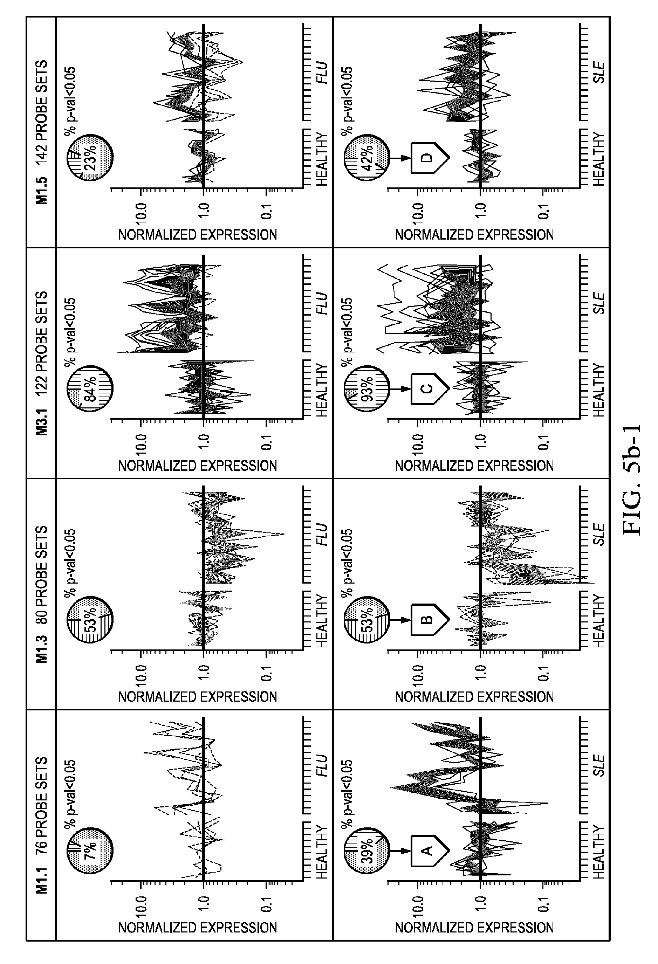

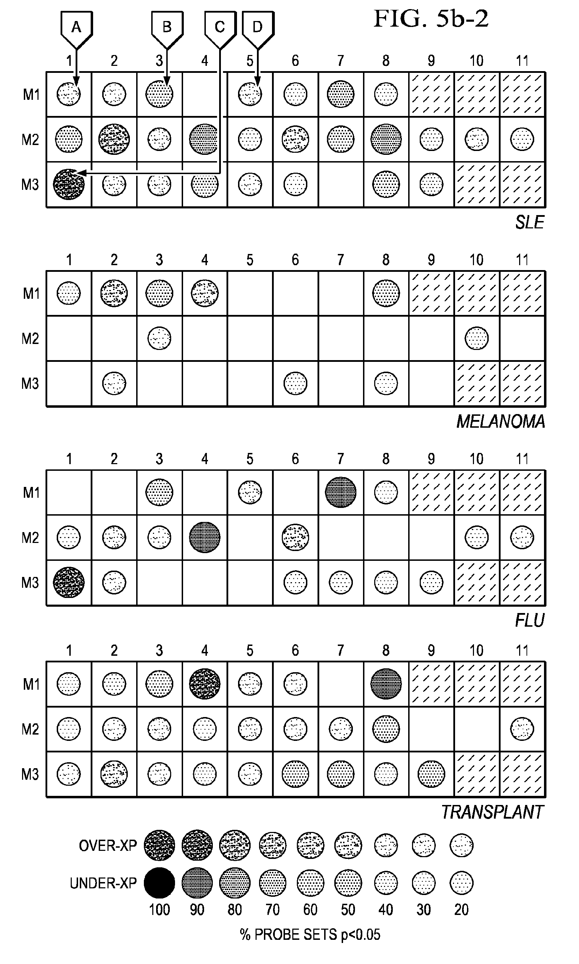

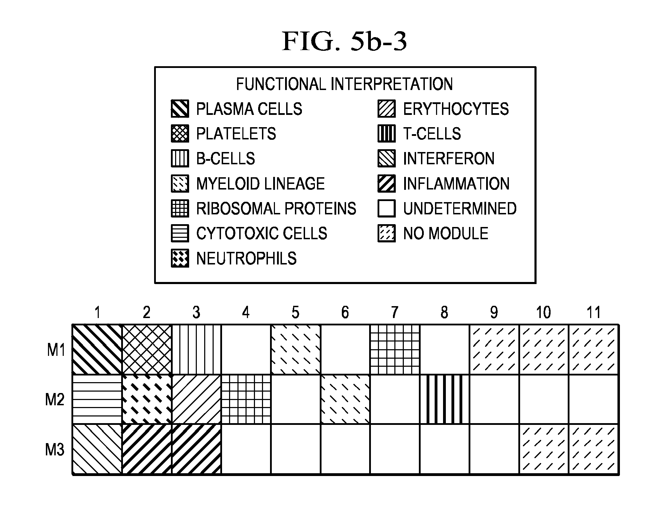

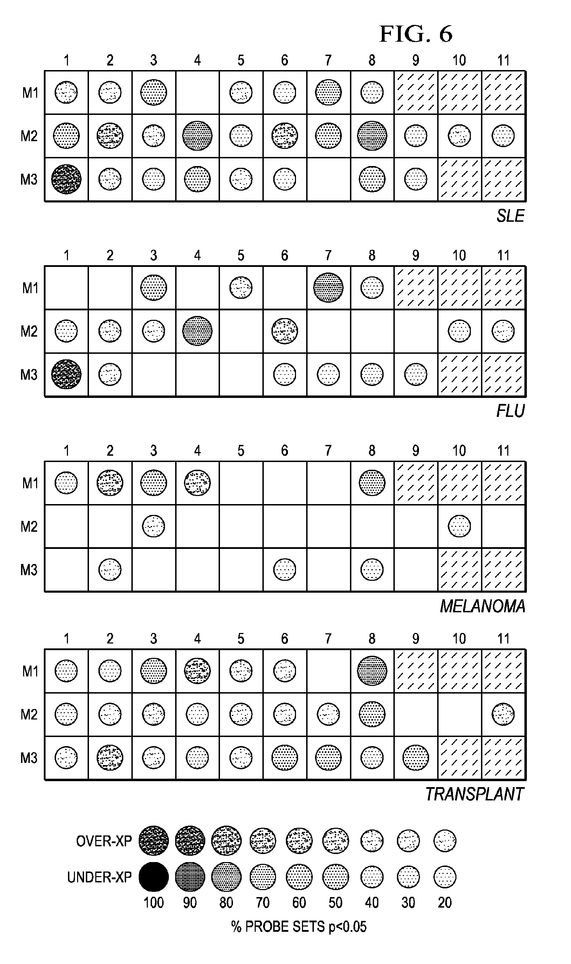

The present invention uses a novel microarray data mining strategy emphasizing the selection of biologically relevant transcripts at an early stage of the analysis. This first step can be carried out using for instance the modular mining algorithm described above in combination with a functional mining tool used for in-depth characterization of each transcriptional module (FIGURE 4: top panel, Step 1). The analysis does not take into consideration differences in gene expression levels between groups. Rather, the present invention focuses instead on complex gene expression patterns that arise due to biological variations (e.g., inter-individual variations among a patient population). After defining the transcriptional components associated to a given biological system the second step of the analysis includes the analysis of changes in gene expression through the comparison of different study groups (FIGURE 4: bottom panel, Step 2). Group comparison analyses are carried out independently for each module. Changes at the module level are expressed as the proportion of genes that meet the significance criteria (represented by a pie chart in FIGURE 5 or a spot in FIGURE 6). Notably, carrying out comparisons at the modular level permits to avoid the noise generated when thousands of tests are performed on "random" collections of genes.

Perturbation of modular PBMC transcriptional profiles in human diseases. To illustrate the second step of the microarray data mining strategy described above (FIGURE 4), gene expression data for PBMC samples obtained from two pediatric patient populations composed of eighteen children with systemic lupus erythematosus (SLE) and sixteen children with acute influenza A infection was obtained, compared and analyzed. Each patient cohort was matched to its respective control group (healthy volunteers: eleven and ten donors were matched to the SLE and influenza groups, respectively). Following the analytical scheme depicted in FIGURE 4, a statistical group comparisons between patient and healthy groups for each individual module and measured the proportion of genes significantly changed in each module (FIGURE 5) was performed. The statistical group comparison approach allows the user to focus the analysis on well defined groups of genes that contain minimal amounts of noise and carry identifiable biological meaning. A key to the graphical representation of these results is provided in Figure 4.

The following findings were made: (1) that a large proportion of genes in M3.1 ("interferon-associated") met the significance level in both Flu and SLE groups (84% and 94%, respectively). This observation confirms earlier work with SLE patients ¹⁹ and identifies the presence of an interferon signature in patients with acute influenza infection. (2) Equivalent proportions of genes in M1.3 ("B-cell-associated") were significantly changed in both groups (53%), with over 50% overlap between the two lists. This time, genes were consistently under-expressed in patient compared to healthy groups. (3) Modules were also found that differentiate the two diseases. The proportion of genes significantly changed in Module 1.1 reaches 39% in SLE patients and is only 7% in Flu patients, which at a significance level of 0.05 is very close to the proportion of genes that would be expected to be differentially expressed only by chance. Interestingly, this module is almost exclusively composed of genes encoding immunoglobulin chains and has been associated with plasma cells. However, this module is clearly distinct from the B-cell associated module (M1.3), both in terms of gene expression level and pattern (not shown). (4) As illustrated by module M1.5, gene-level analysis of individual modules can be used to further discriminate the two diseases. It is also the case for M1.3, where, despite the absence of differences at the module-level (FIGURE 4: 53% under-expressed transcripts), differences between Flu and SLE groups could be identified at the gene-level (only 51% of the under-expressed transcripts in M1.3 were common to the two disease groups). These examples illustrate the use of a modular framework to streamline the analysis and interpretation of microarray results.

Mapping changes in gene expression at the modular level. Data visualization is paramount for the interpretation of complex datasets and the present invention includes a comprehensive graphical illustration of changes that occur at the modular level. Changes in gene expression levels caused by different diseases were represented for the twenty-eight PBMC transcriptional modules (FIGURE 6). Each disease group is compared to its respective control group composed of healthy donors who were matched for age and sex (eighteen patients with SLE, sixteen with acute influenza infection, sixteen with metastatic melanoma and sixteen liver transplant recipients receiving immunosuppressive drug treatment were compared to control groups composed of ten to eleven healthy subjects). Module-level data were represented graphically by spots aligned on a grid, with each position corresponding to a different module (See Table 1 for functional annotations on each of the modules).

The spot intensity indicates the proportion of genes significantly changed for each module. The spot color indicates the polarity of the change (red: proportion of over-expressed genes, blue: proportion of under-expressed genes; modules containing a significant proportion of both over- and under-expressed genes would be purple-though none were observed). This representation permits a rapid assessment of perturbations of the PBMC transcriptional system. Such "module maps" were generated for each disease. When comparing the four maps, we found that diseases were characterized by a unique modular combination. Indeed, results for M1.1 and M1.2 alone sufficed to distinguish all four diseases (M1.1/M1.2: SLE = +/+; FLU=0/0; Melanoma=-/+; transplant=-/-). A number of genes in M3.2 ("inflammation") were over-expressed in all diseases (particularly so in the transplant group), while genes in M3.1 (interferon) were over-expressed in patients with SLE, influenza infection and, to some extent, transplant recipients. "Ribosomal protein" module genes (M1.7 and M2.4) were under-expressed in both SLE and Flu groups. The level of expression of these genes was recently found to be inversely correlated to disease activity in SLE patients (Bennett *et al.,* submitted). M2.8 includes T-cell transcripts which are under-expressed in lymphopenic SLE patients and transplant recipients treated with immunosuppressive drugs targeting T-cells.

Interestingly, differentially expressed genes in each module were predominantly either under-expressed or over-expressed (FIGURE 5 and FIGURE 6). Yet, modules were purely selected on the basis of similarities in gene expression profiles, not changes in expression levels between groups. The fact that changes in gene expression appear highly polarized within each module denotes the functional relevance of modular data. Thus, the present invention enables disease fingerprinting by a modular analysis of patient blood leukocyte transcriptional profiles.

Validation of PBMC modules in a published dataset. Next, the validity of the PBMC transcriptional modules described above in a "third-party" dataset was tested. The study from Connolly, et al., who investigated the effects of exercise on gene expression in human PBMCs²⁰ was tested.

Blood samples were obtained from 35 patients with metastatic melanoma enrolled in three phase I/II clinical trials designed to test the efficacy of a dendritic cell therapeutic vaccine as seen in the table below. Gene expression signatures were generated from blood samples collected prior to the initiation of vaccine therapy, and at least 4 weeks after the last systemic therapy if a patient had undergone such.

**Table 3: Clinical and demographic characteristics of 35 patients with metastatic melanoma**

| ID | Sex | Age | Stage | Diagnosis | Time from Dx to Blood Draw (Months) | Blood Draw | Status | Blood Draw to Time of Death (Months) |
|---|---|---|---|---|---|---|---|---|
| MEL 23 | F | 61 | M1a | 02/14/00 | 16 | 06/28/01 | Deceased | 28 |
| MEL 24 | M | 53 | M1c | 09/01/99 | 22 | 07/05/01 | Deceased | 5 |
| MEL 26 | F | 52 | M1c | 10/01/97 | 45 | 07/18/01 | Deceased | 2 |
| MEL 27 | F | 54 | M1a | 07/10/93 | 98 | 09/14/01 | Deceased | 5 |
| MEL 29 | M | 41 | M1c | 10/26/94 | 83 | 09/26/01 | Deceased | 14 |
| MEL 30 | F | 58 | M1c | 10/04/99 | 23 | 09/25/01 | Deceased | 11 |
| MEL 32 | M | 56 | M1b | 01/17/94 | 95 | 12/17/01 | Deceased | 24 |
| MEL 34 | F | 28 | M1b | 04/01/01 | 10 | 02/05/02 | Deceased | 42 |
| MEL 35 | M | 29 | M1a | 08/25/98 | 43 | 03/12/02 | Deceased | 12 |
| MEL 36 | F | 69 | M1b | 01/01/85 | 205 | 02/19/02 | Deceased | 7 |
| MEL 40 | F | 43 | M1c | 07/02/91 | 132 | 07/19/02 | Deceased | 19 |
| MEL 43 | M | 60 | M1a | 08/14/94 | 100 | 12/04/02 | Alive 05/16/05 | |
| MEL44 | F | 68 | M1c | 05/01/99 | 44 | 01/28/03 | Deceased | 12 |
| MEL 45 | F | 53 | M1a | 02/01/02 | 10 | 12/17/02 | Alive 5/5/05 | * |
| MEL 46 | F | 47 | M1c | 11/18/97 | 61 | 12/27/02 | Deceased | 22 |
| MEL 47 | F | 35 | M1c | 03/02/02 | 10 | 01/09/03 | Deceased | 2 |
| MEL 48 | M | 68 | M1b | *1992 | >120 | 03/12/03 | Alive 05/10/05 | * |
| MEL 49 | M | 71 | M1b | 12/05/97 | 64 | 04/10/03 | Alive 07/05/05 | * |
| MEL 50 | M | 52 | M1c | 07/08/97 | 69 | 04/11/03 | Deceased | 18 |
| MEL 51 | M | 56 | M1c | 10/01/01 | 18 | 04/16/03 | Alive 05/17/05 | * |
| MEL 52 | M | 42 | M1c | 03/01/02 | 13 | 04/17/03 | Deceased | 9 |
| MEL 54 | M | 50 | M1b | 07/20/90 | 153 | 04/25/03 | Alive 04/08/05 | * |
| MEL 56 | M | 71 | M1c | 03/01/01 | 26 | 05/29/03 | Deceased | 9 |
| MEL 57 | F | 36 | M1b | 07/01/02 | 11 | 06/05/03 | Deceased | 20 |
| MEL 58 | M | 67 | M1c | 10/01/99 | 45 | 07/18/03 | Deceased | 10 |
| MEL 59 | M | 61 | M1c | unknown | * | 07/25/03 | Alive 06/22/05 | * |
| MEL 60 | M | 41 | M1c | 11/01/02 | 9 | 08/14/03 | Deceased | 7 |
| MEL 61 | F | 54 | M1a | 03/03/99 | 54 | 09/10/03 | Alive 05/18/05 | * |
| MEL 62 | M | 46 | M1b | 12/01/01 | 22 | 10/09/03 | Deceased | 5 |
| MEL 63 | M | 75 | M1b | 12/01/00 | 34 | 10/29/03 | Alive 03/16/05 | * |
| MEL 64 | F | 53 | M1b | 04/01/00 | 42 | 10/30/03 | Alive 03/05/04 | * |
| MEL 65 | M | 62 | M1b | 08/14/94 | 111 | 11/14/03 | Alive 05/16/05 | * |
| MEL 68 | M | 74 | M1b | 06/09/04 | 1 | 07/29/04 | Deceased | 9 |
| MEL 70 | M | 67 | M1b | 04/06/04 | 5 | 09/23/04 | Alive 8/18/2005 | * |
| MEL 72 | M | 50 | M1c | 09/23/04 | 2 | 11/10/04 | Deceased | * |

The Table provides both clinical and demographic characteristics of 35 patients with metastatic melanoma.

A second group of patients included 39 liver transplant recipients maintaining their graft under pharmacological immunosuppressive therapy, time from transplant had a median of 729 days and a range of between 338 and 1905 days. Outpatients coming for routine exams were recruited for this study. All patients received standard treatment regimens with calcineurin inhibitors (e.g., Tacrolimus: n=25; Cyclosporin A: n=13). The main indications for liver transplant were hepatitis C (n = 19) and Laennec's cirrhosis (n = 7). The table below provides both clinical and demographic characteristics of 39 liver transplant recipients.

**Table 4: Clinical and demographic characteristics of 39 liver transplant recipients**

| Patient ID | Age | Sex | Transpl. to Blood Draw (Days) | TAC | CsA | Primary Dx |
|---|---|---|---|---|---|---|
| R1292 | 47 | M | 1854 | yes | | Hepatitis C |
| R1297 | 48 | M | 1868 | | yes | Hepatitis C |
| R1308 | 66 | F | 1905 | | yes | Primary Biliary Cirrhosis |
| R1322 | 32 | F | 1821 | | yes | Hepatitis C |
| R1323 | 45 | M | 1828 | | yes | Hepatitis C |
| R1325 | 50 | M | 1801 | | yes | Hepatitis C |
| R1329 | 51 | F | 1781 | yes | | Laennec's Cirrhosis |
| R1340 | 50 | M | 1829 | | yes | Laennec's Cirrhosis |
| R1348 | 64 | F | 1802 | yes | | Fuhninant Hepatic Failure |
| R1355 | 61 | M | 1780 | yes | | Cryptogenic |
| R1364 | 42 | M | 1756 | yes | | Hepatitis C |
| R1413 | 52 | M | 1856 | | yes | Laennec's Cirrhosis |
| R1673 | 60 | F | 756 | yes | | Hepatitis B |
| R1674 | 45 | M | 729 | yes | | Hepatitis C |
| R1684 | 65 | F | 721 | yes | | Mx. Carcinoid |
| R1686 | 59 | M | 704 | yes | | Hepatitis B |
| R1689 | 43 | M | 692 | yes | | Hepatitis C |
| R1700 | 53 | M | 732 | | yes | Hepatitis C |
| R1701 | 45 | M | 737 | yes | | Hepatitis C |
| R1702 | 48 | M | 726 | yes | | Hepatitis C |
| R1706 | 57 | M | 721 | yes | | Laennec's Cirrhosis |
| R1710 | 50 | F | 736 | yes | | Cryptogenic |
| R1714 | 63 | F | 718 | yes | | Nonalcoholic Steatohepatitis |
| R1718 | 53 | F | 707 | | yes | Hepatitis C |
| R1754 | 51 | F | 589 | yes | | Primary Sclerosing Cholangitis |
| R1771 | 42 | F | 812 | | | Hepatitis C |
| R1787 | 60 | F | 794 | yes | | Laennec's Cirrhosis |
| R1805 | 42 | M | 735 | yes | | Laennec's Cirrhosis & Postnecrotic Cirrhosis Type C |
| R1814 | 45 | M | 427 | yes | | Hepatitis C |
| R1838 | 66 | F | 360 | yes | | Autoimmune Hepatitis |
| R1839 | 56 | M | 354 | yes | | Cryptogenic |
| R1841 | 52 | M | 363 | yes | | PSC-UC |
| R1843 | 48 | M | 361 | | yes | Hepatitis C |
| R1845 | 44 | F | 338 | yes | | Primary Biliary Cirrhosis |
| R1846 | 41 | F | 338 | | yes | Hepatitis C |
| R1847 | 53 | M | 358 | yes | | Laennec's Cirrhosis |
| R1854 | 50 | M | 350 | | yes | Hepatitis C |
| R1971 | 46 | M | 367 | | yes | Hepatitis C; Hepatocellular Carcinoma with Cirrhosis |
| R1974 | 54 | M | 341 | yes | | Hepatitis C |

Blood samples were also obtained from 25 healthy donors that constituted the control groups. The table below provides the demographic characteristics of the 25 healthy donors.

**Table 5: Demographic characteristics of 25 healthy donors**

| Healthy Volunteers | Sex | Age |
|---|---|---|
| D-001 | M | 41 |
| D-002 | F | 53 |
| D-005 | F | 40 |
| D-007 | F | 44 |
| D-008 | M | 40 |
| D-010 | M | 46 |
| D-011 | F | 43 |
| D-013 | M | 58 |
| D-014 | F | 47 |
| D-015 | M | 42 |
| D-016 | F | 40 |
| D-017 | M | 25 |
| D-018 | M | 46 |
| D-019 | F | 40 |
| D-020 | M | 39 |
| D-021 | M | 45 |
| D-022 | M | 50 |
| D-024 | F | 44 |
| D-025 | F | 48 |
| D-027 | F | 43 |
| D-028 | F | 43 |
| D-029 | M | 43 |
| D-031 | M | 35 |
| D-032 | F | 43 |
| D-033 | F | 43 |

Identification of disease-associated blood leukocyte transcriptional signatures. Blood leukocyte gene expression signatures were identified in patients with metastatic melanoma and liver transplant recipients. Each set of patients was compared to a control group of healthy volunteers. Patient samples were divided into a training set, used to identify disease-associated and predictive expression signatures, and an independent test set. This step-wise analysis allowed validation of results in samples that were not used to establish the disease signature. Stringent criteria were employed to select the samples forming training sets in order to avoid confounding the analysis with biological and/or technical factors. The table below illustrates the composition of sample sets taking into account age, gender and sample processing method used for the identification (training) and validation (testing) of expression signatures associated with metastatic melanoma.

**Table 6: Composition of sample sets associated with metastatic melanoma.**

| **Training** | | | | | | | | | |
|---|---|---|---|---|---|---|---|---|---|
| **Set** | | | | | **Test** | | | | Total n |
| **HV** | | Fresh | Frozen | | | Fresh | Frozen | | |
| | Male | 7 | 7 | 14 | Male | 5 | 0 | 5 | |
| | Female | 0 | 9 | 9 | Female | 8 | 0 | 8 | |
| | | 7 | 16 | 23 | | 13 | 0 | 13 | 36 |
| | | | | | | | | | |

| **Melanoma** | | Fresh | Frozen | | | Fresh | Frozen | | |
|---|---|---|---|---|---|---|---|---|---|
| | Male | 3 | 9 | 12 | Male | 0 | 11 | 11 | |
| | Female | 0 | 10 | 10 | Female | 0 | 5 | 5 | |
| | | 3 | 19 | 22 | | 0 | 16 | 16 | 38 |
| | | | Total | 45 | | | Total | 29 | |

Similarly, the table below provides the composition of sample sets used taking into account age, gender and sample processing method for the identification (training) and validation (testing) of expression signatures associated with liver transplant recipients undergoing immunosuppressive drug therapy.

**Table 7: Composition of sample sets associated with liver transplant recipients undergoing immunosuppressive drug therapy.**

| **Training** | | | | | | | | | |
|---|---|---|---|---|---|---|---|---|---|
| **Set** | | | | | **Test** | | | | Total n |
| **HV** | | Fresh | Frozen | | | Fresh | Frozen | | |
| | Male | 12 | 5 | 17 | Male | 0 | 2 | 2 | |
| | Female | 8 | 2 | 10 | Female | 0 | 7 | 7 | |
| | | 20 | 7 | 27 | | 0 | 9 | 9 | 36 |
| | | | | | | | | | |

| **LTx** | | Fresh | Frozen | | | Fresh | Frozen | | |
|---|---|---|---|---|---|---|---|---|---|
| | Male | 9 | 3 | 12 | Male | 15 | 0 | 15 | |
| | Female | 9 | 1 | 10 | Female | 6 | 0 | 6 | |
| | | 18 | 4 | 22 | | 21 | 0 | 21 | 43 |
| | | | Total | 49 | | | Total | 30 | |

Table 8 lists the genes differentially expressed in patients with metastatic melanoma in comparison to healthy volunteers. Statistical comparison of a group of twenty-two patients with metastatic melanoma versus twenty-three healthy volunteers, training set, identified 899 differentially expressed genes (p<0.01, non parametric Mann-Whitney rank test and >1.25 fold change; 218 overexpressed and 681 underexpressed genes). Tables 8 through 14 are provided in Computer Readable Form (CRF) as part of the Lengthy Table and are incorporated herein by reference. The Tables provide a correlation to the modules from which there were identified, their expression level, various nomenclatures for their individual identification.

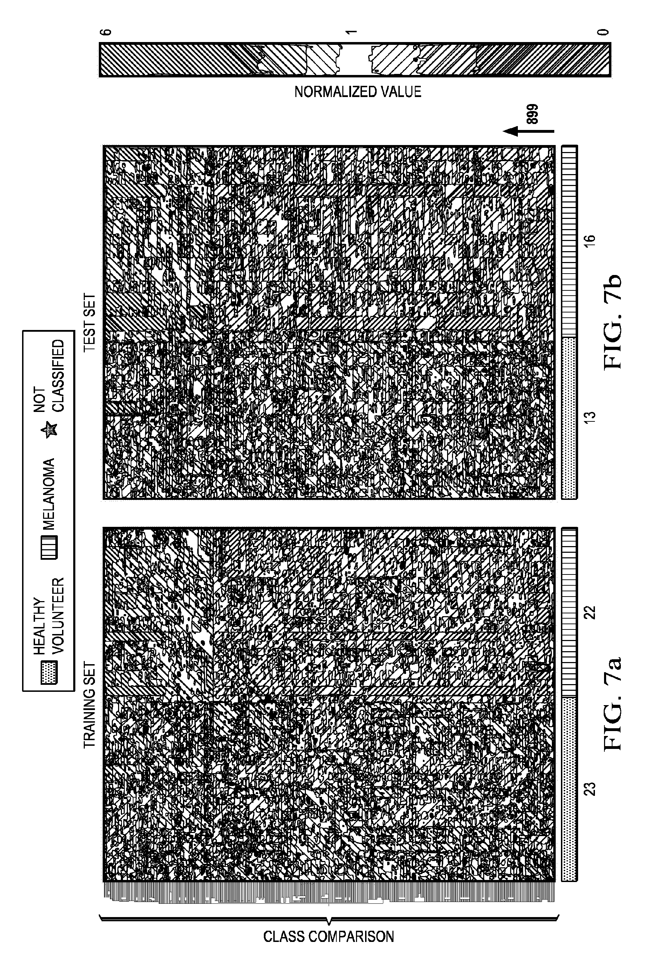

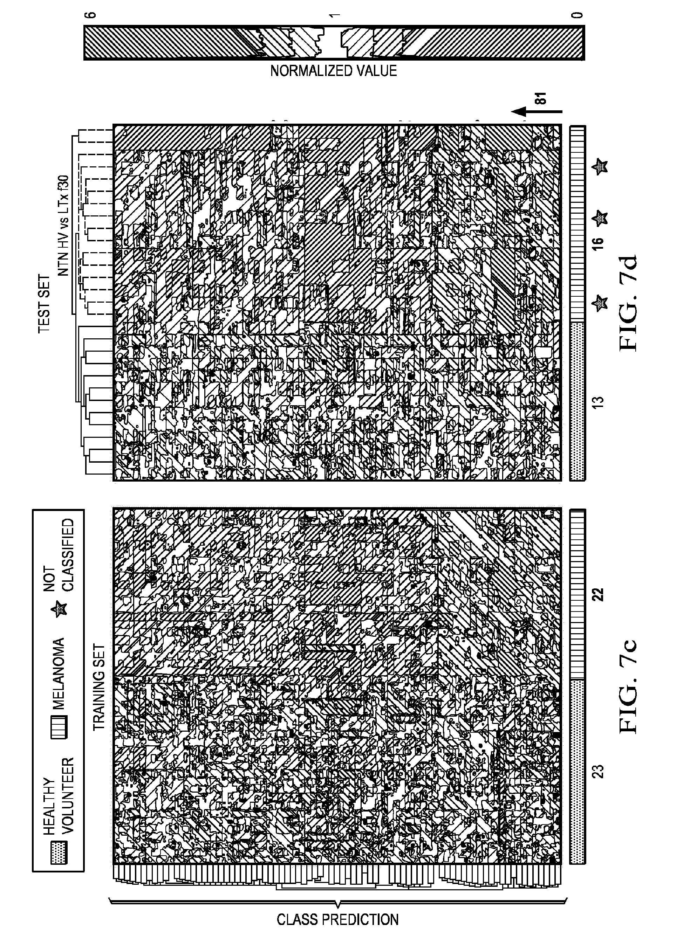

FIGURES 7A-7D are images of the hierarchical clustering of genes. FIGURE 7A illustrates the hierarchical clustering of genes that produce a reciprocal expression pattern; which was confirmed in an independent test set shown in FIGURE 7B. FIGURE 7B displays results from 13 healthy volunteers versus 16 patients. Next, class prediction algorithms were applied to the initial training set. These algorithms yielded 81 genes with the best ability to classify healthy volunteers and patients based on their differential expression as shown in FIGURE 7C and Table 9). Table 9 illustrates the expression levels of a set of transcripts discriminating patients with melanoma from healthy volunteers. Using these 81 genes, an independent test set was classified with 90% accuracy; the class of only three was indeterminate as illustrated in FIGURE 7D.

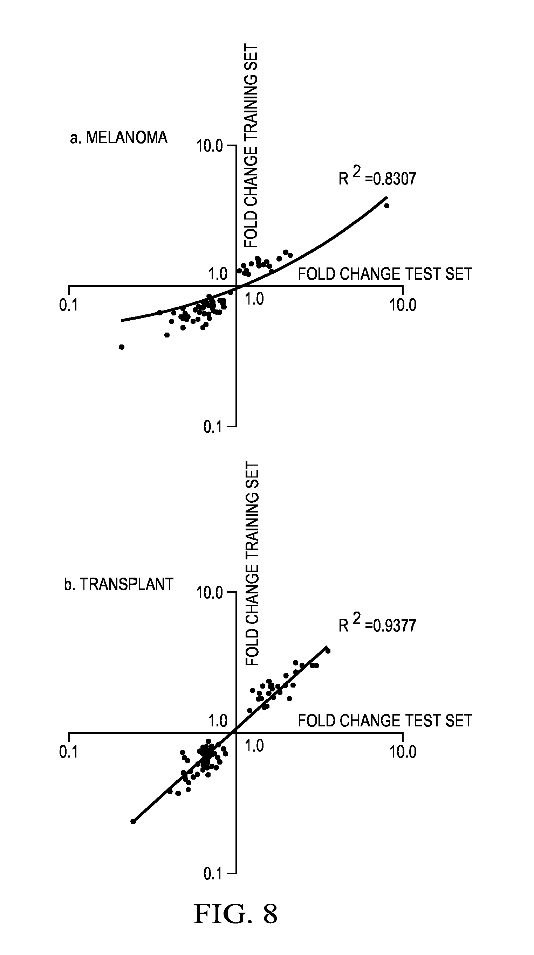

FIGURES 8A and 8B are plots of the microarray results in an independent set of samples to confirm the reliability of the results by correlating expression levels obtained for the training and test sets. Genes with the best ability to discriminate between patients and healthy volunteers were identified in a training set using a class prediction algorithm (k-Nearest Neighbors). Fold change expression levels between healthy controls and patients were measured for discriminative genes in the training set and an independent test set. Fold change values obtained in training and test sets were correlated: FIGURE 8A illustrates results for metastatic melanoma and produced 81 genes with a Pearson correlation of r²=0.83 and a p<0.0001 and FIGURE 8B illustrates results for liver transplant recipients and produced 65 genes with a Pearson correlation of r²=0.94 and a p<0.0001.

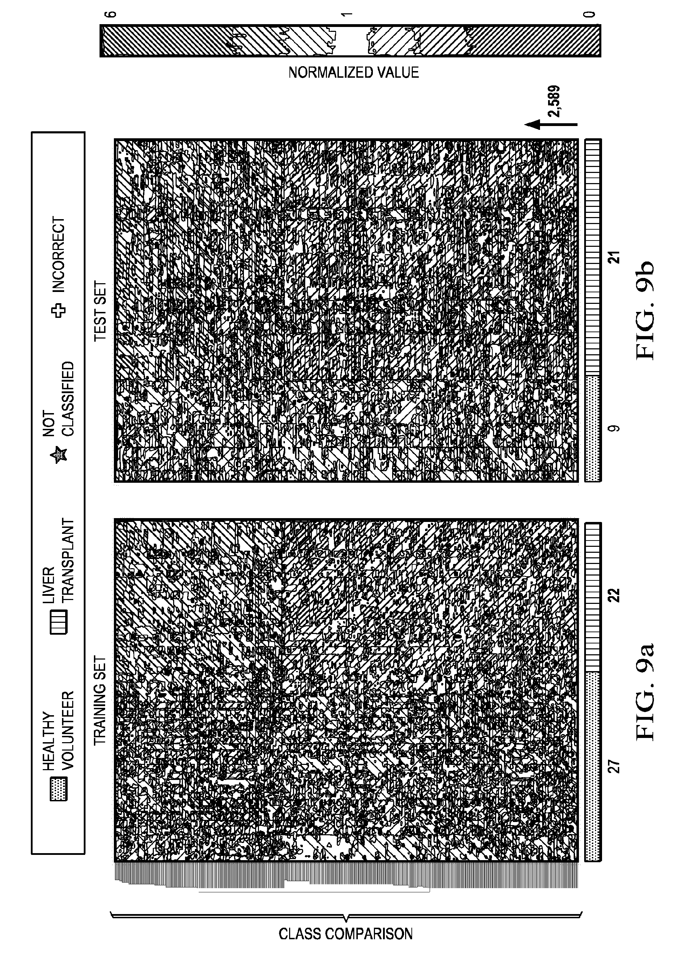

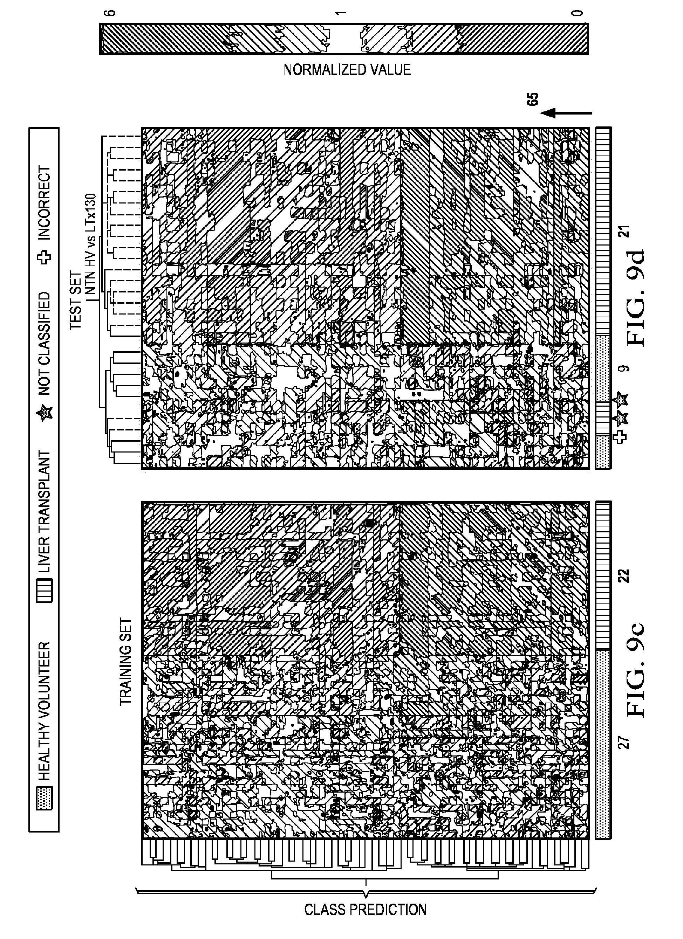

FIGURES 9A-9D are images of the hierarchical clustering of genes for the identification of a blood leukocyte transcriptional signature in transplant recipients under immunosuppressive drug therapy. Samples were divided into a training set (27 healthy, 22 patients) used to identify differentially expressed genes in liver transplant recipients versus healthy volunteers as seen in FIGURE 9A and FIGURE 9C respectively. A test set of nine healthy and 21 patients were used to independently validate this signature as seen in FIGURE 9B and FIGURE 9D. Class comparison identified 2,589 differentially expressed genes (Mann-Whitney test p < 0.01, fold change > 1.25). FIGURE 9A illustrates a similar signature in the test set and FIGURE 9B illustrates the class prediction that identified 81 genes. FIGURE 9C shows that the discrimination of the independent test set with 90% accuracy. FIGURE 9D illustrates the class could not be identified for two samples out of 30; one sample was incorrectly predicted (i.e. transplant recipient classified as healthy).

The same analysis strategy was applied to Liver transplant patients. Statistical comparison of a group of 22 transplant recipients versus 27 healthy volunteers identified 2,589 differentially expressed genes (p<0.01, non-parametric Mann-Whitney rank test and >1.25 fold change; 938 overexpressed and 1651 underexpressed genes; Table 10). Table 10 illustrates genes that are expressed differentially in liver transplant recipients under treatment with immunosuppressive drugs in comparison to healthy volunteers. Hierarchical clustering of genes produced a reciprocal expression pattern that was observed in both training as seen in FIGURE 9A and independent test sets as seen in FIGURE 9B. Sixty-five classifier genes were established in the training set and are illustrates in FIGURE 9C and Table 11. Table 11 illustrates the expression level of a set of transcripts discriminating liver transplant recipients under treatment with immunosuppressive drugs from healthy volunteers. The sixty-five classifier genes were applied to an independent test set of 9 healthy donors and 21 patients. Samples were correctly classified 90% of the time: class could not be determined in two cases and one sample was misclassified as seen in FIGURE 9D. The results obtained for both of these sets were highly correlated, e.g., pearson correlation, r²=0.94, p<0.0001, as seen in FIGURE 8B. Thus, the blood leukocyte transcriptional signatures associated with patients with metastatic melanoma and in liver transplant recipients have been identified and validated.

Module-level analysis of patients PBMC transcriptional profiles was performed. A custom microarray data mining strategy was used to further characterize disease-associated gene expression patterns. The analysis of a set of 239 blood leukocytes transcriptional signatures identified 28 transcriptional modules regrouping 4,742 probe sets. These "transcriptional modules" are sets of genes that follow similar expression patterns across a large number of samples in multiple studies, identified through co-expression meta-analysis. Each module is associated with a unique identifier that indicates the round of selection and order (e.g., M2.8 designates the eighth module of the second round of selection). Upon extraction each transcriptional module is functionally characterized with the help of a literature profiling algorithm (Chaussabel and Sher, 2002).

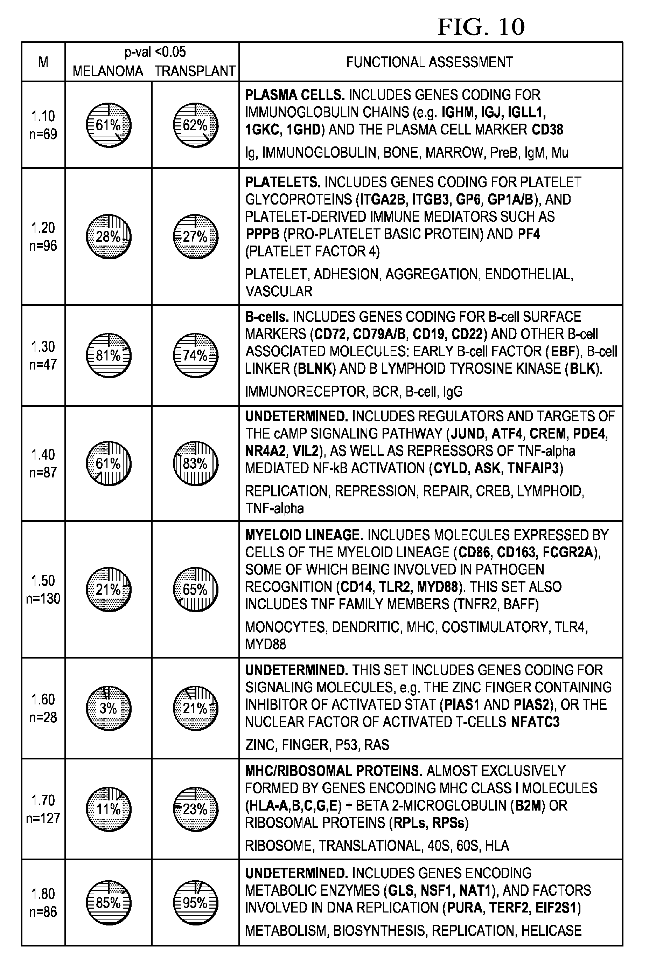

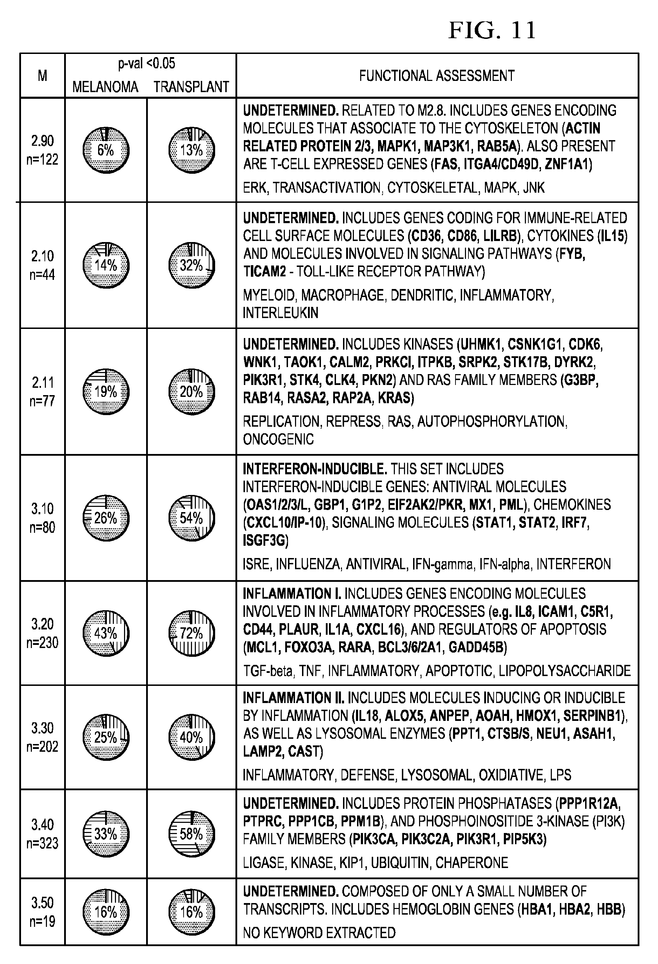

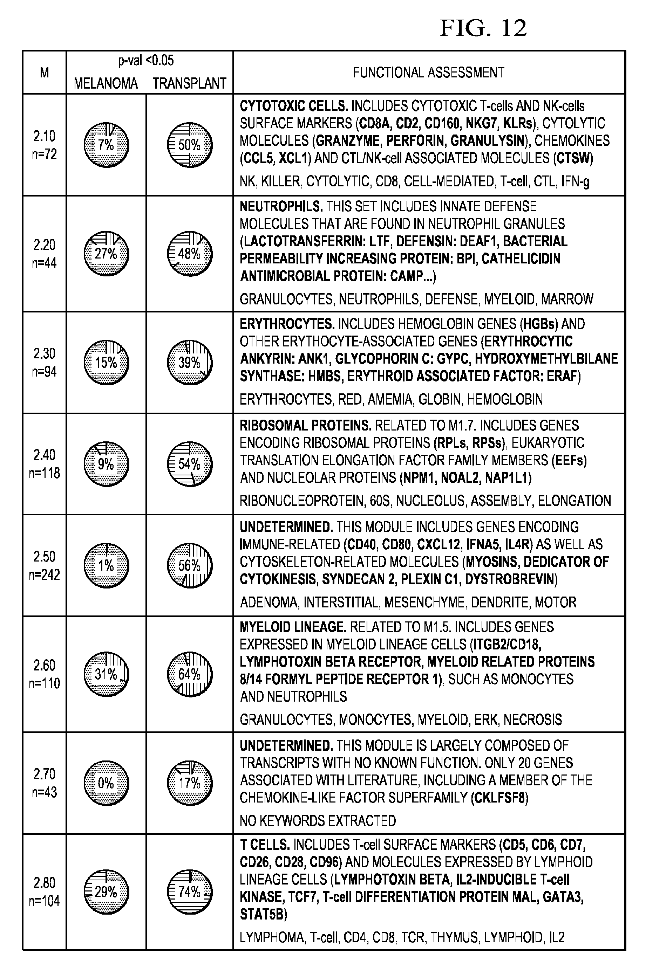

FIGURES 10 to 13 illustrate detailed statistical comparisons between healthy and disease groups of the module-level analysis. For example, twenty-eight sets of coordinately expressed genes, or transcriptional modules, were identified through the analysis of 239 PBMC microarray profiles. For each of these modules changes in expression levels between groups of healthy volunteers and either patients with metastatic melanoma or transplant recipients were tested. A pie chart indicates the proportion of genes that were significantly changed in each module, where red indicates overexpressed genes and blue illustrates underexpressed genes, with a p<0.05 for the Mann-Whitney test. For each module, keywords extracted from the literature are listed in green along with a functional assessment of relationships existing among the genes.

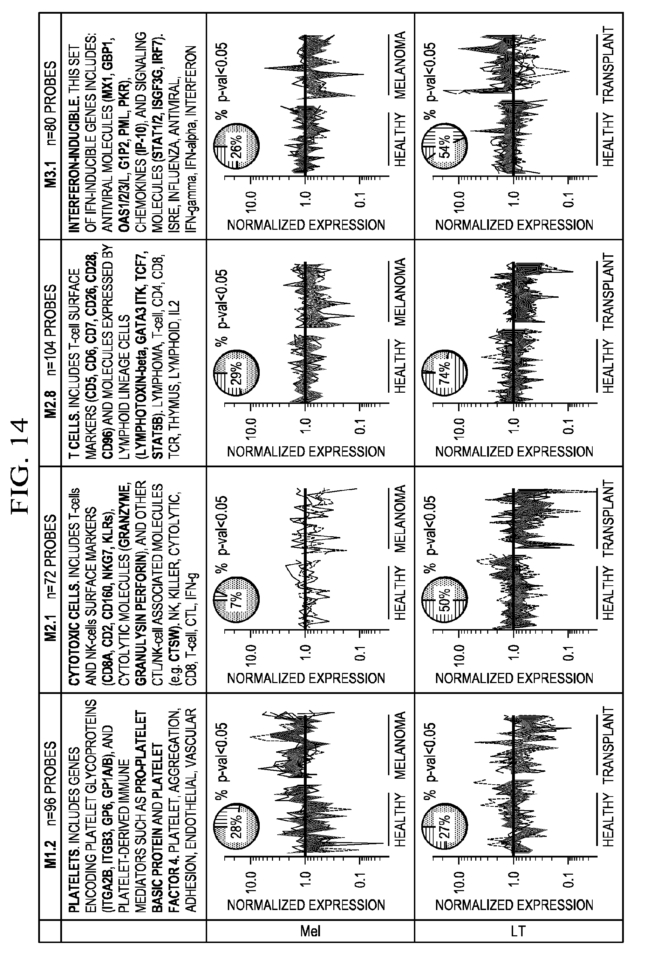

FIGURE 14 is a plot of the changes observed in a few representative modules represented by a transcriptional profile. Each differentially expressed gene is represented by a line that indicates relative levels of expression across healthy volunteer and patient samples. Peaks and dips respectively indicate relatively higher and lower gene expression in a given patient. Genes that were not significantly different are not represented. The levels of expression of genes associated with a platelet signature changed in opposite directions: 28% of the genes forming this signature (M1.2) were overexpressed in patients with melanoma and 27% were underexpressed in transplant recipients. Furthermore, half of the genes belonging to module M2.1 (cytotoxic cell signature) were underexpressed in transplant recipients.

This trend was not observed in patients with melanoma (7% overexpressed with p<0.05 where 5% of changes are expected by chance only). Similarly, a massive down-regulation of genes associated to T cells was observed in transplant recipients (74% of genes in M2.8). This finding most likely reflects pharmacological immunosuppression. In patients with melanoma 29% of these T-cell related genes were down-regulated. In addition, 44% of interferon-inducible genes that form module M3.1 were overexpressed in transplant recipients, while 26% were underexpressed in patients with melanoma. Lists of differentially expressed genes in each module are available in Tables 12 and 13.

Patients with metastatic melanoma and transplant recipients display common transcriptional profiles at the modular level. This analysis identified similarities as well as differences in blood leukocyte transcriptional signatures of patients with metastatic melanoma and liver transplant recipients.

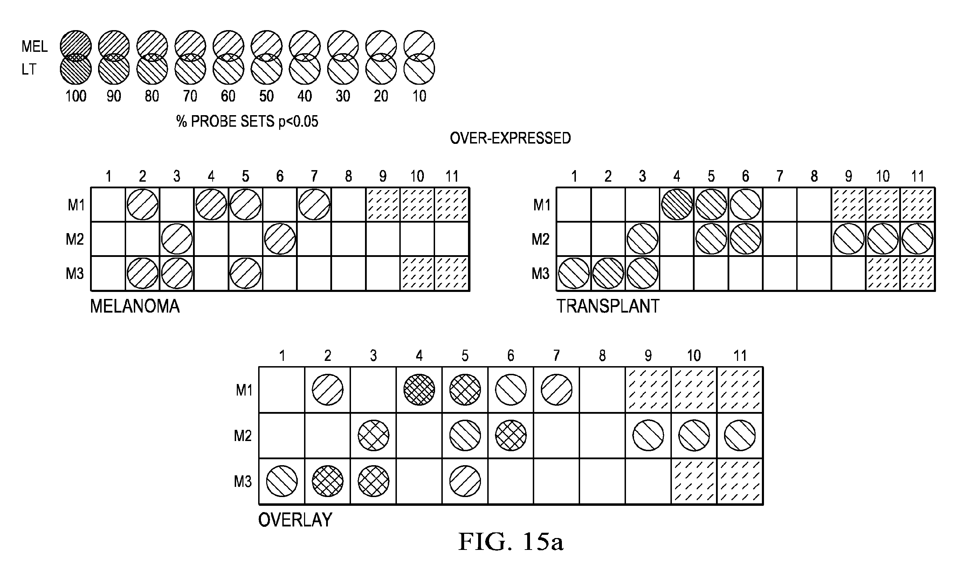

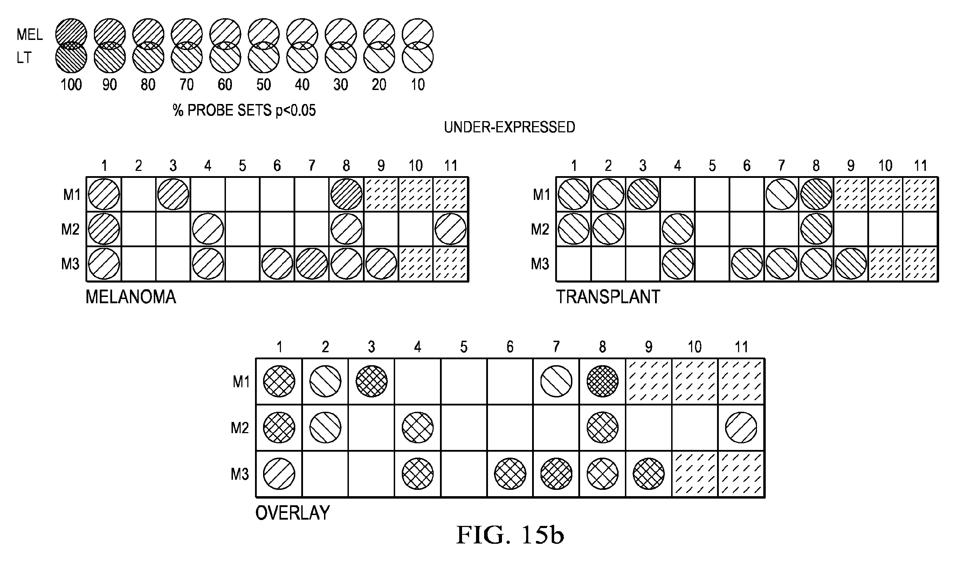

FIGURE 15 is an image of the modular changes observed in both groups of patients vs. their respective healthy control group. The proportion of differentially expressed genes for each module is indicated by a spot of variable intensity. For example, in the overlay a change in transplant is represented by a yellow, while a change in melanoma is indicated by a blue color and a change in both is indicated by a green color. Proportions of underexpressed and overexpressed transcripts are represented on separate grids. Modules that were common between the two groups of patients include M1.4 (regulator of cAMP and NF-kB signaling pathways), M2.6 (including genes expressed in myeloid lineage cells), M3.2 and M3.3 (both M3.2 and 3.3 include factors involved in inflammation; as seen in FIGURES 10-13).

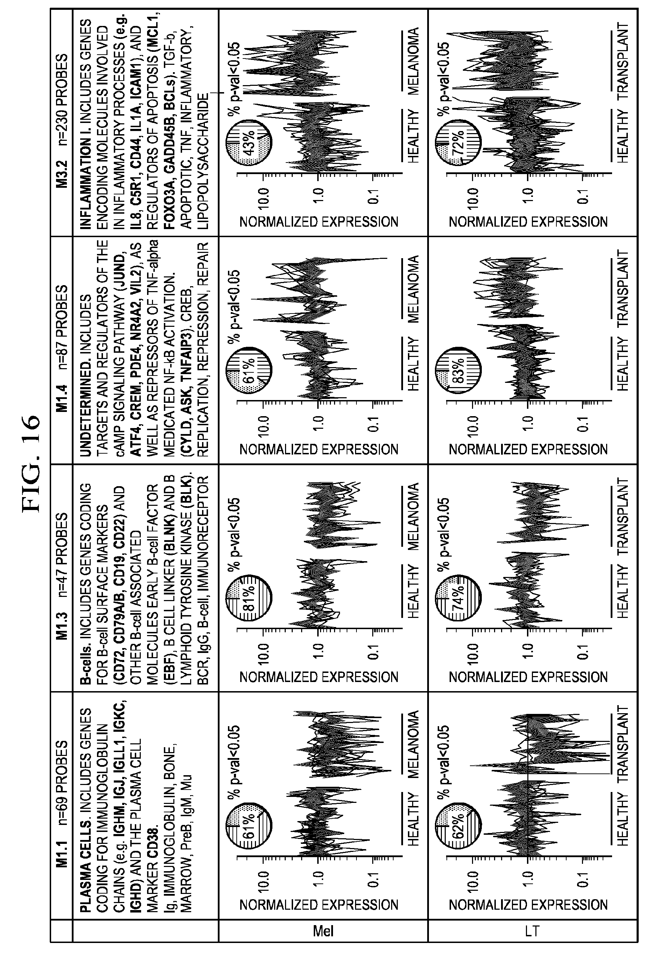

FIGURE 16 is an image illustrating the module-level analysis of the present invention. Common transcriptional signatures in blood from patients with metastatic melanoma and from liver transplant recipients. Expression profiles of genes belonging to blood leukocyte transcriptional modules M1.1, M1.3, M1.4 and M3.2. The total number of probes is indicated for each module (U133A), along with a brief functional interpretation. Keywords extracted by literature profiling are indicated in green. From the total number in each module, the proportion of genes that were significantly changed (Mann-Whitney test, p<0.05) in patients compared to the appropriate healthy control group is indicated in a pie chart with overexpressed genes are expressed in red and underexpressed genes are expressed in blue. Graphs represent transcriptional profiles of the genes that were significantly changed, with each line showing levels of expression on the y-axis of a single transcript across multiple conditions (samples, x-axis).

The association between metastatic melanoma and liver transplant phenotype was strongest for M1.4 and M3.2 as seen in FIGURE 16. Interestingly, a majority of underexpressed modules were common to melanoma and transplant groups, with the most striking similarities in the case of M1.1 (including plasma cell associated genes), M1.3 (including B-cell associated genes) and M1.8 (including genes coding for metabolic enzymes and factors involved in DNA replication).

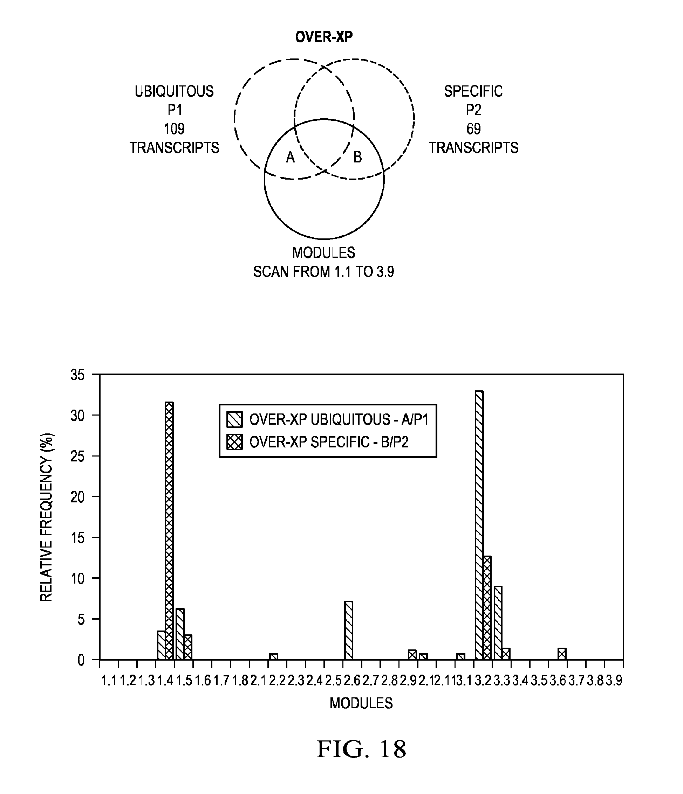

Identification of a common transcriptional signature that is unique to metastatic melanoma and liver transplant patients. The extent of the similarities between patients with metastatic melanoma and liver transplant recipients were specific to these two groups of patients were examined. Statistical group comparison was carried out between patients and healthy controls across all samples (e.g., thirty-eight melanoma, forty-three transplant, thirty-six healthy). Briefly, 323 transcripts were identified that were significantly overexpressed and 918 that were significantly underexpressed in both liver transplant recipients and patients with metastatic melanoma (Mann-Whitney test, p<0.01, filtered >1.25 fold change). Next, group comparisons for these transcripts were carried using samples from patients with Systemic Lupus Erythematosus ("SLE"), acute infections *(S. pneumoniae, S. aureus, E. coli,* and Influenza A) and Graft versus Host Disease ("GVHD") compared to relevant healthy controls. This analysis yielded p-values whose hierarchical clustering identified distinct significance patterns among transcripts common to the melanoma and transplant groups. This analysis identified sets of genes that changed across all diseases as seen in FIGURE 17 with P1 being ubiquitously overexpressed; P3 being ubiquitously underexpressed, while others were associated more specifically with the melanoma and transplant groups as seen in FIGURE 18 with P2 being overexpressed; and P4 being underexpressed. Table 14 illustrates the significance levels across 8 diseases of the genes forming patterns P1, P2, P3 and P4.

FIGURE 17 is an image of the analysis of significance patterns. Genes expressed at higher levels in both stage IV melanoma or liver transplant patients compared to healthy volunteers were selected. P-values were similarly obtained from gene expression profiles generated in other disease models: in PBMCs obtained from patients suffering from systemic lupus erythematosus (SLE), Graft versus Host Disease (GVHD), or acute infections with influenza virus (Influenza A), *Escherichia coli (E. coli), Streptococcus pneumoniae (Strep. Pneumo.)* or *Staphylococcus aureus (Staph. aureus).* Each of these cohorts was compared to the appropriate control group of healthy volunteers accrued in the context of these studies. The genes expressed at significantly higher or lower levels in PBMCs obtained from both patients with melanoma and liver transplant recipients (OVER-XP and UNDER-XP, respectively) were ranked by hierarchical clustering of p-values generated for all the conditions listed above. P-values are represented according to a color scale: Green represents low p-value/significant, while white represents high p-value/not significant. Distinct significant patterns are identified, where P1 and P3 are ubiquitous and P2 and P4 are most specific to melanoma and liver transplant groups.

FIGURE 18 is a chart of the modular distribution of ubiquitous and specific gene signatures common to melanoma and transplant groups. Distribution among 28 PBMC transcriptional modules was determined for genes that form ubiquitous (P1) and specific (P2) transcriptional signatures common to the melanoma and transplant groups. Gene lists of each of the modules were compared in turn to the 109 and 69 transcripts that form P1 and P2. For each module, the proportion of genes shared with either P1 or P2 was recorded. These results are represented by a bar graph of FIGURE 18.

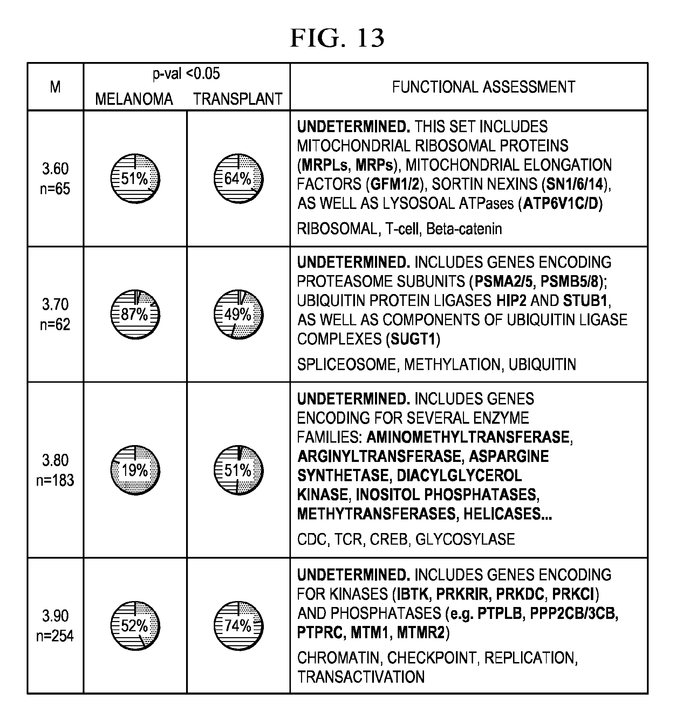

Thus, genes forming transcriptional signatures common to the melanoma and transplant groups can be partitioned into distinct sets based on two properties: (1) coordinated expression as seen in the transcriptional modules of FIGURE 13; and (2) change in expression across diseases as seen in the significance patterns of FIGURE 17. The results from these two different mining strategies were recouped by examining the modular distribution of ubiquitous (P1) and specific (P2) PBMC transcriptional signatures. FIGURE 18 clearly shows that the distribution of P1 and P2 across the 28 PBMC transcriptional modules that have been identified to date is not random. Indeed, P1 transcripts are preferentially found among M3.2 (characterized by transcripts related to inflammation), whereas M1.4 transcripts almost exclusively belonged to P2, which includes genes that are more specifically overexpressed in patients with melanoma and liver transplant recipients.

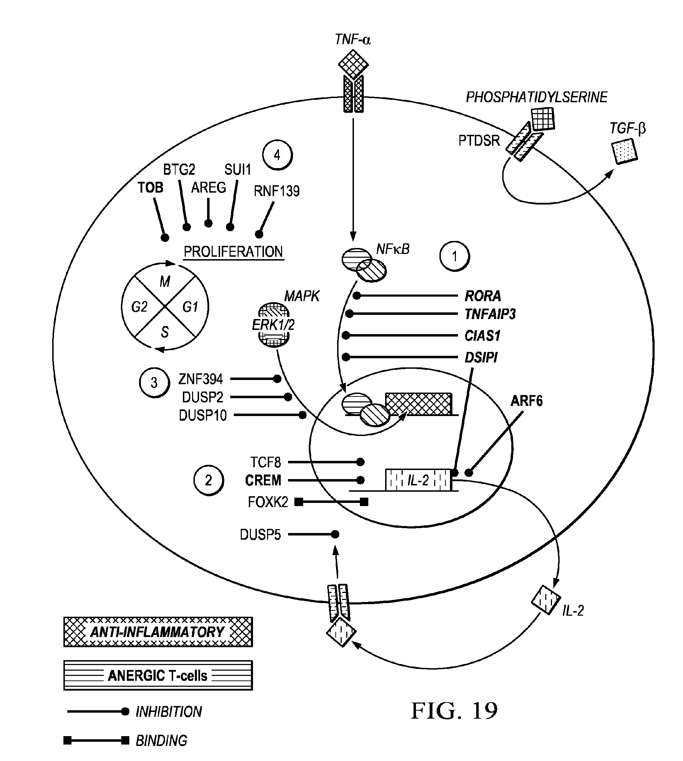

FIGURE 19 is an illustration of the transcriptional signature of immunosuppression. Transcripts overexpressed most specifically in patients with melanoma and transplant recipients (P1) include repressors of immune responses that inhibit: 1) NF-kB translocation; 2) Interleukin 2 production and signaling; 3) MAPK pathways and 4) cell proliferation. Some of these factors are well characterized anti-inflammatory molecules and others are expressed in anergic T-cells.

Molecular signature of immunosuppression. The genes that were most specifically overexpressed in melanoma and transplant groups (P1) were examined. From the 69 probe sets, 55 unique gene identifiers were identified. A query against a literature database indexed by gene, have developed to aid the interpretation of microarray gene expression data, identified 6527 publications associated with 47 genes, 30 of which were associated with more than ten publications. FIGURE 19 illustrates a remarkable functional convergence among the genes forming this signature and includes genes encoding molecules that possess immunoregulatory functions (e.g., anti-proliferative genes: BTG2, TOB1, AREG, SUI1 or RNF139; anti-inflammatory genes: TNFAIP3); inhibitors of transcription: (SON, ZC3HAV1, ZNF394); stress-induced molecules (HERPUD1); while others possess well established immunosuppressive properties. For example, dual specificity phosphatases 2, 5 and 10 (DUSP2, 5, 10) interfere with the MAP kinases ERK1/2, which are known targets of calcineurin inhibitors such as Tacrolimus/FK506. DUSP10 selectively dephosphorylates stress activated kinases(Theodosiou et al., 1999). Interestingly, DUSP5 was found to have a negative feedback role in IL2 signaling in T-cells(Kovanen et al., 2003). CREM, FOXK2 and TCF8 directly bind the IL2 promoter and can contribute to the repression of IL-2 production in T cell anergy(Powell et al., 1999). BHLHB2 (Stra13) negatively regulates lymphocyte development and function in vivo(Seimiya et al., 2004). CIAS1 codes for the protein Cryopyrin, which regulates NF-kappa B activation and production of proinflammatory cytokines. Mutations of this gene have been identified in several inflammatory disorders(Agostini et al., 2004). DSIPI, a leucine zipper protein, is known to mediate the immunosuppressive effects of glucocorticoids and IL10 by interfering with a broad range of signaling pathways (NF-kappa B, NFAT/AP-1, MEK, ERK 1/2), leading to the general inhibition of inflammatory responses in macrophages and down-regulation of the IL2 receptor in T cells. Noatably, the expression of DSIPI in immune cells was found to be augmented after drug treatment (dexamethasone)(D'Adamio et al., 1997) or long term exposure to tumor cells (Burkitt Lymphoma)(Berrebi et al., 2003).

Other immunosuppressive molecules, which did not belong to P1, were also found overexpressed in melanoma and transplant groups. Notably, DDIT4, another dexamethasone-induced gene which was recently found to inhibit mTOR, the mammalian target for rapamycin (Corradetti et al., 2005). Thus, this endogenous factor appears capable of reproducing the action of potent immunosuppressive drugs. HMOX1, a cytoprotective molecule that also demonstrates anti-inflammatory properties. Most recently, HMOX1 expression was found to be induced by FOXP3 and to mediate immunosuppressive effects of CD4+ CD25+ regulatory T cells (Choi et al., 2005). Accordingly, an increase in transcriptional activity of HMOX1 has been correlated with favorable outcomes in experimental transplant models (Soares et al., 1998). Both DDIT4 and HMOX1 genes were also overexpressed in patients with acute *E. coli* or *S. aureus* infections. The immunophilin FKBP1A (FKBP12), a member of the FK506-binding protein family, is a key mediator of T-cell immunosuppression by the drugs FK506 (tacrolimus) and rapamycin (Xu et al., 2002). Expression of this gene was elevated in comparison to healthy donors in all patient groups.

Blood is an accessible tissue and lends itself to comparative analyses across multiple diseases. Parmacological and tumor-mediated immunosuppression would produce a common transcriptional signature in blood leukocytes. Metastatic melanoma and transplant recipients disease-associated transcriptional signatures were identified in the blood of patients. These signatures were identified and confirmed through several analytical approaches. Analysis of transcriptional modules identified alterations in blood leukocytes transcriptional components associated to cell types (e.g., Plasma cells, B-cells, T-cells, Cytotoxic cells) and to immune reactions (e.g., Inflammation, Interferon). Furthermore, using both transcriptional modules and gene expression levels similarities between blood transcriptional signatures in patients with metastatic melanoma and liver transplant recipients were identified. However, this common transcriptional signature could not be entirely attributed to immunosuppression. For instance, expression levels of B-cell associated genes (M1.3) were not only decreased in the melanoma and transplant groups, but also in patients with acute influenza infection and systemic lupus erythematosus (SLE) (53% of genes were underexpressed, in comparison to healthy controls; Chaussabel et al.). Conversely, nearly 40% of the genes associated with plasma cells (M1.1) were overexpressed in SLE patients and there was no change in patients with acute influenza infection (7% of the genes were overexpressed at p<0.05), whereas expression levels were significantly decreased in both patients with melanoma and transplant recipients (61 % and 62% of the genes in M1.1, respectively). In order to select the most specific transcripts common between melanoma and transplant signatures a gene-level analysis was carried out across a total of eight groups of patients. This led to the identification of a set of transcripts that was most specifically overexpressed in immunosuppressed patients. The identified set of genes showed marked functional convergence and included genes coding for repressors of Interleukin-2 transcription, inhibitors of NF-kB or MAPK pathways, and anti-proliferative molecules. Interestingly, these signatures are consistent with the mechanism of action of drugs used for pharmacological immunosuppression, which inhibit the activity of calcineurin, a calcium-dependent serine threonine protein phosphatase responsible for the nuclear translocation of NF-AT and NF-kappaB upon T-cell activation. Indicating a functional convergence between immunosuppressive mechanisms operating in patients with advanced melanoma and pharmacologically treated transplant recipients. The fact that the transcripts more specifically induced in immunosuppressed patients include glucocorticoids-inducible genes (e.g., DSIPI, CXCR4, JUN) and hormone nuclear receptors (NR4A2 and RORA)(Winoto and Littman, 2002) suggest a possible role for steroid hormones in tumor-mediated immunosuppression.

Patients with metastatic melanoma display an endogenous transcriptional signature of immunosuppression similar to that induced by pharmacological treatments in patients who underwent liver transplant. The present invention provides a method and apparatus to identify patients at high risk of melanoma progression. In addition the present invention also provides a method and apparatus for monitoring indicators of immunosuppression could help adjusting the dosage of immunosuppressive drugs and balance risks of rejection and side effects for liver transplant recipients.

Examples of patient information and processing of blood samples include the following. Blood was obtained after informed consent as approved by the institutional IRB (Liver transplant recipients: 002-1570199-017; patients with melanoma: 000-048, 002-094; 003-187). Blood samples were obtained in acid citrate dextrose yellow-top tubes (BD Vaccutainer) at the Baylor University Medical Center in Dallas, TX. Samples were immediately delivered at room temperature to the Baylor Institute for Immunology Research, Dallas, TX, for processing. Fresh PBMCs isolated via Ficoll gradient were either stored in liquid nitrogen (e.g., viable freezing) or immediately lysed in RLT buffer, containing ß-mercaptoethanol (Qiagen, Valencia, CA). Total RNA was extracted from cells previously frozen in liquid nitrogen ("frozen") or from cells that were lysed immediately after isolation ("fresh"), using the RNEASY^{®} Mini Kit according to the manufacturer's recommended protocol (Qiagen, Valencia, CA). This parameter was taken into account in the experimental design taking into account the age, gender and sample processing method used for the identification (training) and validation (testing) of expression signatures associated with metastatic melanoma and liver transplant recipients undergoing immunosuppressive drug therapy.

Microarray assays. Total RNA was isolated using the RNEASY^{®} kit (Qiagen, Valencia, CA) according to the manufacturer's instructions and the RNA integrity was assessed using an Agilent 2100 Bioanalyzer (Agilent, Palo Alto, CA). Although, the skilled artisan will recognize that other methods of isolation may be used. From 2-5 micrograms of total RNA, double-stranded cDNA containing the T7-dT (24) promoter sequence (Operon Biotechnologies, Huntsville, AL) was generated. This cDNA was then used as a template for in vitro transcription single round amplification with biotin labels (Enzo BioArray HighYield RNA Transcript Labeling Kit from Affymetrix Inc, Santa Clara, CA). Biotinylated cRNA targets were purified using the Sample Cleanup Module and subsequently hybridized to human U133A GeneChips (Affymetrix Inc, Santa Clara, CA) according to the manufacturer's standard protocols. Affymetrix U133A GeneChips that contain 22,283 probe sets, represented by ten to twenty unique probe pairs (perfect match and its corresponding mismatch), which allow detection of 14,500 different genes and expressed sequence tags (ESTs). Arrays were scanned using a laser confocal scanner (Agilent). The samples were processed by the same team, at the same core facility, and were randomized between each array run. Raw data are deposited with GEO (www.ncbi.nlm.nih.gov/geo/).

Data analysis. For each Affymetrix U133A GENE CHIP^{®} raw intensity data were normalized to the mean intensity of all measurements on that array and scaled to a target intensity value of 500 (TGT) in Affymetrix Microarray Suite 5.0. With the aid of GeneSpring software, version 7.2, the measurement for each gene per patient sample array was divided by the median of that gene's measurement from the cohort of healthy volunteers. A filter was applied based on Affymetrix flag calls: probe sets were selected if "Present" in at least 75% of samples in either group (healthy controls or patients). This step insured a more reliable intensity measurement of the genes used in downstream analyses. Class comparison was performed using a non-parametric ranking statistical analysis test (Mann-Whitney) applied to the selected set of genes. In the vertical direction, hierarchical clusters of genes were generated using the Pearson correlation around zero, Genespring's standard correlation measure. Normalized gene expression data were examined with a nonparametric univariate analysis (Fisher's exact test) to identify genes potentially discriminating two different groups. A supervised learning algorithm, the K-Nearest Neighbors Method, was applied that assigned a sample to pre-defined classes in three steps: 1) identification of genes (observations) that have strong correlations to classes to be distinguished; 2) confirmation that identified genes distinguish pre-defined classes; and 3) validation with "unknown samples".

Identification of transcriptional modules. A total of 239 blood leukocyte gene expression profiles were generated using Affymetrix U133A&B GENECHIPS (>44K probe sets). Transcriptional data were obtained for 8 groups including Systemic Juvenile Idiopathic Arthritis, SLE, liver transplant recipients, melanoma patients, and patients with acute infections: *Escherichia coli, Staphylococcus aureus* and Influenza A. For each group, transcripts that were present in at least 50% of all conditions were segregated into 30 clusters (k-means clustering: clusters C1 through C30). The cluster assignment for each gene was recorded in a table and distribution patterns were compared among all the genes. Modules were selected using an iterative process, starting with the largest set of genes that belonged to the same cluster in all study groups (i.e. genes that were found in the same cluster in 8 of the 8 groups). The selection was then expanded from this core reference pattern to include genes with 7/8, 6/8 and 5/8 matches. The resulting set of genes formed a transcriptional module and was withdrawn from the selection pool. The process was then repeated starting with the second largest group of genes, progressively reducing the level of stringency. This analysis led to the identification of 4742 transcripts that were distributed among 28 modules. Each module is attributed a unique identifier indicating the round and order of selection (e.g., M3.1 was the first module identified in the third round of selection).

Analysis of significance patterns. Gene expression data were generated for PBMCs obtained from patients and healthy volunteers using Affymetrix HG-U133A GENECHIPS. P values were obtained for six reference datasets by comparing groups of patients to their respective healthy control groups (Mann-Whitney rank test). The groups were composed of patients with: 1) Systemic Lupus Erythematosus (SLE, 16 samples), 2) Influenza A (16 samples), 3) *Escherichia coli* (16 samples), 4) *Staphylococcus aureus* (16 samples), and 5) *Streptococcus pneumoniae* (14 samples); and 7) bone marrow transplant recipients undergoing graft versus host disease (GVHD, 12 samples). Control groups were also formed taking into account age, sex and project (10 samples in each group). Genes significantly changed (p<0.01) in the "study group" (Melanoma and Transplant) were divided in two sets: overexpressed versus control and underexpressed versus control. P-values of the genes forming the overexpressed set were obtained for the "reference groups" (SLE, GVHD and infections with influenza virus, *E. coli, S. aureus, S. pneumoniae).* P-value data were processed with a gene expression analysis software program, GeneSpring, Version 7.2 (Agilent), which was used to perform hierarchical clustering and group genes based on significance patterns.

Example 2. Determination and Analysis of Patterns of Significance are used to identify ubiquitous and disease-specific gene expression signatures in patient peripheral blood leukocytes.

The use of gene expression microarrays in patient-based research creates new prospects for the discovery of diagnostic biomarkers and the identification of genes or pathways linked to pathogenesis. Gene expression signatures were generated from peripheral blood mononuclear cells isolated from over one hundred patients with conditions presenting a strong immunological component (patient with autoimmune, graft versus host and infectious diseases, as well as immunosuppressed transplant recipients). This dataset permitted the opportunity to carry out comparative analyses and define disease signatures in a broader context. It was found that nearly 20% of overlap between lists of genes significantly changed versus healthy controls in patients with Systemic Lupus Erythematosus (SLE) and acute influenza infection. Transcriptional changes of 22,283 probe sets were evaluated through statistical group comparison performed systematically for 7 diseases versus their respective healthy control groups. Patterns of significance were generated by hierarchical clustering of p-values. This "Patterns of Significance" approach led to the identification of a SLE-specific "diagnostic signature", formed by genes that did not change compared to healthy in the other 6 diseases. Conversely, "sentinel signatures" were characterized that were common to all 7 diseases. These findings allow for the use of blood leukocyte expression signatures for diagnostic and early disease detection.

Briefly, blood is a reservoir and migration compartment for immune cells exposed to infectious agents, allergens, tumors, transplants or autoimmune reactions. Leukocytes isolated from the peripheral blood of patients constitute an accessible source of clinically-relevant information and a comprehensive molecular phenotype of these cells can be obtained by microarray analysis. Gene expression microarrays have been extensively used in cancer research, and proof of principle studies analyzing Peripheral Blood Mononuclear Cell (PBMC) samples isolated from patients with Systemic Lupus Erythematosus (SLE) lead to a better understanding of mechanisms of disease onset and responses to treatment. Two main applications have been found for gene expression microarrays in the context of patient-based research: (1) the discovery of biomarkers and establishment of diagnosis / prognosis signatures (e.g. prediction of survival of breast cancer patients) (2) the identification of genes/pathways involved in pathogenesis, leading for instance to the discovery of the role of interleukin-I in the pathogenesis of systemic onset juvenile idiopathic arthritis. However, the analysis of microarray data still constitutes a considerable challenge. The ability to simultaneously acquire data for tens of thousands of features in a single test is one of the most appealing characteristic of microarrays, but it can also be a major shortcoming7. This 'curse of dimensionality' is compounded by the fact that the numbers of samples analyzed is usually small. The imbalance between the numbers of genes and conditions analyzed considerably weakens data interpretation capabilities. A microarray gene expression database was created that constitutes samples obtained from patients with diseases that possess a strong immune component. The meta-analysis strategy of the present invention allows for the identification of ubiquitous as well as disease-specific signatures.

Processing of Blood Samples. Blood samples were collected by venipuncture and immediately delivered at room temperature to the Baylor Institute for Immunology Research, Dallas, TX, for processing. Peripheral blood mononuclear cells (PBMCs) from 3-4 ml of blood were isolated via Ficoll gradient and immediately lysed in RLT reagent (Qiagen, Valencia, CA) with beta-mercaptoethanol (BME) and stored at -80°C prior to the RNA extraction step.

Microarray analysis. Total RNA was isolated using the RNeasy kit (Qiagen, Valencia, CA) according to the manufacturer's instructions and RNA integrity was assessed by using an Agilent 2100 Bioanalyzer (Agilent, Palo Alto, CA). Target labeling was performed according to the manufacturer's standard protocol (Affymetrix Inc, Santa Clara, CA). Biotinylated cRNA targets were purified and subsequently hybridized to Affymetrix HG-U133A GeneChips (22,283 probe sets). Arrays were scanned using an Affymetrix confocal laser scanner. Microarray Suite, Version 5.0 (MAS 5.0; Affymetrix) software was used to assess fluorescent hybridization signals, to normalize signals, and to evaluate signal detection calls. Normalization of signal values per chip was achieved using the MAS 5.0 global method of scaling to the target intensity value of 500 per GeneChip. A gene expression analysis software program, GeneSpring, Version 7.1 (Agilent), was used to perform statistical analysis, hierarchical clustering and classification of samples.

Development and Analysis of Patterns of Significance. Gene expression data were generated for PBMCs obtained from patients and healthy volunteers using Affymetrix HG-U133A GeneChips that were run on the same Affymetrix system, using standard operating procedures. P values were obtained by comparing 7 groups of patients to their respective healthy control groups (Mann-Whitney rank test). The groups were composed of pediatric patients with: 1) Systemic Lupus Erythomatosus (SLE, 16 samples), 2) Influenza A (16 samples), 3) *Staphylococcus aureus* (16 samples), 4) *Escherichia coli* (16 samples) and *5) Streptococcus pneumoniae* (14 samples); as well as adult transplant recipients: 6) Liver transplant patients that have accepted the graft under immunosuppressive therapy (16 samples) and 7) bone marrow transplant recipients undergoing graft versus host disease (GVHD, 12 samples). Control groups were also formed taking into account age, sex and project (10 samples in each group). Genes significantly changed (p<0.01) in the "study group" (Influenza A and/or SLE) were divided in two sets: overexpressed versus control and under-expressed versus control. P-values of the genes forming the overexpressed set were obtained for the "reference groups" (infections with *E. coli, S. aureus, S. pneumoniae,* Liver transplant recipients and graft versus host disease). P-values of the reference groups were set to 1 when genes were under-expressed. The same procedure was used in the set of genes under-expressed in study group, only this time P-values of the reference group were set to 1 when genes were overexpressed. P-value data were processed with a gene expression analysis software program, GeneSpring, Version 7.1 (Agilent), that was used to perform hierarchical clustering and group genes based on significance patterns.

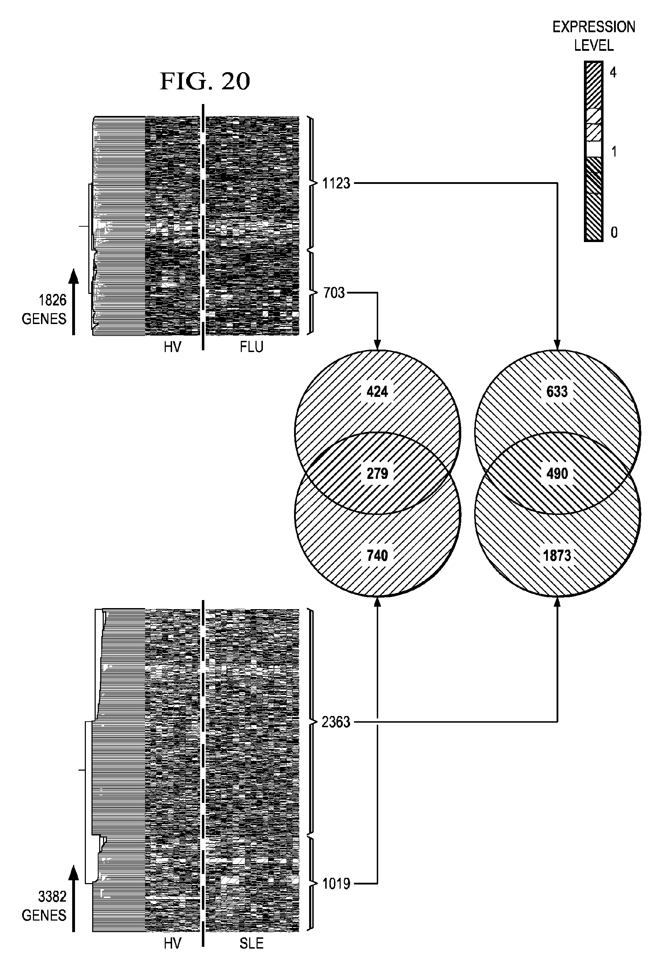

Identification of blood leukocytes transcriptional signatures associated with acute Influenza A infection and SLE. Microarray gene expression data obtained from pediatric patients with either SLE or acute influenza A infections were used to identify transcriptional signatures characteristic of these two diseases (Figure 20). Statistical comparison of a similar number of patients (18 samples) to their respective control group (10 samples) identified: (1) 1826 differentially expressed genes that formed the Influenza signature (of those 703 were over-expressed (red) relative to controls and 1123 were under-expressed (blue), see Figure (20A); 2) 3382 differentially expressed genes formed the SLE signature (of those 1019 were over-expressed relative to controls and 2363 were under-expressed, see Figure 20B).

Figure 20 shows a statistical group comparison between patients and their respective controls. Figure 20A. Microarray expression obtained for PBMC isolated from 16 children with acute Influenza A infection (FLU) and 10 healthy volunteers (HV) were compared (Mann-Whitney rank test, p<0.01). Out of 1826 differentially expressed genes, 703 were over-expressed and 1123 under-expressed in patients. Figure 20B. An equivalent number of children with Systemic Lupus Erythomatosus (SLE) were compared to their respective set of 10 healthy volunteers (HV) (Mann-Whitney rank test, p<0.01). Out of 3382 differentially expressed genes, 1019 were over-expressed and 2363 under-expressed in patients. Figure 20C. Comparison of over-expressed and under-expressed gene lists obtained for SLE and FLU samples relative to their respective control groups (healthy volunteers).

Transformed expression levels are indicated by color scale, with red representing relatively high expression and blue indicating relatively low expression compared to the median expression for each gene across all donors.

Analysis of significance patterns. Next, the specificity of these signatures for each disease was determined. A substantial overlap was found between the sets of genes that were differentially expressed in FLU and SLE (Figure 20C), with 279 over-expressed and 490 under-expressed genes common to both diseases (19% and 16% of similarities, respectively). This observation was used to determine whether a specific disease signature could be obtained in the context of a broader set of diseases.

In order to address this question the analysis was extended to PBMC transcriptional datasets obtained for patients with acute infections caused by bacteria (*E. coli, S. aureus and S. pneumoniae*) as well as transplant recipients (liver recipients who have accepted the allograft under pharmacological immunosuppressive therapy and bone marrow recipients with graft versus host disease). Patterns of significance were analyzed for genes that were specifically over-expressed in SLE compared to Influenza (Figure 1, 740 genes). This approach allowed the visualization of the significance of changes in levels of gene expression for each disease compared to its respective control group (age and sex matched healthy volunteers). Genes were arranged according to significance patterns by hierarchical clustering.

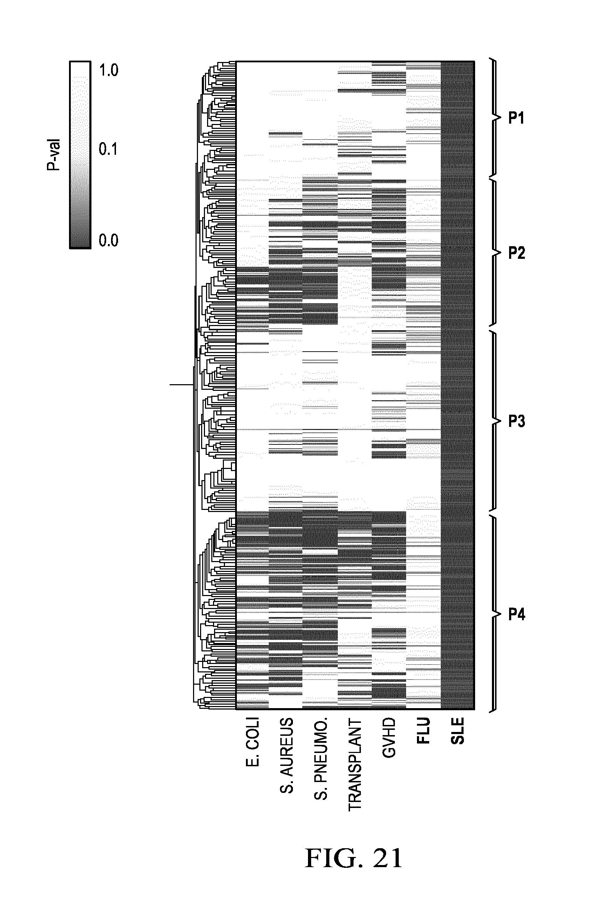

Of the 4 patterns identified, 2 were found to be largely specific to SLE (Figure 21: P1 - 98 genes, and P3 - 193 genes). In conclusion, the method was used to identify sets of genes, particularly among P3, that displayed a high degree of specificity for SLE when compared to 6 other diseases.

Figure 21 is an analysis of patterns of significance for genes over-expressed in SLE patients but not in patients with acute Influenza A infection. The genes used for this analysis were significantly overexpressed in patients with SLE compared to their respective control group (Mann-Whitney P<0.05) and not in patients with acute influenza A infection were selected for this analysis (740 genes). P values were obtained for five additional groups of patients: *E. coli, S. aureus, S. pneumoniae,* Liver transplant recipients and patients with graft vs host disease. The values were imported into a microarray data analysis software package (see methods for details). Four patterns were identified: SLE-1 to 4. Significance levels are indicated by color scale, with darker green representing lower P-values and white indicating a P-value of 1.

Identification of a common disease signature. An important proportion of genes from Figure 21 were induced ubiquitously (P2 - 222 genes and P4 - 225 genes). This finding suggests that these different diseases may share common transcriptional components in the blood constituting a "sickness" signature. In order to investigate this possibility sets of genes were analyzed that were shared between Influenza and SLE signatures (Figure 20C: 279 genes over-expressed, and 490 under-expressed).

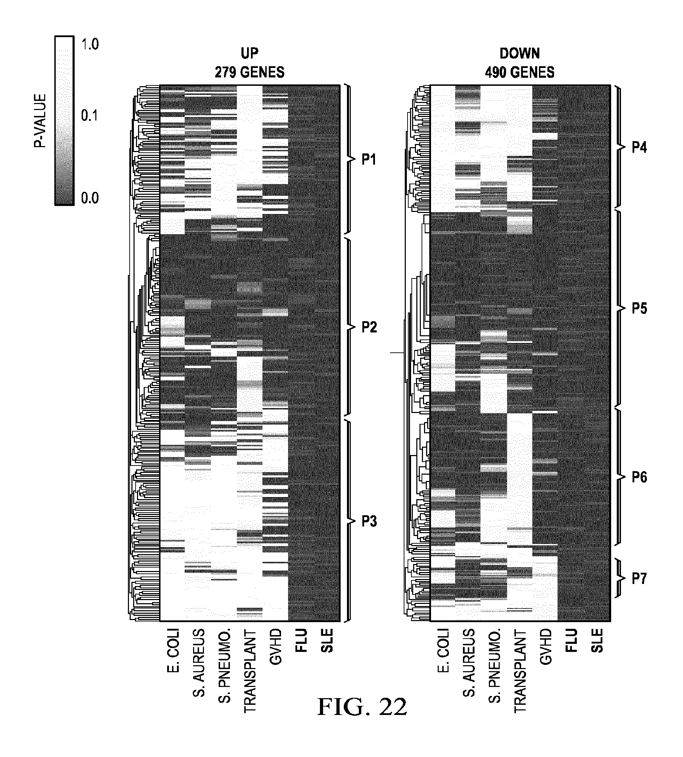

Figure 22 shows Patterns of Significance for genes common to Influenza A and SLE. Genes overexpressed (left panel, OVER) and underexpressed (right panel, UNDER) in both patients with Influenza A (FLU) and SLE were examined in the context of other diseases: acute infections with *E. coli, S. aureus, S. pneumoniae,* liver transplant recipients (transplant) and bone marrow recipients with graft versus host disease (GVHD). Significance levels are indicated by color scale, with dark green representing lower P-values and white indicating a P-value of 1.

Patterns of significance were generated for these genes across all 7 diseases as described above. Three subsets were identified among the genes that were over-expressed in patients with Influenza A infection and SLE: one changing in most diseases, another presenting significant differences in all diseases, while the third was more specific to Influenza and SLE (Figure 22A, respectively P1, P2 and P3). Equivalent patterns can be found upon analysis of a set of under-expressed genes common to Influenza and SLE (Figure 22B, P4-7). Interestingly, the group of patient with significance patterns that were the most similar to Influenza and SLE had Graft Versus Host Disease. The parallelism was particularly striking for the set of under-expressed genes (Figure 22B).

Functional analysis of significance patterns. Finally, functional annotations associated to the patterns identified on Figure 22 were extracted. Genes associated with "defense response" were preferentially found in two patterns (P2-3 on Figures 3 & 4; Fisher's test for over-representation of this functional category: p<0.0005). These genes were expressed at higher levels compared to healthy. The list includes Defensin alpha 3, Azurocidin 1, Stabilin 1 (P2); the tumor necrosis factor family member TRAIL, and Galectin 3 binding protein (P3). Conversely under-expressed genes belonging to patterns P4-6 were preferentially associated with "structural constituent of ribosome" (Fisher's test for over-representation of this functional category in P4-6: p<0.0001). These genes include multiple ribosomal protein family members (e.g. RPS10, RPL37, and RPL13). Genes belonging to the set of over-expressed genes the most specific to Influenza and SLE (P3) were preferentially associated to "interferon response" (p<0.0001, e.g. myxovirus resistance 1, interferon alpha-inducible protein 16, double stranded RNA inducible protein kinase), while genes in P1 were uniquely associated to "heavy metal binding" (p<0.0001, reflecting an overabundance of members of the metallothionein family).

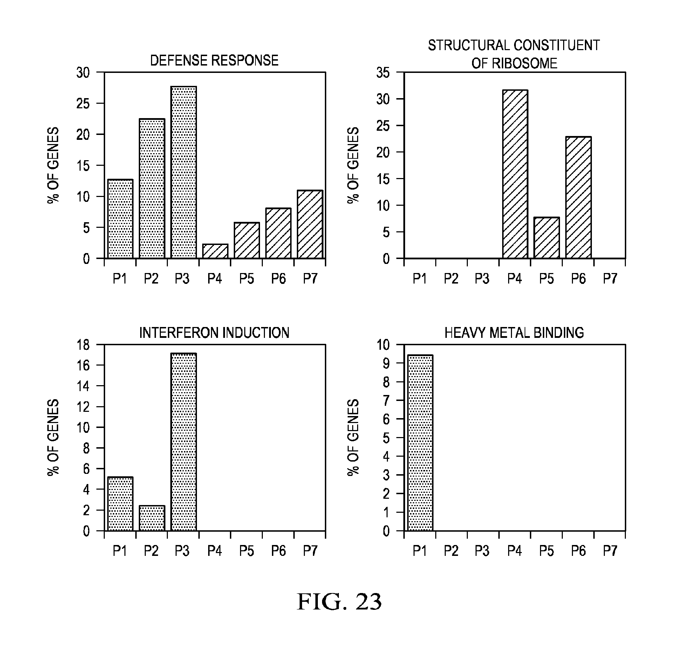

Figure 23 is a functional analysis of genes shared by patients with Influenza infection and Lupus grouped according to significance patterns. Sets of genes forming the different patterns indicated on Figure 22 (P1-7) were subjected to functional analyses. The histograms indicate the percentage of genes associated to a given annotation for each of the sets. Over-expressed genes = red, under-expressed = blue. P1 n= 71 genes; P2 n=118; P3 n=85; P4 n=117; P5 n=184; P6 n=120; P7 n=46.

The comparative analysis of PBMC transcriptional patterns identified disease-specific as well as ubiquitous expression signatures. Different degrees of disease specificity were observed among the genes found to be common between the transcriptional profiles of PBMCs obtained from patients with Influenza infection and SLE. Differences in significance patterns were translated into distinct functional associations. Indeed, the genes that were most specific to Influenza and SLE relative to 5 other diseases were the most strongly associated to biological themes such as: "Interferon induction" (over-expressed genes; Figures 22 and 23: P3) or "structural constituent of ribosome" (under-expressed genes; Figures 22 and 24: P4). These observations permit to validate the relevance of this approach. This analysis facilitates the interpretation of microarray data by placing disease signatures in a much broader context.

In addition to contributing to a better understanding of disease processes the meta-analysis of PBMC transcriptional datasets has important implications for clinical diagnostic with: (1) the identification of discriminatory disease-specific signatures; as the screening of tens of thousands of potential markers will in most cases permit to pinpoint a limited number of transcripts that uniquely characterize a disease; and (2) the identification of a sentinel signature; as sets of genes for which expression changes in a wide range of health disorders could potentially be used in a screening assay for early disease detection.

Patients with metastatic melanoma display an endogenous transcriptional signature of immunosuppression similar to that induced by pharmacological treatments in patients who underwent liver transplant. The present invention provides a method and apparatus to identify patients at high risk of melanoma progression. In addition, the present invention also provides a method and apparatus for monitoring indicators of immunosuppression could help adjusting the dosage of immunosuppressive drugs and balance risks of rejection and side effects for liver transplant recipients.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference.

**Table 10. Genes differentially expressed in liver transplant recipients under treatment with immunosuppressive drugs in comparison to healthy volunteers.**

| Liver transplant recipients (n=22) vs. Healthy Volunteer (n=27) - training set >1.25 fold up and down from Mann Whitney U test p-value<0.01 (2589), no mtc | | | | | | | | |
|---|---|---|---|---|---|---|---|---|
| | | **HV** | | **Liver Transplant** | | | | |
| **Systematic** | **P-value** | **Normalized** | **Raw** | **Normalized** | **Raw** | **Common** | **Genbank** | **Description** |
| 1598_g_at | 4,05E-05 | 0,9104603 | 560,89624 | 1,3507749 | 803,25 | GAS6 | L13720 | Growth arrest-specific 6 |
| 200036_s_at | 1,46E-06 | 1,0014551 | 15184,485 | 0,7471252 | 11178,514 | RPL10A | NM_007104 | Ribosomal protein L10a |
| 200047_s_at | 4,44E-06 | 1,0306468 | 3185,763 | 0,8108783 | 2518,3093 | YY1 | NM_003403 YY1 | transcription factor |
| 200049_at | 8,24E-08 | 1,0070481 | 590,15924 | 1,2933183 | 754,47266 | MYST2 | NM_007067 | MYST histone acetyltransferase 2 |
| 200050_at | 9,06E-12 | 1,0158027 | 1137,2852 | 0,55847067 | 636,85004 | ZNF146 | NM_007145 | Zinc finger protein 146 |
| 200056_s_at | 5,94E-07 | 1,0071732 | 569,50006 | 0,6961763 | 395,66818 | C1D | NM_006333 | Nuclear DNA-binding protein |
| 200069_at | 0,000131 | 0,90388525 | 549,29626 | 0,6566346 | 394,90005 | SART3 | NM_014706 | Squamous cell carcinoma antigen recognised by T cells 3 |
| 200078_s_at | 1,64E-07 | 0,9889188 | 2669,0852 | 1,343849 | 3612,4185 | ATP6V0B | BC005876 | ATPase, H+ transporting, lysosomal 21 kDa, V0 subunit c" |
| 200084_at | 4,82E-08 | 0,98041064 | 3502,4182 | 0,73534584 | 2639,082 | SMAP | BE748698 | Small acidic protein |
| 200090_at | 1,59E-05 | 0,9977675 | 3435,2668 | 0,757509 | 2657,055 | FNTA | BG168896 | Farnesyltransferase, CAAX box, alpha |
| 200595_s_at | 2,27E-05 | 1,0654379 | 2296,0447 | 0,80865556 | 1716,341 | EIF3S10 | NM_003750 | Eukaryotic translation initiation factor 3, subunit 10 theta, |
| 200603_at | 1,79E-05 | 1,0165917 | 3926,493 | 0,7501497 | 2950,6863 | PRKAR1A | AL050038 | Protein kinase, cAMP-dependent, regulatory, type I, alpha (tissue specific extinguisher 1) |
| 200608_s_at | 5,06E-05 | 0,9846552 | 1840,685 | 0,70391756 | 1309,3591 | RAD21 | NM_006265 | RAD21 homolog (S. pombe) |
| 200614_at | 4,02E-08 | 0,98103416 | 4044,474 | 0,7621779 | 3143,3728 | CLTC | NM_004859 | Clathrin, heavy polypeptide (Hc) |
| 200623_s_at | 0,00833 | 1,0488492 | 1269,9998 | 1,4139841 | 1687,9819 | CALM3; PHKD; PHKD3 | NM_005184 | synonyms: PHKD, PHKD3; go_component: cytoplasm [goid 0005737] [evidence ISS]; go_component: plasma membrane [goid 0005886] [evidence ISS]; go_function: protein binding [goid 0005515] [evidence ISS]; go_function: |
| 200648_s_at | 0,0098 | 0,88556826 | 502,7667 | 1,2698482 | 740,0409 | GLUL | NM_002065 | Glutamate-ammonia ligase (glutamine synthase) |
| 200649_at | 1,59E-05 | 0,9789512 | 1435,0703 | 1,2786359 | 1884,409 | NUCB1 | BC002356 | Nucleobindin 1 |
| 200659_s_at | 0,00129 | 1,0499921 | 406,75922 | 0,8054991 | 315,62726 | PHB | NM_002634 | Prohibitin |
| 200661_at 200664_s_at | 0,000107 7,50E-06 | 0,9615666 0,9854703 | 2706,9814 990,6408 | 1,203954 1,6431462 | 3434,5503 1598,7272 | PPGB DNAJB1 | NM_000308 BG537255 | Protective protein for beta-galactosidase (galactosialidosis) |
| 200666_s_at | 3,88E-06 | 1,0260544 | 1925,0222 | 1,4348284 | 2718,4724 | DNAJB1 | NM_006145 | DnaJ (Hsp40) homolog, subfamily B, member 1 |
| 200676_s_at | 0,000196 | 1,0102671 | 403,99997 | 0,7998809 | 322,8773 | UBE2L3 | NM_003347 | Ubiquitin-conjugating enzyme E2L 3 |
| 200678_x_at | 2,27E-05 | 0,9678019 | 6961,189 | 1,3699757 | 9889,847 | GRN | NM_002087 | Granulin |
| 200683_s_at | 3,61 E-05 | 0,99855685 | 1348,2185 | 0,76639515 | 1042,7772 | UBE2L3 | NM_003347 | Ubiquitin-conjugating enzyme E2L 3 |
| 200687_s_at | 4,81 E-08 | 0,9891875 | 851,7185 | 0,7397607 | 638,5409 | SF3B3 | NM_012426 | Splicing factor 3b, subunit 3, 130kDa |
| 200696_s_at | 8,06E-07 | 1,0069044 | 1035,1593 | 1,5581717 | 1583,9272 | GSN | NM_000177 | Gelsolin (amyloidosis, Finnish type) |
| 200700_s_at | 0,00618 | 0,9733115 | 1573,0516 | 1,2201155 | 1982,6864 | KDELR2 | NM_006854 | KDEL (Lys-Asp-Glu-Leu) endoplasmic reticulum protein retention receptor 2 |
| 200701_at | 1,38E-07 | 1,0084656 | 5029,627 | 1,4209206 | 7021,4683 | NPC2 | NM_006432 | Niemann-Pick disease, type C2 |
| 200703_at | 0,000196 | 1,0099849 | 4221,8257 | 0,70055366 | 3085,05 | DNCL1 | NM_003746 | Dynein, cytoplasmic, light polypeptide 1 |
| 200709_at | 0,00078 | 0,97911626 | 4129,5635 | 1,2515693 | 5299,1504 | FKBP1A | NM_000801 | FK506 binding protein 1A, 12kDa |
| 200713_s_at | 1,57E-09 | 1,0121485 | 3324,819 | 1,2696422 | 4177,8135 | MAPRE1 | NM_012325 | Microtubule-associated protein, RP/EB family, member 1 |
| 200719_at | 7,08E-13 | 0,9922775 | 515,1185 | 1,6137085 | 835,5681 | SKP1A | NM_003197 | |
| 200726_at | 0,000119 | 1,0513686 | 3274,289 | 0,8382299 | 2586,7773 | PPP1CC | NM_002710 | Protein phosphatase 1, catalytic subunit, gamma isoform |
| 200727_s_at | 0,00109 | 1,1992867 | 910,05554 | 2,2008042 | 1542,4774 | ACTR2 | AA699583 | ARP2 actin-related protein 2 homolog (yeast) |
| 200730_s_at | 1,78E-07 | 0,91481 | 353,21112 | 1,870016 IMAGE:4290141 | 739,54083 | PTP4A1 | BF576710 | 602135085F1 NIH_MGC_81 Homo sapiens CDNA clone 5', mRNA sequence. |
| 200731_s_at | 1,10E-05 | 0,9785795 | 372,6222 | 1,6035231 IMAGE:4290141 | 605,50903 | PTP4A1 | BF576710 | 602135085F1 NIH_MGC_81 Homo sapiens cDNA clone 5', mRNA sequence. |
| 200732_s_at | 9,70E-11 | 1,0264257 | 939,3371 | 1,5883412 IMAGE:4290141 | 1462 | PTP4A1 | BF576710 | 602135085F1 NIH_MGC_81 Homo sapiens cDNA clone 5', mRNA sequence. |
| 200742_s_at | 0,000107 | 0,93660074 | 3210,8372 | 1,2565378 | 4355,814 | TPP1 | BG231932 | Tripeptidyl peptidase I |
| 200749_at | 4,05E-05 | 0,97768396 | 651,5075 | 0,6749312 | 459,3363 | RAN | BF112006 | RAN, member RAS oncogene family |
| 200765_x_at | 0,00158 | 0,9855839 | 1022,07764 | 1,2377168 | 1267,6411 | CTNNA1 | NM_001903 | Catenin (cadherin-associated protein), alpha 1, 102kDa |
| 200766_at | 0,00468 | 0,9999239 | 1004,3778 | 1,2996724 | 1350,2228 | CTSD | NM_001909 | Cathepsin D (lysosomal aspartyl protease) |
| 200768_s_at | 0,000119 | 0,9630753 | 1785,8 | 0,7493337 | 1357,418 | MAT2A | BC001686 | Methionine adenosyltransferase II, alpha |
| 200779_at | 2,38E-10 | 0,98888814 | 3671,53 | 1,3902841 | 5193,2544 | ATF4 | NM_001675 | Activating transcription factor 4 (tax-responsive enhancer element B67) |
| 200782_at | 4,53E-05 | 1,0018673 | 4280,337 | 1,2992077 | 5478,8228 | ANXA5 | NM_001154 | Annexin A5 |
| 200788_s_at | 2,27E-05 | 1,0364705 | 1898,3593 | 1,352392 | 2464,873 | PEA15 | NM_003768 | Phosphoprotein enriched in astrocytes 15 |
| 200797_s_at | 3,71E-07 | 0,9821959 | 10878,5625 | 1,4001372 | 15188,777 | MCL1 | NM_021960 | Myeloid cell leukemia sequence 1 (BCL2-related) |
| 200798_x_at | 0,00178 | 0,9855381 | 2568,0667 | 1,3812336 | 3658,932 | MCL1 | NM_021960 | Myeloid cell leukemia sequence 1 (BCL2-related) |
| 200799_at | 2,28E-07 | 0,9063665 | 4490,534 | 0,46141994 | 2416,1228 | HSPA1A | NM_005345 | Heat shock 70kDa protein 1A |
| 200800_s_at | 2,28E-07 | 0,8793383 | 1260,7518 | 0,42510474 | 644,52264 | HSPA1A | NM_005345 | Heat shock 70kDa protein 1A |
| 200806_s_at | 0,00178 | 0,92898357 | 1247,5887 | 0,6818645 | 905,7955 | HSPD1 | BE256479 | Heat shock 60kDa protein 1 (chaperonin) |
| 200808_s_at | 0,000131 | 0,99740887 | 2934,8003 | 1,3910868 | 4172,896 | ZYX | NM_003461 | Zyxin |
| 200811_at | 3,61E-05 | 0,98863363 | 2378,1448 | 1,3336936 | 3224,3003 | CIRBP | NM_001280 | Cold inducible RNA binding protein |
| 200813_s_at | 0,00085 | 0,9305404 | 563,6 | 0,6448917 | 389,3909 | PAFAH1B1 | BE256969 | Platelet-activating factor acetylhydrolase, isoform lb, alpha subunit 45kDa |
| 200816_s_at | 2,18E-09 | 0,9471215 | 1280,5779 | 0,57625616 | 786,15454 | PAFAH1B1 | NM_000430 | Platelet-activating factor acetylhydrolase, isoform lb, alpha subunit 45kDa |
| 200833_s_at | 4,53E-05 | 1,0935626 | 7987,563 | 0,8500783 | 6038,141 | RAP1B | NM_015646 | RAP1B, member of RAS oncogene family |
| 200838_at | 0,000262 | 0,9640296 | 2267,8965 | 1,2968433 | 3074,5952 | CTSB | NM_001908 | Cathepsin B |
| 200839_s_at | 2,02E-05 | 0,95315456 | 5262,4185 | 1,2787938 | 6957,081 | CTSB | NM_001908 | Cathepsin B |
| 200842_s_at | 0,00919 | 1,0351046 | 696,87775 | 0,8183989 | 562,09546 | EPRS | A1475965 | Glutamyl-prolyl-tRNA synthetase |
| 200843_s_at | 1,56E-08 | 1,0155656 | 1123,0408 | 0,7790404 | 859,60455 | EPRS | NM_004446 | Glutamyl-prolyl-tRNA synthetase |
| 200852_x_at | 0,000549 | 0,9779183 | 2198,7703 | 1,2859721 | 2905,6543 | GNB2 | NM_005273 | Guanine nucleotide binding protein (G protein), beta polypeptide 2 |
| 200853_at | 0,00152 | 1,0135646 | 3058,6924 | 0,7684802 | 2332,2861 | H2AFZ | NM_002106 | H2A histone family, member Z |
| 200854_at | 6,59E-06 | 1,0110005 | 873,28894 | 0,6860759 | 603,4318 | NCOR1 | NM_006311 | Nuclear receptor co-repressor 1 |
| 200855_at | 4,70E-07 | 0,985203 | 273,84073 | 0,65658504 | 185,79999 | NCOR1 | NM_006311 | Nuclear receptor co-repressor 1 |
| 200866_s_at | 1,59E-05 | 0,9923034 | 11275,965 | 1,2746823 | 14413,522 | PSAP | M32221 | Prosaposin (variant Gaucher disease and variant metachromatic leukodystrophy) |
| 200868_s_at | 2,77E-08 | 0,975763 | 982,6666 | 1,3652259 | 1374,8228 | ZNF313 | NM_018683 | Zinc finger protein 313 |
| 200873_s_at | 0,000238 | 0,98252046 | 3511,048 | 0,7507465 | 2662,2002 | CCT8 | NM_006585 | Chaperonin containing TCP1, subunit 8 (theta) |
| 200875_s_at | 1,10E-05 | 1,0419735 | 1189,2518 | 0,71940655 | 840,9455 | NOL5A | NM_006392 | Nucleolar protein 5A (56kDa with KKE/D repeat) |
| 200877_at | 0,000715 | 0,94682634 | 4238,0635 | 0,75680155 | 3331,9185 | CCT4 | NM_006430 | Chaperonin containing TCP1, subunit 4 (delta) |
| 200892_s_at | 7,81E-05 | 0,9919459 | 1643,1333 | 1,2707542 homolog, | 2119,4863 | SFRS10 | BC000451 | Splicing factor, arginine/serine-rich 10 (transformer 2 Drosophila) |
| 200897_s_at | 7,92E-10 | 0,9822078 | 386,42966 | 0,49142447 | 201,22273 | KIAA0992 | NM_016081 | Palladin |
| 200902_at | 4,63E-09 | 1,0247122 | 2162,874 | 0,68268156 | 1425,2545 | 15.Sep | NM_004261 | 15 kDa selenoprotein |
| 200907_s_at | 0,000419 | 0,9613452 | 233,37407 | 0,62890464 | 156,76363 | KIAA0992 | AK025843 | Palladin |
| 200916_at | 7,02E-05 | 1,0150712 | 5708,908 | 1,3244249 | 7371,505 | TAGLN2 | NM_003564 | Transgelin 2 |
| 200919_at | 5,94E-07 | 0,9817996 | 1203,7407 | 1,295772 | 1602,9454 | PHC2 | NM_004427 | Polyhomeotic-like 2 (Drosophila) |
| 200953_s_at | 1,09E-06 | 1,0356232 | 2791,0962 | 0,74535006 | 1974,5546 | CCND2 | NM_001759 | Cyclin D2 |
| 200961_at | 1,68E-06 | 1,0031503 | 885,3889 | 0,7223069 | 647,5865 | SEPHS2 | NM_012248 | Selenophosphate synthetase 2 |
| 200965_s_at | 6,30E-05 | 0,99331963 | 3064,9075 | 0,6910918 | 2152,082 | ABLIM1 | NM_006720 | Actin binding LIM protein 1 |
| 200972_at | 9,65E-05 | 1,0557096 | 1019,663 | 0,8103602 | 784,25903 | TM4SF8 | BC000704 | Transmembrane 4 superfamily member 8 |
| 200979_at | 5,65E-05 | 0,9404474 | 390,07037 | 0,7284613 | 296,01364 | FLJ16518 | BF739979 | Mitogen-activated protein kinase kinase kinase 15 |
| 200985_s_at | 0,00485 | 1,0138766 | 449,6519 | 0,7830506 | 359,7818 | CD59 | NM_000611 | CD59 antigen p18-20 (antigen identified by monoclonal antibodies 16.3A5, EJ16, EJ30, EL32 and G344) |
| 200986_at | 3,16E-07 | 1,006983 | 158,44446 | 2,1898115 | 375,83182 | SERPING1 | NM_000062 | Serine (or cysteine) proteinase inhibitor, clade G (C1 inhibitor), member 1, (angioedema, hereditary) |
| 200989_at | 3,16E-07 | 1,032005 | 2966,378 | 1,4428962 helix | 4207,5684 | HIF1A | NM_001530 | Hypoxia-inducible factor 1, alpha subunit (basic helix-loop-transcription factor) |
| 200992_at | 2,08E-06 | 0,97372746 | 846,8815 | 0,6867775 | 604,1318 | IPO7 | AL137335 | Importin 7 |
| 200993_at | 9,65E-05 | 0,9791714 | 1102,8776 | 0,7489338 | 860,71814 | IPO7 | AL137335 | Importin 7 |
| 200994_at | 2,57E-06 | 0,9574423 | 713,2296 | 0,6963575 | 522,0273 | IPO7 | AL137335 | Importin 7 |
| 200995_at | 2,27E-05 | 0,9408813 | 422,62225 | 0,7266619 | 324,38184 | IPO7 | AL137335 | Importin 7 |
| 200996_at | 6,92E-07 | 1,0506195 | 5882,874 | 0,73927504 | 4196,9136 | ACTR3 | NM_005721 | ARP3 actin-related protein 3 homolog (yeast) |
| 200997_at | 0,000502 | 0,9880792 | 1725,3074 | 0,7321962 | 1305,8091 | RBM4 | NM_002896 | RNA binding motif protein 4 |
| 200998_s_at | 5,94E-07 | 0,9563119 | 1023,6594 | 1,4211236 | 1513,2592 | CKAP4 | AW029619 | Cytoskeleton-associated protein 4 |
| 200999_s_at | 0,00661 | 0,927395 | 1417,4515 | 1,2142289 | 1792,209 | CKAP4 | NM_006825 | Cytoskeleton-associated protein 4 |
| 201013_s_at | 1,78E-07 | 1,020394 | 529,4297 | 0,74270463 | 387,41815 | PAICS | AA902652 | Phosphoribosylaminoimidazole carboxylase, phosphoribosylaminoimidazole succinocarboxamide synthetase |
| 201016_at | 0,00378 | 1,0360541 | 363,68893 | 0,74656504 | 264,31366 | EIF1AX | BE542684 | Eukaryotic translation initiation factor 1A, X-linked |
| 201023_at | 5,71E-09 | 0,9716754 | 2280,9922 | 0,6797227 | 1605,2772 | TAF7 | NM_005642 | TAF7 RNA polymerase II, TATA box binding protein (TBP)-associated factor, 55kDa |
| 201024_x_at | 0,00378 | 0,8990019 | 656,98145 | 0,66000724 | 484,71817 | EIF5B | NM_015904 | Eukaryotic translation initiation factor 5B |
| 201027_s_at | 5,09E-07 | 1,0124757 | 534,7223 | 0,6647373 | 358,79092 | EIF5B | NM_015904 | Eukaryotic translation initiation factor 5B |
| 201030_x_at | 5,65E-05 | 1,1093816 | 9235,892 | 0,82838356 | 6729,536 | LDHB | NM_002300 | Lactate dehydrogenase B |
| 201036_s_at | 3,04E-08 | 1,0256174 | 347,4741 | 0,70236135 | 236,57727 | HADHSC | NM_005327 | L-3-hydroxyacyl-Coenzyme A dehydrogenase, short chain |
| 201039_s_at | 4,53E-05 | 0,9378352 | 668,20734 | 1,2581472 | 883,9591 | RAD23A | BF572938 | RAD23 homolog A (S. cerevisiae) |
| 201046_s_at | 0,000502 | 0,97012335 | 1202,0962 | 1,2258385 | 1521,0953 | RAD23A | NM_005053 | RAD23 homolog A (S. cerevisiae) |
| 201054_at | 1,85E-10 | 1,0059075 | 1389,2703 | 0,63726723 | 868,05 | HNRPA0 | BE966599 | Heterogeneous nuclear ribonucleoprotein A0 |
| 201055_s_at | 3,16E-07 | 1,0653392 | 743,0037 | 1,5897032 | 1101,368 | HNRPA0 | NM_006805 | Heterogeneous nuclear ribonucleoprotein A0 |
| 201086_x_at | 3,88E-10 | 0,9932776 | 3126,4927 | 0,6626507 | 2112,0503 | SON | NM_003103 | SON DNA binding protein |
| 201087_at | 1,46E-06 | 0,9764763 | 1436,9408 | 1,3623133 | 1982,9818 | PXN | NM_002859 | Paxillin |
| 201098_at | 2,69E-07 | 1,0492086 | 878,037 | 0,7240693 | 614,3682 | COPB2 | NM_004766 | Coatomer protein complex, subunit beta 2 (beta prime) |
| 201111_at | 2,29E-08 | 1,0173384 | 221,42593 | 0,6625244 | 141,85454 | CSE1L | AF053641 | CSE1 chromosome segregation 1-like (yeast) |
| 201118_at | 0,000178 | 1,0097895 | 2202,422 | 1,3733766 | 3014,4045 | PGD | NM_002631 | Phosphogluconate dehydrogenase |
| 201119_s_at | 2,87E-05 | 0,9462517 | 4930,2 | 1,18891 | 6108,327 | COX8A | NM_004074 | Cytochrome c oxidase subunit 8A (ubiquitous) |
| 201129_at | 4,02E-08 | 0,9781365 | 884,25555 | 0,68468815 | 612,65454 | SFRS7 | NM_006276 | Splicing factor, arginine/serine-rich 7, 35kDa |
| 201142_at | 2,29E-08 | 1,0430955 | 771,4037 | 0,7200717 | 538,5636 | EIF2S1 | AA577698 | Eukaryotic translation initiation factor 2, subunit 1 alpha, |
| 201146_at | 0,00661 | 1,0330108 | 1176,3146 | 0,78860736 | 964,1953 | NFE2L2 | NM_006164 | Nuclear factor (erythroid-derived 2)-like 2 |
| 201153_s_at | 0,000289 | 1,0524249 | 5365,852 | 0,82666296 | 4133,977 | MBNL1 | NM_021038 | Muscleblind-like (Drosophila) |
| 201156_s_at | 0,00755 | 1,0089504 | 732,4778 | 1,3069708 | 938,08185 | RAB5C | AF141304 | RAB5C, member RAS oncogene family |
| 201158_at | 4,99E-11 | 0,91091275 | 270,437 | 0,20086259 | 77,277275 | NMT1 | NM_021079 | N-myristoyltransferase 1 |
| 201160_s_at | 0,00101 | 0,8946762 | 3077,696 | 1,2018692 | 4207,577 | CSDA | AL556190 | Cold shock domain protein A |
| 201161_s_at | 2,27E-05 | 1,0570291 | 992,9518 | 1,5985372 | 1510,9501 | CSDA | NM_003651 | Cold shock domain protein A |
| 201167_x_at | 0,00261 | 1,0222646 | 330,36664 | 1,7120847 | 569,2955 | ARHGDIA | D13989 | Rho GDP dissociation inhibitor (GDI) alpha |
| 201168_x_at | 5,09E-07 | 1,0036266 | 1995,3667 | 1,513771 | 2997,3318 | ARHGDIA | NM_004309 | Rho GDP dissociation inhibitor (GDI) alpha |
| 201169_s_at | 4,82E-08 | 0,96865934 | 255,5037 | 2,130702 | 582,12274 | BHLHB2 | BG326045 | Basic helix-loop-helix domain containing, class B, 2 |
| 201170_s_at | 1,92E-12 | 1,0400934 | 1476,485 | 2,1492555 | 3059,9773 | BHLHB2 | NM_003670 | Basic helix-loop-helix domain containing, class B, 2 |
| 201176_s_at | 8,59E-09 | 0,9734205 | 1594,326 | 0,59413874 | 1002,9636 | ARCN1 | NM_001655 | Archain 1 |
| 201177_s_at | 7,81E-05 | 1,0131946 | 1351,248 | 0,7975745 | 1048,941 | UBA2 | NM_005499 | SUMO-1 activating enzyme subunit 2 |
| 201181_at | 0,00224 | 0,9531184 | 399,563 | 0,7270417 | 306,28635 | GNAI3 | NM_006496 | Guanine nucleotide binding protein (G protein), alpha inhibiting activity polypeptide 3 |
| 201182_s_at | 4,43E-06 | 1,016372 | 283,59262 | 1,419565 | 397,83636 | CHD4 | AI761771 | Chromodomain helicase DNA binding protein 4 |
| 201186_at | 0,00538 | 0,95113623 | 1073,8779 | 1,2086347 | 1365,0455 | LRPAP1 | NM_002337 | Low density lipoprotein receptor-related protein associated protein 1 |
| 201200_at | 1,79E-05 | 1,0153191 | 1678,5776 | 1,3790934 | 2278,8271 | CREG1 | NM_003851 | Cellular repressor of E1A-stimulated genes 1 |
| 201202_at | 4,35E-07 | 0,98533225 | 675,1667 | 0,6255863 | 438,53635 | PCNA | NM_002592 | Proliferating cell nuclear antigen |
| 201204_s_at | 0,00352 | 0,8764973 | 383,35925 | 1,3006297 | 549,25 | RRBP1 | AI921320 | Ribosome binding protein 1 homolog 180kDa (dog) |
| 201209_at | 4,82E-08 | 0,9771758 | 2609,1108 | 0,69472593 | 1847,4501 | HDAC1 | NM_004964 | Histone deacetylase 1 |
| 201218_at | 2,28E-07 | 1,0121835 | 972,27045 | 0,5943128 | 595,02277 | CTBP2 | NM_001329 | C-terminal binding protein 2 |
| 201223_s_at | 0,00304 | 1,0458232 | 711,46295 | 0,8330534 | 569,50464 | RAD23B | NM_002874 | RAD23 homolog B (S. cerevisiae) |
| 201236_s_at | 0,000382 | 0,9829352 | 1834,911 | 1,2531438 | 2364,459 | BTG2 | NM_006763 | BTG family, member 2 |
| 201239_s_at | 8,06E-07 | 1,0152892 | 1906,4666 | 0,7607323 | 1451,6226 | SPCS2 | BF530535 | Signal peptidase complex subunit 2 homolog (S. cerevisiae) |
| 201240_s_at | 1,17E-07 | 1,0290754 | 4018,9033 | 0,7609815 | 3003,5955 | SPCS2 | NM_014752 | Signal peptidase complex subunit 2 homolog (S. cerevisiae) |
| 201241_at | 2,44E-09 | 1,0630955 | 1237,1556 | 0,64974976 | 776,841 | DDX1 | NM_004939 | DEAD (Asp-Glu-Ala-Asp) box polypeptide 1 |
| 201248_s_at | 9,65E-05 | 0,94273406 | 397,5111 | 1,3190186 | 548,94086 | SREBF2 | NM_004599 | Sterol regulatory element binding transcription factor 2 |
| 201251_at | 0,00208 | 1,0201864 | 4397,303 | 1,2916971 | 5741,9404 | PKM2 | NM_002654 | Pyruvate kinase, muscle |
| 201260_s_at | 2,56E-05 | 0,9779349 | 943,7742 | 0,7413153 | 720,8227 | SYPL | NM_006754 | Synaptophysin-like protein |
| 201263_at | 1,38E-07 | 1,1047565 | 700,67773 | 0,7341265 | 452,03186 | TARS | NM_003191 | synonyms: ThrRS, MGC9344; threonine--tRNA ligase; go_component: cytoplasm [goid 0005737] [evidence TAS] [pmid 2033077]; go_component: soluble fraction [goid 0005625] [evidence TAS] [pmid 2033077]; go_function: ATP binding [goid 0005524] [evidence IEA]; go |
| 201266_at | 2,29E-08 | 0,9958791 | 980,1852 | 0,7917909 | 780,7363 | TXNRD1 | NM_003330 | Thioredoxin reductase 1 |
| 201274_at | 3,71E-07 | 0,9687593 | 1768,763 | 0,74174327 | 1363,2001 | PSMA5 | NM_002790 | Proteasome (prosome, macropain) subunit, alpha type, 5 |
| 201275_at | 0,0098 | 1,0780723 | 839,5667 | 0,8478139 | 668,66364 | FDPS | NM_002004 | Farnesyl diphosphate synthase (farnesyl pyrophosphate synthetase, dimethylallyltranstransferase, geranyltranstransferase) |
| 201280_s_at | 0,00178 | 1,0526993 | 317,59625 | 0,76514447 (Drosophila) | 233,68182 | DAB2 | NM_001343 | Disabled homolog 2, mitogen-responsive phosphoprotein |
| 201307_at | 1,69E-05 | 0,9966525 | 606,5444 | 0,75507617 | 467,61816 | 11.Sep | AL534972 | Septin 11 |
| 201318_s_at | 5,71E-09 | 0,99659806 | 9316,639 | 0,6940609 | 6381,009 | MRCL3 | NM_006471 | Myosin regulatory light chain MRCL3 |
| 201326_at | 0,000216 | 1,0444318 | 661,337 | 0,7578771 IMAGE:3639098 | 480,75912 | CCT6A | BE737030 | 601304610F1 NIH_MGC_39 Homo sapiens cDNA clone 5', mRNA sequence. |
| 201327_s_at | 3,22E-05 | 1,0272626 | 960,7371 | 0,81265706 | 756,65454 | CCT6A | NM_001762 | Chaperonin containing TCP1, subunit 6A (zeta 1) |
| 201329_s_at | 2,57E-06 | 1,0452671 | 320,95554 | 1,8436267 | 631,9501 | ETS2 | NM_005239 | V-ets erythroblastosis virus E26 oncogene homolog 2 |
| 201344_at | 4,01E-07 | 1,0201223 | 242,18149 | 0,7551994 | 181,06818 | UBE2D2 | NM_003339 | Ubiquitin-conjugating enzyme E2D 2 (UBC4/5 homolog, |
| 201358_s_at | 4,44E-06 | 1,017386 | 2990,0222 | 0,81291777 | 2418,236 | COPB | NM_016451 | Coatomer protein complex, subunit beta |
| 201360_at | 7,50E-06 | 0,9381299 | 7489,852 | 1,3819261 | 11025,95 | CST3 | NM_000099 | Cystatin C (amyloid angiopathy and cerebral hemorrhage) |
| 201362_at | 3,04E-10 | 1,0043048 | 954,7668 | 0,68961334 | 650,88635 | IVNS1ABP | AF205218 | Influenza virus NS1A binding protein |
| 201363_s_at | 5,65E-05 | 0,97974914 | 757,65186 | 0,661177 | 506,0455 | IVNS1ABP | AB020657 | Influenza virus NS1A binding protein |
| 201364_s_at | 0,0006 | 1,0199047 | 1164,6887 | 1,5373597 | 1682,2819 | OAZ2 | AF242521 | Ornithine decarboxylase antizyme 2 |
| 201365_at | 8,06E-07 | 0,9302242 | 1025,6483 | 1,3190212 | 1434,5726 | OAZ2 | NM_002537 | Ornithine decarboxylase antizyme 2 |
| 201369_s_at | 0,00129 | 1,1568302 | 2074,3113 | 2,110903 | 3189,0547 | ZFP36L2 | NM_006887 | Zinc finger protein 36, C3H type-like 2 |
| 201370_s_at | 1,93E-07 | 1,0085968 | 181,90002 | 0,6735451 | 123,23181 | CUL3 | AU145232 | Cullin 3 |
| 201371_s_at | 1,56E-08 | 1,0039946 | 2220,6147 | 0,78688335 | 1744,8682 | CUL3 | AF062537 | Cullin 3 |
| 201375_s_at | 2,87E-05 | 1,0113541 | 1543,6111 | 0,80712193 | 1241,9683 | PPP2CB | NM_004156 | Protein phosphatase 2 (formerly 2A), catalytic subunit, beta isoform |
| 201376_s_at | 0,000502 | 0,96513456 | 1501,3705 | 0,7325314 | 1137,7136 | HNRPF | AI591354 | Heterogeneous nuclear ribonucleoprotein F |
| 201381_x_at | 9,36E-07 | 1,0290171 | 1302,3964 | 0,78049093 | 993,5455 | CACYBP | AF057356 | Calcyclin binding protein |
| 201388_at | 0,000317 | 0,9459193 | 455,5481 | 1,1896237 | 571,85 | PSMD3 | NM_002809 | Proteasome (prosome, macropain) 26S subunit, non-ATPase, 3 |
| 201394_s_at | 0,0006 | 0,91641396 | 1325,2186 | 0,7024697 | 998,25 | RBM5 | U23946 | RNA binding motif protein 5 |
| 201401_s_at | 8,24E-08 | 0,87917113 | 928,13696 | 1,7151883 | 1743,5364 | ADRBK1 | M80776 | Adrenergic, beta, receptor kinase 1 |
| 201407_s_at | 0,00618 | 1,0124456 | 495,36667 | 0,7752164 | 385,50458 | PPP1CB | AI186712 | Protein phosphatase 1, catalytic subunit, beta isoform |
| 201413_at | 1,46E-06 | 0,9619445 | 1228,9297 | 0,70328593 | 906,2818 | HSD17B4 | NM_000414 | Hydroxysteroid (17-beta) dehydrogenase 4 |
| 201424_s_at | 0,0006 | 1,0437167 | 392,31848 | 0,71988595 | 263,9318 | CUL4A | NM_003589 | Cullin 4A |
| 201425_at | 0,0014 | 1,0050931 | 3173,3147 | 1,2780828 | 4060,5957 | ALDH2 | NM_000690 | Aldehyde dehydrogenase 2 family (mitochondrial) |
| 201437_s_at | 2,57E-06 | 0,9514033 | 354,46295 | 0,565731 | 217,3909 | EIF4E | NM_001968 | Eukaryotic translation initiation factor 4E |
| 201446_s_at | 0,00707 | 0,97711897 | 878,6852 | 0,76092046 | 674,4682 | TIA1 | NM_022037 | TIA1 cytotoxic granule-associated RNA binding protein |
| 201447_at | 0,000153 | 0,9676314 | 546,911 | 0,6874588 | 376,3227 | TIA1 | NM_022037 | TIA1 cytotoxic granule-associated RNA binding protein |
| 201448_at | 6,91 E-08 | 0,9803317 | 925,69257 | 0,59924614 | 574,70447 | TIA1 | NM_022037 | TIA1 cytotoxic granule-associated RNA binding protein |
| 201450_s_at | 0,00252 | 0,9647193 | 370,84814 | 0,69711536 | 265,88635 | TIA1 | NM_022037 | TIA1 cytotoxic granule-associated RNA binding protein |
| 201464_x_at | 8,52E-06 | 0,9101929 | 1444,6481 | 2,1307867 | 3154,2317 | JUN | BG491844 | V-jun sarcoma virus 17 oncogene homolog (avian) |
| 201472_at | 2,28E-07 | 1,0212033 | 2030,2999 | 0,7512151 | 1502,8 | VBP1 | NM_003372 | Von Hippel-Lindau binding protein 1 |
| 201473_at | 0,000262 | 1,0157659 | 3695,548 | 1,6726094 | 5453,618 | JUNB | NM_002229 | Jun B proto-oncogene |
| 201478_s_at | 0,00252 | 0,98562706 | 662,3592 | 0,75130653 | 514,75903 | DKC1 | U59151 | Dyskeratosis congenita 1, dyskerin |
| 201479_at | 4,44E-06 | 0,9752218 | 1533,9519 | 0,76519066 | 1205,1636 | DKC1 | NM_001363 | Dyskeratosis congenita 1, dyskerin |
| 201482_at | 1,94E-06 | 0,8936573 | 511,84445 | 1,3045292 | 739,0954 | QSCN6 | NM_002826 | Quiescin Q6 |
| 201483_s_at | 1,26E-06 | 0,92192304 | 1337,6555 | 1,340662 | 1881,7319 | SUPT4H1 | BC002802 | Suppressor of Ty 4 homolog 1 (S. cerevisiae) |
| 201486_at | 3,88E-06 | 0,97099787 | 528,66296 | 0,56441724 | 315,56818 | RCN2 | NM_002902 | Reticulocalbin 2, EF-hand calcium binding domain |
| 201489_at | 4,35E-07 | 0,98894894 | 575,1222 | 1,824625 | 1108,1 | PPIF | BC005020 | Peptidylprolyl isomerase F (cyclophilin F) |
| 201490_s_at | 7,81E-05 | 0,97187245 | 425,70367 | 1,5010444 | 671,9364 | PPIF | NM_005729 | Peptidylprolyl isomerase F (cyclophilin F) |
| 201493_s_at | 5,27E-08 | 0,98987263 | 1394,8259 | 0,679107 | 970,2273 | PUM2 | BE778078 | Vacuolar protein sorting 35 (yeast) |
| 201501_s_at | 9,64E-05 | 1,0135853 | 550,58514 | 0,7574059 | 414,25 | GRSF1 | NM_002092 | G-rich RNA sequence binding factor 1 |
| 201502_s_at | 1,26E-06 | 1,1864247 | 6090,7183 | 2,711446 B-cells | 12045,886 | NFKBIA | AI078167 | Nuclear factor of kappa light polypeptide gene enhancer in inhibitor, alpha |
| 201503_at | 1,22E-11 | 1,0145357 | 1118,7556 | 0,55126774 IMAGE:4668234 | 619,24536 | G3BP | BG500067 | 602545874F1 NIH_MGC_60 Homo sapiens cDNA clone 5', mRNA sequence. |
| 201517_at | 9,96E-10 | 1,026044 | 815,52966 | 0,758045 | 601,6545 | NCBP2 | BC001255 | Nuclear cap binding protein subunit 2, 20kDa |
| 201518_at | 3,75E-09 | 0,99714077 | 1029,9778 | 0,7653827 | 794,7909 | CBX1 | NM_006807 | Chromobox homolog 1 (HP1 beta homolog Drosophila) |
| 201519_at | 1,96E-09 | 1,0144919 | 1242,7001 | 0,765815 A | 932,3637 | TOMM70A | NM_014820 | Translocase of outer mitochondrial membrane 70 homolog (yeast) |
| 201526_at | 6,91 E-08 | 0,9399505 | 1291,6038 | 1,3866929 | 1847,2092 | ARF5 | NM_001662 | ADP-ribosylation factor 5 |
| 201528_at | 0,000161 | 1,0234581 | 1703,2147 | 0,8030358 | 1340,4089 | RPA1 | BG398414 | Replication protein A1, 70kDa |
| 201531_at | 8,59E-09 | 0,91810197 | 3697,0781 | 1,7419353 | 6820,4907 | ZFP36 | NM_003407 | Zinc finger protein 36, C3H type, homolog (mouse) |
| 201534_s_at | 0,00618 | 0,90713024 | 1102,0111 | 0,62642324 | 784,05 | UBL3 | AF044221 | Ubiquitin-like 3 |
| 201536_at | 0,000419 | 0,997501 | 646,9667 | 1,2750632 VH1-related) | 835,6955 | DUSP3 | AL048503 | Dual specificity phosphatase 3 (vaccinia virus phosphatase |
| 201537_s_at | 1,64E-07 | 0,99516827 | 199,0037 | 1,7687837 | 360,90457 | DUSP3 | BC002682 | Dual specificity phosphatase 3 (vaccinia virus phosphatase VH1-related) |
| 201538_s_at | 0,000238 | 0,990096 | 129,6889 | 1,424079 VH1-related) | 185,97273 | DUSP3 | NM_004090 | Dual specificity phosphatase 3 (vaccinia virus phosphatase |
| 201543_s_at | 0,000146 | 0,9990647 | 455,9518 | 1,2790941 | 580,7637 | SARA1 | NM_020150 | SAR1a gene homolog 1 (S. cerevisiae) |
| 201544_x_at | 5,09E-07 | 0,96897805 | 3618,696 | 1,2283113 IMAGE:4274564 | 4577,3325 | PABPN1 | BF675004 | 602138088F1 NIH_MGC_83 Homo sapiens cDNA clone 5', mRNA sequence. |
| 201551_s_at | 0,000382 | 0,8931663 | 693,9185 | 1,7199514 | 1135,2592 | LAMP1 | J03263 | Lysosomal-associated membrane protein 1 |
| 201589_at | 4,02E-08 | 1,0320815 | 1513,1038 | 0,72392154 | 1078,5728 | SMC1L1 | D80000 | SMC1 structural maintenance of chromosomes 1-like 1 |
| 201598_s_at | 9,36E-07 | 0,9318465 | 745,7037 | 1,3441176 | 1046,209 | INPPL1 | NM_001567 | Inositol polyphosphate phosphatase-like 1 |
| 201603_at | 1,64E-07 | 1,0069908 | 1124,8073 | 0,60384583 | 683,5409 | PPP1R12A | NM_002480 | Protein phosphatase 1, regulatory (inhibitor) subunit 12A |
| 201606_s_at | 2,87E-05 | 1,0138417 | 1131,2742 | 0,7398581 | 814,31366 | PWP1 | BE796924 | Nuclear phosphoprotein similar to S. cerevisiae PWP1 |
| 201608_s_at | 1,17E-07 | 0,97342527 | 974,85187 | 0,76360345 | 761,1454 | PWP1 | NM_007062 | Nuclear phosphoprotein similar to S. cerevisiae PWP1 |
| 201612_at | 7,02E-05 | 0,99966425 | 1496,2037 | 0,79907674 | 1200,0728 | ALDH9A1 | NM_000696 | Aldehyde dehydrogenase 9 family, member A1 |
| 201614_s_at | 2,10E-07 | 1,016015 | 276,94077 | 0,71435475 | 195,01363 | RUVBL1 | NM_003707 | RuvB-like 1 (E. coli) |
| 201623_s_at | 0,00378 | 0,9174672 | 2129,6296 | 0,6077197 | 1460,4728 | DARS | BC000629 | Aspartyl-tRNA synthetase |
| 201624_at | 0,00661 | 0,9740832 | 531,8184 | 0,75357705 | 411,75 | DARS | NM_001349 | Aspartyl-tRNA synthetase |
| 201628_s_at | 2,44E-09 | 0,9873999 | 1319,7963 | 0,71796346 | 968,7773 | RRAGA | NM_006570 | Ras-related GTP binding A |
| 201631_s_at | 0,00327 | 1,0206276 | 889,38873 | 1,3328524 | 1169,4819 | IER3 | NM_003897 | Immediate early response 3 |
| 201634_s_at | 0,00224 | 1,0033901 | 207,9185 | 0,7467916 | 156,21364 | CYB5-M | NM_030579 | Hypothetical protein LOC283852 |
| 201636_at | 5,78E-08 | 0,9972065 | 453,6592 | 0,786977 | 359,54544 | FXR1 | AI990766 | Fragile X mental retardation, autosomal homolog 1 |
| 201647_s_at | 0,00502 | 0,85522926 | 198,18149 | 1,2728198 | 297,57727 | SCARB2 | NM_005506 | Scavenger receptor class B, member 2 |
| 201649_at | 7,50E-06 | 0,9630316 | 2579,5886 | 1,3338724 | 3649,4456 | UBE2L6 | NM_004223 | Ubiquitin-conjugating enzyme E2L 6 |
| 201652_at | 2,69E-07 | 0,98715585 | 1499,3444 | 0,7050214 (Arabidopsis) | 1097,5272 | COPS5 | NM_006837 | COP9 constitutive photomorphogenic homolog subunit 5 |
| 201656_at | 0,00352 | 0,90726304 | 447,9704 | 0,606965 | 299,6227 | ITGA6 | NM_000210 | Integrin, alpha 6 |
| 201657_at | 0,000317 | 1,0847791 | 108,470375 | 0,81807584 | 82,24999 | ARL1 | NM_001177 | ADP-ribosylation factor-like 1 |
| 201660_at | 9,36E-07 | 0,9894704 | 324,3556 | 0,64183116 | 217,22726 | ACSL3 | AL525798 | Acyl-CoA synthetase long-chain family member 3 |
| 201661_s_at | 1,68E-06 | 0,9933758 | 303,74072 | 0,6870974 | 211,32274 | ACSL3 | NM_004457 | Acyl-CoA synthetase long-chain family member 3 |
| 201664_at | 8,99E-08 | 0,9643648 | 574,48517 | 0,67158747 | 408,13638 | SMC4L1 | AL136877 | SMC4 structural maintenance of chromosomes 4-like 1 |
| 201666_at | 0,00109 | 0,9496677 | 3727,867 | 1,2413721 | 4781,0684 | TIMP1 | NM_003254 | Tissue inhibitor of metalloproteinase 1 (erythroid potentiating activity, collagenase inhibitor) |
| 201668_x_at | 0,0014 | 0,98477393 | 194,5963 | 1,553799 | 326,72272 | MARCKS | AW163148 | Myristoylated alanine-rich protein kinase C substrate |
| 201670_s_at | 0,00618 | 1,0097607 | 546,9592 | 1,5066307 | 849,0455 | MARCKS | M68956 | Myristoylated alanine-rich protein kinase C substrate |
| 201683_x_at | 0,00406 | 1,0057211 | 324,18152 | 1,2861577 | 425,35455 | C14orf92 | BE783632 | Chromosome 14 open reading frame 92 |
| 201685_s_at | 0,000216 | 1,0578295 | 511,48157 | 0,8434778 | 406,0545 | C14orf92 | NM_014828 | Chromosome 14 open reading frame 92 |
| 201687_s_at | 7,50E-06 | 0,9734487 | 1039,4594 | 0,6937192 | 747,48175 | API5 | NM_006595 | Apoptosis inhibitor 5 |
| 201689_s_at | 0,00468 | 1,03787 | 346,6185 | 0,7795641 | 274,42273 | TPD52 | BE974098 | Tumor protein D52 |
| 201695_s_at | 3,88E-06 | 0,9251387 | 898,04443 | 0,61538756 | 599,43646 | NP | NM_000270 | Nucleoside phosphorylase |
| 201699_at | 9,06E-12 | 1,0150248 | 899,8407 | 0,5501056 | 490,98178 | PSMC6 | NM_002806 | Proteasome (prosome, macropain) 26S subunit, ATPase, 6 |
| 201701_s_at | 0,000574 | 1,0124154 | 251,62598 | 0,67903095 | 173,88182 | PGRMC2 | NM_006320 | Progesterone receptor membrane component 2 |
| 201705_at | 5,96E-05 | 1,0542151 | 1212,3036 | 0,8220848 ATPase, | 924,13184 | PSMD7 | NM_002811 | Proteasome (prosome, macropain) 26S subunit, non-7 (Mov34 homolog) |
| 201713_s_at | 1,17E-07 | 1,0038178 | 828,90735 | 0,63970363 | 530,35455 | RANBP2 | D42063 | RAN binding protein 2 |
| 201735_s_at | 1,05E-08 | 0,9648271 | 415,65558 | 0,6189776 | 262,3909 | CLCN3 | NM_001829 | Chloride channel 3 |
| 201737_s_at | 8,68E-05 | 0,9719124 | 691,03705 | 0,6147626 | 443,2909 | MARCH-VI | NM_005885 | Membrane-associated ring finger (C3HC4) 6 |
| 201751_at | 0,000146 | 0,9907591 | 922,5556 | 1,2963561 | 1222,5637 | KIAA0063 | NM_014876 | KIAA0063 gene product |
| 201753_s_at | 0,00538 | 1,1328672 | 4982,644 | 0,8240693 | 3510,441 | ADD3 | NM_019903 | Adducin 3 (gamma) |
| 201763_s_at | 0,000119 | 1,088384 | 479,13705 | 0,7811143 | 346,15002 | DAXX | NM_001350 | Death-associated protein 6 |
| 201769_at | 5,49E-07 | 1,0248201 | 1389,9073 | 0,72389764 | 1000,8363 | ENTH | NM_014666 | Enthoprotin |
| 201773_at | 1,72E-13 | 0,9997071 | 1207,3553 | 0,55180746 | 678,10895 | ADNP | NM_015339 | Activity-dependent neuroprotector |
| 201778_s_at | 1,11E-10 | 1,030917 | 3350,0002 | 0,8025929 | 2600,982 | KIAA0494 | NM_014774 | KIAA0494 gene product |
| 201784_s_at | 2,27E-05 | 1,0113236 | 2538,637 | 0,71357316 | 1846,5681 | SMAP | NM_014267 | Small acidic protein |
| 201795_at | 6,91 E-08 | 1,008374 | 3276,4226 | 0,6939779 | 2273,5952 | LBR | NM_002296 Lamin | B receptor |
| 201803_at | 0,000131 | 0,9394331 | 1934,2555 | 0,718366 | 1461,1138 | POLR2B | NM_000938 | Polymerase (RNA) II (DNA directed) polypeptide B, 140kDa |
| 201819_at | 0,00282 | 0,949139 | 205,05556 | 1,2224241 | 261,71817 | SCARB1 | NM_005505 | Scavenger receptor class B, member 1 |
| 201824_at | 2,27E-05 | 0,963337 | 302,59628 | 0,7081392 | 221,31819 | RNF14 | AB022663 | Ring finger protein 14 |
| 201825_s_at | 0,000289 | 1,0115093 | 268,57776 | 1,3225054 | 353,15002 | CGI-49 | AL572542 | CGI-49 protein |
| 201829_at | 0,00171 | 0,9620653 | 207,9185 | 0,7449374 | 162,49089 | NET1 | AW263232 | Neuroepithelial cell transforming gene 1 |
| 201832_s_at | 2,64E-12 | 1,0119476 | 1650,6926 | 0,670826 | 1096,2816 | VDP | NM_003715 | Vesicle docking protein p115 |
| 201833_at | 1,64E-07 | 0,9349979 | 885,44073 | 0,5880607 | 553,32733 | HDAC2 | NM_001527 | Histone deacetylase 2 |
| 201841_s_at | 0,000685 | 0,9846983 | 502,30743 | 0,7485447 | 378,8818 | HSPB1 | NM_001540 | Heat shock 27kDa protein 1 |
| 201845_s_at | 9,49E-09 | 0,9428935 | 1084,9407 | 0,6072352 | 706,5909 | RYBP | AB029551 | RING1 and YY1 binding protein |
| 201849_at | 0,000161 | 0,9305821 | 280,65927 | 0,60773593 | 180,55908 | BNIP3 | NM_004052 | synonym: NIP3; BCL2Vadenovirus E1B 19kD-interacting protein 3; go_component: mitochondrion [goid 0005739] [evidence IEA]; go_component: integral to membrane [goid 0016021] [evidence IEA]; go_function: protein binding [goid 0005515] [evidence NAS]; go_pro |
| 201850_at | 0,00468 | 0,982847 | 1504,363 | 1,2499765 | 1945,6956 | CAPG | NM_001747 | Capping protein (actin filament), gelsolin-like |
| 201855_s_at | 1,16E-08 | 1,0307047 | 691,6667 | 0,6600063 | 436,91364 | KIAA0431 | NM_015251 | KIAA0431 protein |
| 201872_s_at | 0,000196 | 0,9983699 | 649,0037 | 0,69959617 | 465,53183 | ABCE1 | AI002002 | ATP-binding cassette, sub-family E (OABP), member 1 |
| 201873_s_at | 0,000161 | 1,0130806 | 540 | 0,766465 | 411,31366 | ABCE1 | NM_002940 | ATP-binding cassette, sub-family E (OABP), member 1 |
| 201874_at | 0,000178 | 0,9905533 | 338,07407 | 1,3477643 | 470,20908 | MPZL1 | BF978611 | Myelin protein zero-like 1 |
| 201877_s_at | 3,88E-06 | 0,97450006 | 2325,9558 | 0,6839644 | 1642,7365 | PPP2R5C | NM_002719 | Protein phosphatase 2, regulatory subunit B (B56), gamma isoform |
| 201880_at | 8,68E-07 | 0,9630877 | 959,4519 | 0,6800893 | 682,97723 | ARIH1 | NM_005744 | Ariadne homolog, ubiquitin-conjugating enzyme E2 binding protein, 1 (Drosophila) |
| 201881_s_at | 0,000146 | 0,9400778 | 306,4222 | 1,1909146 | 389,55905 | ARIH1 | NM_005744 | Ariadne homolog, ubiquitin-conjugating enzyme E2 binding protein, 1 (Drosophila) |
| 201889_at | 4,35E-07 | 0,9455409 | 574,64075 | 0,5461173 | 337,89545 | FAM3C | NM_014888 | Family with sequence similarity 3, member C |
| 201892_s_at | 3,39E-06 | 1,0162967 | 1819,7372 | 0,68620586 | 1231,7183 | IMPDH2 | NM_000884 | IMP (inosine monophosphate) dehydrogenase 2 |
| 201898_s_at | 3,88E-10 | 0,9832801 | 902,06665 | 1,3414917 | 1216,8365 | UBE2A | AI126625 | Ubiquitin-conjugating enzyme E2A (RAD6 homolog) |
| 201917_s_at | 2,95E-06 | 0,99046075 | 493,60373 | 1,5811363 | 806,9 | FLJ10618 | AI694452 | Hypothetical protein FLJ10618 |
| 201918_at | 0,00352 | 0,94996756 | 537,1371 | 1,2622784 | 726,88635 | FLJ10618 | AI694452 | Hypothetical protein FLJ10618 |
| 201919_at | 3,88E-06 | 0,9382228 | 1343,3038 | 1,2365456 | 1766,4364 | FLJ10618 | AI694452 | Hypothetical protein FLJ10618 |
| 201930_at | 3,16E-07 | 0,958949 | 677,5778 | 0,65984696 homolog, | 472,8818 | MCM6 | NM_005915 | MCM6 minichromosome maintenance deficient 6 (MIS5 S. pombe) (S. cerevisiae) |
| 201936_s_at | 0,000656 | 1,1275477 | 243,12593 | 0,7101114 | 153,08182 | EIF4G3 | NM_003760 | Eukaryotic translation initiation factor 4 gamma, 3 |
| 201947_s_at | 1,05E-08 | 0,9849543 | 2292,6667 | 0,7702108 | 1791,7228 | CCT2 | NM_006431 | Chaperonin containing TCP1, subunit 2 (beta) |
| 201963_at | 4,05E-05 | 0,9918443 | 1107,7407 | 1,4820207 | 1670,0272 | ACSL1 | NM_021122 | synonyms: ACS1, LACS, FACL1, FACL2, LACS1, LACS2; long-chain acyl-CoA synthetase 2; fatty-acid-Coenzyme A ligase, long-chain 2; palmitoyl-CoA ligase 2; long-chain acyl-CoA synthetase 1; paltimoyl-CoA ligase 1; fatty-acid-Coenzyme A ligase, long-chain 1; I |
| 201964_at | 4,35E-07 | 0,9978282 | 890,4333 | 0,6409831 | 591,5818 | ALS4 | N64643 | Amyotrophic lateral sclerosis 4 |
| 201967_at | 2,44E-09 | 1,0187714 | 1442,5037 | 0,6376236 | 908,95 | RBM6 | NM_005777 | RNA binding motif protein 6 |
| 201970_s_at | 0,000238 | 1,0050485 | 657,00366 | 0,7947589 | 506,04092 | NASP | NM_002482 | Nuclear autoantigenic sperm protein (histone-binding) |
| 201971_s_at | 0,00378 | 1,0638455 | 428,86667 | 1,4237708 | 567,05457 | ATP6V1A | NM_001690 | ATPase, H+ transporting, lysosomal 70kDa, V1 subunit A |
| 201986_at | 0,00807 | 1,0864375 | 917,3518 | 0,86700827 | 715,8227 | THRAP1 | NM_005121 | Thyroid hormone receptor associated protein 1 |
| 201987_at | 3,88E-06 | 1,0229956 | 1258,1628 | 0,7406186 | 913,9409 | THRAP1 | NM_005121 | Thyroid hormone receptor associated protein 1 |
| 201990_s_at | 1,69E-05 | 1,053646 | 1039,7853 | 0,81340414 | 804,0818 | CREBL2 | NM_001310 | CAMP responsive element binding protein-like 2 |
| 201995_at | 2,28E-07 | 1,0019108 | 208,7667 | 1,4002872 | 293,93637 | EXT1 | NM_000127 | Exostoses (multiple) 1 |
| 201998_at | 4,63E-09 | 0,9760575 | 3200,704 | 0,6738963 | 2241,259 | SIAT1 | AI743792 | Sialyltransferase 1 (beta-galactoside alpha-2,6-sialyltransferase) |
| 201999_s_at | 9,65E-05 | 0,9745332 | 3172,0996 | 1,2422175 | 4093,7227 | TCTEL1 | NM_006519 | T-complex-associated-testis-expressed 1-like 1 |
| 202008_s_at | 0,000119 | 0,90771085 | 154,95183 | 1,408933 | 242,75455 | NID | NM_002508 | Nidogen (enactin) |
| 202014_at | 3,62E-06 | 0,99964213 | 604,53705 | 1,7827573 | 1069,5135 | PPP1R15A | NM_014330 | Protein phosphatase 1, regulatory (inhibitor) subunit 15A |
| 202021_x_at | 1,38E-07 | 1,0643113 | 14814,038 | 1,4410651 | 20036,992 | SUI1 | AF083441 | Putative translation initiation factor |
| 202028_s_at | 6,30E-05 | 0,9948257 | 796,66296 | 1,5584217 | 1256,6909 | RPL38 | BC000603 | Ribosomal protein L38 |
| 202032_s_at | 0,000549 | 0,9730354 | 947,563 | 1,2713555 | 1218,2274 | MAN2A2 | NM_006122 | Mannosidase, alpha, class 2A, member 2 |
| 202033_s_at | 2,27E-05 | 1,0175649 | 883,7926 | 0,7250701 | 636,2637 | RB1CC1 | BG402105 | RB1-inducible coiled-coil 1 |
| 202034_x_at | 0,00164 | 1,0494391 | 326,3926 | 0,76407117 | 232,04544 | RB1CC1 | NM_014781 | RB1-inducible coiled-coil 1 |
| 202038_at | 5,78E-06 | 0,98784727 | 1992,4073 | 0,76371425 | 1548,4727 | UBE4A | NM_004788 | Ubiquitination factor E4A (UFD2 homolog, yeast) |
| 202047_s_at | 5,71 E-09 | 0,9606522 | 1076,0555 | 1,3375646 | 1511,7998 | CBX6 | AI458128 | Chromobox homolog 6 |
| 202056_at | 3,88E-06 | 0,99730474 | 342,32962 | 0,70826733 | 245,55455 | KPNA1 | NM_002264 | Karyopherin alpha 1 (importin alpha 5) |
| 202060_at | 1,63E-11 | 0,9790125 | 1264,4742 | 0,56019104 | 737,5545 | SH2BP1 | NM_014633 | SH2 domain binding protein 1 (tetratricopeptide repeat containing) |
| 202070_s_at | 0,000715 | 0,8998131 | 450,77036 | 1,1803452 | 574,1364 | IDH3A | NM_005530 | Isocitrate dehydrogenase 3 (NAD+) alpha |
| 202076_at | 5,78E-06 | 0,9754029 | 2240,3108 | 0,7352276 | 1699,5681 | BIRC2 | NM_001166 | Baculoviral IAP repeat-containing 2 |
| 202082_s_at | 0,00208 | 0,9371691 | 344,7778 | 1,2193642 | 450,18182 | SEC14L1 | NM_003003 | SEC14-like 1 (S. cerevisiae) |
| 202083_s_at | 0,000262 | 1,0026557 | 171,85925 | 1,4515847 | 260,10452 | SEC14L1 | NM_003003 | SEC14-like 1 (S. cerevisiae) |
| 202088_at | 5,06E-05 | 1,0121424 | 1150,1 | 0,7759123 | 898,49536 | LIV-1 | A1635449 | Transcribed locus |
| 202096_s_at | 5,07E-06 | 1,036346 | 4890,0405 | 1,4368664 | 6764,681 | BZRP | NM_000714 | Benzodiazapine receptor (peripheral) |
| 202122_s_at | 4,52E-05 | 1,0186639 | 944,0926 | 1,3120134 | 1224,2771 | M6PRBP1 | NM_005817 | Mannose-6-phosphate receptor binding protein 1 |
| 202126_at | 3,88E-06 | 0,9776025 | 1103,9147 | 0,70445377 | 811,9909 | PRPF4B | AA156948 | PRP4 pre-mRNA processing factor 4 homolog B (yeast) |
| 202129_s_at | 0,00919 | 0,9041293 | 676,7037 | 1,2038361 | 914,3864 | RIOK3 | AW006290 | RIO kinase 3 (yeast) |
| 202136_at | 5,07E-06 | 0,976774 | 1517,4222 | 0,7289535 | 1146,6638 | ZMYND11 | BE250417 | Zinc finger, MYND domain containing 11 |
| 202145_at | 0,00304 | 0,9788324 | 1228,711 | 1,479134 | 2275,8682 | LY6E | NM_002346 | Lymphocyte antigen 6 complex, locus E |
| 202149_at | 3,74E-09 | 0,9770013 | 596,2481 | 0,7011998 | 430,58633 | dJ761I2.1 | AL136139 | |
| 202152_x_at | 0,00979 | 0,9724188 | 1010,4667 | 1,2256902 | 1274,3226 | USF2 | NM_003367 | Upstream transcription factor 2, c-fos interacting |
| 202163_s_at | 8,24E-08 | 1,0630932 | 547,774 | 0,72882307 | 367,09543 | CNOT8 | NM_004779 | CCR4-NOT transcription complex, subunit 8 |
| 202169_s_at | 1,05E-08 | 0,9672459 | 1022,763 | 0,5746553 | 613,89087 | AASDHPPT | AF302110 | Aminoadipate-semialdehyde dehydrogenase-phosphopantetheinyl transferase |
| 202172_at | 1,56E-08 | 1,0543224 | 789,6963 | 0,6806696 | 511,00906 | ZNF161 | NM_007146 | Zinc finger protein 161 |
| 202174_s_at | 1,25E-09 | 0,9871436 | 1032,9294 | 0,5944152 | 617,44543 | PCM1 | | NM_006197 Pericentriolar material 1 |
| 202180_s_at | 0,000238 | 1,0253328 | 1700,374 | 1,3391023 | 2253,973 | MVP | NM_017458 | Major vault protein |
| 202184_s_at | 3,88E-06 | 0,97576684 | 593,837 | 0,64019674 | 378,0364 | NUP133 | NM_018230 | Nucleoporin 133kDa |
| 202191_s_at | 0,00109 | 1,0954328 | 1139,9926 | 1,6114064 | 1665,0863 | GAS7 | NM_005890 | synonyms: MGC1348, KIAA0394; isoform b is encoded by transcript variant b; go_function: transcription factor activity [goid 0003700] [evidence TAS] [pmid 9736752]; go_process: development [goid 0007275] [evidence IEA]; go_process: neurogenesis [goid 00073 |
| 202194_at | 1,24E-05 | 0,9705794 | 1825,4519 | 0,70803 | 1344,3774 | CGI-100 | AL117354 | |
| 202197_at | 9,15E-05 | 0,9447259 | 939,30005 | 1,1985025 | 1160,3865 | MTMR3 | NM_021090 | Myotubularin related protein 3 |
| 202201_at | 0,000178 | 0,98595357 | 1230,963 | 1,2998526 | 1666,3319 | BLVRB | NM_000713 | Biliverdin reductase B (flavin reductase (NADPH)) |
| 202205_at | 0,000382 | 1,0330527 | 1319,5259 | 1,5152283 | 1941,3497 | VASP | NM_003370 | Vasodilator-stimulated phosphoprotein |
| 202214_s_at | 5,76E-08 | 0,9934712 | 1016,44434 | 0,7282338 | 748,3091 | CUL4B | NM_003588 | Cullin 4B |
| 202225_at | 7,07E-13 | 1,0313045 | 337,61105 | 0,68488634 | 223,55455 | CRK | NM_016823 | V-crk sarcoma virus CT10 oncogene homolog (avian) |
| 202227_s_at | 3,04E-10 | 0,9597996 | 768,46295 | 0,6381114 | 510,92728 | BRD8 | NM_006696 | Bromodomain containing 8 |
| 202245_at | 0,00538 | 0,9381215 | 669,50366 | 1,198775 | 840,59546 | LSS | AW084510 | Lanosterol synthase (2,3-oxidosqualene-lanosterol cyclase) |
| 202251_at | 7,80E-05 | 1,0330714 | 479,58145 | 0,7387061 | 344,54092 | PRPF3 | NM_004698 | PRP3 pre-mRNA processing factor 3 homolog (yeast) |
| 202253_s_at | 0,00085 | 1,0057279 | 660,67035 | 1,3127002 | 880,36816 | DNM2 | NM_004945 | Dynamin 2 |
| 202266_at | 7,00E-09 | 0,9790363 | 1452,8148 | 0,6245043 | 928,2318 | TTRAP | NM_016614 | TRAF and TNF receptor associated protein |
| 202268_s_at | 1,38E-07 | 0,998851 | 1332,3964 | 0,6602552 | 890,4954 | APPBP1 | NM_003905 | Amyloid beta precursor protein binding protein 1, 59kDa |
| 202271_at | 1,79E-05 | 0,9603916 | 455,9001 | 0,65914375 | 313,86368 | FBXO28 | AB007952 | F-box protein 28 |
| 202272_s_at | 0,000317 | 0,9739378 | 445,7074 | 0,7119524 | 326,73633 | FBXO28 | NM_015176 | F-box protein 28 |
| 202275_at | 2,57E-06 | 1,0229657 | 768,5038 | 1,8844464 | 1451,2408 | G6PD | NM_000402 | Glucose-6-phosphate dehydrogenase |
| 202284_s_at | 1,22E-11 | 0,9747769 | 870,0222 | 2,2741055 | 2071,4182 | CDKN1A | NM_000389 | Cyclin-dependent kinase inhibitor 1A (p21, Cip1) |
| 202294_at | 0,00224 | 0,98579293 | 510,94446 | 0,7872304 | 415,9 | STAG1 | NM_005862 | Stromal antigen 1 |
| 202303_x_at | 0,000238 | 1,0310571 | 377,52597 | 0,6666375 | 240,78181 | SMARCA5 | NM_003601 | SWI/SNF related, matrix associated, actin dependent regulator of chromatin, subfamily a, member 5 |
| 202318_s_at | 1,81E-06 | 1,0048311 | 676,5074 | 0,75776845 | 515,5409 | SENP6 | AF306508 | SUMO1/sentrin specific protease 6 |
| 202321_at | 3,38E-06 | 0,9643617 | 173,38147 | 0,7025212 | 126,08635 | GGPS1 | AW299507 | Geranylgeranyl diphosphate synthase 1 |
| 202322_s_at | 2,38E-10 | 0,96380264 | 1088,9594 | 0,5837694 | 660,5046 | GGPS1 | NM_004837 | Geranylgeranyl diphosphate synthase 1 |
| 202324_s_at | 1,79E-05 | 1,0012443 | 815,6704 | 0,714576 | 591,94543 | ACBD3 | NM_022735 | Acyl-Coenzyme A binding domain containing 3 |
| 202330_s_at | 2,29E-08 | 0,9920462 | 210,51854 | 0,5930167 | 124,418175 | UNG | NM_003362 | Uracil-DNA glycosylase |
| 202336_s_at | 8,23E-05 | 0,9714011 | 469,74445 | 0,64090705 | 332,4818 | PAM | NM_000919 | Peptidylglycine alpha-amidating monooxygenase |
| 202346_at | 0,000459 | 0,9913964 | 483,91113 | 0,7884763 | 388,44547 | HIP2 | NM_005339 | Huntingtin interacting protein 2 |
| 202351_at | 2,87E-05 | 0,9747409 | 313,96664 | 0,71299535 | 227,46362 | ITGAV | AI093579 | Integrin, alpha V (vitronectin receptor, alpha polypeptide, antigen CD51) |
| 202365_at | 2,23E-06 | 0,93775934 | 565,3147 | 0,6447546 | 391,8591 | MGC5139 | BC004815 | Hypothetical protein MGC5139 |
| 202373_s_at | 2,28E-07 | 0,9753948 | 1157,4924 GAP150 | 0,7252422 | 862,11365 | RAB3- | AF255648 | Rab3 GTPase-activating protein, non-catalytic subunit (150kD) |
| 202374_s_at | 0,00755 | 1,0183579 | 404,23703 | 0,766299 | 316,4409 | RAB3- GAP150 | NM_012414 | Rab3 GTPase-activating protein, non-catalytic subunit (150kD) |
| 202381_at | 0,00109 | 1,0476936 | 432,67404 | 1,3181776 | 550,0681 | ADAM9 | NM_003816 | A disintegrin and metalloproteinase domain 9 (meltrin |
| 202386_s_at | 0,000317 | 0,9900868 | 1248,1704 | 0,7700231 | 965,2136 | LKAP | NM_019081 | Limkain b1 |
| 202388_at | 0,00119 | 1,0239632 | 8924,408 | 0,790993 | 6777,714 | RGS2 | NM_002923 | Regulator of G-protein signalling 2, 24kDa |
| 202391_at | 0,00282 | 0,98868376 | 2518,0813 | 1,2440742 | 3246,7908 | BASP1 | NM_006317 | Brain abundant, membrane attached signal protein 1 |
| 202393_s_at | 7,92E-10 | 0,9684237 | 3545,9443 | 1,6433018 | 5813,8364 | KLF10 | NM_005655 | Kruppel-like factor 10 |
| 202397_at | 0,00129 | 1,1562154 | 755,12964 | 1,8970674 | 1079,5591 | NUTF2 | NM_005796 | Nuclear transport factor 2 |
| 202401_s_at | 3,22E-05 | 0,97040683 | 280,25558 | 1,2525287 binding | 356,1 | SRF | NM_003131 | Serum response factor (c-fos serum response element-transcription factor) |
| 202413_s_at | 2,77E-08 | 0,9778298 | 1608,1593 | 0,66678184 | 1107,409 | USP1 | NM_003368 | Ubiquitin specific protease 1 |
| 202416_at | 0,00538 | 1,0170212 | 823,42596 | 1,2973423 | 1055,0908 | DNAJC7 | NM_003315 | DnaJ (Hsp40) homolog, subfamily C, member 7 |
| 202426_s_at | 1,68E-06 | 0,95674735 | 402,0074 | 1,581221 | 678,36365 | RXRA | NM_002957 | Retinoid X receptor, alpha |
| 202429_s_at | 1,85E-10 | 0,9586196 | 1470,7593 | 0,64196783 | 990,5318 | PPP3CA | AL353950 | Protein phosphatase 3 (formerly 2B), catalytic subunit, alpha isoform (calcineurin A alpha) |
| 202430_s_at | 0,00661 | 1,0368799 | 486,2296 | 1,363126 | 645,97723 | PLSCR1 | NM_021105 | Phospholipid scramblase 1 |
| 202431_s_at | 2,95E-06 | 0,96128374 | 1111,4296 | 0,5446967 | 636,4227 | MYC | NM_002467 | V-myc myelocytomatosis viral oncogene homolog (avian) |
| 202445_s_at | 1,25E-09 | 1,0059859 | 222,87405 | 1,4806868 | 324,83185 | NOTCH2 | NM_024408 | Notch homolog 2 (Drosophila) |
| 202446_s_at | 7,02E-05 | 0,9687274 | 1757,5074 | 1,4919062 | 2887,6138 | PLSCR1 | AI825926 | Phospholipid scramblase 1 |
| 202450_s_at | 0,000196 | 0,9656572 | 381,44443 | 1,309234 | 514,24536 | CTSK | NM_000396 | Cathepsin K (pycnodysostosis) |
| 202451_at | 1,57E-09 | 0,99827236 | 396,4741 | 0,5049722 | 204,82272 | GTF2H1 | BC000365 | General transcription factor IIH, polypeptide 1, 62kDa |
| 202453_s_at | 2,27E-05 | 0,9990303 | 400,5741 | 0,75895065 | 303,64093 | GTF2H1 | NM_005316 | General transcription factor IIH, polypeptide 1, 62kDa |
| 202454_s_at | 0,00861 | 1,0677482 | 90,90741 | 1,3958697 (avian) | 120,67728 | ERBB3 | NM_001982 | V-erb-b2 erythroblastic leukemia viral oncogene homolog 3 |
| 202464_s_at | 2,69E-07 | 0,9608148 | 359,51855 | 2,194377 | 929,59546 | PFKFB3 | NM_004566 | 6-phosphofructo-2-kinase/fructose-2,6-biphosphatase 3 |
| 202474_s_at | 1,40E-05 | 0,96397537 | 641,7888 | 1,2423062 | 827,39996 | HCFC1 | NM_005334 | Host cell factor C1 (VP16-accessory protein) |
| 202477_s_at | 1,40E-05 | 0,9785889 | 965,3073 | 1,2744365 | 1250,4591 | TUBGCP2 | NM_006659 | Tubulin, gamma complex associated protein 2 |
| 202478_at | 0,0014 | 0,9465306 | 1328,789 | 0,6453778 | 933,61365 | TRIB2 | NM_021643 | Tribbles homolog 2 (Drosophila) |
| 202481_at | 4,44E-06 | 0,97696674 | 457,45923 | 0,62728965 | 291,9636 | DHRS3 | NM_004753 | Dehydrogenase/reductase (SDR family) member 3 |
| 202491_s_at | 6,59E-06 | 1,012238 | 847,6704 | 0,74279857 | 618,6227 | IKBKAP | NM_003640 | Inhibitor of kappa light polypeptide gene enhancer in B-cells, kinase complex-associated protein |
| 202497_x_at | 3,62E-12 | 1,0646983 | 638,26294 | 2,8210952 | 1777,9045 | SLC2A3 | NM_006931 | Solute carrier family 2 (facilitated glucose transporter), member 3 |
| 202498_s_at | 5,08E-07 | 0,9357419 | 230,56668 | 2,029889 | 576,6136 | SLC2A3 | NM_006931 | Solute carrier family 2 (facilitated glucose transporter), member 3 |
| 202499_s_at | 4,94E-10 | 1,047085 | 1338,4 | 2,4583585 | 3225,277 | SLC2A3 | NM_006931 | Solute carrier family 2 (facilitated glucose transporter), member 3 |
| 202502_at | 8,68E-05 | 0,9405343 | 681,51855 | 0,6289723 | 461,3864 | ACADM | NM_000016 | Acyl-Coenzyme A dehydrogenase, C-4 to C-12 straight |
| 202510_s_at | 3,39E-06 | 0,9200859 | 3177,1929 | 1,4800683 | 5027,518 | TNFAIP2 | NM_006291 | Tumor necrosis factor, alpha-induced protein 2 |
| 202519_at | 0,00618 | 0,9389696 | 1191,5962 | 0,6938831 | 891,7727 | MONDOA | NM_014938 | Mlx interactor |
| 202521_at | 3,54E-13 | 0,98976773 | 1463,0225 | 0,7218413 | 1067,4773 | CTCF | NM_006565 | CCCTC-binding factor (zinc finger protein) |
| 202527_s_at | 1,26E-06 | 1,0133392 | 387,34814 | 0,5746211 | 228,15909 | SMAD4 | NM_005359 | SMAD, mothers against DPP homolog 4 (Drosophila) |
| 202531_at | 5,06E-05 | 0,99280864 | 2023,8778 | 1,2985219 | 2711,4685 | IRF1 | NM_002198 | Interferon regulatory factor 1 |
| 202536_at | 4,53E-05 | 1,0437725 | 584,67786 | 0,80243224 | 453,50452 | DKFZP564O 123 | AK002165 | DKFZP564O123 protein |
| 202540_s_at | 3,16E-07 | 0,9133055 | 294,03705 | 0,45874095 | 147,28181 | HMGCR | NM_000859 | 3-hydroxy-3-methylglutaryl-Coenzyme A reductase |
| 202557_at | 2,77E-08 | 1,0432832 | 351,97778 | 0,6008763 | 201,96819 | STCH | AI718418 | Stress 70 protein chaperone, microsome-associated, 60kDa |
| 202558_s_at | 0,00919 | 0,9185842 | 132,63335 | 1,2851489 | 191,72726 | STCH | NM_006948 | Stress 70 protein chaperone, microsome-associated, 60kDa |
| 202561_at | 0,000715 | 0,9787442 | 337,96664 | 0,74866533 | 256,70456 | TNKS | AF070613 | Tankyrase, TRF1-interacting ankyrin-related ADP-ribose polymerase |
| 202562_s_at | 0,00178 | 0,93469125 | 419,7148 | 0,71906275 | 333,28183 | C14orf1 | AL136658 | Chromosome 14 open reading frame 1 |
| 202594_at | 0,000459 | 1,0566347 | 1902,0962 | 0,74794024 | 1329,3909 | LEPROTL1 | NM_015344 | Leptin receptor overlapping transcript-like 1 |
| 202606_s_at | 1,79E-05 | 0,99092436 | 1233,2964 | 0,72822356 | 906,4636 | TLK1 | NM_012290 | Tousled-like kinase 1 |
| 202610_s_at | 6,64E-05 | 1,0318984 | 252,82594 | 0,6437779 2, 150kDa | 164,02725 | CRSP2 | AF135802 | Cofactor required for Sp1 transcriptional activation, subunit |
| 202626_s_at | 0,00129 | 0,9757152 | 3993,3037 | 1,2999232 | 5100,7456 | LYN | NM_002350 | V-yes-1 Yamaguchi sarcoma viral related oncogene |
| 202630_at | 9,06E-12 | 0,97073954 | 345,35928 | 0,45248964 | 164,60455 | APPBP2 | AA046411 | Amyloid beta precursor protein (cytoplasmic tail) binding protein 2 |
| 202633_at | 1,17E-07 | 1,017545 | 748,3074 | 0,7914722 | 586,6318 | TOPBP1 | NM_007027 | Topoisomerase (DNA) II binding protein 1 |
| 202635_s_at | 0,000138 | 0,9838186 | 583,88885 | 0,7764524 | 463,26813 | POLR2K | NM_005034 | Polymerase (RNA) II (DNA directed) polypeptide K, 7.0kDa |
| 202637_s_at | 6,53E-11 | 1,0409771 | 522,87775 | 1,8513285 | 914,84546 | ICAM1 | AI608725 | Intercellular adhesion molecule 1 (CD54), human rhinovirus receptor |
| 202638_s_at | 1,63E-11 | 0,9437592 | 293,67407 | 1,7901559 | 551,94995 | ICAM1 | NM_000201 | Intercellular adhesion molecule 1 (CD54), human rhinovirus receptor |
| 202640_s_at | 0,00208 | 0,9338758 | 355,99634 | 0,73867327 | 274,8591 | RANBP3 | NM_003624 | RAN binding protein 3 |
| 202642_s_at | 0,000549 | 0,99394584 | 616,57404 | 0,79308385 | 493,76364 | TRRAP | NM_003496 | Transformation/transcription domain-associated protein |
| 202643_s_at | 2,28E-07 | 1,2393924 | 1403,0409 | 2,8812716 | 3012,6772 | TNFAIP3 | AI738896 | Tumor necrosis factor, alpha-induced protein 3 |
| 202644_s_at | 1,94E-07 | 1,1101542 | 3577,6667 | 2,2692 | 7068,455 | TNFAIP3 | NM_006290 | Tumor necrosis factor, alpha-induced protein 3 |
| 202653_s_at | 0,000146 | 1,015888 | 1091,9409 | 0,67426205 | 754,6818 | AXOT | BC003404 | Membrane-associated ring finger (C3HC4) 7 |
| 202654_x_at | 0,00861 | 0,95252603 | 617,1186 | 0,7096626 | 478,5864 | AXOT | NM_022826 | Membrane-associated ring finger (C3HC4) 7 |
| 202656_s_at | 0,00101 | 1,1195729 | 1229,2853 | 1,4886601 | 1618,5045 | SERTAD2 | BG107456 | SERTA domain containing 2 |
| 202657_s_at | 4,35E-07 | 0,97430253 | 852,70734 | 1,5239964 | 1361,7773 | SERTAD2 | NM_014755 | SERTA domain containing 2 |
| 202663_at | 6,90E-08 | 1,0378739 | 757,95557 | 0,55739564 | 390,17273 | WASPIP | AI005043 | Wiskott-Aldrich syndrome protein interacting protein |
| 202664_at | 7,01E-09 | 1,0185959 | 3771,2627 | 0,62828016 | 2313,4998 | WASPIP | AI005043 | Wiskott-Aldrich syndrome protein interacting protein |
| 202666_s_at | 1,64E-07 | 0,9259245 | 273,84073 | 0,54136515 | 161,58183 | ACTL6A | NM_004301 | Actin-like 6A |
| 202671_s_at | 8,06E-07 | 1,0117295 | 680,51483 | 1,4189376 | 964,89545 | PDXK | NM_003681 | Pyridoxal (pyridoxine, vitamin B6) kinase |
| 202672_s_at | 1,56E-08 | 0,9962252 | 224,37407 | 2,3119717 | 556,5364 | ATF3 | NM_001674 | Activating transcription factor 3 |
| 202673_at | 6,92E-07 | 1,01997 | 1274,5334 | 0,78169805 | 978,19543 | DPM1 | NM_003859 | Dolichyl-phosphate mannosyltransferase polypeptide 1, catalytic subunit |
| 202689_at | 0,00152 | 0,96884716 | 604,8852 | 1,2523429 | 783,4954 | RBM15B | NM_013286 | RNA binding motif protein 15B |
| 202693_s_at | 7,92E-10 | 0,9435052 | 1902,3667 | 0,48402593 | 987,89996 | STK17A | NM_004760 | Serine/threonine kinase 17a (apoptosis-inducing) |
| 202695_s_at | 1,17E-07 | 0,97111815 | 454,0259 | 0,6262709 | 298,5546 | STK17A | NM_004760 | Serine/threonine kinase 17a (apoptosis-inducing) |
| 202704_at | 0,00406 | 1,0058854 | 2297,47 | 1,2645943 | 2776,4863 | TOB1 | AA675892 | Transducer of ERBB2, 1 |
| 202706_s_at | 7,50E-06 | 0,8874496 | 317,58154 | 0,58042586 | 210,23183 | UMPS | D86227 | Uridine monophosphate synthetase (orotate phosphoribosyl transferase and orotidine-5'-decarboxylase) |
| 202710_at | 1,38E-12 | 0,9734927 | 392,67773 | 0,431754 | 170,01364 | BET1 | BC000899 | BET1 homolog (S. cerevisiae) |
| 202717_s_at | 0,00178 | 0,9618352 | 841,0222 | 0,7694557 | 664,4409 | CDC16 | NM_003903 | CDC16 cell division cycle 16 homolog (S. cerevisiae) |
| 202720_at | 1,59E-05 | 0,96573025 | 2742,026 | 0,68431747 | 1982,6184 | TES | NM_015641 | Testis derived transcript (3 LIM domains) |
| 202724_s_at | 3,88E-06 | 0,96672827 | 854,7223 | 0,63803273 | 583,5045 | FOXO1A | NM_002015 | Forkhead box O1A (rhabdomyosarcoma) |
| 202734_at | 0,00078 | 0,8549906 | 188,1074 | 1,2324792 | 256,5545 | TRIP10 | NM_004240 | Thyroid hormone receptor interactor 10 |
| 202735_at | 1,24E-05 | 1,146861 | 369,65552 | 0,71584314 | 221,79546 | EBP | NM_006579 | Emopamil binding protein (sterol isomerase) |
| 202741_at | 6,71E-12 | 0,98023903 | 4130,0967 | 0,46846685 | 2009,9548 | PRKACB | AA130247 | Protein kinase, cAMP-dependent, catalytic, beta |
| 202742_s_at | 7,50E-06 | 0,98316693 | 676,34814 | 0,5091942 | 379,83636 | PRKACB | NM_002731 | Protein kinase, cAMP-dependent, catalytic, beta |
| 202749_at | 4,02E-08 | 0,95604277 | 536,07776 | 0,59483635 | 333,3045 | WRB | NM_004627 | Tryptophan rich basic protein |
| 202779_s_at | 4,05E-05 | 1,142385 | 378,0111 | 1,6311637 | 529,32275 | UBE2S | NM_014501 | Ubiquitin-conjugating enzyme E2S |
| 202781_s_at | 9,65E-05 | 0,99014264 | 577,42975 | 1,3030119 | 744,27734 | SKIP | AI806031 | Skeletal muscle and kidney enriched inositol phosphatase |
| 202786_at | 0,000146 | 1,0127823 | 465,1704 | 0,7503901 | 348,33633 | STK39 | NM_013233 | Serine threonine kinase 39 (STE20/SPS1 homolog, yeast) |
| 202788_at | 6,30E-05 | 0,98696953 | 2381,529 | 1,2559692 | 3006,6228 | MAPKAPK3 | NM_004635 | Mitogen-activated protein kinase-activated protein kinase 3 |
| 202789_at | 0,00406 | 1,0017363 | 674,32965 | 0,67628086 | 492,3909 | | AL022394 | |
| 202797_at | 4,99E-11 | 1,0653548 | 1448,0963 | 0,5899879 | 803,8091 | SACM1L | NM_014016 | SAC1 suppressor of actin mutations 1-like (yeast) |
| 202801_at | 0,00224 | 1,0228817 | 539,85187 | 1,4054021 | 750,4272 | PRKACA | NM_002730 | Protein kinase, cAMP-dependent, catalytic, alpha |
| 202812_at | 0,00078 | 0,95250726 | 1367,911 | 1,293069 | 1827,3773 | GAA | NM_000152 | Glucosidase, alpha; acid (Pompe disease, glycogen storage disease type II) |
| 202814_s_at | 0,000161 | 1,0183332 | 415,85187 | 0,78748596 | 323,42273 | HIS1 | AW193511 | HMBA-inducible |
| 202818_s_at | 0,000348 | 1,0349036 | 356,99258 | 1,4357736 (110kDa, | 498,51355 | TCEB3 | AI344128 | Transcription elongation factor B (SIII), polypeptide 3 elongin A) |
| 202820_at | 0,00365 | 1,002147 | 403,163 | 0,7615475 | 301,71817 | AHR | NM_001621 | Aryl hydrocarbon receptor |
| 202832_at | 0,000317 | 0,9136283 | 1070,6259 | 0,633983 | 730,14545 | GCC2 | NM_014635 | GRIP and coiled-coil domain containing 2 |
| 202841_x at | 3,39E-06 | 1,0094877 | 961,21106 | 1,3672463 | 1313,4454 | OGFR | NM_007346 | Opioid growth factor receptor |
| 202842_s_at | 4,05E-05 | 1,0135684 | 1211,774 | 1,3720074 | 1636,4955 | DNAJB9 | AL080081 | DnaJ (Hsp40) homolog, subfamily B, member 9 |
| 202846_s_at | 1,24E-05 | 0,97493505 | 710,6815 | 0,70127696 | 499,0909 | PIGC | NM_002642 | Phosphatidylinositol glycan, class C |
| 202850_at | 1,28E-08 | 1,008869 | 711,28516 | 0,57343954 | 420,44995 | ABCD3 | NM_002858 | ATP-binding cassette, sub-family D (ALD), member 3 |
| 202853_s_at | 4,34E-07 | 1,0016005 | 473,94812 | 0,7411313 | 348,05453 | RYK | NM_002958 | RYK receptor-like tyrosine kinase |
| 202861_at | 1,22E-11 | 1,0865383 | 453,863 | 3,9906387 | 1669,2998 | PER1 | NM_002616 | Period homolog 1 (Drosophila) |
| 202877_s_at | 0,00661 | 0,9296173 | 545,5666 | 1,2551627 | 715,29095 | C1QR1 | W72082 | Complement component 1, q subcomponent, receptor 1 |
| 202887_s_at | 3,34E-08 | 1,0380032 | 1705,4777 | 2,2940643 | 3534,3228 | DDIT4 | NM_019058 | DNA-damage-inducible transcript 4 |
| 202888_s_at | 0,00129 | 0,93996346 | 628,34814 | 1,6081569 | 1001,0363 | ANPEP | NM_001150 | Alanyl (membrane) aminopeptidase (aminopeptidase N, aminopeptidase M, microsomal aminopeptidase, CD13, |
| 202892_at | 2,87E-05 | 0,9387734 | 410,22595 | 0,7100713 | 309,44092 | CDC23 | NM_004661 | CDC23 (cell division cycle 23, yeast, homolog) |
| 202895_s_at | 0,000419 | 0,9329813 | 185,84444 | 1,4323627 | 298,40912 | PTPNS1 | D86043 | Protein tyrosine phosphatase, non-receptor type substrate 1 |
| 202896_s_at | 4,05E-05 | 0,96835923 | 635,88153 | 1,3245438 | 882,25 | PTPNS1 | NM_004648 | synonyms: BIT, MFR, P84, SIRP, MYD-1, SHPS1, SHPS-1, SIRPalpha, SIRPalpha2, SIRP-ALPHA-1; myd-1 antigen; signal regulatory protein, alpha type 2; SHP substrate-1; brain-immunoglobulin-like molecule with tyrosine-based activation motifs; signal regulatory |
| 202897_at | 3,22E-05 | 0,9624587 | 976,2 | 1,3448828 | 1385,0317 | PTPNS1 | AB023430 | Protein tyrosine phosphatase, non-receptor type substrate 1 |
| 202900_s_at | 0,000238 | 1,0500642 | 685,77405 | 0,8032802 | 522,76825 | NUP88 | NM_002532 | Nucleoporin 88kDa |
| 202902_s_at | 0,000131 | 1,0513055 | 12322,325 | 1,3615466 | 15904,499 | CTSS | NM_004079 | Cathepsin S |
| 202906_s_at | 0,00577 | 0,96505815 | 583,55554 | 0,76193887 | 474,1091 | NBS1 | AI796269 | Nijmegen breakage syndrome 1 (nibrin) |
| 202911_at | 2,02E-05 | 0,9606382 | 691,9259 | 0,75835437 | 547,45 | MSH6 | NM_000179 | MutS homolog 6 (E. coli) |
| 202912_at | 4,93E-12 | 0,9875565 | 285,7185 | 2,798082 | 838,2637 | ADM | NM_001124 | Adrenomedullin |
| 202918_s_at | 4,35E-07 | 1,0146581 | 723,9223 | 0,7307607 | 526,75 | PREI3 | AF151853 | Preimplantation protein 3 |
| 202924_s_at | 6,71 E-12 | 0,93108314 | 309,5148 | 1,5240024 | 502,33633 | PLAGL2 | AL562280 | Pleiomorphic adenoma gene-like 2 |
| 202930_s_at | 2,27E-05 | 0,97662634 | 372,7815 | 0,7028876 | 270,41815 | SUCLA2 | NM_003850 | Succinate-CoA ligase, ADP-forming, beta subunit |
| 202943_s_at | 0,00085 | 1,0135581 | 1317,5594 | 1,2840145 | 1667,741 | NAGA | M38083 | N-acetylgalactosaminidase, alpha- |
| 202944_at | 9,36E-07 | 1,0293252 | 1018,15186 | 1,4894457 | 1462,0455 | NAGA | NM_000262 | N-acetylgalactosaminidase, alpha- |
| 202950_at | 1,05E-08 | 1,0179362 | 514,7889 | 0,59251386 | 303,93182 | CRYZ | NM_001889 | Crystallin, zeta (quinone reductase) |
| 202951_at | 7,02E-05 | 1,0090624 | 3865,048 | 0,7814543 | 2990,8545 | STK38 | BE048506 | Serine/threonine kinase 38 |
| 202956_at | 6,16E-06 | 1,0206624 | 924,63715 | 0,76075256 | 685,0318 | ARFGEF1 | NM_006421 | ADP-ribosylation factor guanine nucleotide-exchange factor 1 (brefeldin A-inhibited) |
| 202959_at | 0,00406 | 0,92848647 | 216,3741 | 0,72505486 | 168,81819 | MUT | AI433712 | Methylmalonyl Coenzyme A mutase |
| 202960_s_at | 5,94E-07 | 0,9445777 | 364,02222 | 0,57974786 | 235,2182 | MUT | NM_000255 | Methylmalonyl Coenzyme A mutase |
| 202969_at | 1,89E-08 | 0,99560905 | 506,24814 | 0,5497994 | 275,2091 | DYRK2 | Y09216 | Dual-specificity tyrosine-(Y)-phosphorylation regulated |
| 202970_at | 6,59E-06 | 0,9613753 | 280,36664 | 0,58071446 | 175,07272 | DYRK2 | Y09216 | Dual-specificity tyrosine-(Y)-phosphorylation regulated |
| 202983_at | 9,93E-13 | 0,9846736 | 503,9889 | 0,49123746 | 254,05002 | SMARCA3 | AI760760 | SWI/SNF related, matrix associated, actin dependent regulator of chromatin, subfamily a, member 3 |
| 203008_x_at | 3,88E-10 | 1,0365083 | 1121,3002 | 0,6361453 | 698,6773 | TXNDC9 | NM_005783 | Thioredoxin domain containing 9 |
| 203011_at | 2,69E-07 | 0,9699919 | 975,9185 | 0,6875143 | 705,10913 | IMPA1 | NM_005536 | Inositol(myo)-1(or4)-monophosphatase 1 |
| 203020_at | 1,56E-08 | 1,0111244 | 1133,9481 | 0,7047746 | 794,49097 | RABGAP1L | NM_014857 | RAB GTPase activating protein 1-like |
| 203023_at | 8,68E-05 | 0,9563204 | 173,7259 | 0,69801044 | 131,1182 | HSPC111 | NM_016391 | Hypothetical protein HSPC111 |
| 203026_at | 0,00755 | 0,9499978 | 519,69257 | 1,2111583 | 670,3682 | ZBTB5 | NM_014872 | Zinc finger and BTB domain containing 5 |
| 203037_s_at | 1,72E-13 | 1,0101433 | 2511,6445 | 0,5473882 | 1357,3864 | MTSS1 | NM_014751 | Metastasis suppressor 1 |
| 203045_at | 1,89E-08 | 0,9234391 | 939,28156 | 1,6124197 | 1661,7864 | NINJ1 | NM_004148 | Ninjurin 1 |
| 203049_s_at | 5,78E-06 | 0,96465445 | 424,44443 | 0,63894576 | 281,24545 | KIAA0372 | NM_014639 | KIAA0372 |
| 203051_at | 0,000153 | 1,0064437 | 307,54443 | 0,7807118 | 237,41821 | BAHD1 | NM_014952 | Bromo adjacent homology domain containing 1 |
| 203060_s_at | 0,000119 | 1,0817077 | 185,94073 SK2; | 0,69330186 | 119,42272 | PAPSS2; ATPSK2 | AF074331 | Homo sapiens PAPS synthetase-2 (PAPSS2) mRNA, complete cds. |
| 203064_s_at | 4,82E-08 | 1,0032023 | 306,76666 | 1,4944321 | 449,0409 | FOXK2 | NM_004514 | Forkhead box K2 |
| 203074_at | 0,000161 | 0,87488085 | 274,7815 | 1,2510664 | 379,14093 | ANXA8 | NM_001630 | Annexin A8 |
| 203079_s_at | 0,00352 | 1,0072823 | 396,75186 | 0,791244 | 311,89093 | CUL2 | NM_003591 | Cullin 2 |
| 203082_at | 5,41 E-06 | 1,0238705 | 667,0852 | 0,765181 | 505,8227 | BMS1L | NM_014753 | BMS1-like, ribosome assembly protein (yeast) |
| 203086_at | 0,00468 | 0,99931186 | 406,34076 | 0,7927144 | 322,7 | HSA9761 | BE872563 | Putative dimethyladenosine transferase |
| 203087_s_at | 0,000216 | 1,012954 | 914,0408 | 0,7762735 | 716,37274 | KIF2 | NM_004520 | Kinesin heavy chain member 2 |
| 203095_at | 9,07E-06 | 1,0093305 | 641,65186 | 0,74261725 | 471,5318 | MTIF2 | NM_002453 | Mitochondrial translational initiation factor 2 |
| 203098_at | 0,00755 | 1,0652183 | 334,3556 | 0,7578216 | 249,41817 | CDYL | AL050164 | Chromodomain protein, Y-like |
| 203102_s_at | 1,43E-10 | 0,9636995 | 911,4963 | 0,39211872 | 385,8091 | MGAT2 | NM_002408 | Mannosyl (alpha-1,6-)-glycoprotein beta-1,2-N-acetylglucosaminyltransferase |
| 203109_at | 0,00755 | 1,0895478 | 541,98895 | 1,43462 | 697,4136 | UBE2M | NM_003969 | Ubiquitin-conjugating enzyme E2M (UBC12 homolog, yeast) |
| 203123_s_at | 2,95E-06 | 0,89209425 | 312,58148 | 1,2939347 | 445,22275 | SLC11A2 | AU154469 | Solute carrier family 11 (proton-coupled divalent metal ion transporters), member 2 |
| 203124_s_at | 0,00661 | 0,9957223 | 261,74075 | 1,2841849 | 332,0182 | SLC11A2 | NM_000617 | Solute carrier family 11 (proton-coupled divalent metal ion transporters), member 2 |
| 203126_at | 3,16E-07 | 0,9814559 | 754,1295 | 1,5531262 | 1202,1136 | IMPA2 | NM_014214 | Inositol(myo)-1(or4)-monophosphatase 2 |
| 203127_s_at | 2,38E-10 | 0,97180384 | 1066,8073 | 1,4702554 | 1635,8593 | SPTLC2 | BC005123 | Serine palmitoyltransferase, long chain base subunit 2 |
| 203137_at | 5,07E-06 | 1,0075976 | 1822,4816 | 0,7842226 | 1433,4456 | WTAP | NM_004906 | Wilms tumor 1 associated protein |
| 203138_at | 1,92E-12 | 1,0178207 | 717,47406 | 0,6005414 | 424,90002 | HAT1 | NM_003642 | Histone acetyltransferase 1 |
| 203140_at | 3,03E-09 | 0,9484933 | 1628,7482 | 1,7535702 | 2911,0227 | BCL6 | NM_001706 | B-cell CLL/lymphoma 6 (zinc finger protein 51) |
| 203146_s_at | 0,000656 | 0,8687248 | 874,57043 | 1,2959452 | 1240,4088 | GABBR1 | NM_001470 | Gamma-aminobutyric acid (GABA) B receptor, 1 |
| 203148_s_at | 5,71E-09 | 0,95866066 | 1234,5332 | 0,5711226 | 754,7818 | TRIM14 | NM_014788 | Tripartite motif-containing 14 |
| 203150_at | 0,00114 | 0,9702607 | 322,87408 | 0,76826143 | 259,8409 | RAB9P40 | NM_005833 | Rab9 effector p40 |
| 203156_at | 0,000238 | 1,0082703 | 1396,4333 | 0,7847548 | 1099,3135 | AKAP11 | NM_016248 | A kinase (PRKA) anchor protein 11 |
| 203157_s_at | 0,000348 | 1,1055926 | 374,60367 | 0,7655018 | 264,8182 | GLS | AB020645 | Glutaminase |
| 203158_s_at | 0,00807 | 1,0139403 | 189,26666 | 0,7618233 | 141,96362 | GLS | AF097493 | Glutaminase |
| 203159_at | 5,78E-08 | 0,93976194 | 1039,1888 | 0,6017023 | 662,05 | GLS | NM_014905 | Glutaminase |
| 203165_s_at | 1,94E-06 | 1,005895 | 122,78148 | 0,6868843 | 83,931816 | SLC33A1 | NM_004733 | Solute carrier family 33 (acetyl-CoA transporter), member 1 |
| 203167_at | 1,79E-05 | 1,0224354 | 592,2074 | 1,4624978 | 868,0273 | TIMP2 | NM_003255 | Tissue inhibitor of metalloproteinase 2 |
| 203169_at | 0,00661 | 1,0214165 | 378,3185 | 0,7817143 | 303,8682 | KIAA0258 | NM_014785 | |
| 203175_at | 1,05E-08 | 0,99880236 | 4769,959 | 1,4167831 | 6761,3813 | RHOG | NM_001665 | Ras homolog gene family, member G (rho G) |
| 203196_at | 0,00216 | 1,055188 | 206,73703 | 0,7988129 | 155,6727 | ABCC4 | AI948503 | ATP-binding cassette, sub-family C (CFTR/MRP), member |
| 203200_s_at | 8,59E-09 | 0,9883986 | 874,94824 | 0,6599473 | 589,1318 | MTRR | NM_024010 | 5-methyltetrahydrofolate-homocysteine methyltransferase reductase |
| 203201_at | 1,59E-05 | 1,0390605 | 318,96664 | 0,683614 | 210,64093 | PMM2 | NM_000303 | Phosphomannomutase 2 |
| 203202_at | 8,52E-06 | 1,002013 | 339,6407 | 0,638004 | 219,26364 | HRB2 | AI950314 | HIV-1 rev binding protein 2 |
| 203208_s_at | 0,00178 | 0,9706087 | 149,43333 | 0,68752253 | 102,93182 | CHPPR | NM_014637 | Likely ortholog of chicken chondrocyte protein with a polyproline region |
| 203211_s_at | 2,56E-05 | 1,0002215 | 207,91112 | 0,7422248 | 153,33182 | MTMR2 | AK027038 | Myotubularin related protein 2 |
| 203224_at | 6,90E-08 | 0,9958852 | 473,5297 | 0,6826188 | 324,74545 | RFK | BF340123 | Riboflavin kinase |
| 203234_at | 1,28E-08 | 0,9681056 | 638,4482 | 1,4500011 | 944,5818 | UPP1 | NM_003364 | Uridine phosphorylase 1 |
| 203236_s_at | 0,000146 | 1,041711 | 1012,237 | 1,5632302 | 1594,3999 | LGALS9 | NM_009587 | Lectin, galactoside-binding, soluble, 9 (galectin 9) |
| 203246_s_at | 0,000238 | 0,970727 | 1075,2704 | 0,685142 | 790,3636 | TUSC4 | NM_006545 | Tumor suppressor candidate 4 |
| 203253_s_at | 1,46E-06 | 0,9302394 | 631,4667 | 0,61838996 | 431,50906 | KIAA0433 | NM_015216 | KIAA0433 protein |
| 203276_at | 5,09E-07 | 0,96317184 | 313,0667 | 1,5114206 | 478,98187 | LMNB1 | NM_005573 | Lamin B1 |
| 203291_at | 8,68E-05 | 1,00541 | 285,7778 | 0,74971944 | 210,82727 | CNOT4 | | NM_013316 CCR4-NOT transcription complex, subunit 4 |
| 203302_at | 1,63E-11 | 1,0208229 | 830,0705 | 0,54795265 | 452,58182 | DCK | NM_000788 | Deoxycytidine kinase |
| 203304_at | 0,000849 | 0,98618346 | 91,31852 | 1,4026911 (Xenopus | 125,3636 | BAMBI | NM_012342 | BMP and activin membrane-bound inhibitor homolog laevis) |
| 203309_s_at | 0,0006 | 1,0254831 | 265,14444 | 1,2926149 | 329,92725 | HPS1 | NM_000195 | Hermansky-Pudlaksyndrome 1 |
| 203310_at | 0,00124 | 0,99139446 | 750,4815 | 0,7455329 | 573,5591 | STXBP3 | NM_007269 | Syntaxin binding protein 3 |
| 203311_s_at | 7,02E-05 | 0,942923 | 927,8778 | 1,4398619 | 1319,8363 | ARF6 | M57763 | ADP-ribosylation factor 6 |
| 203327_at | 1,24E-05 | 0,9761812 | 486,25186 | 0,74993026 | 375,15002 | IDE | N22903 | Insulin-degrading enzyme |
| 203334_at | 2,87E-05 | 0,93255955 | 338,80368 | 0,6850027 | 248,46364 | DHX8 | NM_004941 | DEAH (Asp-Glu-Ala-His) box polypeptide 8 |
| 203339_at | 6,30E-05 | 0,94434017 | 169,66666 | 0,6797891 | 122,83636 | SLC25A12 | AI887457 | Solute carrier family 25 (mitochondrial carrier, Aralar), member 12 |
| 203343_at | 0,00755 | 1,0274897 | 302,23703 | 0,81795853 | 246,07272 | UGDH | NM_003359 | UDP-glucose dehydrogenase |
| 203345_s_at | 7,99E-06 | 1,050168 | 1162,6185 | 0,7910062 | 858,42725 | M96 | A1566096 | Likely ortholog of mouse metal response element binding transcription factor 2 |
| 203347_s_at | 9,97E-10 | 0,95887583 | 210,44073 | 0,5171669 | 115,89091 | M96 | NM_007358 | Likely ortholog of mouse metal response element binding transcription factor 2 |
| 203366_at | 5,94E-07 | 0,98681635 | 506,18875 | 0,7523978 | 388,32724 | POLG | NM_002693 | Polymerase (DNA directed), gamma |
| 203370_s_at | 9,65E-05 | 0,9958516 | 230,77037 | 1,4242097 | 328,4136 | PDLIM7 | NM_005451 | PDZ and LIM domain 7 (enigma) |
| 203378_at | 3,71 E-07 | 0,96065927 | 694,152 | 0,62086993 | 448,95 | PCF11 | AB020631 | Pre-mRNA cleavage complex II protein Pcf11 |
| 203380_x_at | 2,27E-05 | 1,0024277 | 6833,6255 | 0,77961034 | 5258,691 | SFRS5 | NM_006925 | Splicing factor, arginine/serine-rich 5 |
| 203386_at | 0,000502 | 1,050933 | 661,60364 | 0,72749275 | 453,84088 | TBC1D4 | AI650848 | TBC1 domain family, member 4 |
| 203387_s_at | 0,00661 | 1,015145 | 632,8259 | 0,7501037 | 475,0637 | TBC1D4 | NM_014832 | TBC1 domain family, member 4 |
| 203388_at | 0,000459 | 1,0132676 | 2887,2925 | 1,3894184 | 4064,2864 | ARRB2 | NM_004313 | Arrestin, beta 2 |
| 203397_s_at | 0,00351 | 0,98633015 | 237,27777 | 0,7546546 | 178,28636 | GALNT3 | BF063271 | UDP-N-acetyl-alpha-D-galactosamine:polypeptide N-acetylgalactosaminyltransferase 3 (GalNAc-T3) |
| 203403_s_at | 9,06E-12 | 1,0394195 | 1255,3854 | 0,6614804 | 800,42737 | RNF6 | NM_005977 | Ring finger protein (C3H2C3 type) 6 |
| 203406_at | 5,09E-07 | 0,8586999 | 747,3482 | 0,5190862 | 432,31363 | MFAP1 | NM_005926 | Microfibrillar-associated protein 1 |
| 203413_at | 0,000178 | 1,0256761 | 1880,8964 | 0,68551236 | 1259,2318 | NELL2 | NM_006159 | NEL-like 2 (chicken) |
| 203427_at | 3,75E-14 | 0,98394966 | 656,8926 | 0,36861083 | 252,89091 | ASF1A | NM_014034 | ASF1 anti-silencing function 1 homolog A (S. cerevisiae) |
| 203428_s_at | 2,64E-12 | 1,0288419 | 433,48517 | 0,4765293 | 198,85909 | ASF1A | AB028628 | ASF1 anti-silencing function 1 homolog A (S. cerevisiae) |
| 203436_at | 2,72E-09 | 0,99717784 | 674,44446 | 0,7680814 | 519,60913 | RPP30 | NM_006413 | Ribonuclease P/MRP 30kDa subunit |
| 203449_s_at | 1,38E-12 | 1,0091074 | 408,47775 | 0,593306 | 244,23636 | TERF1 | NM_017489 | Telomeric repeat binding factor (NIMA-interacting) 1 |
| 203460_sat | 1,09E-06 | 1,0027255 | 1024,1149 | 0,7446809 | 771,5682 | PSEN1 | | NM_007318 Presenilin 1 (Alzheimer disease 3) |
| 203468_at | 7,40E-05 | 0,91973937 | 166,91483 | 0,52038467 | 108,13182 | CDK10 | NM_003674 | Cyclin-dependent kinase (CDC2-like) 10 |
| 203470_s_at | 0,000196 | 0,9870398 | 2253,3108 | 1,4815354 | 3347,5728 | PLEK | AI433595 | Pleckstrin |
| 203481_at | 0,000196 | 0,90869063 | 186,09631 | 0,665812 | 135,95453 | C10orf6 | AI655902 | Hypothetical protein LOC143286 |
| 203487_s_at | 1,28E-08 | 0,9547998 | 415,05927 | 0,6208995 | 266,60455 | ARMC8 | NM_015396 | Armadillo repeat containing 8 |
| 203490_at | 0,000459 | 0,9491285 | 497,7037 | 1,265274 | 648,5 | ELF4 | NM_001421 | E74-like factor 4 (ets domain transcription factor) |
| 203493_s_at | 6,30E-05 | 1,0222377 | 322,67776 | 0,7098257 | 221,87727 | PIG8 | AI123527 | Translokin |
| 203494_s_at | 2,95E-06 | 1,015183 | 878,6963 | 0,7082246 | 618,1273 | PIG8 | NM_014679 | Translokin |
| 203500_at | 1,40E-05 | 1,0347713 | 344,9852 | 0,78035724 | 258,54092 | GCDH | NM_000159 | Glutaryl-Coenzyme A dehydrogenase |
| 203509_at | 0,000459 | 1,0486062 | 6484,923 | 0,7550802 | 4642,691 | SORL1 | NM_003105 | Sortilin-related receptor, L(DLR class) A repeats-containing |
| 203523_at | 0,00538 | 0,9593601 | 2969,1853 | 1,2692122 | 3933,1414 | LSP1 | NM_002339 | Lymphocyte-specific protein 1 |
| 203525_s_at | 0,00146 | 0,9882769 | 243,26295 | 0,7362932 | 180,88637 | APC | AI375486 | Adenomatosis polyposis coli |
| 203526_s_at | 0,00025 | 1,0327724 | 193,60739 | 0,7561237 | 144,12276 | APC | M74088 | Adenomatosis polyposis coli |
| 203531_at | 4,05E-05 | 0,95840627 | 1001,0704 | 0,76306605 | 799,57733 | CUL5 | BF435809 | Cullin 5 |
| 203537_at | 8,24E-08 | 0,9781722 | 874,8222 | 0,698882 | 632,58636 | PRPSAP2 | NM_002767 | Phosphoribosyl pyrophosphate synthetase-associated |
| 203542_s_at | 4,53E-05 | 1,0681031 | 420,8481 | 2,0385208 | 808,83185 | KLF9 | BF438302 | Kruppel-like factor 9 |
| 203543_s_at | 0,00577 | 1,0795614 | 307,21115 | 1,596519 | 425,7 | KLF9 | NM_001206 | Kruppel-like factor 9 |
| 203556_at | 7,81E-05 | 0,9421885 | 784,1815 | 0,6732126 | 571,75 | ZHX2 | NM_014943 | Zinc fingers and homeoboxes 2 |
| 203566_s_at | 3,34E-08 | 0,9714065 | 468,7889 | 0,5329329 | 262,70456 | AGL | NM_000645 | Amylo-1, 6-glucosidase, 4-alpha-glucanotransferase (glycogen debranching enzyme, glycogen storage disease |
| 203574_at | 2,17E-11 | 1,0665983 | 887,41125 | 3,015483 | 2425,1638 | NFIL3 | NM_005384 | Nuclear factor, interleukin 3 regulated |
| 203583_at | 4,05E-05 | 0,9164908 | 1020,9741 | 0,66937745 | 735,0683 | UNC50 | NM_014044 | Unc-50 homolog (C. elegans) |
| 203584_at | 1,40E-05 | 0,9306103 | 363,2963 | 0,6778313 | 264,5 | KIAA0103 | NM_014673 | KIAA0103 |
| 203591_s_at | 5,07E-06 | 0,98817265 | 1385,5259 | 1,4547428 | 2015,6637 | CSF3R | NM_000760 | Colony stimulating factor 3 receptor (granulocyte) |
| 203593_at | 3.71E-07 | 0,9934564 | 352,67032 | 0,60444033 | 211,41817 | CD2AP | NM_012120 | CD2-associated protein |
| 203598_s_at | 0,0004 | 0,9535495 | 93,09259 | 0,7550881 | 72,54091 | WBP4 | NM_007187 | WW domain binding protein 4 (formin binding protein 21) |
| 203605_at | 1,38E-07 | 1,00834 | 757,86285 | 0,7479999 | 563,02277 | SRP54 | NM_003136 | Signal recognition particle 54kDa |
| 203611_at | 2,23E-06 | 1,037657 | 892,6704 | 0,7998739 | 690,0409 | TERF2 | NM_005652 | Telomeric repeat binding factor 2 |
| 203614_at | 5,71 E-09 | 0,89793265 | 535,45557 | 0,50219196 | 291,74088 | LOC440138 | NM_021645 | Similar to hypothetical protein B230397C21 |
| 203617_x_at | 0,0098 | 0,9909248 | 383,52222 | 1,2980717 | 520,0091 | ELK1 | NM_005229 | ELK1, member of ETS oncogene family |
| 203625_x_at | 7,50E-06 | 1,0191911 | 440,7371 | 0,7028441 | 313,06363 | SKP2 | BG105365 | S-phase kinase-associated protein 2 (p45) |
| 203640_at | 5,09E-07 | 0,9292938 | 459,32965 | 0,58896756 IMAGE:3145289 | 295,03177 | MBNL2 | BE328496 | hs98f09.x1 NCI_CGAP_Kid13 Homo sapiens cDNA clone 3', mRNA sequence. |
| 203646_at | 0,000317 | 1,0123695 | 278,21484 | 0,67105615 | 185,55455 | FDX1 | NM_004109 | Ferredoxin 1 |
| 203650_at | 6,29E-05 | 1,1939998 | 76,35186 | 0,6759145 | 44,499996 | PROCR | NM_006404 | Protein C receptor, endothelial (EPCR) |
| 203651_at | 3,22E-05 | 1,0383961 | 258,3704 | 0,719991 | 176,65909 | ZFYVE16 | NM_014733 | Zinc finger, FYVE domain containing 16 |
| 203659_s_at | 1,59E-05 | 0,9692402 | 549,59625 | 1,3628507 | 778,19086 | RFP2 | NM_005798 | Ret finger protein 2 |
| 203665_at | 0,000459 | 0,984966 | 1187,4221 | 1,4191387 | 1722,9591 | HMOX1 | NM_002133 | Heme oxygenase (decycling) 1 |
| 203666_at | 0,00282 | 0,9185271 | 153,18518 | 1,2870414 factor | 215,85002 | CXCL12 | NM_000609 | Chemokine (C-X-C motif) ligand 12 (stromal cell-derived 1) |
| 203668_at | 0,00109 | 0,90375584 | 697,2668 | 1,2058574 | 908,75446 | MAN2C1 | NM_006715 | Mannosidase, alpha, class 2C, member 1 |
| 203685_at | 0,000161 | 1,0205845 | 1392,2852 | 0,71823096 | 968,0682 | BCL2 | NM_000633 | B-cell CLL/lymphoma 2 |
| 203689_s_at | 2,29E-08 | 1,0305977 | 839,3853 | 0,652725 | 550,04083 | FMR1 | AI743037 | Fragile X mental retardation 1 |
| 203690_at | 8,52E-06 | 0,95708203 | 550,0704 | 0,56849504 | 329,33182 | TUBGCP3 | NM_006322 | Tubulin, gamma complex associated protein 3 |
| 203710_at | 0,000317 | 0,99166137 | 619,7 | 0,74966645 | 478,0318 | ITPR1 | NM_002222 | Inositol 1,4,5-triphosphate receptor, type 1 |
| 203711_s_at | 1,08E-06 | 1,0024323 | 116,14815 | 0,5216869 | 67,19091 | HIBCH | NM_014362 | 3-hydroxyisobutyryl-Coenzyme A hydrolase |
| 203715_at | 0,000178 | 0,9638445 | 97,4074 | 0,6693409 | 71,28636 | TBCE | NM_003193 | Tubulin-specific chaperone e |
| 203721_s_at | 6,91E-08 | 0,9733257 | 1247,5553 | 0,73288107 | 947,59094 | CGI-48 | NM_016001 | CGI-48 protein |
| 203723_at | 1,89E-08 | 0,99722004 | 1180,0778 | 0,64309406 | 777,3091 | ITPKB | NM_002221 | Inositol 1,4,5-trisphosphate 3-kinase B |
| 203733_at | 1,10E-05 | 0,95603305 | 352,7926 | 0,6747378 | 244,44543 | DEXI | NM_014015 | Dexamethasone-induced transcript |
| 203739_at | 1,79E-05 | 1,0045418 | 1050,2219 | 0,5776432 | 579,559 | ZNF217 | NM_006526 | Zinc finger protein 217 |
| 203743_s_at | 1,38E-07 | 1,0207138 | 657,51117 | 0,66352755 | 428,27725 | TDG | NM_003211 | Thymine-DNA glycosylase |
| 203745_at | 2,57E-06 | 0,97793293 | 377,1889 | 0,7326525 | 282,24542 | HCCS | AI801013 | Holocytochrome c synthase (cytochrome c heme-lyase) |
| 203748_x_at | 0,000502 | 1,0501571 | 2655,43 | 1,3130279 | 3330,1362 | RBMS1 | NM_016839 | RNA binding motif, single stranded interacting protein 1 |
| 203749_s_at | 0,00119 | 0,91738486 | 746,91846 | 1,2617736 | 1032,0908 | RARA | AI806984 | Retinoic acid receptor, alpha |
| 203751_x_at | 2,56E-05 | 0,8892348 | 548,25934 | 2,1508067 | 1308,6865 | JUND | NM_005354 | Jun D proto-oncogene |
| 203752_s_at | 4,94E-10 | 1,0220581 | 7781,9893 | 1,473734 | 11121,354 | JUND | NM_005354 | Jun D proto-oncogene |
| 203753_at | 0,00406 | 1,029655 | 390,47406 | 0,752515 | 285,18637 | TCF4 | NM_003199 | Transcription factor 4 |
| 203761_at | 0,00101 | 0,9380812 | 2311,5073 | 0,7473099 | 1856,3182 | SLA | NM_006748 | Src-like-adaptor |
| 203773_x_at | 0,000419 | 0,965885 | 1399,5371 | 1,3253227 | 1909,9635 | BLVRA | NM_000712 | Biliverdin reductase A |
| 203774_at | 3,03E-09 | 0,9932652 | 502,137 | 0,61005694 | 306,66815 | MTR | NM_000254 | 5-methyltetrahydrofolate-homocysteine methyltransferase |
| 203776_at | 0,000146 | 1,0292366 | 636,60736 | 0,79458797 | 495,80908 | GPKOW | NM_015698 | G patch domain and KOW motifs |
| 203787_at | 8,68E-05 | 1,0139012 | 346,30737 | 0,754936 | 257,61365 | SSBP2 | NM_012446 | Single-stranded DNA binding protein 2 |
| 203791_at | 2,23E-06 | 0,9870227 | 502,1074 | 0,6116398 | 324,57272 | DMXL1 | NM_005509 | Dmx-like 1 |
| 203793_x_at | 0,00282 | 0,8546516 | 263,26297 | 1,2032527 | 348,2682 | PCGF2 | NM_007144 | Polycomb group ring finger 2 |
| 203801_at | 4,82E-08 | 1,0052642 | 265,27774 | 0,7096847 | 190,73637 | MRPS14 | NM_022100 | Mitochondrial ribosomal protein S14 |
| 203803_at | 1,32E-05 | 0,99886 | 83,49629 | 0,5606531 | 49,62727 | PCYOX1 | N45309 | Prenylcysteine oxidase 1 |
| 203817_at | 0,00406 | 0,9738554 | 329,04446 | 0,6397933 | 240,97728 | GUCY1B3 | W93728 | Guanylate cyclase 1, soluble, beta 3 |
| 203821_at | 9,96E-10 | 0,8229103 | 280,04443 | 5,822297 | 1348,5773 | HBEGF | NM_001945 | Heparin-binding EGF-like growth factor |
| 203822_s_at | 0,000382 | 0,95071524 | 545,6185 | 1,3824373 | 794,23645 | ELF2 | NM_006874 | Protein phosphatase 1, regulatory (inhibitor) subunit 14B |
| 203833_s_at | 2,28E-07 | 1,01803 | 485,34818 | 1,4979886 | 717,7091 | TGOLN2 | BF061845 | Trans-golgi network protein 2 |
| 203839_s_at | 4,53E-05 | 0,93952936 | 600,7593 | 1,2267247 | 779,8136 | TNK2 | NM_005781 | Tyrosine kinase, non-receptor, 2 |
| 203843_at | 0,00261 | 0,93657726 | 873,0926 | 0,7477946 | 696,3454 | RPS6KA3 | AA906056 | Ribosomal protein S6 kinase, 90kDa, polypeptide 3 |
| 203846_at | 0,000574 | 0,89616466 | 316,36667 | 0,63579375 | 219,72275 | TRIM32 | BC003154 | Tripartite motif-containing 32 |
| 203856_at | 2,87E-05 | 1,0499178 | 418,70004 | 0,8031043 | 324,0864 | VRK1 | NM_003384 | Vaccinia related kinase 1 |
| 203880_at | 0,0006 | 1,0123609 | 885,69635 | 0,7978375 | 707,3818 | COX17 | NM_005694 | COX17 homolog, cytochrome c oxidase assembly protein (yeast) |
| 203887_s_at | 1,72E-08 | 0,9254434 | 152,39629 | 2,005826 | 363,65002 | THBD | NM_000361 | Thrombomodulin |
| 203893_at | 3,80E-11 | 1,0042478 | 446,13336 | 0,5559564 | 251,07271 | TAF9 | NM_016283 | TAF9 RNA polymerase II, TATA box binding protein (TBP)-associated factor, 32kDa |
| 203921_at | 4,43E-06 | 1,0761663 | 774,10736 | 0,6770906 | 467,8773 | CHST2 | NM_004267 | Carbohydrate (N-acetylglucosamine-6-O) sulfotransferase 2 |
| 203922_s_at | 0,000317 | 1,0484772 | 1382,463 | 1,4990106 | 1905,3407 | CYBB | AI308863 | Cytochrome b-245, beta polypeptide (chronic granulomatous disease) |
| 203925_at | 8,24E-08 | 1,0524719 | 335,16296 | 0,63879323 | 210,85455 | GCLM | NM_002061 | Glutamate-cysteine ligase, modifier subunit |
| 203927_at | 9,97E-10 | 1,0148965 | 358,94443 | 1,489083 B-cells | 527,18634 | NFKBIE | NM_004556 | Nuclear factor of kappa light polypeptide gene enhancer in inhibitor, epsilon |
| 203936_s_at | 0,00129 | 0,95839816 | 274,52594 | 1,6602832 92kDa | 805,2363 | MMP9 | NM_004994 | Matrix metalloproteinase 9 (gelatinase B, 92kDa gelatinase, type IV collagenase) |
| 203939_at | 6,17E-06 | 1,0043432 | 209,67035 | 0,52644056 | 118,43181 | NT5E | NM_002526 | 5'-nucleotidase, ecto (CD73) |
| 203943_at | 1,79E-05 | 0,9478236 | 550,65186 | 0,6841632 | 389,75455 | KIF3B | NM_004798 | Kinesin family member 3B |
| 203956_at | 8,68E-05 | 0,8905104 | 579,1592 | 0,51691365 | 329,30457 | ZCWCC1 | NM_014941 | Zinc finger, CW type with coiled-coil domain 1 |
| 203960_s_at | 0,000238 | 0,9708011 | 219,72221 | 0,7432413 | 167,37727 | C1orf41 | NM_016126 | Chromosome 1 open reading frame 41 |
| 203970_s_at | 1,25E-09 | 0,9438555 | 221,0889 | 0,55765086 | 130,4909 | PEX3 | NM_003630 | Peroxisomal biogenesis factor 3 |
| 203972_s_at | 7,92E-10 | 1,0098289 | 315,9111 | 0,5703704 | 181 | PEX3 | AB035307 | Peroxisomal biogenesis factor 3 |
| 203973_s_at | 0,00378 | 0,9691702 | 5687,955 | 1,269514 | 7356,55 | CEBPD | NM_005195 | CCAAT/enhancer binding protein (C/EBP), delta |
| 203983_at | 2,77E-08 | 0,99821943 | 1023,7778 | 0,68488014 | 699,8409 | TSNAX | NM_005999 | Translin-associated factor X |
| 203989_x_at | 0,00216 | 0,92932177 | 293,83334 | 0,56210405 | 177,61362 | F2R | NM_001992 | Coagulation factor II (thrombin) receptor |
| 203991_s_at | 0,000161 | 1,0254914 | 125,14444 | 0,6276833 | 78,66364 | UTX | NM_021140 | Ubiquitously transcribed tetratricopeptide repeat, X chromosome |
| 204003_s_at | 1,64E-07 | 0,925789 | 412,5667 | 0,58016014 | 264,1864 | NUPL2 | NM_007342 | Nucleoporin like 2 |
| 204020_at | 3,88E-10 | 0,970653 | 1403,3778 | 0,61142415 | 886,5955 | PURA | BF739943 | Purine-rich element binding protein A |
| 204023_at | 7,02E-05 | 1,0270268 | 445,43335 | 0,78758293 | 342,0682 | RFC4 | NM_002916 | Replication factor C (activator 1) 4, 37kDa |
| 204025_s_at | 2,95E-06 | 1,1328983 | 182,97778 | 0,7048775 | 110,02273 | PDCD2 | NM_002598 | Programmed cell death 2 |
| 204039_at | 0,00101 | 0,9923924 | 1285,7666 | 1,347791 | 1753,8318 | CEBPA | NM_004364 | CCAAT/enhancer binding protein (C/EBP), alpha |
| 204040_at | 0,000925 | 1,0020561 | 344,24817 | 0,7599569 | 265,1727 | RNF144 | NM_014746 | Ring finger protein 144 |
| 204054_at | 9,80E-08 | 0,9967142 | 379,32962 | 1,3401618 | 508,70908 | PTEN | NM_000314 | Phosphatase and tensin homolog (mutated in multiple advanced cancers 1) |
| 204063_s_at | 0,000549 | 0,8894703 | 196,45557 | 1,145933 | 247,32724 | ULK2 | NM_014683 | Unc-51-like kinase 2 (C. elegans) |
| 204064_at | 0,00085 | 0,9730552 | 492,07776 | 0,73989016 | 366,44095 | THOC1 | NM_005131 | THO complex 1 |
| 204075_s_at | 0,000502 | 1,0870148 | 72,30741 | 0,6915651 | 46,190914 | KIAA0562 | NM_014704 | Glycine-, glutamate-, thienylcyclohexylpiperidine-binding |
| 204079_at | 0,00164 | 1,0360526 | 1560,9888 | 0,8109726 | 1254,8729 | TPST2 | NM_003595 | Tyrosylprotein sulfotransferase 2 |
| 204085_s_at | 0,000238 | 0,967192 | 163,47035 | 0,7086423 | 117,722725 | CLN5 | NM_006493 | Ceroid-lipofuscinosis, neuronal 5 |
| 204091_at | 0,00208 | 1,0070306 | 510,91107 | 0,7957859 | 409,3909 | PDE6D | NM_002601 | Phosphodiesterase 6D, cGMP-specific, rod, delta |
| 204099_at | 0,00078 | 0,9420211 | 513,2741 | 1,2409158 | 680,2637 | SMARCD3 | NM_003078 | SWI/SNF related, matrix associated, actin dependent regulator of chromatin, subfamily d, member 3 |
| 204141_at | 0,00164 | 0,94064546 | 256,51855 | 1,6737925 | 432,77725 | TUBB2 | NM_001069 | Tubulin, beta 2 |
| 204142_at | 5,06E-06 | 1,0856959 | 210,54074 | 0,6031561 A | 113,81819 | HSRTSBET | NM_017512 | Enolase superfamily member 1 |
| 204150_at | 0,00304 | 0,9150964 | 859,8445 | 1,3598773 | 1313,9817 | STAB1 | NM_015136 | Stabilin 1 |
| 204155_s_at | 3,22E-05 | 1,0228702 | 441,2074 | 0,80227923 | 344,76367 | KIAA0999 | AA044154 | KIAA0999 protein |
| 204160_s_at | 0,000131 | 1,0022492 | 452,01483 | 0,6912237 (putative | 331,82727 | ENPP4 | AW194947 | Ectonucleotide pyrophosphatase/phosphodiesterase 4 function) |
| 204161_s_at | 9,65E-05 | 1,0051891 | 137,09631 | 0,6605582 (putative | 92,568184 | ENPP4 | NM_014936 | Ectonucleotide pyrophosphatase/phosphodiesterase 4 function) |
| 204172_at | 4,35E-07 | 0,9484446 | 463,77774 | 0,48829022 | 236,7 | CPOX | NM_000097 | Coproporphyrinogen oxidase |
| 204180_s_at | 0,000656 | 0,93999964 | 234,44447 | 1,1823313 | 300,21365 | ZNF297B | NM_014007 | Zinc finger protein 297B |
| 204181_s_at | 4,99E-11 | 0,95005083 | 285,15558 | 1,5485634 | 464,02725 | ZNF297B | NM_014007 | Zinc finger protein 297B |
| 204182_s_at | 4,53E-05 | 1,0492203 | 176,64449 | 1,7596768 | 314,59094 | ZNF297B | NM_014007 | Zinc finger protein 297B |
| 204184_s_at | 0,00224 | 0,9562719 | 225,44073 | 1,2532274 | 301,80453 | ADRBK2 | NM_005160 | Adrenergic, beta, receptor kinase 2 |
| 204186_s_at | 5,06E-05 | 1,05261 | 656,50354 | 0,7749383 | 476,47275 | PPID | AI014573 | Peptidylprolyl isomerase D (cyclophilin D) |
| 204208_at | 2,27E-05 | 0,93344 | 276,91483 | 0,56834 | 169,74544 | RNGTT | NM_003800 | RNA guanylyltransferase and 5'-phosphatase |
| 204216_s_at | 0,000146 | 1,0059981 | 589,7334 | 0,7766 | 456,3818 | FLJ11806 | NM_024824 | Nuclear protein UKp68 |
| 204218_at | 0,000419 | 0,89788 | 709,92957 | 1,1428808 | 883,5227 | DKFZP564M 082 | NM_014042 | DKFZP564M082 protein |
| 204228_at | 6,17E-06 | 1,0192747 | 634,26294 | 0,747665 | 469,57733 | PPIH | NM_006347 | Peptidyl prolyl isomerase H (cyclophilin H) |
| 204234_s_at | 0,000317 | 1,0136213 | 312,40002 | 0,7865457 | 238,60454 | ZNF195 | AI476267 | Zinc finger protein 195 |
| 204236_at | 1,17E-07 | 1,0076883 | 1469,0371 | 0,63111746 | 927,0863 | FLI1 | NM_002017 | Friend leukemia virus integration 1 |
| 204264_at | 0,00101 | 0,945816 | 154,3 | 0,70445085 | 116,4409 | CPT2 | NM_000098 | Carnitine palmitoyltransferase II |
| 204274_at | 9,36E-07 | 0,9531487 | 938,04443 | 0,6147219 | 615,1317 | EBAG9 | AA812215 | Estrogen receptor binding site associated, antigen, 9 |
| 204285_s_at | 3,04E-10 | 1,0492105 | 632,3296 | 2,8037434 | 1630,4865 | PMAIP1 | AI857639 | Phorbol-12-myristate-13-acetate-induced protein 1 |
| 204291_at | 0,00252 | 1,0273749 | 175,35925 | 0,7881357 | 130,95456 | ZNF518 | NM_014803 | Zinc finger protein 518 |
| 204297_at | 0,0014 | 1,0305958 | 632,92224 | 0,8167932 | 504,59546 | PIK3C3 | NM_002647 | Phosphoinositide-3-kinase, class 3 |
| 204314_s_at | 1,26E-06 | 0,9925956 | 589,9333 | 0,5416162 | 308,65454 | CREB1 | NM_004379 | CAMP responsive element binding protein 1 |
| 204326_x_at | 0,00129 | 1,0165195 | 961,7223 | 1,3229591 | 1261,9319 | MT1L | NM_002450 | |
| 204335_at | 0,00436 | 1,0441048 | 246,09628 | 0,7661066 | 190,19092 | FLJ10374 | NM_018074 | Hypothetical protein FLJ10374 |
| 204349_at | 0,00707 | 0,96735823 | 153,93333 | 0,7467004 | 119,50454 | CRSP9 | BC005250 | Cofactor required for Sp1 transcriptional activation, subunit 9, 33kDa |
| 204351_at | 2,29E-08 | 0,67675525 | 381,24814 | 2,0862625 | 1008,5364 | S100P | NM_005980 | S100 calcium binding protein P |
| 204352_at | 3,34E-08 | 0,93380165 | 1172,4 | 0,4951636 | 624,0773 | TRAF5 | NM_004619 | TNF receptor-associated factor 5 |
| 204353_s_at | 3,22E-05 | 0,9891831 | 290,7778 | 0,78552157 | 231,4182 | POT1 | BC002923 | POT1 protection of telomeres 1 homolog (S. pombe) |
| 204354_at | 2,02E-05 | 0,98968774 | 399,64813 | 0,7383445 | 304,60458 | POT1 | NM_015450 | POT1 protection of telomeres 1 homolog (S. pombe) |
| 204370_at | 2,64E-12 | 0,9939122 | 593,43335 | 1,8120357 | 1070,8046 | HEAB | NM_006831 | ATP/GTP-binding protein |
| 204376_at | 0,000655 | 0,90594584 | 136,12222 | 1,202019 | 177,9 | VprBP | NM_014703 | Vpr-binding protein |
| 204385_at | 0,000925 | 1,0489208 | 628,22217 | 1,49615 | 879,18634 | KYNU | NM_003937 | Kynureninase (L-kynurenine hydrolase) |
| 204393_s_at | 9,67E-06 | 0,9087756 | 279,35184 | 1,41129 | 429,68637 | ACPP | NM_001099 | Acid phosphatase, prostate |
| 204396_s_at | 0,00224 | 1,0159026 | 923,3185 | 0,7608276 | 723,49084 | GRK5 | NM_005308 | G protein-coupled receptor kinase 5 |
| 204404_at | 0,000502 | 0,98542726 | 225,16669 | 1,2932795 | 293,80905 | SLC12A2 | NM_001046 | Solute carrier family 12 (sodium/potassium/chloride transporters), member 2 |
| 204405_x_at | 3,34E-08 | 0,9916721 | 1068,2853 | 0,6799997 | 742,3045 | HSA9761 | NM_014473 | Putative dimethyladenosine transferase |
| 204407_at | 0,00979 | 1,0483017 | 102,70369 | 0,78705174 | 80,08182 | TTF2 | AF080255 | Transcription termination factor, RNA polymerase II |
| 204415_at | 0,00861 | 0,9572622 | 545,5074 | 1,4503528 | 814,4501 | G1P3 | NM_022873 | Interferon, alpha-inducible protein (clone IFI-6-16) |
| 204425_at | 0,000656 | 0,9697807 | 865,7222 | 1,2432648 | 1116,7545 | ARHGAP4 | NM_001666 | Rho GTPase activating protein 4 |
| 204440_at | 3,71 E-07 | 0,95303696 | 445,4185 | 1,813694 | 906,3681 | CD83 | NM_004233 | CD83 antigen (activated B lymphocytes, immunoglobulin superfamily) |
| 204449_at | 1,09E-06 | 0,980352 | 134,53703 | 0,6428997 | 87,38636 | PDCL | NM_005388 | Phosducin-like |
| 204451_at | 0,00192 | 0,9777025 | 229,9111 | 0,71691984 | 172,43182 | FZD1 | NM_003505 | Frizzled homolog 1 (Drosophila) |
| 204453_at | 1,57E-09 | 0,9774952 | 226,35555 | 0,46448475 | 106,63182 | ZNF84 | NM_003428 | Zinc finger protein 84 (HPF2) |
| 204458_at | 0,00109 | 0,9702747 | 168,77037 | 1,2268599 | 212,4409 | LYPLA3 | AL110209 | Lysophospholipase 3 (lysosomal phospholipase A2) |
| 204477_at | 2,02E-05 | 0,9895674 | 232,92963 | 0,64172053 | 152,25453 | RABIF | U74324 | RAB interacting factor |
| 204494_s_at | 1,40E-05 | 0,9141512 | 826,4297 | 1,4387931 | 1299,509 | DKFZP434H 132 | AW516789 | DKFZP434H132 protein |
| 204497_at | 0,00806 | 0,92980546 | 226,81482 | 0,633175 | 163,62274 | ADCY9 | AB011092 | Adenylate cyclase 9 |
| 204523_at | 4,34E-07 | 0,9510932 | 218,04076 | 0,50249434 | 119,0591 | ZNF140 | NM_003440 | Zinc finger protein 140 (clone pHZ-39) |
| 204529_s_at | 2,87E-05 | 0,9997511 | 279,82962 | 0,6553723 | 191,74544 | TOX | AI961231 | Thymus high mobility group box protein TOX |
| 204544_at | 6,30E-05 | 0,91666555 | 399,57034 | 0,6178716 | 274,14093 | HPS5 | NM_007216 | Hermansky-Pudlak syndrome 5 |
| 204564_at | 0,00919 | 0,8714452 | 154,2926 | 1,3590484 | 211,33182 | PCGF3 | NM_006315 | Polycomb group ring finger 3 |
| 204566_at | 0,000262 | 1,0114993 | 287,55554 | 0,7574433 | 215,56366 | PPM1D | NM_003620 | Protein phosphatase 1D magnesium-dependent, delta |
| 204567_s_at | 0,00129 | 0,9733746 | 165,55556 | 0,70571655 | 119,009094 | ABCG1 | | NM_004915 ATP-binding cassette, sub-family G (WHITE), member 1 |
| 204569_at | 6,17E-06 | 1,0637633 | 71,89629 | 0,5820094 | 39,245453 | ICK | NM_014920 | Intestinal cell (MAK-like) kinase |
| 204577_s_at | 0,00577 | 1,0187457 | 105,37777 | 0,7737334 | 80,200005 | CLUAP1 | NM_024793 | Clusterin associated protein 1 |
| 204581_at | 0,00208 | 0,9622335 | 304,4149 | 0,50714886 | 189,86363 | CD22 | NM_001771 | CD22 antigen |
| 204612_at | 0,00119 | 0,931538 | 352,22592 | 0,6862652 | 252,77728 | PKIA | NM_006823 | Protein kinase (cAMP-dependent, catalytic) inhibitor alpha |
| 204615_x_at | 0,00078 | 1,00505 | 930,6481 | 1,344376 | 1274,1726 | IDI1 | NM_004508 | Isopentenyl-diphosphate delta isomerase |
| 204617_s_at | 4,43E-06 | 0,95337284 | 496,15186 | 0,66635305 | 353,11362 | ACD | NM_022914 | Adrenocortical dysplasia homolog (mouse) |
| 204619_s_at | 0,00185 | 1,0382656 | 1745,1926 | 1,3153639 | 2204,0454 | CSPG2 | BF590263 | Chondroitin sulfate proteoglycan 2 (versican) |
| 204622_x_at | 5,93E-07 | 1,1020252 | 228,88521 | 3,3681297 | 611,14087 | NR4A2 | NM_006186 | Nuclear receptor subfamily 4, group A, member 2 |
| 204632_at | 0,00192 | 0,9569119 | 769,28156 | 1,3875163 | 1039,1138 | RPS6KA4 | NM_003942 | Ribosomal protein S6 kinase, 90kDa, polypeptide 4 |
| 204633_s_at | 1,40E-05 | 1,0318108 | 563,76294 | 0,6860644 | 368,5864 | RPS6KA5 | AF074393 | Ribosomal protein S6 kinase, 90kDa, polypeptide 5 |
| 204634_at | 0,00242 | 0,9345886 | 308,2519 | 0,7328626 | 245,79544 | NEK4 | NM_003157 | NIMA (never in mitosis gene a)-related kinase 4 |
| 204635_at | 6,30E-05 | 1,0305355 | 329,45187 | 0,7492119 | 247,0591 | RPS6KA5 | NM_004755 | Ribosomal protein S6 kinase, 90kDa, polypeptide 5 |
| 204642_at | 2,87E-05 | 1,0047289 | 967,7592 | 0,6497644 | 659,31824 | EDG1 | NM_001400 | Endothelial differentiation, sphingolipid G-protein-coupled receptor, 1 |
| 204647_at | 0,00292 | 0,92539155 | 302,53705 | 1,2503273 | 393,64545 | HOMER3 | NM_004838 | Homer homolog 3 (Drosophila) |
| 204662_at | 0,000178 | 1,0313936 | 304,97403 | 0,7244451 | 215,66818 | CP110 | NM_014711 | CP110 protein |
| 204665_at | 0,00208 | 0,9715485 | 72,16666 | 1,3481517 | 101,2409 | FLJ21168 | NM_025073 | Hypothetical protein FLJ21168 |
| 204676_at | 3,71 E-07 | 0,9421055 | 290,8111 | 0,6203881 | 192,03635 | DKFZP564K 2062 | NM_015421 | DKFZP564K2062 protein |
| 204686_at | 0,00292 | 0,97376424 | 90,60371 | 0,69759804 | 69,82727 | IRS1 | NM_005544 | Insulin receptor substrate 1 |
| 204699_s_at | 7,02E-05 | 0,91062915 | 223,44075 | 0,6707279 | 163,19545 | MGC29875 | N30910 | Hypothetical protein MGC29875 |
| 204700_x_at | 1,17E-07 | 0,9168042 | 214,0815 | 0,5850331 | 134,06818 | MGC29875 | NM_014388 | Hypothetical protein MGC29875 |
| 204716_at | 5,05E-05 | 0,9552914 | 213,14075 | 1,3264003 | 295,15457 | CCDC6 | NM_005436 | Coiled-coil domain containing 6 |
| 204725_s_at | 0,000178 | 0,997808 | 786,4334 | 0,69022 | 562,61816 | NCK1 | NM_006153 NCK | adaptor protein 1 |
| 204731_at | 0,000146 | 0,9558266 | 904,79626 | 0,49034446 300kDa) | 531,2818 | TGFBR3 | NM_003243 | Transforming growth factor, beta receptor III (betaglycan, |
| 204745_x_at | 0,000262 | 0,9635469 | 1006,4259 | 1,3357643 | 1366,1455 | MT1G | NM_005950 | Metallothionein 1G |
| 204751_x_at | 0,000196 | 0,91558826 | 81,05556 | 1,5436628 | 133,95453 | DSC2 | NM_004949 | Desmocollin 2 |
| 204767_s_at | 0,000502 | 0,9988101 | 294,78522 | 0,68687695 | 202,1091 | FEN1 | BC000323 | Flap structure-specific endonuclease 1 |
| 204769_s_at | 1,59E-05 | 0,99675524 | 432,55557 | 1,5429893 | 664,4728 | TAP2 | M74447 | Transporter 2, ATP-binding cassette, sub-family B |
| 204771_s_at | 0,00502 | 1,0454253 | 551,7111 | 0,82346725 | 429,55002 | TTF1 | AI632304 | Transcription termination factor, RNA polymerase I |
| 204794_at | 2,77E-08 | 0,97416824 | 867,826 | 2,2363083 | 1888,7819 | DUSP2 | NM_004418 | Dual specificity phosphatase 2 |
| 204805_s_at | 5,07E-06 | 0,98099095 | 2494,2002 | 1,6048268 | 4152,959 | H1FX | NM_006026 | H1 histone family, member X |
| 204809_at | 2,02E-05 | 1,0074317 | 358,05557 | 0,76060605 | 270,8136 | CLPX | NM_006660 | ClpX caseinolytic protease X homolog (E. coli) |
| 204813_at | 0,00109 | 0,80719125 | 254,14815 | 1,384326 | 387,89093 | MAPK10 | NM_002753 | Mitogen-activated protein kinase 10 |
| 204820_s_at | 9,81 E-08 | 0,99716485 | 6324,4414 | 0,69658995 | 4523,3047 | BTN3A3 | NM_006994 | Butyrophilin, subfamily 3, member A3 |
| 204821_at | 0,000382 | 0,93991107 | 1053,7258 | 0,7403002 | 847,0817 | BTN3A3 | NM_006994 | Butyrophilin, subfamily 3, member A3 |
| 204847_at | 1,59E-05 | 0,9111371 | 750,7555 | 0,6483796 | 527,3227 | ZBTB11 | NM_014415 | Zinc finger and BTB domain containing 11 |
| 204849_at | 6,30E-05 | 1,0143976 | 355,71854 | 0,74413776 | 267,8727 | TCFL5 | NM_006602 | Transcription factor-like 5 (basic helix-loop-helix) |
| 204858_s_at | 2,28E-07 | 0,92813206 | 1666,3483 | 2,086364 | 3800,4504 | ECGF1 | NM_001953 | Endothelial cell growth factor 1 (platelet-derived) |
| 204859_s_at | 0,000715 | 0,96271485 | 463,68893 | 0,7204058 | 348,15912 | APAF1 | NM_013229 | Apoptotic protease activating factor |
| 204872_at | 3,71 E-07 | 0,9796812 | 1341,3297 | 0,66526717 Drosophila) | 928,03186 | TLE4 | NM_007005 | Transducin-like enhancer of split 4 (E(sp1) homolog, |
| 204873_at | 0,0006 | 0,96853465 | 112,025925 | 0,61089337 | 73,045456 | PEX1 | NM_000466 | Peroxisome biogenesis factor 1 |
| 204882_at | 0,00242 | 0,8813916 | 1847,8739 | 0,68132365 | 1380,0319 | ARHGAP25 | NM_014882 | Rho GTPase activating protein 25 |
| 204890_s_at | 0,000119 | 0,97790825 | 2471,9668 | 0,76879305 | 1948,4956 | LCK | U07236 | Lymphocyte-specific protein tyrosine kinase |
| 204891_s_at | 5,07E-06 | 0,9720543 | 5262,8 | 0,6620779 | 3602,4773 | LCK | NM_005356 | Lymphocyte-specific protein tyrosine kinase |
| 204897_at | 1,46E-06 | 1,0277061 | 2107,6406 | 0,7572931 | 1547,441 | PTGER4 | NM_000958 | Prostaglandin E receptor 4 (subtype EP4) |
| 204900_x_at | 0,00208 | 0,9964403 | 429,6519 | 1,3534057 | 607,3 | SAP30 | NM_003864 | Sin3-associated polypeptide, 30kDa |
| 204905_s_at | 2,87E-11 | 1,0123733 | 563,80005 | 0,6584104 | 367,54086 | EEF1E1 | NM_004280 | Eukaryotic translation elongation factor 1 epsilon 1 |
| 204908_s_at | 2,17E-1 | 0,96715677 | 479,54446 | 2,7747996 | 1482,2634 | BCL3 | NM_005178 | B-cell CLL/lymphoma 3 |
| 204937_s_at | 9,65E-05 | 0,9347225 | 943,7074 | 0,72618985 | 723,5409 | ZNF274 | NM_016325 | Zinc finger protein 274 |
| 204957_at | 0,000196 | 0,91510445 | 430,10004 | 0,7209002 | 336,59088 | ORC5L | NM_002553 | Origin recognition complex, subunit 5-like (yeast) |
| 204960_at | 0,00192 | 0,92837745 | 2317,974 | 0,70409715 | 1748,9229 | PTPRCAP | NM_005608 | Protein tyrosine phosphatase, receptor type, C-associated protein |
| 204961_s_at | 0,000131 | 0,93466085 | 2402,2368 | 1,4124333 | 3737,459 | NCF1 | NM_000265 | Neutrophil cytosolic factor 1 (47kDa, chronic granulomatous disease, autosomal 1) |
| 204978_at | 9,36E-07 | 0,87328386 | 720,6074 | 1,3532656 | 1111,3364 | SFRS16 | NM_007056 | Splicing factor, arginine/serine-rich 16 (suppressor-of-white-apricot homolog, Drosophila) |
| 204981_at | 0,000289 | 1,0069501 | 470,7222 | 1,3395011 | 623,29553 | SLC22A18 | NM_002555 | Solute carrier family 22 (organic cation transporter), member |
| 204998_s_at | 6,59E-06 | 1,0395643 | 264,45926 | 1,5485944 | 401,36365 | ATF5 | NM_012068 | Activating transcription factor 5 |
| 205005_s_at | 0,000332 | 1,0138167 | 280,07404 | 0,69599414 | 199,18181 | NMT2 | AW293531 | N-myristoyltransferase 2 |
| 205006_s_at | 0,00661 | 0,97489995 | 317,92587 | 0,7689631 | 244,19545 | NMT2 | NM_004808 | N-myristoyltransferase 2 |
| 205013_s_at | 3,22E-05 | 1,02085 | 424,72595 | 0,675473 | 282,81818 | ADORA2A | NM_000675 | Adenosine A2a receptor |
| 205021_s_at | 0,00378 | 1,0543414 | 469,74442 | 1,4119326 | 635,90466 | CHES1 | AA860806 | Checkpoint suppressor 1 |
| 205022_s_at | 0,00119 | 1,0695117 | 1270,737 | 1,3882107 | 1550,1908 | CHES1 | NM_005197 | Checkpoint suppressor 1 |
| 205038_at | 0,000365 | 0,97062796 | 854,75195 | 0,7319199 IMAGE:4693783 | 632,2045 | ZNFN1A1 | BG540504 | 602569230F1 NIH_MGC_77 Homo sapiens cDNA clone 5', mRNA sequence. |
| 205052_at | 1,49E-05 | 0,96894914 | 442,8889 | 0,7432168 | 340,80453 | AUH | NM_001698 | AU RNA binding protein/enoyl-Coenzyme A hydratase |
| 205059_s_at | 0,00468 | 0,8850737 | 527,8482 | 1,154826 | 664,61816 | IDUA | NM_000203 | Iduronidase, alpha-L- |
| 205060_at | 6,92E-07 | 0,9778948 | 295,64813 | 0,59499 | 178,17726 | PARG | NM_003631 | Poly (ADP-ribose) glycohydrolase |
| 205063_at | 9,81E-08 | 1,0318217 | 99,5148 | 0,61174285 | 61,27728 | SIP1 | NM_003616 | Survival of motor neuron protein interacting protein 1 |
| 205068_s_at | 5,71 E-09 | 1,0093666 | 397,07785 | 1,5495704 | 602,9637 | ARHGAP26 | BE671084 | Rho GTPase activating protein 26 |
| 205070_at | 0,000289 | 1,0357746 | 894,82965 | 0,74853617 | 630,4409 | ING3 | NM_019071 | Inhibitor of growth family, member 3 |
| 205076_s_at | 0,000196 | 0,940161 | 789,62225 | 1,2611289 | 1047,7457 | CRA | NM_006697 | Cisplatin resistance associated |
| 205078_at | 0,0006 | 1,0068537 | 315,23697 | 0,77600104 | 243,2818 | PIGF | NM_002643 | Phosphatidylinositol glycan, class F |
| 205087_at | 0,00048 | 0,95707667 | 308,09998 | 0,75683063 | 243,63635 | RWDD3 | NM_015485 | RWD domain containing 3 |
| 205089_at | 6,71E-12 | 1,007607 | 294,0074 | 0,6795735 | 196,13637 | ZNF7 | NM_003416 | Zinc finger protein 7 (KOX 4, clone HF.16) |
| 205091_x_at | 1,40E-05 | 1,0195564 | 411,73703 | 0,654926 | 275,25452 | RECQL | NM_002907 | RecQ protein-like (DNA helicase Q1-like) |
| 205096_at | 0,00327 | 1,0174794 | 170,91112 | 1,2722559 | 212,77727 | | NM_014833 | |
| 205097_at | 1,94E-06 | 0,9749175 | 338,17776 | 0,6454752 | 223,97726 | SLC26A2 | AI025519 | Solute carrier family 26 (sulfate transporter), member 2 |
| 205119_s_at | 0,00378 | 0,9647928 | 5382,281 | 1,2639554 | 7020,004 | FPR1 | NM_002029 | Formyl peptide receptor 1 |
| 205133_s_at | 1,64E-07 | 0,9539221 | 1089,8666 | 0,6052175 | 688,1181 | HSPE1 | NM_002157 | Heat shock 10kDa protein 1 (chaperonin 10) |
| 205140_at | 8,68E-05 | 0,89062476 | 217,51852 | 0,6061927 | 150,4091 | FPGT | NM_003838 | Fucose-1-phosphate guanylyltransferase |
| 205142_x_at | 5,94E-07 | 1,0126455 | 451,05923 | 1,6485653 | 741,1227 | ABCD1 | NM_000033 | ATP-binding cassette, sub-family D (ALD), member 1 |
| 205147_x_at | 0,00101 | 0,9822368 | 991,863 | 1,2545438 | 1273,4773 | NCF4 | NM_000631 | Neutrophil cytosolic factor 4, 40kDa |
| 205171_at | 0,00557 | 1,0595675 | 989,52966 | 0,8065103 | 759,8091 | PTPN4 | NM_002830 | Protein tyrosine phosphatase, non-receptor type 4 (megakaryocyte) |
| 205176_s_at | 0,000348 | 0,96011895 | 575,78516 | 0,7276782 | 442,49088 | ITGB3BP | NM_014288 | Integrin beta 3 binding protein (beta3-endonexin) |
| 205202_at | 0,000178 | 1,009492 | 1620,3999 | 0,6985618 | 1183,9681 | PCMT1 | NM_005389 | Protein-L-isoaspartate (D-aspartate) O-methyltransferase |
| 205205_at | 7,50E-06 | 0,96593976 | 375,7926 | 1,2948334 | 508,19998 | RELB | NM_006509 | V-rel reticuloendotheliosis viral oncogene homolog B, nuclear factor of kappa light polypeptide gene enhancer in B-cells 3 (avian) |
| 205207_at | 8,68E-05 | 0,9639657 | 106,566666 | 1,3910784 | 153,19545 | IL6 | NM_000600 | Interleukin 6 (interferon, beta 2) |
| 205211_s_at | 0,000161 | 0,9983341 | 253,0926 | 1,3233036 | 335,26358 | RIN1 | NM_004292 | Ras and Rab interactor 1 |
| 205214_at | 1,72E-13 | 1,1014491 | 1109,4742 | 3,15141 | 2899,0364 | STK17B | NM_004226 | Serine/threonine kinase 17b (apoptosis-inducing) |
| 205237_at | 2,23E-06 | 0,9579506 | 16252,811 | 1,4065354 | 23492,887 | FCN1 | NM_002003 | Ficolin (collagen/fibrinogen domain containing) 1 |
| 205239_at | 1,56E-08 | 1,0921788 | 94,537025 | 3,3775365 | 252,75908 | AREG | NM_001657 | Amphiregulin (schwannoma-derived growth factor) |
| 205241_at | 9,97E-10 | 1,0092635 | 1679,2407 | 2,560986 | 4211,4683 | SCO2 | NM_005138 | SCO cytochrome oxidase deficient homolog 2 (yeast) |
| 205255_x_at | 5,65E-05 | 0,98771024 | 8233,648 | 0,6453767 | 5395,9956 | TCF7 | NM_003202 | Transcription factor 7 (T-cell specific, HMG-box) |
| 205256_at | 0,000107 | 0,98817956 | 165,05928 | 1,2359644 | 206,67729 | KIAA0352 | NM_014830 | KIAA0352 gene product |
| 205260_s_at | 0,000549 | 1,0402536 | 324,52225 | 0,81343347 | 255,89543 | ACYP1 | NM_001107 | Acylphosphatase 1, erythrocyte (common) type |
| 205267_at | 5,78E-06 | 1,0090047 | 1391,5444 | 0,55226755 | 816,43634 | POU2AF1 | NM_006235 | POU domain, class 2, associating factor 1 |
| 205269_at | 1,40E-05 | 1,0319345 | 1700,0776 | 0,749774 | 1235,0591 | LCP2 | AI123251 | Lymphocyte cytosolic protein 2 (SH2 domain containing leukocyte protein of 76kDa) |
| 205281_s_at | 7,90E-10 | 0,9822947 | 153,8926 | 1,7758312 | 281,541 | PIGA | NM_002641 | Phosphatidylinositol glycan, class A (paroxysmal nocturnal hemoglobinuria) |
| 205291_at | 9,36E-07 | 1,1022296 | 3241,6592 | 0,6173404 | 1840,4818 | IL2RB | NM_000878 | Interleukin 2 receptor, beta |
| 205294_at | 0,00327 | 0,8832726 | 210,88887 | 1,2341459 | 272,96362 | BAIAP2 | NM_017450 | BAI1-associated protein 2 |
| 205296_at | 0,00339 | 0,8637742 | 70,36666 | 0,6275384 | 51,654545 | | AL365505 | |
| 205297_s_at | 1,05E-08 | 1,0169411 | 804,1297 | 0,58845145 | 469,49094 | CD79B | NM_000626 CD79B | antigen (immunoglobulin-associated beta) |
| 205312_at | 0,00085 | 1,0001123 | 876,18146 | 1,7506669 | 1694,6089 | SPI1 | NM_003120 | Spleen focus forming virus (SFFV) proviral integration oncogene spi1 |
| 205327_s_at | 0,00224 | 0,9434082 | 346,0074 | 1,2019713 | 454,17725 | ACVR2 | NM_001616 | Activin A receptor, type II |
| 205340_at | 0,000459 | 0,99565405 | 391,06668 | 0,7373088 | 286,3682 | ZBTB24 | NM_014797 | Zinc finger and BTB domain containing 24 |
| 205349_at | 0,000656 | 0,9857199 | 438,1333 | 1,2444167 (Gq | 564,95905 | GNA15 | NM_002068 | Guanine nucleotide binding protein (G protein), alpha 15 class) |
| 205352_at | 2,48E-07 | 0,9808759 | 200,6037 | 0,6243108 | 134,93184 | SERPINI1 | NM_005025 | Serine (or cysteine) proteinase inhibitor, clade I (neuroserpin), member 1 |
| 205359_at | 0,00485 | 0,86276877 | 107,5037 | 1,2302525 | 144,9818 | AKAP6 | NM_004274 | A kinase (PRKA) anchor protein 6 |
| 205361_s_at | 0,00101 | 1,0129254 | 671,55914 | 0,7663548 | 513,36816 | PFDN4 | AI718295 | Prefoldin 4 |
| 205367_at | 0,00352 | 0,9694002 | 1075,2037 | 1,2121093 2 | 1342,4274 | APS | NM_020979 | Adaptor protein with pleckstrin homology and src homology domains |
| 205372_at | 0,00129 | 0,9550155 | 139,55925 | 0,58821684 | 93,13182 | PLAG1 | NM_002655 | Pleiomorphic adenoma gene 1 |
| 205400_at | 8,68E-05 | 1,0277737 | 279,39633 | 1,7098689 | 478,5591 | WAS | NM_000377 | Wiskott-Aldrich syndrome (eczema-thrombocytopenia) |
| 205401_at | 0,00807 | 0,8939685 | 284,04813 | 1,1727049 | 367,14093 | AGPS | NM_003659 | Alkylglycerone phosphate synthase |
| 205407_at | 2,87E-11 | 0,9849309 | 228,61852 | 0,45270061 | 102,98182 | RECK | NM_021111 | Reversion-inducing-cysteine-rich protein with kazal motifs |
| 205442_at | 0,00468 | 0,9410932 | 403,21854 | 0,53875446 | 268,2682 | MFAP3L | NM_021647 | Microfibrillar-associated protein 3-like |
| 205443_at | 0,000302 | 1,0116042 | 103,93704 | 0,75278306 | 76,28182 | SNAPC1 | NM_003082 | Small nuclear RNA activating complex, polypeptide 1, |
| 205474_at | 1,89E-08 | 0,9439981 | 3165,0854 | 0,6845867 | 2283,873 | CRLF3 | NM_015986 | Cytokine receptor-like factor 3 |
| 205488_at | 0,00538 | 0,9809195 | 3655,7556 | 0,64296144 | 2567,0137 | GZMA | NM_006144 | Granzyme A (granzyme 1, cytotoxic T-lymphocyte-associated serine esterase 3) |
| 205541_s_at | 6,26E-10 | 1,0153433 | 377,45554 | 0,65276945 | 244,84547 | GSPT2 | NM_018094 | G1 to S phase transition 2 |
| 205548_s_at | 7,75E-09 | 1,0267464 | 288,2852 | 1,8985933 | 552,6727 | BTG3 | | NM_006806 BTG family, member 3 |
| 205566_at | 0,00617 | 0,9328991 | 304,53336 | 1,1945984 | 393,83636 | ABHD2 | NM_007011 | Abhydrolase domain containing 2 |
| 205568_at | 0,00078 | 0,8642223 | 818,6408 | 1,3070877 | 1242,5137 | AQP9 | NM_020980 | Aquaporin 9 |
| 205571_at | 8,59E-09 | 1,0338027 | 529,57776 | 0,63797826 | 323,47723 | LIPT1 | NM_015929 | Lipoyltransferase 1 |
| 205584_at | 0,00352 | 0,94352317 | 224,41112 | 0,5580098 | 146,26364 | CXorf45 | NM_024810 | Chromosome X open reading frame 45 |
| 205585_at | 1,94E-06 | 0,97945803 | 142,72223 | 1,4019083 | 203,03181 | ETV6 | NM_001987 | Ets variant gene 6 (TEL oncogene) |
| 205590_at | 7,81 E-05 | 0,93755233 | 2219,6257 | 0,63803494 | 1525,5774 | RASGRP1 | | NM_005739 RAS guanyl releasing protein 1 (calcium and DAG- |
| 205603_s_at | 0,00152 | 1,0716748 | 359,5926 | 1,4054474 | 451,7727 | DIAPH2 | NM_007309 | Diaphanous homolog 2 (Drosophila) |
| 205607_s_at | 1,24E-05 | 0,9554782 | 281,6778 | 0,6077279 | 186,71364 | PACE-1 | NM_020423 | Ezrin-binding partner PACE-1 |
| 205619_s_at | 7,81 E-05 | 1,1053953 | 91 | 0,7472655 | 62,236366 | MEOX1 | NM_004527 | Mesenchyme homeo box 1 |
| 205621_at | 5,65E-05 | 0,9194622 | 530,85925 | 0,656056 | 369,6136 | ALKBH | NM_006020 | AlkB, alkylation repair homolog (E. coli) |
| 205627_at | 0,00618 | 0,945601 | 640,963 | 1,3051381 | 896,2228 | CDA | NM_001785 | Cytidine deaminase |
| 205636_at | 0,00502 | 0,754427 | 120,062965 | 1,2366732 | 177,80908 | SH3GL3 | AF036269 | SH3-domain GRB2-like 3 |
| 205639_at | 0,00078 | 1,0607694 | 1273,7888 | 1,3553429 | 1642,3318 | AOAH | NM_001637 | Acyloxyacyl hydrolase (neutrophil) |
| 205644_s_at | 8,52E-06 | 0,9860101 | 2380,052 | 0,6891122 | 1688,8181 | SNRPG | NM_003096 | Small nuclear ribonucleoprotein polypeptide G |
| 205660_at | 0,00282 | 0,96424 | 415,9 | 1,4159331 | 671,4182 | OASL | NM_003733 | 2'-5'-oligoadenylate synthetase-like |
| 205672_at | 0,000178 | 1,0320396 | 531,05194 | 0,8234175 | 426,04996 | XPA | NM_000380 | Xeroderma pigmentosum, complementation group A |
| 205681_at | 9,65E-05 | 1,0058682 | 1264,6187 | 1,5152181 | 1880,5092 | BCL2A1 | NM_004049 | BCL2-related protein A1 |
| 205684_s_at | 4,02E-08 | 1,00963 | 454,71848 | 0,61903846 | 280,59094 | C9orf55 | NM_017925 | Chromosome 9 open reading frame 55 |
| 205698_s_at | 4,52E-05 | 0,9989111 | 392,2148 | 1,2817268 | 507,8818 | MAP2K6 | NM_002758 | Mitogen-activated protein kinase kinase 6 |
| 205708_s_at | 5,34E-05 | 0,8880073 | 219,15184 | 1,4590863 | 348,96362 | TRPM2 | A1051254 | Transient receptor potential cation channel, subfamily M, member 2 |
| 205739_x_at | 2,77E-08 | 0,90595984 | 155,42221 | 0,3749777 | 67,17273 | ZFD25 | NM_016220 | synonym: smap-7; C2H2 type zinc-finger protein; go_component: nucleus [goid 0005634] [evidence IEA]; go-function: DNA binding [goid 0003677] [evidence IEA]; go_function: zinc ion binding [goid 0008270] [evidence IEA]; go_process: regulation of transcripti |
| 205760_s_at | 0,000656 | 0,96562314 | 328,06665 | 0,7273411 | 247,34093 | OGG1 | NM_016821 | 8-oxoguanine DNA glycosylase |
| 205767_at | 0,000419 | 1,1133398 | 41,837036 | 2,1318667 | 76,704544 | EREG | NM_001432 | Epiregulin |
| 205771_s_at | 9,65E-05 | 1,0107746 | 518,01843 | 0,78560394 | 398,3727 | AKAP7 | AL137063 | |
| 205781_at | 0,00807 | 1,0119042 | 288,25928 | 1,2885865 | 366,23636 | C16orf7 | NM_004913 | Chromosome 16 open reading frame 7 |
| 205798_at | 8,68E-05 | 0,9490081 | 7370,6367 | 0,55871075 | 4292,345 | IL7R | NM_002185 | Interleukin 7 receptor |
| 205804_s_at | 0,000161 | 1,0159029 | 2163,0444 | 0,75641376 | 1615,0044 | T3JAM | NM_025228 | TRAF3-interacting Jun N-terminal kinase (JNK)-activating modulator |
| 205831_at | 2,57E-06 | 0,93635136 | 4983,911 | 0,61131835 | 3336,6455 | CD2 | NM_001767 | CD2 antigen (p50), sheep red blood cell receptor |
| 205849_s_at | 0,00078 | 0,96189433 | 8683,074 | 0,75183785 | 6698,0454 | UQCRB | NM_006294 | Ubiquinol-cytochrome c reductase binding protein |
| 205861_at | 8,59E-09 | 0,9876178 | 652,5444 | 0,49984264 | 333,01822 | SPIB | NM_003121 | Spi-B transcription factor (Spi-1/PU.1 related) |
| 205863_at | 6,59E-06 | 0,9728798 | 5657,5815 | 1,4452617 | 8535,97 | S100A12 | NM_005621 | S100 calcium binding protein A12 (calgranulin C) |
| 205873_at | 1,09E-06 | 0,8546061 | 242,72964 | 1,3139188 | 354,09094 | PIGL | NM_004278 | Phosphatidylinositol glycan, class L |
| 205887_x at | 0,00327 | 1,0761358 | 246,13335 | 0,74438643 | 152,15457 | MSH3 | NM_002439 | MutS homolog 3 (E. coli) |
| 205896_at | 3,88E-06 | 0,98461145 | 186,2963 | 1,3787628 | 259,10455 | SLC22A4 | NM_003059 | Solute carrier family 22 (organic cation transporter), member |
| 205898_at | 4,94E-10 | 1,003197 | 9469,832 | 0,45819956 | 4854,5366 | CX3CR1 | U20350 | Chemokine (C-X3-C motif) receptor 1 |
| 205928_at | 1,64E-07 | 0,957803 | 281,7704 | 0,39075977 | 135,70454 | ZNF443 | NM_005815 | Zinc finger protein 443 |
| 205930_at | 2,56E-05 | 0,95518565 | 534,8889 | 0,69352925 | 391,62723 | GTF2E1 | NM_005513 | General transcription factor IIE, polypeptide 1, alpha 56kDa |
| 205931_s at | 0,000814 | 0,97320986 | 132,18889 | 1,5848708 | 196,48183 | CREB5 | NM_004904 | CAMP responsive element binding protein 5 |
| 205936_s_at | 1,79E-05 | 0,96616024 | 1912,6852 | 1,4814912 | 2868,0273 | HK3 | NM_002115 | Hexokinase 3 (white cell) |
| 205945_at | 5,78E-06 | 0,939898 | 1033,4594 | 0,64158833 | 699,7318 | IL6R | NM_000565 | Interleukin 6 receptor |
| 205949_at | 0,0052 | 0,7638101 | 87,55926 | 1,3017106 | 120,722725 | CA1 | M33987 | Carbonic anhydrase I |
| 205950_s_at | 0,000317 | 1,0968126 | 208,87038 | 2,101613 | 406,9091 | CA1 | NM_001738 | Carbonic anhydrase I |
| 205953_at | 2,87E-05 | 0,9791038 | 311,16666 | 1,2769772 | 398,82272 | LRIG2 | NM_014813 | Leucine-rich repeats and immunoglobulin-like domains 2 |
| 205961_s at | 2,57E-06 | 0,9751219 | 1070,3702 | 0,59289265 | 676,0318 | PSIP2 | NM_004682 | |
| 205964_at | 1,94E-07 | 1,0759542 | 152,8852 | 0,48679373 | 74,99546 | ZNF426 | NM_024106 | Zinc finger protein 426 |
| 205987_at | 0,00755 | 0,98993284 | 801,8 | 0,7748195 | 640,4 | CD1C | NM_001765 | CD1C antigen, c polypeptide |
| 205997_at | 6,29E-05 | 0,9121615 | 340,37778 | 0,5454981 | 223,57272 | ADAM28 | NM_021778 | synonyms: MDCL, ADAM23, MDC-Lm, MDC-Ls, eMDCII; isoform 1 preproprotein is encoded by transcript variant 1; metalloproteinase-like, disintegrin-like, and cysteine-rich protein-L; go_component: integral to membrane [goid 0016021] [evidence IEA]; go_functio |
| 206003_at | 0,000502 | 0,9869305 | 274,00742 | 0,7489111 | 211,57274 | KIAA0635 | NM_014645 | |
| 206044_s_at | 0,00485 | 0,9828316 | 137,07036 | 1,4717232 | 189,93636 | BRAF | NM_004333 | V-raf murine sarcoma viral oncogene homolog B1 |
| 206050_s_at | 2,95E-06 | 0,91543883 | 3390,6704 | 1,3953271 | 5089,4053 | RNH | NM_002939 | Ribonuclease/angiogenin inhibitor |
| 206059_at | 0,00208 | 0,95179147 | 1889,9962 | 0,69559884 | 1330,5544 | ZNF91 | NM_003430 | Zinc finger protein 91 (HPF7, HTF10) |
| 206082_at | 4,99E-11 | 0,9781559 | 645,61475 | 0,45467013 | 301,38184 | HCP5 | NM_006674 | HLA complex P5 |
| 206095_s_at | 1,68E-06 | 1,0139017 | 879,0075 | 0,70354265 | 613,3501 | FUSIP1 | NM_006625 | FUS interacting protein (serine-arginine rich) 1 |
| 206111_at | 0,000715 | 0,9690798 | 2099,4075 | 1,3870484 | 3222,6138 | RNASE2 | NM_002934 | Ribonuclease, RNase A family, 2 (liver, eosinophil-derived neurotoxin) |
| 206118_at | 7,81E-05 | 0,90816057 | 1531,9742 | 0,67373896 | 1147,0137 | STAT4 | NM_003151 | Signal transducer and activator of transcription 4 |
| 206129_s_at | 0,000459 | 0,9120293 | 133,06296 | 1,2989491 | 181,17273 | ARSB | NM_000046 | Arylsulfatase B |
| 206130_s_at | 4,35E-07 | 0,98453707 | 501,57034 | 1,9876539 | 1078,4226 | ASGR2 | NM_001181 | Asialoglycoprotein receptor 2 |
| 206134_at | 1,90E-05 | 0,8554028 | 62,17407 | 1,4803754 | 97,65454 | ADAMDEC1 | NM_014479 | ADAM-like, decysin 1 |
| 206150_at | 1,40E-05 | 0,96213406 | 2425,363 | 0,6255607 | 1592,2183 | TNFRSF7 | NM_001242 | Tumor necrosis factor receptor superfamily, member 7 |
| 206167_s_at | 0,00707 | 0,9658439 | 181,8778 | 0,6749217 | 132,58638 | ARHGAP6 | NM_001174 | Rho GTPase activating protein 6 |
| 206181_at | 0,000317 | 0,9738705 | 437,04816 | 0,72151303 | 323,91364 | SLAMF1 | NM_003037 | Signaling lymphocytic activation molecule family member 1 |
| 206188_at | 5,78E-06 | 1,0606097 | 94,87038 | 0,57892036 | 52,87727 | ZNF623 | NM_014789 | Zinc finger protein 623 |
| 206206_at | 1,94E-07 | 1,0036927 | 615,7259 | 0,55194575 (mouse) | 340,8818 | LY64 | NM_005582 | Lymphocyte antigen 64 homolog, radioprotective 105kDa |
| 206212_at | 0,00406 | 0,89964956 | 208,66667 | 1,2222555 | 268,27725 | CPA2 | NM_001869 | Carboxypeptidase A2 (pancreatic) |
| 206214_at | 5,78E-06 | 0,9467313 | 333,47037 | 1,5513179 | 527,2545 | PLA2G7 | NM_005084 | Phospholipase A2, group VII (platelet-activating factor acetylhydrolase, plasma) |
| 206245_s_at | 0,0006 | 0,96891665 | 1318,6482 | 0,7549086 | 1037,8455 | IVNS1ABP | NM_006469 | Influenza virus NS1A binding protein |
| 206255_at | 0,0006 | 1,0148677 | 259,13702 | 0,7553745 | 198,36818 | BLK | NM_001715 | B lymphoid tyrosine kinase |
| 206283_s_at | 0,00129 | 0,97842056 | 356,47775 | 0,68749416 | 263,33636 | TAL1 | NM_003189 | T-cell acute lymphocytic leukemia 1 |
| 206332_s_at | 0,00502 | 1,0075828 | 3379,8074 | 0,8017261 | 2790,4043 | IFI16 | NM_005531 | Interferon, gamma-inducible protein 16 |
| 206337_at | 1,40E-05 | 1,0059352 | 2629,196 | 0,5546521 | 1488,541 | CCR7 | NM_001838 | Chemokine (C-C motif) receptor 7 |
| 206342_x_at | 1,17E-07 | 1,0226202 | 728,29254 | 1,5328966 | 1110,2955 | IDS | NM_006123 | Iduronate 2-sulfatase (Hunter syndrome) |
| 206359_at | 7,92E-10 | 1,0424414 | 98,84815 | 3,99703 | 316,84998 | SOCS3 | BG035761 | Suppressor of cytokine signaling 3 |
| 206366_x_at | 6,30E-05 | 0,8988548 | 637,2148 | 0,47311822 | 344,7363 | XCL1 | U23772 | Chemokine (C motif) ligand 2 |
| 206374_at | 9,36E-07 | 0,9154671 | 170,28148 | 1,9131181 | 375,3682 | DUSP8 | NM_004420 | Dual specificity phosphatase 8 |
| 206380_s_at | 2,56E-05 | 0,9283159 | 2388,0593 | 1,3377103 | 3431,3455 | PFC | NM_002621 | Properdin P factor, complement |
| 206383_s_at | 0,00109 | 0,9776289 | 230,80742 | 1,4631647 | 347,45456 | G3BP2 | NM_012297 | Ras-GTPase activating protein SH3 domain-binding protein |
| 206420_at | 1,79E-05 | 0,97862685 | 587,4667 | 1,4394106 | 869,42725 | IGSF6 | NM_005849 | Immunoglobulin superfamily, member 6 |
| 206437_at | 0,00224 | 0,96383053 | 1089,7926 | 0,7429548 | 850,07275 | EDG6 | NM_003775 | Endothelial differentiation, G-protein-coupled receptor 6 |
| 206472_s_at | 3,79E-11 | 0,97358805 | 256,05185 | 1,6357483 | 434,8045 | TLE3 | NM_005078 | Transducin-like enhancer of split 3 (E(sp1) homolog, Drosophila) |
| 206474_at | 0,00538 | 0,9701197 | 139,20741 | 1,2798501 | 186,08635 | PCTK2 | NM_002595 | PCTAIRE protein kinase 2 |
| 206488_s_at | 0,00304 | 0,92366505 | 1649,0334 | 1,2454797 | 2190,7817 | CD36 | NM_000072 | CD36 antigen (collagen type I receptor, thrombospondin receptor) |
| 206499_s_at | 0,00861 | 0,96946037 | 218,51851 | 1,216277 | 271,1182 | CHC1 | NM_001269 | RNA, U17D small nucleolar |
| 206525_at | 0,000502 | 0,946749 | 58,714817 | 1,4686053 | 91,013626 | GABRR1 | NM_002042 | Gamma-aminobutyric acid (GABA) receptor, rho 1 |
| 206542_s_at | 6,59E-06 | 0,97071654 | 933,0852 | 0,669143 | 649,7954 | SMARCA2 | AV725365 | SWI/SNF related, matrix associated, actin dependent regulator of chromatin, subfamily a, member 2 |
| 206555_s_at | 0,00618 | 1,0439512 | 1014,7333 | 0,8083851 | 761,7637 | THUMPD1 | NM_017736 | THUMP domain containing 1 |
| 206571_s_at | 2,95E-06 | 0,9570565 | 724,80005 | 1,2688771 | 962,4001 | MAP4K4 | NM_004834 | Mitogen-activated protein kinase kinase kinase kinase 4 |
| 206572_x_at | 1,59E-05 | 1,0195923 | 433,12958 | 0,7128081 | 298,5182 | ZNF85 | NM_003429 | Zinc finger protein 85 (HPF4, HTF1) |
| 206583_at | 8,59E-09 | 1,0364846 | 313,02597 | 0,6682447 | 203,87274 | FLJ20344 | NM_017776 | Hypothetical protein FLJ20344 |
| 206613_s_at | 0,000262 | 1,0025631 | 112,262955 | 0,6453613 | 71,49547 | TAF1A | NM_005681 | TATA box binding protein (TBP)-associated factor, RNA polymerase I, A, 48kDa |
| 206618_at | 0,000216 | 0,98184896 | 192,97777 | 0,6293124 | 123,72274 | IL18R1 | NM_003855 | Interleukin 18 receptor 1 |
| 206637_at | 1,26E-06 | 0,92596304 | 312,62964 | 0,4968452 | 176,3682 | P2RY14 | NM_014879 | Purinergic receptor P2Y, G-protein coupled, 14 |
| 206649_s_at | 0,000119 | 0,97970337 | 323,8111 | 1,3196398 | 439,4727 | TFE3 | NM_006521 | Transcription factor binding to IGHM enhancer 3 |
| 206666_at | 0,00661 | 1,0269649 | 3077,5593 | 0,7038321 | 2165,2227 | GZMK | NM_002104 | Granzyme K (serine protease, granzyme 3; tryptase II) |
| 206695_x_at | 0,000419 | 0,94053787 | 275,37405 | 0,6908804 | 203,46819 | ZNF43 | NM_003423 | Zinc finger protein 43 (HTF6) |
| 206710_s_at | 0,00178 | 0,95811856 | 444,98148 | 1,4360967 | 683,21826 | EPB41L3 | NM_012307 | Erythrocyte membrane protein band 4.1-like 3 |
| 206715_at | 0,000317 | 1,0215279 | 427,82962 | 1,4083345 | 578,4636 | TFEC | NM_012252 | Transcription factor EC |
| 206723_s_at | 0,00282 | 0,9117563 | 492,6111 | 1,2884289 | 669,2864 | EDG4 | AF011466 | Endothelial differentiation, lysophosphatidic acid G-protein-coupled receptor, 4 |
| 206724_at | 0,00378 | 1,0489131 | 324,35556 | 1,6674724 | 546,44086 | CBX4 | NM_003655 | Chromobox homolog 4 (Pc class homolog, Drosophila) |
| 206734_at | 1,17E-07 | 0,9364866 | 239,1037 | 0,45287612 | 122,67727 | JRKL | NM_003772 | Jerky homolog-like (mouse) |
| 206743_s_at | 0,00185 | 0,9420949 | 366,65924 | 1,2946452 | 496,10452 | ASGR1 | NM_001671 | Asialoglycoprotein receptor 1 |
| 206761_at | 4,05E-05 | 0,91992515 | 754,71844 | 0,561376 | 451,39545 | CD96 | NM_005816 | CD96 antigen |
| 206770_s_at | 0,00661 | 1,0495725 | 340,67035 | 0,83711576 GIcNAc) | 266,45453 | SLC35A3 | NM_012243 | Solute carrier family 35 (UDP-N-acetylglucosamine (UDP-transporter), member A3 |
| 206828_at | 8,52E-06 | 1,0133451 | 475,28513 | 0,5724742 | 262,90457 | TXK | NM_003328 | TXK tyrosine kinase |
| 206829_x_at | 0,000107 | 0,99755496 | 873,6963 | 0,7052455 | 605,12726 | ZNF430 | NM_025189 | Zinc finger protein 430 |
| 206874_s_at | 4,82E-08 | 0,98092055 | 607,5296 | 0,66982996 | 414,9455 | SLK | AL138761 | |
| 206881_s at | 0,0098 | 0,869484 | 978,21857 | 1,3938819 (without | 1199,0682 | LILRA3 | NM_006865 | Leukocyte immunoglobulin-like receptor, subfamily A TM domain), member 3 |
| 206896_s_at | 0,000886 | 0,9885693 | 265,7667 | 0,7495058 | 204,8318 | GNG7 | NM_005145 | synonym: FLJ00058; go_component: heterotrimeric G-protein complex [goid 0005834] [evidence IEA]; go_function: signal transducer activity [goid 0004871] [evidence IEA]; go_process: signal transduction [goid 0007165] [evidence IEA]; go_process: regulation o |
| 206907_at | 1,94E-07 | 0,97714937 | 116,29259 | 1,4927702 | 177,52274 | TNFSF9 | NM_003811 | Tumor necrosis factor (ligand) superfamily, member 9 |
| 206934_at | 0,000502 | 0,96148753 | 275,82593 | 1,4363945 | 404,0136 | SIRPB1 | NM_006065 | Signal-regulatory protein beta 1 |
| 206976_s_at | 2,87E-11 | 0,93982273 | 968,12213 | 0,48571798 | 495,2364 | HSPH1 | NM_006644 | Heat shock 105kDa/110kDa protein 1 |
| 206978_at | 0,00164 | 0,8393954 | 3009,2368 | 0,53181165 | 1939,7637 | CCR2 | NM_000647 | Chemokine (C-C motif) receptor 2 |
| 206983_at | 1,05E-08 | 0,97520065 | 270,72223 | 0,5031669 | 143,65456 | CCR6 | NM_004367 | Chemokine (C-C motif) receptor 6 |
| 206989_s_at | 7,01 E-09 | 1,1548706 | 2128,2703 | 0,690697 | 1222,5908 | SFRS2IP | NM_004719 | Splicing factor, arginine/serine-rich 2, interacting protein |
| 206991_s at | 0,00216 | 0,9555151 | 549,3629 | 0,5771179 | 372,1818 | CCR5 | NM_000579 | Chemokine (C-C motif) receptor 5 |
| 207001_x at | 4,53E-05 | 0,8213901 | 862 | 1,566674 | 1631,7953 | DSIPI | NM_004089 | Delta sleep inducing peptide, immunoreactor |
| 207008_at | 2,02E-05 | 1,046576 | 268,99634 | 0,6368181 | 208,40909 | IL8RB | NM_001557 | Interleukin 8 receptor, beta |
| 207038_at | 6,59E-06 | 1,0038418 | 227,15556 | 1,7118729 | 375,84998 | SLC16A6 | NM_004694 | Solute carrier family 16 (monocarboxylic acid transporters), member 6 |
| 207072_at | 0,00152 | 0,93798107 | 752,12225 | 0,55936176 | 437,32727 | IL18RAP | NM_003853 | Interleukin 18 receptor accessory protein |
| 207075_at | 0,000107 | 0,93826854 | 379,14813 | 1,4517694 | 614,5364 | CIAS1 | NM_004895 | Cold autoinflammatory syndrome 1 |
| 207079_s_at | 0,00224 | 1,0232959 | 424,02963 | 0,807015 homolog | 341,12274 | MED6 | NM_005466 | Mediator of RNA polymerase II transcription, subunit 6 (yeast) |
| 207098_s_at | 2,87E-05 | 0,9922679 | 235,77779 | 0,6400355 | 150,36818 | MFN1 | NM_017927 | Mitofusin 1 |
| 207108_s_at | 7,02E-06 | 0,90142196 | 300,28522 | 0,4866979 | 167,9909 | NIPBL | NM_015384 | Nipped-B homolog (Drosophila) |
| 207131_x_at | 0,000549 | 0,9898282 | 376,28885 | 1,2604837 | 486,85913 | GGT1 | NM_013430 | Gamma-glutamyltransferase 1 |
| 207163_s_at | 3,22E-05 | 0,9505957 | 947,86664 | 1,2405987 | 1230,4409 | AKT1 | NM_005163 | V-akt murine thymoma viral oncogene homolog 1 |
| 207187_at | 2,55E-05 | 0,9156119 | 442,66296 | 1,4657593 | 678,0909 | JAK3 | NM_000215 | Janus kinase 3 (a protein tyrosine kinase, leukocyte) |
| 207205_at | 0,000107 | 0,94531757 | 248,81111 | 1,3083093 | 344,47272 | CEACAM4 | NM_001817 | Carcinoembryonic antigen-related cell adhesion molecule 4 |
| 207223_s_at | 0,00101 | 1,0103309 | 207,59998 | 1,3298135 | 275,52725 | ROD1 | NM_005156 | ROD1 regulator of differentiation 1 (S. pombe) |
| 207233_s_at | 0,00436 | 1,049985 | 232,04445 | 1,3255228 | 285,30002 | MITF | NM_000248 | Microphthalmia-associated transcription factor |
| 207238_s_at | 0,000925 | 0,9814381 | 6045,93 | 0,72977746 | 4548,5 | PTPRC | NM_002838 | Protein tyrosine phosphatase, receptor type, C |
| 207239_s_at | 0,000925 | 0,8993004 | 193,6 | 1,2086886 | 259,84094 | PCTK1 | NM_006201 | PCTAIRE protein kinase 1 |
| 207275_s_at | 3,88E-06 | 0,91429746 | 739,30365 | 1,6456845 | 1375,7136 | ACSL1 | NM_001995 | Acyl-CoA synthetase long-chain family member 1 |
| 207304_at | 6,64E-05 | 0,8829249 | 106,037025 | 0,5788494 | 68,81362 | ZNF45 | NM_003425 | Zinc finger protein 45 |
| 207332_s_at | 1,40E-05 | 0,98878455 | 1008,226 | 1,3905629 | 1474,1091 | TFRC | NM_003234 | Transferrin receptor (p90, CD71) |
| 207338_s_at | 1,72E-13 | 0,99163634 | 398,71854 | 0,41501635 | 165,85455 | ZNF200 | NM_003454 | Zinc finger protein 200 |
| 207339_s_at | 0,00129 | 1,0136005 | 6646,74 | 0,75580007 | 4978,405 | LTB | NM_002341 | Lymphotoxin beta (TNF superfamily, member 3) |
| 207351_s_at | 0,00707 | 1,0417856 | 596,3296 | 0,83023864 | 481,7182 | SH2D2A | NM_003975 | SH2 domain protein 2A |
| 207391_s_at | 0,00807 | 0,88157976 | 469,2555 | 1,1884971 | 617,34546 | PIP5K1A | NM_003557 | Phosphatidylinositol-4-phosphate 5-kinase, type I, alpha |
| 207405_s_at | 7,01 E-09 | 1,0661775 | 438,9926 | 0,632504 | 260,7909 | RAD17 | NM_002873 | RAD17 homolog (S. pombe) |
| 207435_s_at | 0,000146 | 0,8535312 | 1611,974 | 1,1725703 | 2156,1682 | SRRM2 | NM_016333 | Serine/arginine repetitive matrix 2 |
| 207436_x_at | 0,00085 | 1,0145249 | 1308,0964 | 0,77990735 | 1015,4545 | KIAA0894 | NM_014896 | |
| 207438_s_at | 0,000131 | 0,9869958 | 656,8667 | 0,7491889 | 516,2363 | RNUT1 | NM_005701 | RNA, U transporter 1 |
| 207483_s_at | 1,59E-05 | 0,98045343 | 517,42584 | 0,69500697 | 365,55457 | TIP120A | NM_018448 | TBP-interacting protein |
| 207513_s_at | 2,87E-11 | 1,0376413 | 368,03705 | 0,444551 | 165,4409 | ZNF189 | NM_003452 | Zinc finger protein 189 |
| 207535_s_at | 1,68E-06 | 1,0187523 | 458,51483 | 1,5827341 B-cells | 719,7455 | NFKB2 | NM_002502 | Nuclear factor of kappa light polypeptide gene enhancer in 2 (p49/p100) |
| 207571_x_at | 8,52E-06 | 0,98730636 | 2887,7927 | 1,3811675 | 3989,2366 | C1orf38 | NM_004848 | Chromosome 1 open reading frame 38 |
| 207574_s_at | 2,87E-11 | 0,97425103 | 1343,0074 | 1,9265866 | 2673,5457 | GADD45B | NM_015675 | Growth arrest and DNA-damage-inducible, beta |
| 207630_s_at | 0,00078 | 1,0122817 | 346,7296 | 1,3737528 | 487,25452 | CREM | NM_001881 | CAMP responsive element modulator |
| 207643_s_at | 0,00406 | 0,85770273 | 1744,8702 | 0,6710538 | 1308,7092 | TNFRSF1A | NM_001065 | Tumor necrosis factor receptor superfamily, member 1A |
| 207651_at | 0,000107 | 0,8820024 | 805,1852 | 0,5203913 | 505,45453 | GPR171 | NM_013308 | G protein-coupled receptor 171 |
| 207655_s_at | 4,53E-05 | 1,0733275 | 631,2333 | 0,72450244 | 444,2909 | BLNK | NM_013314 | B-cell linker |
| 207667_s_at | 0,000925 | 0,95617604 | 723,7297 | 1,2626284 | 951,2909 | MAP2K3 | NM_002756 | Mitogen-activated protein kinase kinase 3 |
| 207674_at | 2,02E-05 | 0,96931463 | 256,85925 | 1,7891539 | 494,1 | FCAR | NM_002000 | Fc fragment of IgA, receptor for |
| 207677_s_at | 0,00233 | 0,9708517 | 942,64075 | 1,2679921 | 1268,9773 | NCF4 | NM_013416 | Neutrophil cytosolic factor 4, 40kDa |
| 207721_x_at | 2,56E-05 | 0,9706819 | 7202,4004 | 0,76230186 | 5610,254 | HINT1 | NM_005340 | Histidine triad nucleotide binding protein 1 |
| 207734_at | 1,49E-05 | 1,0042657 | 612,3222 | 0,7302414 | 458,59546 | LAX | NM_017773 | Hypothetical protein FLJ20340 |
| 207780_at | 0,00706 | 0,96808034 | 62,151844 | 1,2827252 | 79,172714 | CYLC2 | NM_001340 | Cylicin, basic protein of sperm head cytoskeleton 2 |
| 207812_s_at | 3,80E-11 | 0,9689855 | 1162,6222 | 0,6776414 | 815,7455 | GORASP2 | NM_015530 | Golgi reassembly stacking protein 2, 55kDa |
| 207845_s_at | 4,82E-08 | 1,0042965 | 222,21854 | 0,6473207 | 149,14091 | ANAPC10 | NM_014885 | Anaphase promoting complex subunit 10 |
| 207847_s_at | 0,00436 | 0,8730956 | 115,41482 | 1,2885568 | 172,37271 | MUC1 | NM_002456 | Mucin 1, transmembrane |
| 207855_s_at | 0,00152 | 1,0152326 | 69,6 | 0,7070893 | 50,395454 | MCLC | NM_015127 | Mid-1-related chloride channel 1 |
| 207922_s_at | 2,29E-08 | 1,0070108 | 1518,515 | 0,6933947 | 1035,341 | MAEA | NM_005882 | Macrophage erythroblast attacher |
| 207951_at | 0,00979 | 0,98450094 | 108,570366 | 1,4119322 | 165,42728 | CSN2 | NM_001891 | Casein beta |
| 207969_x_at | 0,00807 | 0,9203953 | 184,1148 | 1,2512091 | 250,46365 | ACRV1 | NM_020109 | Acrosomal vesicle protein 1 |
| 207993_s_at | 2,29E-08 | 0,97460216 | 194,1926 | 1,9397616 | 365,31366 | CHP | NM_007236 | Calcium binding protein P22 |
| 207996_s_at | 6,91 E-08 | 0,9944365 | 182,87776 | 0,645627 | 118,100006 | C18orf1 | NM_004338 | Chromosome 18 open reading frame 1 |
| 208003_s_at | 0,00918 | 1,0078622 | 186,54073 | 1,3503218 | 255,64546 | NFAT5 | NM_006599 | Nuclear factor of activated T-cells 5, tonicity-responsive |
| 208010_s_at | 0,00304 | 1,0558908 | 184,9074 | 0,7018276 | 127,636375 | PTPN22 | NM_012411 | Protein tyrosine phosphatase, non-receptor type 22 |
| 208055_s_at | 2,23E-06 | 0,94093585 | 113,12222 | 0,43955356 | 58,409092 | HERC4 | NM_015601 | Hect domain and RLD 4 |
| 208078_s_at | 9,06E-12 | 1,217317 | 571,1963 | 4,0775585 | 1761,959 | TCF8 | NM_030751 | Transcription factor 8 (represses interleukin 2 expression) |
| 208089_s_at | 5,34E-05 | 1,0123385 | 200 | 0,6853437 | 129,99545 | TDRD3 | NM_030794 | Tudor domain containing 3 |
| 208092_s_at | 1,96E-09 | 0,98712504 | 614,11487 | 1,6081595 | 991,9181 | FAM49A | NM_030797 | Family with sequence similarity 49, member A |
| 208120_x_at | 0,000925 | 1,0801834 | 835,65186 | 1,4207629 | 1132,7864 | | NM_031221 | |
| 208127_s_at | 1,69E-05 | 0,9518257 | 213,82962 | 0,6639329 | 151,4909 | SOCS5 | NM_014011 | Suppressor of cytokine signaling 5 |
| 208130_s_at | 0,00208 | 1,0494566 | 1004,84814 | 1,331283 family | 1267,4773 | TBXAS1 | NM_030984 | Thromboxane A synthase 1 (platelet, cytochrome P450, 5, subfamily A) |
| 208137_x_at | 7,02E-05 | 1,0493256 | 903,62585 | 0,655341 | 533,64087 | ZNF611 | NM_030972 | Zinc finger protein 611 |
| 208200_at | 5,09E-07 | 1,0649408 | 163,49629 | 1,6929816 | 253,64548 | IL1A | NM_000575 | Interleukin 1, alpha |
| 208248_x_at | 0,000348 | 0,97037005 | 6165,303 | 1,3472277 | 8466,431 | APLP2 | NM_001642 | Amyloid beta (A4) precursor-like protein 2 |
| 208284_x_at | 3,61 E-05 | 0,94734406 | 357,59628 | 1,2467024 | 474,60907 | GGT1 | NM_013421 | Gamma-glutamyltransferase 1 |
| 208296_x_at | 1,94E-07 | 0,978316 | 2639,4739 | 0,66343063 | 1831,3864 | TNFAIP8 | NM_014350 | Tumor necrosis factor, alpha-induced protein 8 |
| 208313_s_at | 5,07E-06 | 0,9764321 | 2737,3486 | 1,3047135 | 3711,4998 | SF1 | NM_004630 | Splicing factor 1 |
| 208322_s_at | 0,000656 | 0,8706426 | 349,19632 | 0,40479997 | 205,57274 | SIAT4A | NM_003033 | Sialyltransferase 4A (beta-galactoside alpha-2,3-sialyltransferase) |
| 208360_s_at | 0,00406 | 0,8801147 | 138,40738 | 1,4020115 | 211,09999 | | NM_015870 | |
| 208398_s_at | 3,04E-05 | 1,010518 | 675,2778 | 0,74776757 | 507,71368 | TBPL1 | NM_004865 | TBP-like 1 |
| 208415_x_at | 0,00208 | 0,965897 | 1093,6111 | 1,2668879 | 1441,6091 | ING1 | NM_005537 | Inhibitor of growth family, member 1 |
| 208424_s_at | 0,000317 | 0,9710698 | 334,8111 | 0,76202935 | 263,0864 | CIAPIN1 | NM_020313 | Cytokine induced apoptosis inhibitor 1 |
| 208442_s_at | 0,0014 | 1,0576371 | 1391,185 | 0,7336332 | 930,641 | ATM | NM_000051 | Ataxia telangiectasia mutated (includes complementation groups A, C and D) |
| 208445_s_at | 0,000317 | 0,94771916 | 507,20364 | 0,7234049 | 388,69998 | BAZ1B | NM_023005 | Bromodomain adjacent to zinc finger domain, 1B |
| 208452_x_at | 0,00327 | 0,8911629 | 279,42963 | 1,3000413 | 426,09546 | MYO9B | NM_004145 | Myosin IXB |
| 208478_s_at | 0,0052 | 0,9423569 | 269,35928 | 1,2715645 | 365,00912 | BAX | NM_004324 | BCL2-associated X protein |
| 208488_s_at | 0,0014 | 0,89084876 | 230,01114 | 1,2859637 | 346,1273 | CR1 | NM_000651 | Complement component (3b/4b) receptor 1, including Knops blood group system |
| 208498_s_at | 0,00406 | 0,9812354 | 239,75185 | 0,66664076 | 167,24092 | AMY1A | NM_004038 | Amylase, alpha 1A; salivary |
| 208581_x_at | 0,000925 | 0,97296906 | 1488,3445 | 1,4133755 | 2235,382 | MT1X | NM_005952 | Metallothionein 1X |
| 208594_x_at | 0,000419 | 0,8969827 | 1534,3854 | 1,2911274 TM | 2199,6863 | LILRB3 | NM_024318 | Leukocyte immunoglobulin-like receptor, subfamily B (with and ITIM domains), member 6 |
| 208622_s_at | 9,65E-05 | 0,94272894 | 1255,6519 | 1,4229616 | 1901,4454 | VIL2 | NM_003379 | Villin 2 (ezrin) |
| 208626_s_at | 0,00192 | 1,0077794 | 594,16296 | 1,2671428 | 752,9227 | VAT1 | BC001913 | Vesicle amine transport protein 1 homolog (T californica) |
| 208632_at | 9,67E-06 | 1,01152 | 752,38885 | 1,3326536 | 1002,9591 | RNF10 | AL578551 | Ring finger protein 10 |
| 208644_at | 1,94E-07 | 1,0035154 | 1031,4185 | 0,78230196 | 806,6 | PARP1 | M32721 | Poly (ADP-ribose) polymerase family, member 1 |
| 208646_at | 0,00208 | 1,0666835 | 7976,5034 | 0,6825166 | 4997,277 | RPS14 | AF116710 | PRO2640 |
| 208648_at | 0,0006 | 1,0164026 | 728,3369 | 1,3022143 | 933,38635 | VCP | W60953 | Valosin-containing protein |
| 208650_s_at | 0,00352 | 1,0807047 | 175,31113 | 0,65996605 | 126,622734 | CD24 | BG327863 | CD24 antigen (small cell lung carcinoma cluster 4 antigen) |
| 208671_at | 2,02E-05 | 0,9734908 | 2416,0999 | 0,72120506 | 1808,2091 | TDE2 | AF164794 | Tumor differentially expressed 2 |
| 208677_s_at | 0,000502 | 0,952018 | 916,0667 | 1,3219589 | 1303,5365 | BSG | AL550657 | Basigin (OK blood group) |
| 208686_s_at | 0,00618 | 0,9108491 | 1016,30743 | 1,2391678 | 1357,891 | BRD2 | D42040 | Bromodomain containing 2 |
| 208687_x_at | 8,59E-09 | 1,0262376 | 10999,117 | 0,67020625 | 7088,3774 | HSPA8 | AF352832 | Heat shock 70kDa protein 8 |
| 208691_at | 1,94E-07 | 0,9467626 | 1178,6703 | 1,3983712 | 1745,591 | TFRC | BC001188 | Transferrin receptor (p90, CD71) |
| 208694_at | 4,35E-07 | 1,0867181 | 569,60004 | 0,68965906 | 360,34546 | PRKDC | U47077 | Protein kinase, DNA-activated, catalytic polypeptide |
| 208699_x_at | 8,52E-06 | 1,0111339 | 4652,418 | 1,409021 | 6452,3315 | TKT | BF696840 | Transketolase (Wernicke-Korsakoff syndrome) |
| 208700_s_at | 2,02E-05 | 0,9815242 | 6568,466 | 1,2647492 | 8394,386 | TKT | L12711 | Transketolase (Wernicke-Korsakoff syndrome) |
| 208702_x_at | 0,0006 | 1,0229028 | 4255,4927 | 1,3528019 | 5656,4683 | APLP2 | BC000373 | Amyloid beta (A4) precursor-like protein 2 |
| 208703_s_at | 3,41E-05 | 0,97797394 | 2943,196 | 1,3913344 | 4156,949 | APLP2 | BC000373 | Amyloid beta (A4) precursor-like protein 2 |
| 208704_x_at | 0,000382 | 0,9419732 | 5896,6143 | 1,2648691 | 7783,2314 | APLP2 | BC000373 | Amyloid beta (A4) precursor-like protein 2 |
| 208708_x_at | 1,94E-06 | 0,96547127 | 1093,7742 | 0,7241907 | 817,6727 | EIF5 | AL080102 | Eukaryotic translation initiation factor 5 |
| 208726_s_at | 1,89E-08 | 1,0125123 | 2644,437 | 0,80315673 | 2070,441 | EIF2S2 | BC000461 | Eukaryotic translation initiation factor 2, subunit 2 beta, |
| 208727_s_at | 0,000925 | 1,08024 | 3443,7485 | 1,9909154 | 5313,086 | CDC42 | BC002711 | Cell division cycle 42 (GTP binding protein, 25kDa) |
| 208731_at | 0,000107 | 0,9835797 | 761,87775 | 0,73110205 | 580,11816 | RAB2 | NM_002865 RAB2, | member RAS oncogene family |
| 208734_x_at | 0,000196 | 1,0218952 | 1002,75183 | 1,2816571 | 1241,4271 | RAB2 | M28213 | RAB2, member RAS oncogene family |
| 208735_s_at | 9,36E-07 | 0,9619532 | 726,3555 | 1,2983685 | 967,97723 | CTDSP2 | AF022231 | CTD (carboxy-terminal domain, RNA polymerase II, polypeptide A) small phosphatase 2 |
| 208741_at | 0,00706 | 0,9939511 | 255,69258 | 0,7769452 | 201,12727 | SAP18 | NM_005870 | Sin3-associated polypeptide, 18kDa |
| 208743_s_at | 0,00208 | 1,0355184 | 3567,0107 | 0,7872519 | 2658,7002 | YWHAB | BC001359 | Tyrosine 3-monooxygenase/tryptophan 5-monooxygenase activation protein, beta polypeptide |
| 208744_x_at | 0,000502 | 0,97828186 | 271,1185 | 0,59591025 | 159,12726 | HSPH1 | BG403660 | Heat shock 105kDa/110kDa protein 1 |
| 208754_s_at | 4,53E-05 | 0,9965347 | 1505,289 | 0,773861 | 1173,8317 | NAP1L1 | AL162068 | Nucleosome assembly protein 1-like 1 |
| 208757_at | 2,02E-05 | 0,9735318 | 780,0334 | 1,425495 | 1130,9589 | TMED9 | BC001123 | Transmembrane emp24 protein transport domain containing |
| 208763_s_at | 2,27E-05 | 1,0358891 | 8490,966 | 1,4164978 | 11701,75 | DSIPI | AL110191 | Delta sleep inducing peptide, immunoreactor |
| 208773_s_at | 5,65E-05 | 0,95292044 | 1671,159 | 0,76214546 | 1334,2454 | ANKHD1 | AL136943 | Eukaryotic translation initiation factor 4E binding protein 3 |
| 208775_at | 5,71E-09 | 1,0342396 | 3013,1038 | 0,7807361 | 2276,2864 | XPO1 | D89729 | Exportin 1 (CRM1 homolog, yeast) |
| 208783_s_at | 3,38E-06 | 1,0068383 | 1632,326 | 0,69338953 | 1134,6818 | MCP | AL570661 | Membrane cofactor protein (CD46, trophoblast-lymphocyte cross-reactive antigen) |
| 208787_at | 3,80E-11 | 0,99683917 | 1843,537 | 0,70605487 | 1313,1227 | MRPL3 | BC003375 | Mitochondrial ribosomal protein L3 |
| 208796_s_at | 6,92E-07 | 1,0219916 | 2174,237 | 0,73462445 | 1536,241 | CCNG1 | BC000196 | Cyclin G1 |
| 208798_x_at | 0,00807 | 0,91476613 | 1789,9333 | 0,6774445 | 1344,0636 | GOLGIN-67 | AF204231 | Golgin-67 |
| 208799_at | 0,00129 | 0,91671604 | 583,5704 | 0,7110762 | 444,68182 | PSMB5 | BC004146 | Homo sapiens cDNA clone IMAGE:2967251, partial cds. |
| 208818_s_at | 0,000262 | 0,98245597 | 1773,5369 | 1,247909 | 2236,4407 | COMT | BC000419 | Catechol-O-methyltransferase |
| 208820_at | 0,00152 | 1,031651 | 163,03333 | 0,64457107 | 111,01818 | PTK2 | AL037339 | PTK2 protein tyrosine kinase 2 |
| 208824_x_at | 6,30E-05 | 0,9970258 | 311,71854 | 1,3565942 | 420,74545 | PCTK1 | BC001048 | PCTAIRE protein kinase 1 |
| 208829_at | 3,39E-06 | 1,0252376 | 5064,555 | 1,308085 | 6447,982 | TAPBP | AF029750 | TAP binding protein (tapasin) |
| 208838_at | 9,81E-08 | 0,97765243 | 332,33704 | 0,5838766 | 208,12274 | TIP120A | AB020636 | TBP-interacting protein |
| 208839_s_at | 1,94E-07 | 0,9479045 | 298,59262 | 0,57983506 | 184,26817 | TIP120A | AL136810 | TBP-interacting protein |
| 208840_s_at | 0,000419 | 1,0278113 | 728,14453 | 1,3621982 | 967,83185 | G3BP2 | AU149503 | Ras-GTPase activating protein SH3 domain-binding protein |
| 208841_s_at | 6,30E-05 | 1,0091382 | 1281,2036 | 0,78623927 | 1000,2318 | G3BP2 | AB014560 | Ras-GTPase activating protein SH3 domain-binding protein |
| 208843_s_at | 0,000161 | 1,0046314 | 943,87415 | 0,7843423 | 740,7363 | GORASP2 | BC001408 | Golgi reassembly stacking protein 2, 55kDa |
| 208847_s_at | 1,17E-07 | 1,0717624 | 1379,5298 | 0,81943333 | 1045,9773 | ADH5 | M29872 | Alcohol dehydrogenase 5 (class III), chi polypeptide |
| 208848_at | 0,000656 | 0,96963304 | 590,963 | 0,7679652 | 471,3137 | ADH5 | M30471 | Alcohol dehydrogenase 5 (class III), chi polypeptide |
| 208863_s_at | 7,81E-05 | 0,9608691 | 836,7074 | 0,69773054 | 602,9818 | SFRS1 | M72709 | |
| 208868_s_at | 0,00171 | 0,95371234 | 164,09258 | 1,4690069 | 256,58182 | GABARAPL 1 | BF125756 | GABA(A) receptor-associated protein like 1 |
| 208869_s_at | 3,71 E-07 | 1,0170785 | 242,82593 1 | 1,7236671 | 426,25 | GABARAPL | AF087847 | GABA(A) receptor-associated protein like 1 |
| 208873_s_at | 5,93E-07 | 1,0296546 | 2009,0408 | 0,8154238 | 1595,2047 | C5orf18 | BC000232 | Chromosome 5 open reading frame 18 |
| 208881_x_at | 0,00661 | 0,9958938 | 1172,2928 | 1,2617602 | 1514,7908 | IDI1 | BC005247 | Isopentenyl-diphosphate delta isomerase |
| 208882_s_at | 0,000216 | 1,00209 | 602,38513 | 0,7327089 | 445,9 | EDD | U69567 | E3 identified by differential display |
| 208883_at | 1,46E-06 | 1,059677 | 295,47406 | 0,6361741 | 176,24998 | EDD | U69567 | E3 identified by differential display |
| 208890_s_at | 0,000459 | 0,9703115 | 1984,2815 | 1,3253411 | 2608,409 | PLXNB2 | BC004542 | Plexin B2 |
| 208891_at | 2,57E-06 | 0,8770902 | 3190,4817 | 0,4125511 | 1523,609 | DUSP6 | BC003143 | Dual specificity phosphatase 6 |
| 208892_s_at | 0,00152 | 0,88096803 | 1889,3999 | 0,54926383 | 1076,3455 | DUSP6 | BC003143 | Dual specificity phosphatase 6 |
| 208896_at | 1,10E-05 | 0,9608175 | 655,9519 | 0,6738201 | 464,85452 | DDX18 | BC003360 | DEAD (Asp-Glu-Ala-Asp) box polypeptide 18 |
| 208897_s_at | 4,53E-05 | 0,95227474 | 1068,2518 | 0,75422543 | 841,1136 | DDX18 | BC003360 | DEAD (Asp-Glu-Ala-Asp) box polypeptide 18 |
| 208909_at | 2,02E-05 | 1,0264992 | 4071,1003 | 1,3136953 | 5167,128 | UQCRFS1 | BC000649 | Ubiquinol-cytochrome c reductase, Rieske iron-sulfur polypeptide 1 |
| 208923_at | 3,16E-07 | 0,9181789 | 1551,5741 | 1,3322194 | 2180,6228 | CYFIP1 | BC005097 | Cytoplasmic FMR1 interacting protein 1 |
| 208931_s_at | 0,00577 | 0,9036532 | 689,80743 MMP4; | 1,1689048 | 896,61365 | ILF3; DRBF; MPP4; NF90; NFAR; TCP80; DRBP76; NFAR-1; MPHOSPH4 ; NF-AT-90 | AF147209 | Homo sapiens double-stranded RNA-binding nuclear protein DRBP76 mRNA, complete cds. |
| 208935_s_at | 0,00948 | 1,0566405 | 540,0296 Gal-8; | 0,79899615 | 384,60907 | LGALS8; PCTA-1; Po66-CBP | L78132 | Human prostate carcinoma tumor antigen (pcta-1) mRNA, complete cds. |
| 208937_s_at | 1,43E-10 | 0,9649949 | 208,51111 | 2,5979123 helix | 556,1909 | ID1 | D13889 | Inhibitor of DNA binding 1, dominant negative helix-loop-protein |
| 208943_s_at | 6,53E-11 | 0,95496064 | 1695,6926 | 0,57245463 | 1026,5547 | TLOC1 | U93239 | Translocation protein 1 |
| 208944_at | 2,95E-06 | 1,0016059 | 2735,2297 | 0,78084165 | 2146,1633 | TGFBR2 | D50683 | Homo sapiens mRNA for TGF-betalIR alpha, complete cds. |
| 208949_s_at | 0,000146 | 1,0007781 | 4847,2334 | 1,2583526 | 6122,055 | LGALS3 | BC001120 | Lectin, galactoside-binding, soluble, 3 (galectin 3) |
| 208962_s_at | 0,00242 | 1,0202118 | 256,6445 | 1,3546548 | 344,7864 | FADS1 | BE540552 | Fatty acid desaturase 1 |
| 208975_s_at | 0,000459 | 0,97870874 | 678,1333 | 0,73427445 | 520,7819 | KPNB1 | L38951 | Karyopherin (importin) beta 1 |
| 208984_x_at | 5,65E-05 | 0,9801513 | 814,6184 | 0,77508223 | 649,4182 | RBM10 | BC004181 | RNA binding motif protein 10 |
| 208985_s_at | 3,75E-09 | 0,96642345 | 869,6037 | 0,677203 | 609,1228 | EIF3S1 | BC002719 | Eukaryotic translation initiation factor 3, subunit 1 alpha, |
| 208986_at | 1,64E-07 | 0,98583025 | 806,2259 | 0,6883916 factors | 555,9682 | TCF12 | AL559478 | Transcription factor 12 (HTF4, helix-loop-helix transcription 4) |
| 208988_at | 7,02E-05 | 0,9179325 | 878,4703 | 0,6364627 | 606,35913 | FBXL11 | AK024505 | F-box and leucine-rich repeat protein 11 |
| 208989_s_at | 0,00085 | 0,97335726 | 335,5963 | 1,2707187 | 457,50452 | FBXL11 | AF179221 | F-box and leucine-rich repeat protein 11 |
| 208990_s_at | 4,05E-05 | 0,9748265 | 1715,4926 | 0,7480613 | 1323,6135 | HNRPH3 | AF132362 | Heterogeneous nuclear ribonucleoprotein H3 (2H9) |
| 208992_s_at | 0,000262 | 1,0197647 | 1166,274 | 1,3816953 | 1608,5592 | STAT3 | BC000627 | Signal transducer and activator of transcription 3 (acute-phase response factor) |
| 208995_s_at | 2,02E-05 | 0,95025194 | 218,29259 | 0,69378823 | 158,92728 | PPIG | U40763 | Peptidyl-prolyl isomerase G (cyclophilin G) |
| 209002_s_at | 0,00134 | 1,0090185 | 1246,0404 | 1,2874182 | 1594,2043 | KIAA1536 | BC003177 | KIAA1536 protein |
| 209005_at | 0,00078 | 0,9788726 | 1104,1815 | 1,2600818 | 1430,2727 | FBXL5 | AF157323 | F-box and leucine-rich repeat protein 5 |
| 209015_s_at | 0,000317 | 1,0720053 | 362,17413 | 0,76991004 | 262,15 | DNAJB6 | BC002446 | DnaJ (Hsp40) homolog, subfamily B, member 6 |
| 209018_s_at | 0,000459 | 0,95310867 | 552,7853 | 1,20772 | 709,2046 | PINK1 | BF432478 | PTEN induced putative kinase 1 |
| 209020_at | 4,63E-09 | 1,0086797 | 641,2704 | 1,555843 | 1005,9591 | C20orf111 | AF217514 | Chromosome 20 open reading frame 111 |
| 209022_at | 3,80E-11 | 1,0267103 | 2826,1367 | 0,74086154 | 2049,0774 | STAG2 | AK026678 | Stromal antigen 2 |
| 209023_s_at | 0,00152 | 1,0706891 | 1062,8147 | 0,7528449 | 692,4591 | STAG2 | BC001765 | Stromal antigen 2 |
| 209028_s_at | 0,00171 | 0,9848749 | 1003,64813 | 0,7752966 | 806,2727 | ABI1 | AF006516 | Abl-interactor 1 |
| 209039_x_at | 1,79E-05 | 0,99776995 | 2195,1958 | 0,75322616 | 1643,9817 | EHD1 | AF001434 | EH-domain containing 1 |
| 209056_s_at | 4,63E-09 | 0,9912756 | 572,3111 | 0,7130298 | 409,36816 | CDC5L | AW268817 | xv38c01.x1 Soares_NFL_T_GBC_S1 Homo sapiens cDNA clone IMAGE:2815392 3' similar to TR:O08837 008837 CDC-LIKE PROTEIN ;, mRNA sequence. |
| 209061_at | 2,57E-06 | 0,93237066 | 648,5666 | 0,6194361 | 424,79547 | NCOA3 | AF012108 | Nuclear receptor coactivator 3 |
| 209066_x_at | 0,00352 | 0,97451526 | 8048,1074 | 0,7557264 | 6264,7314 | UQCRB | M26700 | Ubiquinol-cytochrome c reductase binding protein |
| 209069_s_at | 0,000262 | 1,0047017 | 10863,203 | 1,3333247 | 14139,341 | H3F3B | BC001124 | H3 histone, family 3B (H3.3B) |
| 209088_s_at | 0,0098 | 1,0099993 | 425,45184 | 1,2853788 | 564,2137 | UBN1 | T70262 | Ubinuclein 1 |
| 209096_at | 9,65E-05 | 1,0666069 | 606,6445 | 0,84778816 | 472,63184 | UBE2V2 | U62136 | Ubiquitin-conjugating enzyme E2 variant 2 |
| 209115_art | 2,57E-06 | 1,0226785 | 1336,348 | 0,7039517 | 930,2227 | UBE1 C | AL117566 | Ubiquitin-activating enzyme E1C (UBA3 homolog, yeast) |
| 209117_at | 0,00618 | 0,9387965 | 1036,8704 | 1,2089158 | 1342,5637 | WBP2 | U79458 | WBP-2; binds to the WW domain of Yes-associated protein (YAP) through a proline-rich motif PPPPY (PY motif) contained within the sequence.; Human WW domain binding protein-2 mRNA, complete cds. |
| 209128_s_at | 2,69E-07 | 0,9300747 | 735,26666 | 0,57800996 | 458,25 | SART3 | D63879 | Squamous cell carcinoma antigen recognised by T cells 3 |
| 209137_s_at | 0,0014 | 0,926008 | 816,4259 | 0,69720894 | 623,6818 | USP10 | BC000263 | Ubiquitin specific protease 10 |
| 209138_x_at | 0,000178 | 1,0315566 | 8949,182 | 0,5179802 | 5852,959 | IGLJ3 | M87790 | |
| 209142_s_at | 0,000196 | 0,99842274 | 898,52216 | 0,7768985 | 710,2045 | UBE2G1 | BC002775 | Ubiquitin-conjugating enzyme E2G 1 (UBC7 homolog, C. elegans) |
| 209146_at | 1,03E-05 | 0,9800952 | 288,55557 | 0,6838449 | 200,15001 | SC4MOL | AV704962 | Sterol-C4-methyl oxidase-like |
| 209153_s_at | 0,000238 | 1,06003 | 748,6629 | 0,75445354 | 542,2 | TCF3 | M31523 | Transcription factor 3 (E2A immunoglobulin enhancer binding factors E12/E47) |
| 209160_at | 1,57E-09 | 0,94197506 | 501,17407 | 0,27575457 hydroxysteroid | 147,68636 | AKR1C3 | AB018580 | Aldo-keto reductase family 1, member C3 (3-alpha dehydrogenase, type II) |
| 209161_at | 3,16E-07 | 0,982837 | 721,98145 | 0,74517965 | 548,7818 | PRPF4 | AI184802 | PRP4 pre-mRNA processing factor 4 homolog (yeast) |
| 209179_s_at | 0,000178 | 1,0137528 | 695,863 | 1,43902 | 1001,3727 | LENG4 | BC003164 | Leukocyte receptor cluster (LRC) member 4 |
| 209184_s_at | 3,16E-07 | 1,117908 | 928,74084 | 1,8960588 | 1580,6864 | IRS2 | BF700086 | 602127569F1 NIH_MGC_56 Homo sapiens cDNA clone IMAGE:4284401 5', mRNA sequence. |
| 209185_s_at | 1,26E-06 | 1,1245315 | 1944,9222 | 1,8648564 | 3278,3547 | IRS2 | AF073310 | Insulin receptor substrate 2 |
| 209187_at | 0,000178 | 1,0038811 | 1332,5815 | 0,7167791 | 963,17267 | DR1 | AW516932 | Down-regulator of transcription 1, TBP-binding (negative cofactor 2) |
| 209198_s_at | 0,00468 | 1,0458233 | 256,84814 | 0,786021 | 188,12273 | SYT11 | BC004291 | SynaptotagminXl |
| 209200_at | 3,39E-06 | 1,0328263 | 668,44824 | 0,6508316 (myocyte | 437,89542 | MEF2C | N22468 | MADS box transcription enhancer factor 2, polypeptide C enhancer factor 2C) |
| 209201_x_at | 1,10E-05 | 1,0095849 | 6848,4893 | 1,5609655 | 10631,432 | CXCR4 | L01639 | Chemokine (C-X-C motif) receptor 4 |
| 209205_s_at | 0,00109 | 1,0348264 | 637,7112 | 1,3190914 | 811,609 | LMO4 | BC003600 | LIM domain only 4 |
| 209206_at | 4,35E-07 | 1,0441844 | 412,82593 | 0,6444249 | 257,25 | SEC22L1 | AV701283 | AV701283 ADA Homo sapiens cDNA clone ADAAGD06 5', mRNA sequence. |
| 209233_at | 5,07E-06 | 1,0017588 | 793,1815 | 0,7862015 | 623,18646 | C2F | U72514 | C2f protein |
| 209250_at | 8,06E-07 | 1,0236421 | 981,45917 | 0,58397365 (Drosophila) | 556,5591 | DEGS1 | BC000961 | Degenerative spermatocyte homolog 1, lipid desaturase |
| 209252_at | 8,52E-06 | 0,9851304 | 524,7889 | 0,7372976 | 388,39545 | HARSL | U18937 | Histidyl-tRNA synthetase-like |
| 209259_s_at | 1,24E-05 | 0,9396412 | 622,87396 | 0,6880908 | 464,73184 | CSPG6 | AF020043 | Chondroitin sulfate proteoglycan 6 (bamacan) |
| 209265_s_at | 0,00119 | 1,0277747 | 1076,4446 | 0,80271757 | 823,6138 | METTL3 | BC001650 | Methyltransferase like 3 |
| 209296_at | 0,000715 | 0,9918702 | 1004,67017 | 0,767439 dependent, | 771,1226 | PPM1B B | AF136972 | Protein phosphatase 1 B (formerly 2C), magnesium-beta isoform |
| 209300_s_at | 0,000138 | 0,99550664 | 441,04443 | 1,3813301 | 615,85455 | NECAP1 | BC002888 | Adaptin-ear-binding coat-associated protein 1 |
| 209304_x_at | 5,02E-13 | 0,9957385 | 902,3519 | 2,2043376 | 1994,5997 | GADD45B | AF087853 | Growth arrest and DNA-damage-inducible, beta |
| 209305_s_at | 6,53E-11 | 0,9277067 | 505,91113 | 1,9424969 | 1048,4546 | GADD45B | AF078077 | Growth arrest and DNA-damage-inducible, beta |
| 209306_s_at | 5,65E-05 | 1,0715419 | 591,8703 | 0,67890537 | 362,19998 | SWAP70 | BC000616 | SWAP-70 protein |
| 209307_at | 9,81 E-08 | 0,96071994 | 530,85547 | 0,4728623 | 262,83636 | SWAP70 | BC000616 | SWAP-70 protein |
| 209323_at | 6,91E-08 | 0,9896674 | 1999,9185 | 0,6923091 | 1412,2455 | PRKRIR | AF081567 | Protein-kinase, interferon-inducible double stranded RNA dependent inhibitor, repressor of (P58 repressor) |
| 209330_s_at | 0,000419 | 1,0332369 | 1851,6962 | 0,8179155 | 1450,7635 | HNRPD | D55674 | Heterogeneous nuclear ribonucleoprotein D (AU-rich element RNA binding protein 1, 37kDa) |
| 209333_at | 0,00129 | 0,9145252 | 275,1037 | 1,2257495 | 366,41818 | ULK1 | AB018265 | Unc-51-like kinase 1 (C. elegans) |
| 209337_at | 6,92E-07 | 0,973137 | 2721,937 | 0,6693427 | 1857,3457 | PSIP1 | AF063020 | PC4 and SFRS1 interacting protein 1 |
| 209340_at | 0,00109 | 1,0412407 | 430,1148 | 0,7396623 | 324,55 | UAP1 | S73498 | UDP-N-acteylglucosamine pyrophosphorylase 1 |
| 209341_s_at | 5,65E-05 | 0,99954015 | 1639,811 | 0,75725615 | 1241,6816 | IKBKB | AU153366 | Inhibitor of kappa light polypeptide gene enhancer in B-cells, kinase beta |
| 209345_s_at | 1,59E-05 | 1,0124363 | 524,1778 | 1,3716135 | 726,7591 | PI4KII | AL561930 | Phosphatidylinositol 4-kinase type II |
| 209348_s_at | 1,17E-07 | 0,97210944 | 633,9778 | 0,48593464 homolog | 333,1909 | MAF | AF055376 | V-maf musculoaponeurotic fibrosarcoma oncogene (avian) |
| 209357_at | 1,40E-05 | 0,95910037 | 735,68524 | 0,687729 | 510,04553 | CITED2 | AF109161 | Cbp/p300-interacting transactivator, with Glu/Asp-rich carboxy-terminal domain, 2 |
| 209367_at | 0,000459 | 0,9702378 | 1850,2297 | 1,3230071 | 2576,559 | STXBP2 | AB002559 | Syntaxin binding protein 2 |
| 209370_s_at | 0,000262 | 0,85685766 | 935,50745 | 1,2709565 | 1287,9592 | SH3BP2 | AB000462 | SH3-domain binding protein 2 |
| 209374_s_at | 6,30E-05 | 0,9966258 | 5095,5405 | 0,59940046 | 3293,0908 | IGHM | BC001872 | Immunoglobulin heavy constant mu |
| 209389_x_at | 0,000146 | 0,9713842 | 3124,8372 | 1,2620839 Coenzyme | 3959,8499 | DBI | M15887 | Diazepam binding inhibitor (GABA receptor modulator, acyl-A binding protein) |
| 209398_at | 0,00242 | 1,01287 | 271,7926 H1.2; | 1,4922266 | 415,91364 | HIST1H1C; H1F2; MGC:3992 | BC002649 | synonyms: MGC:3992, H1F2, H1.2; Homo sapiens histone 1, H1c, mRNA (cDNA clone MGC:3992 IMAGE:3608862), complete cds. |
| 209406_at | 2,29E-08 | 1,0059221 | 136,94073 | 0,57757515 | 81,42273 | BAG2 | AF095192 | BCL2-associated athanogene 2 |
| 209417_s_at | 0,00861 | 0,9490148 | 896,36304 | 1,2891953 | 1271,3408 | IFI35 | BC001356 | Interferon-induced protein 35 |
| 209421_at | 5,78E-06 | 0,9998575 | 356,70737 | 0,7044834 | 255,31818 | MSH2 | U04045 | MutS homolog 2, colon cancer, nonpolyposis type 1 (E. coli) |
| 209422_at | 3,22E-05 | 1,0033666 | 1082,7297 | 0,76298726 | 827,89545 | C20orf104 | AL109965 | |
| 209425_at | 0,000419 | 1,0249356 | 86,13333 | 0,7611324 | 63,613632 | AMACR | NM_014324 | Alpha-methylacyl-CoAracemase |
| 209431_s_at | 0,000107 | 1,0043696 | 327,72223 | 0,77589047 | 251,86365 | ZNF278 | AF254083 | Zinc finger protein 278 |
| 209446_s_at | 0,0014 | 0,9617798 | 136,50742 | 1,4468955 | 181,12274 | FLJ10803 | BC001743 | |
| 209447_at | 0,00109 | 0,9803575 | 1224,0668 | 0,7602135 | 978,07275 | SYNE1 | AF043290 | Spectrin repeat containing, nuclear envelope 1 |
| 209451_at | 0,00327 | 1,0013353 | 498,18884 | 0,7121422 | 356,5727 | TANK | U59863 | TRAF family member-associated NFKB activator |
| 209457_at | 3,88E-10 | 0,9544883 | 531,9741 | 1,7254795 | 948,2273 | DUSP5 | U16996 | Dual specificity phosphatase 5 |
| 209459_s_at | 5,34E-05 | 0,994216 | 268,6741 | 0,65472597 | 177,4682 | ABAT | AF237813 | 4-aminobutyrate aminotransferase |
| 209460_at | 0,00806 | 1,0148578 | 315,62222 | 0,75338537 | 240,32272 | ABAT | AF237813 | 4-aminobutyrateaminotransferase |
| 209471_s_at | 2,38E-10 | 1,0772403 | 1081,2261 | 0,72705007 | 712,1181 | FNTA | L00634 | Farnesyltransferase, CAAX box, alpha |
| 209477_at | 5,41 E-06 | 0,9553331 | 309,22223 | 1,2959431 | 415,64545 | EMD | BC000738 | Emerin (Emery-Dreifuss muscular dystrophy) |
| 209484_s_at | 0,000216 | 0,9794895 | 1114,937 | 0,76384753 | 890,2818 | C1orf48 | AF201941 | Chromosome 1 open reading frame 48 |
| 209486_at | 6,92E-07 | 0,9622084 | 587,55927 | 0,6541782 | 400,93637 | SAS10 | BC004546 | Disrupter of silencing 10 |
| 209507_at | 5,96E-05 | 1,0170126 | 627,0889 | 0,7524576 | 473,54095 | RPA3 | BC005264 | Replication protein A3, 14kDa |
| 209510_at | 0,000178 | 1,1221293 | 1213,0295 | 1,5410844 | 1633,0364 | RNF139 | AF064801 | Ring finger protein 139 |
| 209514_s_at | 0,000459 | 1,0168586 | 1634,8999 | 1,2889502 | 2043,9183 | RAB27A | BE502030 | RAB27A, member RAS oncogene family |
| 209520_s_at | 0,00152 | 0,9591297 | 175,82222 | 0,7320769 | 134,90907 | NCBP1 | BC001450 | Nuclear cap binding protein subunit 1, 80kDa |
| 209523_at | 4,35E-07 | 1,017527 | 645,00745 | 0,659708 | 426,3273 | TAF2 | AK001618 | TAF2 RNA polymerase II, TATA box binding protein (TBP)-associated factor, 150kDa |
| 209526_s_at | 0,00392 | 0,9545891 | 163,86667 | 1,2999163 | 222,16364 | HDGFRP3 | AB029156 | Hepatoma-derived growth factor, related protein 3 |
| 209538_at | 0,000131 | 1,0326606 | 418,48517 | 0,7578043 | 308,12726 | ZNF32 | U69645 | Zinc finger protein 32 (KOX 30) |
| 209545_s_at | 1,38E-12 | 1,0142642 | 862,6742 | 1,7830235 | 1572,1547 | RIPK2 | AF064824 | Receptor-interacting serine-threonine kinase 2 |
| 209551_at | 2,02E-05 | 0,95087 | 81,403694 | 0,66037863 | 57,231815 | MGC11061 | BC004875 | Hypothetical protein MGC11061 |
| 209555_s_at | 1,94E-06 | 0,9649459 | 1601,4777 | 1,8147894 | 2908,8862 | CD36 | M98399 | CD36 antigen (collagen type I receptor, thrombospondin receptor) |
| 209566_at | 8,16E-14 | 0,9761047 | 316,2852 | 0,5257659 | 175,59093 | INSIG2 | AL080184 | Insulin induced gene 2 |
| 209567_at | 0,00436 | 0,9268287 | 640,80743 | 0,736175 | 510,34546 | RRS1 | BC001811 | RRS1 ribosome biogenesis regulator homolog (S. |
| 209572_s_at | 0,000382 | 0,9903533 | 638,6518 | 0,71396524 | 465,38635 | EED | AF080227 | Embryonic ectoderm development |
| 209580_s_at | 7,50E-06 | 0,9768675 | 609,56305 | 0,7442676 | 460,55905 | MBD4 | AF114784 | Methyl-CpG binding domain protein 4 |
| 209583_s_at | 0,00192 | 0,9546376 | 197,27406 | 0,6349243 | 140,71819 | CD200 | AF063591 | CD200 antigen |
| 209584_x_at | 0,00327 | 0,9612889 | 1270,7999 | 0,7390705 | 1011,3457 | APOBEC3C | AF165520 | Apolipoprotein B mRNA editing enzyme, catalytic polypeptide-like 3C |
| 209585_s_at | 5,65E-05 | 1,0463392 | 217,78888 | 0,59952056 | 131 | MINPP1 | AF084943 | Multiple inositol polyphosphate histidine phosphatase, 1 |
| 209586_s_at | 7,02E-05 | 1,0227504 | 1134,2667 | 0,78785646 | 880,1727 | PRUNE | AF123539 | Prune homolog (Drosophila) |
| 209602_s_at | 2,69E-07 | 0,9869953 | 242,00002 | 0,583933 | 141,17728 | GATA3 | AI796169 | GATA binding protein 3 |
| 209604_s_at | 4,05E-05 | 1,0266063 | 2746,9778 | 0,69551426 | 1823,2136 | GATA3 | BC003070 | GATA binding protein 3 |
| 209616_s_at | 0,00152 | 0,91564006 | 495,24814 | 1,5922948 | 960,4091 | CES1 | S73751 | Carboxylesterase 1 (monocyte/macrophage serine esterase |
| 209630_s_at | 3,71 E-07 | 0,97393584 | 1157,5852 | 0,6765311 | 808,5364 | FBXW2 | AL043967 | F-box and WD-40 domain protein 2 |
| 209635_at | 0,0073 | 0,96104664 | 219,85184 | 1,2401264 | 280,00906 | AP1S1 | BC003561 | Adaptor-related protein complex 1, sigma 1 subunit |
| 209648_x_at | 0,000227 | 0,9895155 | 111,700005 | 0,6031378 | 70,56364 | SOCS5 | AL136896 | Suppressor of cytokine signaling 5 |
| 209649_at | 1,79E-05 | 0,94995964 | 148,3222 | 0,41217512 | 81,972725 | STAM2 | AL133600 | Signal transducing adaptor molecule (SH3 domain and ITAM motif) 2 |
| 209654_at | 3,88E-10 | 0,9693182 | 864,48883 | 0,6334835 | 561,7864 | KIAA0947 | BC004902 | KIAA0947 protein |
| 209658_at | 0,00861 | 0,95884305 | 1016,10364 | 0,6463309 | 741,9091 | CDC16 | AF164598 | CDC16 cell division cycle 16 homolog (S. cerevisiae) |
| 209662_at | 2,96E-13 | 0,92929286 | 458,22592 | 0,33402583 | 165,43636 | CETN3 | BC005383 | Centrin, EF-hand protein, 3 (CDC31 homolog, yeast) |
| 209666_s_at | 0,00618 | 1,0571386 | 238,46669 | 0,84073824 | 189,10909 | CHUK | AF080157 | Conserved helix-loop-helix ubiquitous kinase |
| 209671_x_at | 8,68E-05 | 0,91065294 | 7997,4297 | 0,6197762 | 5512,5454 | TRA@ | M12423 | T cell receptor alpha locus |
| 209674_at | 0,00192 | 1,0195742 | 352,95184 | 0,7049784 | 253,41362 | CRY1 | D83702 | Cryptochrome 1 (photolyase-like) |
| 209684_at | 0,000216 | 0,87944716 | 519,33704 | 0,61835283 | 373,2727 | RIN2 | AL136924 | Ras and Rab interactor 2 |
| 209701_at | 2,75E-06 | 0,8846133 | 151,9185 | 1,699721 | 285,3909 | CAST | D16217 | Calpastatin |
| 209704_at | 0,00129 | 0,97041065 | 164,7 | 0,71768636 | 123,35455 | M96 | AF073293 | Likely ortholog of mouse metal response element binding transcription factor 2 |
| 209711_at | 0,00919 | 1,0266724 | 339,72592 | 0,78098154 | 251,88635 | SLC35D1 | N80922 | Solute carrier family 35 (UDP-glucuronic acid/UDP-N-acetylgalactosamine dual transporter), member D1 |
| 209724_s_at | 5,78E-06 | 0,9848759 | 390,40372 | 0,6856697 | 270,3046 | ZFP161 | AL534416 | Zinc finger protein 161 homolog (mouse) |
| 209732_at | 0,000178 | 0,96722925 | 3383,6519 | 1,2564468 domain) | 4412,8003 | CLECSF2 | BC005254 | C-type (calcium dependent, carbohydrate-recognition lectin, superfamily member 2 (activation-induced) |
| 209734_at | 5,65E-05 | 0,8818876 | 969,32227 | 1,4892085 | 1604,2864 | HEM1 | BC001604 | Hematopoietic protein 1 |
| 209771_x_at | 0,00208 | 1,0859869 | 777,36304 | 0,6677166 | 575,89545 | CD24 | AA761181 | CD24 antigen (small cell lung carcinoma cluster 4 antigen) |
| 209780_at | 5,09E-07 | 1,004808 | 763,1741 | 0,7462745 | 565,5545 | PHTF2 | AL136883 | Putative homeodomain transcription factor 2 |
| 209787_s_at | 1,94E-06 | 1,0014104 | 2326,1519 | 0,73899835 | 1727,759 | HMGN4 | BC001282 | High mobility group nucleosomal binding domain 4 |
| 209788_s_at | 0,00861 | 0,8203899 | 868,98883 | 0,4657645 | 479,2682 | ARTS-1 | AF183569 | Type 1 tumor necrosis factor receptor shedding aminopeptidase regulator |
| 209795_at | 4,44E-06 | 0,93527216 | 1910,6074 | 1,7605383 | 3270,7815 | CD69 | L07555 | CD69 antigen (p60, early T-cell activation antigen) |
| 209798_at | 1,94E-07 | 0,9778289 | 625,31104 | 0,6452966 | 410,7273 | NPAT | D83243 | Nuclear protein, ataxia-telangiectasia locus |
| 209803_s_at | 1,40E-05 | 0,96958596 | 48,985184 | 1,893842 | 89,718185 | PHLDA2 | AF001294 | Pleckstrin homology-like domain, family A, member 2 |
| 209808_x_at | 1,57E-09 | 0,9869211 | 451,50003 | 1,522473 | 703 | ING1 | AW193656 | Inhibitor of growth family, member 1 |
| 209813_x_at | 0,000549 | 0,9692167 | 3728,637 | 0,5725228 | 2548,0637 | TRGV9 | M16768 | T cell receptor gamma variable 9 |
| 209815_at | 1,17E-07 | 0,9895052 | 455,737 | 0,56487894 | 261,59998 | PTCH | U43148 | Patched homolog (Drosophila) |
| 209827_s_at | 3,80E-11 | 0,9510428 | 2335,322 | 0,62989086 | 1535,3364 | IL16 | NM_004513 | Interleukin 16 (lymphocyte chemoattractant factor) |
| 209828_s_at | 6,71E-12 | 0,9857973 | 479,94073 | 0,46047497 | 227,28183 | IL16 | M90391 | Interleukin 16 (lymphocyte chemoattractant factor) |
| 209829_at | 2,44E-09 | 1,009024 | 2094,1997 | 0,6041988 | 1275,0771 | C6orf32 | AB002384 | Chromosome 6 open reading frame 32 |
| 209831_x_at | 0,000131 | 0,9746494 | 1115,7852 | 1,2833457 | 1458,2046 | DNASE2 | AB004574 | Deoxyribonuclease II, lysosomal |
| 209835_x_at | 1,79E-05 | 0,9908666 | 3742,6003 | 1,3684287 | 5154,8774 | CD44 | BC004372 | CD44 antigen (homing function and Indian blood group |
| 209841_s_at | 1,57E-09 | 0,97063434 | 625,18146 | 0,36319947 | 200,97273 | LRRN3 | AL442092 | Leucine rich repeat neuronal 3 |
| 209846_s_at | 7,02E-05 | 1,0112212 | 2864,863 | 0,595794 | 1704,0184 | BTN3A2 | BC002832 | Butyrophilin, subfamily 3, member A2 |
| 209861_s_at | 5,08E-07 | 1,0141659 | 1035,2332 | 0,6937027 | 697,4455 | METAP2 | U13261 | Methionyl aminopeptidase 2 |
| 209862_s_at | 0,00577 | 1,0150728 | 371,6667 | 0,796237 | 299,5909 | PIG8 | BC001233 | Translokin |
| 209863_s_at | 0,000685 | 0,86998856 | 117,07778 | 1,3250049 | 167,37274 | TP73L | AF091627 | Tumor protein p73-like |
| 209865_at | 0,000348 | 1,0449532 | 191,48148 | 0,68818104 GIcNAc) | 118,65455 | SLC35A3 | BC005136 | Solute carrier family 35 (UDP-N-acetylglucosamine (UDP-transporter), member A3 |
| 209881_s_at | 0,00406 | 0,96066666 | 1163,5259 | 0,7645436 | 936,0499 | LAT | AF036905 | Linker for activation of T cells |
| 209884_s_at | 0,00755 | 1,0333498 | 302,41483 | 0,7348167 | 222,39998 | SLC4A7 | AF047033 | Solute carrier family 4, sodium bicarbonate cotransporter, member 7 |
| 209892_at | 1,89E-08 | 0,96794707 | 304,41486 | 1,715818 | 542,10913 | FUT4 | AF305083 | Fucosyltransferase 4 (alpha (1,3) fucosyltransferase, myeloid-specific) |
| 209894_at | 0,00109 | 1,0097831 | 131,86667 | 0,71466154 | 93,34544 | LEPR | U50748 | Leptin receptor |
| 209898_x_at | 0,000365 | 0,99498326 | 1818,1742 | 0,75025773 | 1385,3136 | ITSN2 | U61167 | Intersectin 2 |
| 209901_x_at | 0,000419 | 1,0691345 | 5564,734 | 1,4141469 | 7353,1904 | AIF1 | U19713 | Allograft inflammatory factor 1 |
| 209903_s_at | 8,52E-06 | 1,0023721 | 629,3038 | 0,6951178 | 441,41818 | ATR | U49844 | Ataxia telangiectasia and Rad3 related |
| 209906_at | 7,02E-05 | 1,0774156 | 1117,1259 | 0,5722228 | 607,94543 | C3AR1 | U62027 | Complement component 3a receptor 1 |
| 209919_x_at | 1,59E-05 | 0,96403825 | 383,6148 | 1,2953156 | 519,80914 | GGT1 | L20490 | Gamma-glutamyltransferase 1 |
| 209927_s_at | 0,000382 | 0,9757185 | 332,03705 1010 | 0,7383456 | 255,92728 | DKFZP547E | AF261137 | DKFZP547E1010 protein |
| 209943_at | 2,87E-05 | 0,9818499 | 123,262955 | 0,58792615 | 76,31364 | FBXL4 | AF176699 | F-box and leucine-rich repeat protein 4 |
| 209944_at | 2,02E-05 | 0,9548239 | 1239,1146 | 0,7599223 | 986,2318 | ZNF410 | BC000330 | Zinc finger protein 410 |
| 209949_at | 0,000107 | 0,99572825 | 6451,7188 | 1,269406 disease, | 8255,109 | NCF2 | BC001606 | Neutrophil cytosolic factor 2 (65kDa, chronic granulomatous autosomal 2) |
| 209969_s_at | 2,27E-05 | 1,138798 | 1095,4037 | 1,9287251 | 1789,3955 | STAT1 | BC002704 | Signal transducer and activator of transcription 1, 91 kDa |
| 209972_s_at | 0,000131 | 1,0370973 | 141,21852 | 1,4435527 | 195,63637 | JTV1 | AF116615 | JTV1 gene |
| 209974_s_at | 3,16E-07 | 0,99746937 | 1857,5408 | 0,66447115 (yeast) | 1263,2452 | BUB3 | AF047473 | BUB3 budding uninhibited by benzimidazoles 3 homolog |
| 209989_at | 1,92E-12 | 1,0263772 | 138,6963 | 0,3284349 | 47,695454 | ZNF268 | AF317549 | Zinc finger protein 268 |
| 209993_at | 5,06E-05 | 0,96046704 | 273,9 | 0,64352727 | 188,15453 | ABCB1 | AF016535 | ATP-binding cassette, sub-family B (MDR/TAP), member 1 |
| 209994_s_at | 4,39E-08 | 1,011705 | 217,58148 | 0,50374496 | 118,15454 | ABCB1 | AF016535 | ATP-binding cassette, sub-family B (MDR/TAP), member 1 |
| 209995_s_at | 0,00129 | 0,9252073 | 1534,6075 | 0,5780071 | 1030,5546 | TCL1A | BC003574 | T-cell leukemia/lymphoma 1A |
| 210017_at | 0,000107 | 1,0030724 | 522,8333 | 0,7134568 | 379,2682 | MALT1 | AF070528 | Mucosa associated lymphoid tissue lymphoma translocation gene 1 |
| 210031_at | 6,30E-05 | 0,9455938 | 4943,033 | 0,6212156 | 3295,6636 | CD3Z | J04132 | CD3Z antigen, zeta polypeptide (TiT3 complex) |
| 210044_s_at | 0,00282 | 1,0096148 | 830,06287 | 0,75783145 | 655,49554 | LYL1 | BC002796 | Lymphoblastic leukemia derived sequence 1 |
| 210046_s_at | 5,65E-05 | 0,9845913 | 1326,774 | 1,2650143 | 1713,7046 | IDH2 | U52144 | Isocitrate dehydrogenase 2 (NADP+), mitochondrial |
| 210053_at | 7,99E-06 | 0,9511277 | 337,46298 | 0,701292 | 242,38635 | TAF5 | AW138827 | TAF5 RNA polymerase II, TATA box binding protein (TBP)-associated factor, 100kDa |
| 210054_at | 0,000348 | 1,0865843 | 439,49258 | 1,5010746 | 611,31354 | C4orf15 | BC003648 | Chromosome 4 open reading frame 15 |
| 210073_at | 2,28E-07 | 1,0401504 | 68,85185 | 0,5114 | 32,340908 | SIAT8A | L32867 | Sialyltransferase 8A (alpha-N-acetylneuraminate: alpha-2,8-sialyltransferase, GD3 synthase) |
| 210093_s_at | 0,00378 | 1,036864 | 598,2593 | 0,793885 | 450,05908 | MAGOH | AF067173 | Mago-nashi homolog, proliferation-associated (Drosophila) |
| 210104_at | 1,40E-05 | 0,95272803 | 297,30368 | 0,6624413 | 208,21364 | MED6 | AF074723 | Mediator of RNA polymerase II transcription, subunit 6 homolog (yeast) |
| 210116_at | 1,25E-09 | 0,9761796 | 950,637 | 0,4690674 (lymphoproliferative | 462,62723 | SH2D1A | AF072930 | SH2 domain protein 1A, Duncan's disease syndrome) |
| 210128_s_at | 0,00085 | 0,96912295 | 293,93704 | 1,2404506 | 371,28635 | LTB4R | U41070 | Leukotriene B4 receptor |
| 210144_at | 0,000119 | 0,9810836 | 334,73703 | 0,7209459 | 248,66821 | TBC1D22A | AK000851 | TBC1 domain family, member 22A |
| 210164_at | 0,00304 | 0,98340553 | 4230,2593 | 0,6022648 | 2714,4907 | GZMB | J03189 | Granzyme B (granzyme 2, cytotoxic T-lymphocyte-associated serine esterase 1) |
| 210176_at | 0,00861 | 0,98549664 | 428,3556 | 0,7425552 | 315,2727 | TLR1 | AL050262 | Toll-like receptor 1 |
| 210180_s_at | 1,43E-10 | 1,0144763 | 273,95557 | 2,0717814 homolog, | 566,9818 | SFRS10 | U87836 | Splicing factor, arginine/serine-rich 10 (transformer 2 Drosophila) |
| 210190_at | 1,38E-07 | 0,8856651 | 291,2852 | 1,7914631 | 561,7409 | STX11 | AF071504 | Syntaxin 11 |
| 210195_s_at | 0,00364 | 0,7673966 | 84,437035 | 1,245017 | 124,52273 | PSG1 | M34715 | Pregnancy specific beta-1-glycoprotein 1 |
| 210200_at | 2,23E-06 | 0,95752925 | 283,41486 | 1,7149206 | 507,23178 | WWP2 | BC000108 | WW domain containing E3 ubiquitin protein ligase 2 |
| 210216_x_at | 0,00339 | 0,9705505 | 369,99255 | 0,7601768 | 306,53638 | RAD1 | AF084513 | RAD1 homolog (S. pombe) |
| 210220_at | 0,000238 | 0,9568269 | 265,58893 | 0,68087804 | 189,46362 | FZD2 | L37882 | Frizzled homolog 2 (Drosophila) |
| 210222_s_at | 0,000715 | 0,9153933 | 669,663 | 1,2215145 | 880,8545 | RTN1 | BC000314 | Reticulon 1 |
| 210225_x_at | 0,0098 | 0,9659603 | 1675,6929 | 1,220123 TM | 2094,65 | LILRB3 | AF009635 | Leukocyte immunoglobulin-like receptor, subfamily B (with and ITIM domains), member 6 |
| 210240_s_at | 2,64E-12 | 0,98957527 | 686,45184 | 2,0106993 | 1395,3408 | CDKN2D | U20498 | Cyclin-dependent kinase inhibitor 2D (p19, inhibits CDK4) |
| 210260_s_at | 1,46E-06 | 1,0178134 | 3514,0042 | 0,6952452 | 2423,8 | TNFAIP8 | BC005352 | Tumor necrosis factor, alpha-induced protein 8 |
| 210264_at | 1,56E-08 | 0,9491653 | 307,02963 | 1,5835927 | 519,59094 | GPR35 | AF089087 | G protein-coupled receptor 35 |
| 210279_at | 0,00178 | 0,9696442 | 612,48895 | 0,61084616 | 377,59995 | GPR18 | AF261135 | G protein-coupled receptor 18 |
| 210281_s_at | 0,000216 | 0,96218824 | 158,15187 | 1,4562756 | 235,52274 | ZNF198 | AL136621 | Zinc finger protein 198 |
| 210288_at | 9,65E-05 | 1,0354366 | 1088,3632 | 0,66883045 | 726,8272 | KLRG1 | AF081675 | Killer cell lectin-like receptor subfamily G, member 1 |
| 210296_s_at | 2,44E-09 | 0,97628987 | 640,47784 | 0,6978179 | 457,38635 | PXMP3 | BC005375 | Peroxisomal membrane protein 3, 35kDa (Zellweger |
| 210325_at | 0,00661 | 0,9373816 | 213,17036 | 1,2254744 | 273,84094 | CD1A | M28825 | CD1A antigen, a polypeptide |
| 210336_x_at | 0,000925 | 0,99826306 | 411,7704 | 0,7408569 | 309,18637 | ZNF42 | AF055078 | Zinc finger protein 42 (myeloid-specific retinoic acid-responsive) |
| 210338_s_at | 0,00078 | 1,0108705 | 11111,126 | 0,7971723 | 8608,946 | HSPA8 | AB034951 | Heat shock 70kDa protein 8 |
| 210346_s_at | 6,59E-06 | 0,9656228 | 1000,74817 | 0,60007775 | 597,58185 | CLK1 | AF212224 | CDC-like kinase 1 |
| 210347_s_at | 8,52E-06 | 0,96796596 | 560,3704 | 0,69194406 | 401,45453 | BCL11A | AF080216 | B-cell CLL/lymphoma 11A (zinc finger protein) |
| 210354_at | 0,00661 | 1,0707568 | 147,4222 | 1,7006853 | 296,50452 | IFNG | M29383 | Human mRNA for HuIFN-gamma interferon. |
| 210360_s_at | 0,00807 | 0,9548284 | 107,94445 | 1,2687757 | 143,49545 | MTSS1 | AF116674 | Metastasis suppressor 1 |
| 210365_at | 6,25E-10 | 0,94508654 | 107,00741 | 1,8453925 1; | 203,65001 | RUNX1 | D43967 | Runt-related transcription factor 1 (acute myeloid leukemia aml1 oncogene) |
| 210370_s_at | 1,26E-06 | 0,92427284 | 615,4704 | 0,6100024 | 406,45908 | LY9 | AF244129 | Lymphocyte antigen 9 |
| 210389_x_at | 6,92E-07 | 0,9493305 | 333,75555 | 0,64580846 | 227,29091 | TUBD1 | BC000258 | Tubulin, delta 1 |
| 210422_x_at | 0,000715 | 0,93279094 | 497,46664 | 1,333749 | 702,7909 | SLC11A1 | D50402 | Solute carrier family 11 (proton-coupled divalent metal ion transporters), member 1 |
| 210423_s_at | 0,00242 | 0,9323293 | 1169,8074 | 1,3490515 | 1678,1544 | SLC11A1 | L32185 | Solute carrier family 11 (proton-coupled divalent metal ion transporters), member 1 |
| 210426_x_at | 2,55E-05 | 1,085995 | 261,4963 | 1,75444 | 435,0773 | RORA | U04897 | RAR-related orphan receptor A |
| 210443_x_at | 9,36E-07 | 0,96137774 | 756,337 | 1,3097308 | 1032,2592 | OGFR | AF172452 | Opioid growth factor receptor |
| 210466_s_at | 0,000715 | 0,99699306 | 3395,1816 | 0,69846225 | 2498,8774 | PAI-RBP1 | BC002488 | PAI-1 mRNA-binding protein |
| 210479_s_at | 0,000178 | 1,0020066 | 266,11108 | 1,5086194 | 409,24094 | RORA | L14611 | RAR-related orphan receptor A |
| 210502_s_at | 3,75E-09 | 1,0180436 | 819,0482 | 0,76124406 | 608,7865 | PPIE | AF042386 | Peptidylprolyl isomerase E (cyclophilin E) |
| 210512_s_at | 8,68E-05 | 1,0008179 | 245,52225 | 1,7502754 | 460,93637 | VEGF | AF022375 | Vascular endothelial growth factor |
| 210513_s_at | 0,00224 | 0,98393786 | 240,14813 | 1,2988998 | 320,65 | VEGF | AF091352 | Vascular endothelial growth factor |
| 210524_x_at | 0,000549 | 0,9686111 | 789,5889 | 1,2139307 Accession | 998,9592 | | AF078844 | C-terminus similar to human metallothionein-IF: Swiss-Prot Number P04733; Homo sapiens hqp0376 protein mRNA, complete cds. |
| 210538_s_at | 2,29E-08 | 1,0531293 | 663,2889 AIP1; | 0,58278185 | 362,91367 | BIRC3; API2; MIHC; CIAP2; HAIP1; HIAP1; MALT2; RNF49 | U37546 | IAP homolog C; interacts with TRAF1 and TRAF2 in yeast two hybrid system; homolog of Baculovirus IAP genes; Mammalian IAP homolog C; Human IAP homolog C (MIHC) mRNA, complete cds. |
| 210542_s_at | 0,0006 | 0,94701165 | 349,31854 | 1,2227876 | 451,17273 | SLCO3A1 | BC000585 | Solute carrier organic anion transporter family, member 3A1 |
| 210555_s_at | 0,000178 | 1,0237039 | 1227,8296 | 0,7773004 | 953,15466 | NFATC3 | U85430 | Nuclear factor of activated T-cells, cytoplasmic, calcineurin-dependent 3 |
| 210567_s_at | 9,81 E-08 | 0,9940372 | 176,22223 | 0,65875316 | 117,35909 | SKP2 | BC001441 | S-phase kinase-associated protein 2 (p45) |
| 210568_s_at | 0,000925 | 0,98062533 | 345,17773 | 0,72486365 | 251,53183 | RECQL | BC001052 | RecQ protein-like (DNA helicase Q1-like) |
| 210582_s_at | 6,64E-05 | 0,95270836 | 281,95926 | 1,2569784 | 375,45453 | LIMK2 | AL117466 | LIM domain kinase 2 |
| 210645_s_at | 0,00164 | 1,0491235 | 1637,1 | 0,8014824 | 1234,8273 | TTC3 | D83077 | Tetratricopeptide repeat domain 3 |
| 210691_s_at | 8,52E-06 | 1,0952842 | 320,31485 | 0,7226169 | 201,81363 | CACYBP | AF275803 | Calcyclin binding protein |
| 210695_s_at | 0,00406 | 0,9741622 | 620,03326 | 1,2617334 | 791,5636 | WWOX; FOR; FRA16D; HHCMA56; PRO0128; WWOX v8; D16S432E | U13395 | Human oxidoreductase (HHCMA56) mRNA, complete cds. |
| 210719_s_at | 0,000119 | 1,0217583 | 723,0889 | 1,285163 | 913,54083 | HMG20B | BC002552 | High-mobility group 20B |
| 210740_s_at | 1,79E-05 | 1,0359404 | 1372,8369 | 1,3838129 | 1821,5682 | ITPK1 | AF279372 | Inositol 1,3,4-triphosphate 5/6 kinase |
| 210754_s_at | 0,00242 | 0,9955218 | 4216,8335 | 1,2788246 | 5195,1045 | LYN | M79321 | V-yes-1 Yamaguchi sarcoma viral related oncogene |
| 210763_x_at | 6,30E-05 | 1,0543817 | 649,49255 | 0,7251378 | 445,8909 | NCR3 | AF031137 | Natural cytotoxicity triggering receptor 3 |
| 210766_s_at | 2,27E-05 | 0,9791445 | 413,0148 | 0,67712003 | 290,20004 | CSE1L | AF053640 | CSE1 chromosome segregation 1-like (yeast) |
| 210776_x_at | 8,06E-07 | 0,99414635 | 655,2999 | 0,7039209 binding | 470,33182 | TCF3 | M31222 | Transcription factor 3 (E2A immunoglobulin enhancer factors E12/E47) |
| 210785_s_at | 1,24E-05 | 0,9874622 | 3070,8152 | 1,3660866 | 4201,982 | C1orf38 | AB035482 | Chromosome 1 open reading frame 38 |
| 210797_s_at | 0,00468 | 0,9999012 | 530,4296 | 1,4444886 | 800,98175 | OASL | AF063612 | interferon-induced protein; 30 kDa; alternatively spliced; Homo sapiens 2'-5'oligoadenylate synthetase-related protein p30 (OASL) mRNA, alternatively spliced, complete |
| 210807_s_at | 0,00557 | 0,9291355 | 186,0074 | 1,1924859 | 234,83635 | SLC16A7 | AF049608 | Solute carrier family 16 (monocarboxylic acid transporters), member 7 |
| 210829_s_at | 0,000187 | 0,98191154 | 270,04813 | 0,67840004 | 186,84546 | SSBP2 | AF077048 | Single-stranded DNA binding protein 2 |
| 210836_x_at | 1,26E-06 | 0,96529824 | 91,1963 | 1,6676489 E3 | 163,63637 | PDE4D | AF012073 | Phosphodiesterase 4D, cAMP-specific (phosphodiesterase dunce homolog, Drosophila) |
| 210837_s_at | 7,99E-06 | 1,0475407 | 74,703705 | 1,8571388 E3 | 132,75908 | PDE4D | AF012074 | Phosphodiesterase 4D, cAMP-specific (phosphodiesterase dunce homolog, Drosophila) |
| 210844_x_at | 2,56E-05 | 1,0345217 | 1297,2705 | 1,3963574 | 1730,118 | CTNNA1 | D14705 | Catenin (cadherin-associated protein), alpha 1, 102kDa |
| 210845_s_at | 8,52E-11 | 0,96848804 | 1146,1592 | 1,8863494 | 2320,8547 | PLAUR | U08839 | Plasminogen activator, urokinase receptor |
| 210858_x_at | 9,65E-05 | 1,087328 | 2738,4001 | 0,72291964 | 1759,418 | ATM | U26455 | Ataxia telangiectasia mutated (includes complementation groups A, C and D) |
| 210865_at | 2,87E-05 | 0,9276961 | 256,13333 | 0,54501647 | 156,5318 | FASLG | D38122 | Fas ligand (TNF superfamily, member 6) |
| 210889_s_at | 0,00242 | 0,99237293 | 392,34073 | 0,76645654 | 299,48636 | FCGR2B | M31933 | Fc fragment of IgG, low affinity IIb, receptor for (CD32) |
| 210907_s_at | 2,77E-08 | 1,0405185 | 2778,9663 | 0,76186126 | 2036,8772 | PDCD10 | BC002506 | Programmed cell death 10 |
| 210915_x_at | 0,000925 | 1,0105072 | 11048,626 | 0,7956562 | 8690,519 | TCRB | M15564 | T-cell receptor rearranged beta-chain V-region (V-D-J) |
| 210942_s_at | 0,00327 | 0,88341784 | 124,28149 | 1,1901288 | 164,0409 | SIAT10 | AB022918 | Sialyltransferase 10 (alpha-2,3-sialyltransferase VI) |
| 210943_s_at | 1,68E-06 | 1,0399607 | 656,8445 | 1,7128006 | 1154,8228 | LYST | U84744 | Lysosomal trafficking regulator |
| 210951_x_at | 5,78E-08 | 0,94735414 | 2013,8591 | 1,3537457 | 2849,65 | RAB27A | AF125393 | RAB27A, member RAS oncogene family |
| 210959_s_at | 0,0098 | 1,0378262 | 288,0037 | 1,3019774 | 370,6273 | SRD5A1 | AF113128 | Steroid-5-alpha-reductase, alpha polypeptide 1 (3-oxo-5 alpha-steroid delta 4-dehydrogenase alpha 1) |
| 210962_s_at | 5,09E-07 | 1,0005924 | 645,82965 | 0,6966203 | 446,13638 | AKAP9 | AB019691 | A kinase (PRKA) anchor protein (yotiao) 9 |
| 210970_s_at | 0,00661 | 1,0691904 | 412,15927 | 0,8233421 | 315,90454 | IBTK | AF235049 | Inhibitor of Bruton agammaglobulinemia tyrosine kinase |
| 210971_s_at | 0,00618 | 1,0115857 | 251,07408 | 0,7524625 | 190,85457 | ARNTL | AB000815 | Aryl hydrocarbon receptor nuclear translocator-like |
| 210972_x_at | 0,000131 | 0,9155198 | 8567,111 | 0,618035 | 5844,4136 | TCRA | M15565 | T-cell receptor rearranged alpha-chain V-region (V-D-J) |
| 211005_at | 0,000107 | 0,9775174 | 2050,1074 | 0,72708774 | 1506,9636 | LAT | AF036906 | Linker for activation of T cells |
| 211009_s_at | 4,53E-05 | 0,96128315 | 405,39996 | 0,7111121 | 301,09998 | ZNF271 | AF159567 | Zinc finger protein 271 |
| 211014_s_at | 5,94E-07 | 1,0085051 | 254,79259 | 1,4535959 | 369,0773 | PML | AF230410 | Promyelocytic leukemia |
| 211015_s_at | 0,000715 | 1,0593997 | 468,94073 | 0,77917063 | 325,54086 | HSPA4 | L12723 | Heat shock 70kDa protein 4 |
| 211023_at | 4,94E-10 | 0,9692619 | 2001,6815 | 0,59546214 | 1247,45 | PDHB | AL117618 | Pyruvate dehydrogenase (lipoamide) beta |
| 211061_s_at | 4,15E-06 | 1,0191569 | 765,2741 | 0,7041737 | 527,93634 | MGAT2 | BC006390 | Mannosyl (alpha-1,6-)-glycoprotein beta-1,2-N-acetylglucosaminyltransferase |
| 211063_s_at | 1,05E-08 | 0,9870602 | 639,6926 | 0,641082 | 420,80908 | NCK1 | BC006403 | NCK adaptor protein 1 |
| 211067_s_at | 8,52E-06 | 1,0029919 | 697,0815 | 1,3909883 | 952,46814 | GAS7 | BC006454 | Growth arrest-specific 7 |
| 211074_at | 0,00078 | 1,0401784 | 1092,7665 FBP; | 1,607227 | 1721,6819 | FOLR1; MOv18; FR-alpha | AF000381 | Homo sapiens folate binding protein mRNA, partial cds. |
| 211097_s_at | 0,000107 | 1,0136861 | 219,19998 HOX12; | 1,3959799 | 289,3773 | PBX2; G17; PBX2MHC | BC003111 | Homo sapiens pre-B-cell leukemia transcription factor 2, mRNA (cDNA clone IMAGE:3501925), with apparent retained intron. |
| 211100_x_at | 0,00101 | 0,9972845 | 1797,7074 | 1,3293542 TM | 2400,2908 | LILRA2 | U82278 | Leukocyte immunoglobulin-like receptor, subfamily A (with domain), member 2 |
| 211101_x_at | 0,000317 | 0,9826831 | 1744,8851 | 1,3437636 TM | 2398,9 | LILRA2 | U82276 | Leukocyte immunoglobulin-like receptor, subfamily A (with domain), member 2 |
| 211106_at | 0,00129 | 0,92932963 | 250,7 | 1,3392253 | 337,9364 | SUPT3H | AF064804 | Suppressor of Ty 3 homolog (S. cerevisiae) |
| 211139_s_at | 0,000656 | 0,95281744 | 272,50366 | 1,2367948 | 356,8318 | NAB1 | AF045452 | NGFI-A binding protein 1 (EGR1 binding protein 1) |
| 211144_x_at | 0,000715 | 0,99174637 | 3333,1628 | 0,6171851 1/2 | 2388,85 | CD3G | M30894 | Similar to T-cell receptor gamma chain V region PT-gamma-precursor |
| 211152_s_at | 0,00242 | 0,9657275 | 393,75552 | 0,74420065 | 311,91367 | PRSS25 | AF184911 | Protease, serine, 25 |
| 211212_s_at | 0,000382 | 1,010652 | 99,61482 | 0,7599604 | 75,977264 | ORC5L | AF081459 | Origin recognition complex, subunit 5-like (yeast) |
| 211251_x_at | 0,00129 | 0,93286145 | 599,537 | 1,174958 | 746,4273 | NFYC | U78774 | Nuclear transcription factor Y, gamma |
| 211284_s_at | 8,52E-06 | 1,0094445 | 2773,715 | 1,4523213 | 4024,2139 | GRN | BC000324 | Granulin |
| 211339_s_at | 0,000502 | 1,0010741 | 3751,0186 | 0,7521113 | 2842,9958 | ITK | D13720 | IL2-inducible T-cell kinase |
| 211395_x_at | 0,00861 | 0,9849375 | 1166,1628 | 1,312976 | 1501,2681 | FCGR2B | U90940 | Fc fragment of IgG, low affinity IIb, receptor for (CD32) |
| 211404_s_at | 0,000119 | 1,0099691 | 2391,5151 | 1,3054508 | 3076,8684 | APLP2 | BC004371 | Amyloid beta (A4) precursor-like protein 2 |
| 211416_x_at | 1,26E-06 | 0,8899997 | 245,66666 | 1,331963 | 367,1773 | GGTLA4 | L20492 | Gamma-glutamyltransferase-like activity 4 |
| 211417_x_at | 5,65E-05 | 0,9790086 | 408,3185 | 1,2676402 | 527,41815 | GGT1 | L20493 | Gamma-glutamyltransferase 1 |
| 211430_s_at | 2,23E-06 | 1,0306439 | 4548,656 | 0,25486428 | 2239,9727 | IGHG3 | M87789 | |
| 211433_x_at | 0,00129 | 1,010843 | 737,2999 | 1,3936001 | 1016,15894 | KIAA1539 | AL583909 | KIAA1539 |
| 211454_x_at | 2,56E-05 | 1,0184976 | 711,21106 | 1,3908132 FKSG70; | 992,3683 | FKSG51 | AF336878 | similar to other members in this gene family FKSG48 to Homo sapiens FKSG51 (FKSG51) mRNA, complete cds. |
| 211456_x_at | 0,00109 | 1,0631893 | 1321,0295 | 1,6683632 | 2040,7229 | | AF333388 | Similar to 60S ribosomal protein L35 |
| 211458_s_at | 4,02E-08 | 0,9661188 | 807,49634 3 | 1,881213 | 1640,868 | GABARAPL | AF180519 | LOC440308 |
| 211464_x_at | 0,00378 | 1,1128523 | 241,34445 | 0,73580366 | 151,11818 | CASP6 | U20537 | Caspase 6, apoptosis-related cysteine protease |
| 211572_s_at | 4,05E-05 | 0,9828207 | 108,303696 | 1,4913203 | 161,9 | SLC23A2 | AF092511 | Solute carrier family 23 (nucleobase transporters), member |
| 211596_s_at | 0,00833 | 1,0164862 | 391,52222 | 0,80635273 | 306,39093 | LRIG1 | AB050468 | Leucine-rich repeats and immunoglobulin-like domains 1 |
| 211597_s_at | 0,000502 | 1,0088419 | 2040,2999 | 0,639287 | 1289,3364 | HOP | AB059408 | Homeodomain-only protein |
| 211600_at | 0,00378 | 0,9255078 | 8383,455 PTPU2; | 1,1809856 | 9639,899 | PTPRO; GLEPP1; PTP-U2 | U20489 | membrane protein tyrosine phosphatase; Human glomerular epithelial protein 1 (GLEPP1) mRNA, complete cds. |
| 211612_s_at | 0,00101 | 0,9322991 | 394,30002 | 1,2625418 | 535,3954 | IL13RA1 | U62858 | IL-13 receptor; IL-13R or IL-13Ralpha; this protein together with the 140 kDa IL-4 binding protein, IL-4R or IL-4Ralpha, can form a functional receptor for IL-13; IL-13R plus IL-4R is also one of the functional forms of the IL-4 receptor, the |
| 211615_s_at | 2,29E-08 | 0,97806656 | 687,7852 | 0,7198936 | 505,25455 | LRPPRC | M92439 | Leucine-rich PPR-motif containing |
| 211644_x_at | 0,000382 | 1,0106673 | 871,6556 | 0,5211471 | 570,0091 | IGKC | L14458 | HRV Fab 026-VL |
| 211645_x_at | 6,59E-06 | 1,0296928 | 1275,4926 | 0,5159448 | 875,50006 | IgK | M85256 | Cationic anti-DNA autoantibody |
| 211671_s_at | 0,00192 | 1,1005856 | 1970,3258 | 0,8620287 | 1528,3411 | NR3C1 | U01351 | |
| 211716_x_at | 5,78E-08 | 0,98182553 | 2678,4368 | 1,4782009 | 4072,4863 | ARHGDIA | BC005851 | Rho GDP dissociation inhibitor (GDI) alpha |
| 211717_at | 1,17E-07 | 0,97382915 | 339,83704 | 0,43662548 | 168,39998 | MGC15396 | BC005853 | Hypothetical protein MGC15396 |
| 211721_s_at | 0,000119 | 1,0758232 | 132,27777 | 0,6115242 | 71,12273 | ZNF551 | BC005868 | Zinc finger protein 551 |
| 211729_x_at | 2,87E-05 | 0,9898688 | 1633,8817 | 1,3806758 | 2302,3274 | BLVRA | BC005902 | Biliverdin reductase A |
| 211747_s_at | 2,23E-06 | 0,96437216 | 1378,1406 | 0,69715405 | 1010,83185 | LSM5 | BC005938 | LSM5 homolog, U6 small nuclear RNA associated (S. cerevisiae) |
| 211748_x_at | 0,00109 | 1,0281676 | 1685,2925 | 0,577958 | 982,25916 | PTGDS | BC005939 | Prostaglandin D2 synthase 21 kDa (brain) |
| 211758_x_at | 1,85E-10 | 1,0128669 | 1325,5629 | 0,6399388 | 849,30005 | TXNDC9 | BC005968 | Thioredoxin domain containing 9 |
| 211761_s_at | 4,02E-08 | 1,0028944 | 1296,6259 | 0,7511687 | 971,1864 | CACYBP | BC005975 | Calcyclin binding protein |
| 211768_at | 0,000113 | 1,053732 | 195,44815 LAB; NTAL; | 1,4212145 | 258,89093 | WBSCR5; WSCR5; HSPC046; WBSCR15 | BC006080 | synonyms: HSPC046, LAB, NTAL, WBSCR15, WSCR5; Homo sapiens Williams-Beuren syndrome chromosome region 5, mRNA (cDNA clone MGC:12566 IMAGE:3621218), complete cds. |
| 211776_s_at | 0,00919 | 0,8826441 | 362,69623 | 1,1525629 | 473,43185 | EPB41 L3 | BC006141 | Erythrocyte membrane protein band 4.1-like 3 |
| 211784_s_at | 7,92E-10 | 0,9889373 | 2523,6038 | 0,6666943 | 1699,9363 | | BC006181 | |
| 211787_s_at | 3,75E-09 | 1,0099398 | 8269,667 | 1,3037341 | 10672,555 | EIF4A1 | BC006210 | Eukaryotic translation initiation factor 4A, isoform 1 |
| 211794_at | 1,59E-05 | 1,0319756 | 372,38516 | 1,4614798 | 527,12274 | FYB | AF198052 | FYN binding protein (FYB-120/130) |
| 211798_x_at | 0,00085 | 1,0213583 | 531,3816 | 0,56347245 | 331,82272 | IGLJ3 | AB001733 | Immunoglobulin lambda joining 3 |
| 211810_s_at | 0,00164 | 0,9648431 | 237,03703 | 1,3114824 | 321,32272 | GALC | D25284 | Galactosylceramidase (Krabbe disease) |
| 211815_s_at | 0,00101 | 1,0073216 | 200,09631 | 1,464465 binding | 299,42276 | GGA3 | AF219138 | Golgi associated, gamma adaptin ear containing, ARF protein 3 |
| 211833_s_at | 0,00192 | 0,8950861 | 446,01483 | 1,2875525 | 620,43634 | BAX | U19599 | BCL2-associated X protein |
| 211863_x_at | 0,00538 | 0,91886467 | 210,11482 | 1,2097462 | 267,60004 | HFE | AF079408 | Hemochromatosis |
| 211883_x_at | 0,00538 | 0,89338547 | 173,94814 | 1,317264 | 252,23637 | CEACAM1 | M76742 | Carcinoembryonic antigen-related cell adhesion molecule 1 (biliary glycoprotein) |
| 211902_x_at | 0,000196 | 1,0371852 | 3654,0664 | 0,6706089 | 2254,7498 | TCRA | L34703 | T cell receptor alpha chain |
| 211919_s_at | 4,05E-05 | 0,97988856 | 6760,1113 | 1,4891179 | 10352,941 | CXCR4 | AF348491 | Chemokine (C-X-C motif) receptor 4 |
| 211924_s_at | 3,88E-10 | 0,9729251 | 494,5926 | 2,498538 | 1382,1681 | PLAUR | AY029180 | Plasminogen activator, urokinase receptor |
| 211928_at | 0,000459 | 1,0439823 | 1260,6594 | 0,78824204 | 944,34094 | DNCH1 | AB002323 | Dynein, cytoplasmic, heavy polypeptide 1 |
| 211929_at | 7,99E-06 | 1,0222622 | 1518,1111 | 0,66293085 | 974,99994 | HNRPA3 | BE867771 | Heterogeneous nuclear ribonucleoprotein A3 |
| 211935_at | 1,40E-05 | 1,0093172 | 1773,6035 | 0,7412872 | 1312,5092 | ARL6IP | D31885 | ADP-ribosylation factor-like 6 interacting protein |
| 211938_at | 3,22E-05 | 1,0400327 | 5491,4185 | 0,8085629 | 4282,696 | PRO1843 | BF247371 | Hypothetical protein PRO1843 |
| 211946_s_at | 0,00119 | 1,0642943 | 2589,663 | 0,8391627 | 2018,1545 | XTP2 | AL096857 | HBxAg transactivated protein 2 |
| 211948_x_at | 0,00119 | 1,0183944 | 1280,0779 | 1,2843984 | 1619,4044 | XTP2 | AL096857 | HBxAg transactivated protein 2 |
| 211951_at | 0,00085 | 1,0331386 | 1518,959 | 0,8042789 | 1174,4865 | NOLC1 | D21262 | Nucleolar and coiled-body phosphoprotein 1 |
| 211953_s_at | 3,22E-05 | 1,0154723 | 355,77408 | 0,7169531 | 250,23181 | RANBP5 | NM_002271 | RAN binding protein 5 |
| 211954_s_at | 5,07E-06 | 1,0029068 | 794,989 | 0,7621909 | 608,18634 | RANBP5 | NM_002271 | RAN binding protein 5 |
| 211960_s_at | 9,67E-06 | 1,0007331 | 938,1297 CMT2B; | 1,2924621 | 1203,4181 | RAB7; PSN; PRO2706 | AK000826 | unnamed protein product; Homo sapiens cDNA FLJ20819 fis, clone ADSE00511. |
| 211971_s_at | 1,05E-08 | 1,001206 | 1685,1853 | 0,7642256 | 1286,6683 | LRPPRC | AF052133 | Leucine-rich PPR-motif containing |
| 211977_at | 0,00078 | 0,94222575 | 261,63702 | 1,2470837 | 339,67728 | GPR107 | AK024651 | LOC441469 |
| 211987_at | 5,09E-07 | 0,99810827 | 1763,3555 | 0,71720314 | 1300,9 | TOP2B | NM_001068 | Topoisomerase (DNA) II beta 180kDa |
| 211997_x_at | 0,00406 | 0,97175586 | 17090,752 | 1,2439859 | 21190,25 | H3F3B | NM_005324 | H3 histone, family 3B (H3.3B) |
| 211998_at | 4,94E-10 | 0,99191123 | 2782,1965 | 1,8850294 | 5213,9365 | H3F3B | NM_005324 | H3 histone, family 3B (H3.3B) |
| 211999_at | 1,89E-08 | 1,0133227 | 7208,9966 | 1,407858 | 10026,301 | H3F3B | NM_005324 | H3 histone, family 3B (H3.3B) |
| 212002_at | 0,00807 | 0,98102844 | 594,5815 0424 | 1,2326484 | 792,16364 | DKFZp566C | AL050028 | Putative MAPK activating protein PM20,PM21 |
| 212005_at | 0,00755 | 0,9802732 | 389,93707 0424 | 1,3510575 | 547,43634 | DKFZp566C | AL050028 | Putative MAPK activating protein PM20,PM21 |
| 212014_x_at | 1,24E-05 | 1,0018442 | 3196,5667 | 1,4345969 | 4497,2725 | CD44 | AJ251595 | CD44 antigen (homing function and Indian blood group |
| 212037_at | 0,00242 | 0,9556239 | 552,6666 | 0,691339 | 388,24542 | PNN | Y09703 | Pinin, desmosome associated protein |
| 212044_s_at | 0,00861 | 1,0216703 | 494,0333 | 1,3182285 | 643,32733 | RPL27A | BE737027 | Hypothetical protein MGC10850 |
| 212060_at | 0,00164 | 1,0472335 | 262,4889 | 0,8252646 | 205,94545 | SR140 | AB002330 | U2-associated SR140 protein |
| 212071_s_at | 3,39E-06 | 1,0470334 | 2621,4072 | 0,7345401 IMAGE:3933782 | 1853,332 | SPTBN1 | BE968833 | 601649861F1 NIH_MGC_74 Homo sapiens cDNA clone 5', mRNA sequence. |
| 212090_at | 0,000317 | 0,93059397 | 811,6778 | 1,4642198 | 1351,3499 | GRINA | AL571424 | Glutamate receptor, ionotropic, N-methyl D-asparate-associated protein 1 (glutamate binding) |
| 212115_at | 6,91 E-08 | 0,9986523 | 503,49997 | 0,5820244 | 316,35 | C16orf34 | AK023154 | Chromosome 16 open reading frame 34 |
| 212116_at | 5,77E-08 | 0,9501283 | 917,7296 | 0,6577207 | 637,02277 | RFP | NM_006510 | Ret finger protein |
| 212130_x_at | 8,59E-09 | 1,0611563 | 15185,458 | 1,4865524 | 21208,357 | SUI1 | AL537707 | Putative translation initiation factor |
| 212139_at | 4,14E-06 | 0,9726818 | 636,2593 | 0,7571001 | 494,99545 | GCN1L1 | D86973 | GCN1 general control of amino-acid synthesis 1-like 1 |
| 212145_at | 7,01E-09 | 0,9803696 | 816,2519 | 0,7392148 | 614,7546 | MRPS27 | D87453 | Mitochondrial ribosomal protein S27 |
| 212149_at | 0,000317 | 0,99081683 | 1137,1406 | 0,70918727 IMAGE:1290318 | 848,8591 | KIAA0143 | AA805651 | nz41a04.s1 NCI_CGAP_GCB1 Homo sapiens cDNA clone 3', mRNA sequence. |
| 212150_at | 5,71 E-09 | 1,024101 | 823,4555 | 0,6957146 | 564,7818 | KIAA0143 | AA805651 | nz41a04.s1 NCI_CGAP_GCB1 Homo sapiens cDNA clone IMAGE:1290318 3', mRNA sequence. |
| 212157_at | 0,000145 | 0,9703585 | 128,68518 | 1,4959275 | 191,29999 | SDC2 | J04621 | Syndecan 2 (heparan sulfate proteoglycan 1, cell surface-associated, fibroglycan) |
| 212160_at | 0,00352 | 1,0107316 | 744,8074 | 0,80674374 | 592,89996 | XPOT | AI984005 | Exportin, tRNA (nuclear export receptor for tRNAs) |
| 212168_at | 7,81E-05 | 1,011467 | 1124,5852 | 0,77121013 | 858,3546 | RBM12 | AB018308 | RNA binding motif protein 12 |
| 212170_at | 9,47E-09 | 0,99645317 | 225,42223 | 0,59164655 | 136,23634 | RBM12 | AB018308 | RNA binding motif protein 12 |
| 212171_x_at | 9,36E-07 | 0,9160572 | 362,5889 | 1,4339925 | 582,75464 | VEGF | H95344 | Vascular endothelial growth factor |
| 212179_at | 0,00538 | 0,9248783 | 1290,5852 | 0,70231056 | 952,36816 | C6orf111 | AL080186 | Chromosome 6 open reading frame 111 |
| 212180_at | 6,58E-06 | 0,9416765 | 1321,5259 | 0,7373823 | 1031,868 | CRKL | AK000311 | V-crk sarcoma virus CT10 oncogene homolog (avian)-like |
| 212184_s_at | 8,06E-07 | 0,97703016 | 1335,0814 | 0,7223809 | 990,21826 | MAP3K7IP2 | AL117407 | Mitogen-activated protein kinase kinase kinase 7 interacting protein 2 |
| 212185_x_at | 2,56E-05 | 0,92484426 | 3173,5261 | 1,3796692 | 4694,6914 | MT2A | NM_005953 | Metallothionein 2A |
| 212187_x_at | 0,00468 | 0,94808006 | 1461,6669 | 0,55198145 | 929,92267 | PTGDS | NM_000954 | Prostaglandin D2 synthase 21kDa (brain) |
| 212191_x_at | 0,00078 | 0,9713422 | 28546,262 | 0,7649245 | 22357,139 | RPL13 | AW574664 | Similar to ribosomal protein L13; 60S ribosomal protein L13; breast basic conserved protein 1 |
| 212195_at | 0,000715 | 1,0349362 | 1515,6371 | 0,72666305 | 1070,4271 | IL6ST | AL049265 | Interleukin 6 signal transducer (gp130, oncostatin M |
| 212199_at | 1,38E-12 | 1,0107112 | 1518,0703 | 0,51332426 | 788,1637 | PP784 | AL566962 | PP784 protein |
| 212200_at | 0,00282 | 1,0416572 | 264,26297 | 0,8247934 | 211,59546 | KIAA0692 | AB014592 | KIAA0692 protein |
| 212215_at | 7,01E-09 | 0,96816784 | 422,96295 | 0,5524318 | 247,19547 | KIAA0436 | AB007896 | Putative prolyl oligopeptidase |
| 212216_at | 0,000549 | 0,96960646 | 249,09258 | 0,7022748 | 182,61365 | KIAA0436 | AB007896 | Putative prolyl oligopeptidase |
| 212217_at | 9,64E-05 | 1,0172393 | 343,28882 | 0,6666685 | 235,74547 | KIAA0436 | AB007896 | Putative prolyl oligopeptidase |
| 212218_s_at | 0,00101 | 0,9019671 | 291,12964 | 1,2348093 | 395,3727 | FBXO9 | NM_012347 | F-box protein 9 |
| 212222_at | 2,75E-06 | 0,9247017 | 359,5074 | 0,5887438 | 224,8091 | PSME4 | D38521 | Proteasome (prosome, macropain) activator subunit 4 |
| 212225_at | 0,00352 | 0,959843 | 980,6148 | 1,5321555 | 1650,4681 | SUI1 | AL516854 | Putative translation initiation factor |
| 212227_x_at | 1,89E-08 | 1,0573559 | 14852,556 | 1,455076 | 20379,617 | SUI1 | AL516854 | Putative translation initiation factor |
| 212240_s_at | 0,00282 | 0,9027999 | 1566,5592 | 1,3543122 | 2205,255 | PIK3R1 | M61906 | Phosphoinositide-3-kinase, regulatory subunit 1 (p85 alpha) |
| 212241_at | 9,80E-08 | 1,0100873 | 427,1148 | 1,5098699 | 641,5454 | GRINL1A | AI632774 | Glutamate receptor, ionotropic, N-methyl D-aspartate-like |
| 212243_at | 0,000348 | 1,0082645 | 595,79266 | 1,3095009 | 784,0636 | GRINL1A | AI632774 | Glutamate receptor, ionotropic, N-methyl D-aspartate-like |
| 212247_at | 2,87E-05 | 1,0105963 | 456,95178 | 0,698551 | 322,07272 | NUP205 | AW008531 | Nucleoporin 205kDa |
| 212262_at | 6,91E-08 | 0,9982981 | 603,5296 QK3 | 1,4920624 | 901,2773 | QKI; Hqk; | AF142419 | QUAKING-6; Homo sapiens QUAKING isoform 6 (QUAKING) mRNA, complete cds. |
| 212264_s_at | 0,000459 | 1,0245625 | 653,75183 | 0,7856625 | 511,83182 | KIAA0261 | D87450 | KIAA0261 |
| 212267_at | 8,52E-06 | 1,0083219 | 1042,822 | 0,76312244 | 794,07275 | KIAA0261 | D87450 | KIAA0261 |
| 212281_s_at | 1,10E-05 | 0,99320376 | 154,26295 | 0,7324007 | 113,3409 | MAC30 | L19183 | Hypothetical protein MAC30 |
| 212289_at | 0,000119 | 1,0472916 | 436,09253 | 0,7295085 | 297,83188 | ANKRD12 | AW572909 | Ankyrin repeat domain 12 |
| 212297_at | 6,91 E-08 | 1,0781971 | 381,83337 | 0,69003624 | 237,29091 | ATP13A3 | BF218804 | ATPase type 13A3 |
| 212303_x_at | 0,00577 | 0,89388514 | 238,06297 | 1,1998131 IMAGE:4475875 | 322,05002 | KHSRP | BG255575 | 602367874F1 NIH_MGC_91 Homo sapiens cDNA clone 5', mRNA sequence. |
| 212306_at | 0,00152 | 0,8871033 | 432,47778 | 0,61838603 | 308,51816 | CLASP2 | AB014527 | Cytoplasmic linker associated protein 2 |
| 212310_at | 5,77E-06 | 1,028968 | 247,90369 | 0,7536253 | 180,98183 | FLJ39207 | D87742 | C219-reactive peptide |
| 212313_at | 1,43E-10 | 1,0434551 | 1131,0778 | 0,66512156 | 707,83636 | MGC29816 | BC004344 | Hypothetical protein MGC29816 |
| 212314_at | 9,36E-07 | 1,0224276 | 1817,6742 | 0,7229356 | 1291,8907 | KIAA0746 | AB018289 | KIAA0746 protein |
| 212331_at | 0,00164 | 1,017985 | 1913,0187 | 0,8090902 | 1529,7318 | RBL2 | NM_005611 | Retinoblastoma-like 2 (p130) |
| 212333_at | 1,57E-09 | 0,95672417 | 407,2963 0522 | 0,59286904 | 249,15 | DKFZP564F | AL049943 | DKFZP564F0522 protein |
| 212351_at | 0,00152 | 1,0053196 | 609,9408 | 0,80177546 82kDa | 484,92267 | EIF2B5 | U23028 | Eukaryotic translation initiation factor 2B, subunit 5 epsilon, |
| 212352_s_at | 3.71E-07 | 0,9855415 | 3940,9333 | 0,7512632 | 2979,1182 | TMP21 | BE780075 | Transmembrane trafficking protein |
| 212360_at | 0,000131 | 0,85334915 | 1681,2925 | 1,1799467 | 2231,3271 | AMPD2 | AI916249 | Adenosine monophosphate deaminase 2 (isoform L) |
| 212361_s_at | 0,00468 | 0,9015144 | 583,237 | 1,1911564 | 756,14087 | ATP2A2 | AK000300 | ATPase, Ca++transporting, cardiac muscle, slow twitch 2 |
| 212366_at | 0,000289 | 0,9982088 | 167,20001 | 0,7001395 | 114,39091 | ZNF292 | AA972711 | Zinc finger protein 292 |
| 212367_at | 3,02E-09 | 1,0574574 | 274,54074 | 0,5964118 | 150,69092 | FEM1B | NM_015322 | Fem-1 homolog b (C. elegans) |
| 212373_at | 0,00502 | 1,0190051 | 223,12222 | 1,2894217 | 282,6227 | FEM1B | NM_015322 | Fem-1 homolog b (C. elegans) |
| 212374_at | 2,17E-11 | 1,0086262 | 203,9037 | 2,2393217 | 432,4591 | FEM1B | NM_015322 | Fem-1 homolog b (C. elegans) |
| 212375_at | 0,00352 | 0,9550311 | 455,47034 | 0,7627531 | 364,11816 | EP400 | BE880591 | E1A binding protein p400 |
| 212378_at | 1,11E-10 | 1,0045868 | 354,74075 | 0,66538376 | 234,85909 | GART | BE966876 | Phosphoribosylglycinamide formyltransferase, phosphoribosylglycinamide synthetase, phosphoribosylaminoimidazole synthetase |
| 212381_at | 3,61E-05 | 1,0472031 | 329,64072 | 0,6929115 | 219,3818 | USP24 | AB028980 | Ubiquitin specific protease 24 |
| 212383_at | 0,00119 | 1,0322106 | 623,74445 | 1,3893352 | 849,4728 | ATP6V0A1 | AL096733 | ATPase, H+ transporting, lysosomal V0 subunit a isoform 1 |
| 212386_at | 0,000502 | 1,0731921 | 957,8111 | 0,80461085 | 716,36816 | TCF4 | AK021980 | Transcription factor 4 |
| 212388_at | 9,49E-09 | 0,9707065 | 472,39258 | 0,5764192 | 284,59094 | USP24 | AB028980 | Ubiquitin specific protease 24 |
| 212397_at | 4,78E-05 | 0,9861255 | 403,18518 812 | 0,7691436 | 317,20453 | DKFZp434I0 | AL137751 | radixin (Homo sapiens); Homo sapiens mRNA; cDNA DKFZp434I0812 (from clone DKFZp434I0812); partial cds. |
| 212399_s_at | 0,000849 | 1,0113149 | 445,55554 | 0,80782706 | 357,89093 | VGLL4 | D50911 | Vestigial like 4 (Drosophila) |
| 212402_at | 3,80E-11 | 1,0236617 | 787,3149 | 0,57609224 | 458,33636 | KIAA0853 | BE895685 | KIAA0853 |
| 212405_s_at | 3,88E-10 | 0,94724804 | 440,8111 | 0,50479674 | 242,22273 | KIAA0859 | AL049669 | KIAA0859 |
| 212406_s_at | 6,30E-05 | 1,0143616 | 1505,5852 | 0,71639806 | 1101,2726 | MYT1 | AB028973 | Myelin transcription factor 1 |
| 212407_at | 2,87E-05 | 1,0084373 | 366,99625 | 0,7398826 | 273,07727 | KIAA0859 | AL049669 | KIAA0859 |
| 212408_at | 3,03E-09 | 0,9913982 | 2265,1187 | 0,7289842 | 1682,6727 | LAP1B | AK023204 | Lamina-associated polypeptide 1B |
| 212410_at | 5,07E-06 | 1,0009319 | 1243,1555 | 0,73294723 | 910,6091 | EFHA1 | AI346431 | EF hand domain family, member A1 |
| 212412_at | 3,61E-05 | 0,9927872 | 1281,5704 | 0,7912861 | 1030,1864 | PDLIM5 | AV715767 | PDZ and LIM domain 5 |
| 212430_at | 0,00436 | 1,0546043 | 791,31854 | 1,3465458 | 1031,532 | RNPC1 | AL109955 | |
| 212432_at | 1,78E-07 | 0,9842823 | 658,65186 | 1,3382767 | 898,72723 | GRPEL1 | AL542571 | GrpE-like 1, mitochondrial (E. coli) |
| 212434_at | 1,43E-10 | 0,9977885 | 538,25183 | 1,5537717 | 837,44543 | GRPEL1 | AL542571 | GrpE-like 1, mitochondrial (E. coli) |
| 212435_at | 0,00208 | 0,9997357 | 619,3889 | 0,73553133 | 466,65002 | TRIM33 | AI967961 | Tripartite motif-containing 33 |
| 212445_s_at | 0,000656 | 0,94854456 | 120,23333 | 1,355039 | 168,18637 | NEDD4L | AB007899 | Neural precursor cell expressed, developmentally down-regulated 4-like |
| 212450_at | 2,02E-05 | 0,98186606 | 414,02594 | 0,7153035 | 304,60452 | KIAA0256 | D87445 | KIAA0256 gene product |
| 212453_at | 3,39E-06 | 0,906523 | 272,05927 | 0,555952 | 159,42728 | KIAA1279 | AB033105 | KIAA1279 |
| 212472_at | 0,00119 | 0,9089375 | 848,03705 | 1,2778027 IMAGE:3886131 | 1181,2682 | | BE965029 | 601658812R1 NIH_MGC_69 Homo sapiens cDNA clone 3', mRNA sequence. |
| 212474_at | 0,000119 | 0,96435344 | 479,24814 | 0,7325578 | 362,4091 | KIAA0241 | BE503381 | KIAA0241 protein |
| 212477_at | 0,0078 | 0,8391337 | 55,45926 | 1,2634282 | 71,00001 | CENTB2 | D26069 | Centaurin, beta 2 |
| 212483_at | 0,000119 | 1,040671 | 442,82224 | 0,7828108 | 334,6091 | NIPBL | AB019494 | Nipped-B homolog (Drosophila) |
| 212486_s_at | 8,52E-11 | 1,0130296 | 746,4926 | 0,68567294 | 510,18182 | FYN | N20923 | FYN oncogene related to SRC, FGR, YES |
| 212498_at | 3,88E-10 | 0,9892991 | 1323,2703 | 0,6220035 | 829,6272 | MARCH-VI | AF056433 | Membrane-associated ring finger (C3HC4) 6 |
| 212501_at | 1,43E-10 | 0,9733732 | 9878,504 | 1,5348922 | 15566,36 | CEBPB | AL564683 | CCAAT/enhancer binding protein (C/EBP), beta |
| 212507_at | 2,87E-11 | 0,9814308 | 1153,3481 | 0,67418754 | 793,4227 | RW1 | D87446 | RW1 protein |
| 212513_s_at | 9,36E-07 | 0,96958834 | 1282,5742 | 0,71202546 | 940,9863 | USP33 | AB029020 | Ubiquitin specific protease 33 |
| 212522_at | 9,67E-06 | 0,93746394 | 406,95557 | 0,5939192 | 255,53636 | PDE8A | BE568219 | Phosphodiesterase 8A |
| 212523_s_at | 0,00078 | 0,9662193 | 152,27406 | 0,6785134 | 109,00455 | KIAA0146 | D63480 | KIAA0146 protein |
| 212526_at | 2,55E-05 | 0,9231903 | 336,36667 | 0,58599406 | 218,34544 | SPG20 | AK002207 | Spastic paraplegia 20, spartin (Troyer syndrome) |
| 212527_at | 0,000238 | 0,94173974 | 328,93704 | 1,17928 | 411,22726 | D15Wsu75e | AL023553 | |
| 212530_at | 1,89E-08 | 0,98963547 | 1764,1149 | 0,61835104 | 1115,7273 | NEK7 | AL080111 | NIMA (never in mitosis gene a)-related kinase 7 |
| 212532_s_at | 5,94E-07 | 0,9775388 | 905,2444 | 1,2707468 | 1169,6547 | FLJ30656 | BF967094 | Hypothetical protein FLJ30656 |
| 212536_at | 5,94E-07 | 0,97469985 | 607,30743 | 0,67672896 | 422,18634 | ATP11B | AB023173 | ATPase, Class VI, type 11B |
| 212538_at | 4,44E-06 | 0,92979616 | 456,41107 | 0,49669656 | 256,26816 | DOCK9 | AL576253 | Dedicator of cytokinesis 9 |
| 212546_s_at | 2,48E-07 | 1,0042422 | 860,09265 | 0,6995858 | 606,3592 | KIAA0826 | AB020633 | KIAA0826 |
| 212557_at | 2,48E-07 | 1,0176414 | 471,60367 | 0,7518984 | 348,89996 | ZNF451 | AB011148 | KIAA1702 protein |
| 212560_at | 8,06E-07 | 1,0537369 | 4292,4775 | 0,77931976 | 3188,3958 | SORL1 | AV728268 | Sortilin-related receptor, L(DLR class) A repeats-containing |
| 212561_at | 0,00119 | 0,8935444 | 2079,178 | 1,1417994 | 2614,5044 | RAB6IP1 | AA349595 | RAB6 interacting protein 1 |
| 212565_at | 2,91E-07 | 0,99649304 | 171,52223 | 0,55477846 | 104,74091 | STK38L | AB023182 | Serine/threonine kinase 38 like |
| 212568_s_at | 0,000289 | 0,9649667 | 314,66296 | 0,7716318 | 254,28183 | DLAT | BF978872 | Dihydrolipoamide S-acetyltransferase (E2 component of pyruvate dehydrogenase complex) |
| 212569_at | 0,00327 | 1,0333823 | 703,3777 | 0,72806406 | 523,09546 | KIAA0650 | AA868754 | KIAA0650 protein |
| 212574_x_at | 0,00208 | 0,9566229 | 453,79257 | 1,6080947 | 755,8363 | R32184_3 | AC004528 | |
| 212576_at | 0,00233 | 0,9396626 | 896,26666 | 1,1801946 | 1102,9498 | MGRN1 | AB011116 | Mahogunin, ring finger 1 |
| 212579_at | 0,00261 | 0,9654427 | 806,6334 | 0,72087455 | 611,941 | KIAA0650 | AA868754 | KIAA0650 protein |
| 212580_at | 0,00617 | 0,9481051 | 123,559265 | 1,2291763 | 159,1682 | CAST | BG111635 | Calpastatin |
| 212585_at | 0,00282 | 1,0595865 | 1907,652 | 0,8237982 | 1485,6909 | OSBPL8 | AB040884 | Oxysterol binding protein-like 8 |
| 212589_at | 7,02E-05 | 0,99579567 | 244,52222 | 0,60016865 | 151,78636 | SCP2 | BG168858 | Sterol carrier protein 2 |
| 212591_at | 8,52E-06 | 0,9872091 | 1235,5259 | 0,70366603 | 896,0818 | KIAA0117 | AA887480 | KIAA0117 protein |
| 212592_at | 8,06E-07 | 1,0268843 | 1194,1445 | 0,35290387 | 647,8092 | IGJ | AV733266 | Immunoglobulin J polypeptide, linker protein for immunoglobulin alpha and mu polypeptides |
| 212593_s_at | 7,02E-05 | 0,9501869 | 3563,8813 | 0,6656436 | 2540,986 | PDCD4 | N92498 | Programmed cell death 4 (neoplastic transformation |
| 212594_at | 2,14E-05 | 1,1218851 | 664,19995 | 0,6984555 | 385,2909 | PDCD4 | N92498 | Programmed cell death 4 (neoplastic transformation |
| 212595_s_at | 0,00707 | 0,89588845 | 3624,9634 | 1,2055101 | 4920,413 | DAZAP2 | AL534321 | DAZ associated protein 2 |
| 212599_at | 8,06E-07 | 1,006378 | 921,0187 | 0,5636404 | 502,79547 | AUTS2 | AK025298 | Autism susceptibility candidate 2 |
| 212603_at | 5,78E-06 | 0,9837614 | 424,35553 | 0,71782774 | 312,77722 | MRPS31 | NM_005830 | Mitochondrial ribosomal protein S31 |
| 212604_at | 0,00577 | 0,9669669 | 719,60364 | 0,7559949 | 577,6909 | MRPS31 | NM_005830 | Mitochondrial ribosomal protein S31 |
| 212607_at | 0,00152 | 1,0065175 | 663,69257 | 0,8050385 | 533,1636 | AKT3 | U79271 | V-akt murine thymoma viral oncogene homolog 3 (protein kinase B, gamma) |
| 212610_at | 5,78E-06 | 0,98332816 | 1457,289 | 0,7710438 (Noonan | 1145,4729 | PTPN11 | U79291 | Protein tyrosine phosphatase, non-receptor type 11 syndrome 1) |
| 212613_at | 5,65E-05 | 0,90856975 | 1216,3705 | 0,49887466 | 745,55914 | BTN3A2 | AI991252 | Butyrophilin, subfamily 3, member A2 |
| 212615_at | 0,000178 | 1,0245358 | 257,25925 | 0,67884886 | 167,99998 | CHD9 | AB002306 | Chromodomain helicase DNA binding protein 9 |
| 212616_at | 0,00101 | 0,97540337 | 1007,07043 | 0,75906664 | 769,191 | CHD9 | AB002306 | Chromodomain helicase DNA binding protein 9 |
| 212621_at | 3,37E-09 | 0,9578644 | 268,6926 | 0,60541165 | 170,05453 | KIAA0286 | AW205215 | KIAA0286 protein |
| 212622_at | 0,000317 | 0,9416061 | 607,6 | 0,66821 | 427,53183 | TMEM41B | D26067 | Transmembrane protein 41 B |
| 212623_at | 5,07E-06 | 0,94651675 | 369,63333 | 0,5742425 | 221,51367 | TMEM41B | D26067 | Transmembrane protein 41 B |
| 212625_at | 0,0014 | 0,96649927 | 1077,011 | 1,2195867 | 1333,3456 | STX10 | NM_003765 | Syntaxin 10 |
| 212631_at | 0,00261 | 0,9595377 | 934,89996 | 0,7522769 | 734,87714 | STX7 | AF131808 | Syntaxin 7 |
| 212633_at | 1,26E-06 | 1,0019544 | 505,37402 | 0,6083008 | 309,89545 | KIAA0776 | AW298092 | UI-H-BW0-ajs-b-03-0-UI.s1 NCI_CGAP_Sub6 Homo sapiens cDNA clone IMAGE:2732860 3', mRNA sequence. |
| 212635_at | 0,00707 | 1,0386527 | 526,86664 | 0,8083663 | 401,73184 | TNPO1 | AW161626 | Transportin 1 |
| 212637_s_at | 1,59E-05 | 0,96342915 | 179,82964 | 0,5648845 | 109,3409 | WWP1 | BF131791 | WW domain containing E3 ubiquitin protein ligase 1 |
| 212642_s_at | 0,00304 | 1,0528435 | 867,0741 | 0,6914453 | 568,5182 | HIVEP2 | AL023584 | |
| 212648_at | 3,16E-06 | 1,0284519 | 418,2852 | 0,72233915 | 294,8909 | DHX29 | AL079292 | DEAH (Asp-Glu-Ala-His) box polypeptide 29 |
| 212649_at | 0,0014 | 0,96718234 | 146,69258 | 1,4103271 | 203,48636 | DHX29 | AL079292 | DEAH (Asp-Glu-Ala-His) box polypeptide 29 |
| 212653_s_at | 7,50E-06 | 0,95109886 | 298,11856 | 0,6634029 | 209,7227 | EHBP1 | AB020710 | EH domain binding protein 1 |
| 212655_at | 0,000289 | 0,92946136 | 257,0593 | 0,6720518 | 184,80455 | ZCCHC14 | AB011151 | Zinc finger, CCHC domain containing 14 |
| 212657_s_at | 1,09E-06 | 0,992546 | 654,3 | 1,5781707 | 1100,65 | IL1RN | AW083357 | Interleukin 1 receptor antagonist |
| 212659_s_at | 5,94E-07 | 0,9739689 | 303,4111 | 1,5954442 | 482,6227 | IL1RN | AW083357 | Interleukin 1 receptor antagonist |
| 212660_at | 4,35E-07 | 0,9892201 | 1913,7927 | 0,73698807 | 1435,6636 | PHF15 | AI735639 | PHD finger protein 15 |
| 212665_at | 5,71E-09 | 1,0477262 | 867,41846 | 1,9098371 | 1611,1636 | TIPARP | AL556438 | TCDD-inducible poly(ADP-ribose) polymerase |
| 212672_at | 1,05E-08 | 0,9700303 | 1434,4814 | 0,46686128 | 708,191 | | U82828 | |
| 212675_s_at | 1,64E-07 | 0,97258425 | 594,14453 | 0,5138445 | 324,72272 | KIAA0582 | AB011154 | KIAA0582 |
| 212678_at | 9,67E-06 | 1,0185575 | 221,86665 | 0,723854 disease, | 163,31819 | NF1 | AW293356 | Neurofibromin 1 (neurofibromatosis, von Recklinghausen Watson disease) |
| 212679_at | 3,22E-05 | 0,85134137 | 91,77037 | 1,2846789 | 133,5909 | TBL2 | AK026529 | Transducin (beta)-like 2 |
| 212685_s_at | 1,64E-07 | 1,0241802 | 393,89993 | 0,6848971 | 265,48636 | TBL2 | AK026529 | Transducin (beta)-like 2 |
| 212687_at | 5,06E-05 | 1,0141388 | 2548,1926 | 0,64631116 | 1720,5681 | LIMS1 | AL110164 | LIM and senescent cell antigen-like domains 1 |
| 212688_at | 2,75E-06 | 1,0057503 | 471,80737 | 0,707726 | 338,26367 | PIK3CB | BC003393 | Phosphoinositide-3-kinase, catalytic, beta polypeptide |
| 212690_at | 4,04E-05 | 0,9667656 | 710,1814 | 0,7186428 | 524,60455 | DDHD2 | AB018268 | DDHD domain containing 2 |
| 212692_s_at | 0,00242 | 0,9799008 | 975,3999 | 0,77651745 | 775,7455 | LRBA | W60686 | Transcribed locus |
| 212696_s_at | 4,82E-08 | 1,0201188 | 1593,2704 | 0,73954934 | 1165,2363 | RNF4 | BF968633 | Ring finger protein 4 |
| 212698_s_at | 0,00919 | 1,0401366 | 158,48148 | 0,710178 | 114,29091 | 10.Sep | BF966021 | Septin 10 |
| 212706_at | 0,00119 | 0,93721807 | 684,0704 | 0,7133349 | 518,9 | RASA4 | AB011110 | RAS p21 protein activator 4 |
| 212709_at | 2,44E-09 | 1,0095263 | 217,16666 | 0,5706159 | 125,00001 | NUP160 | D83781 | Nucleoporin 160kDa |
| 212722_s_at | 3,03E-09 | 0,9782589 | 289,9741 | 1,5261807 | 459,94547 | PTDSR | AK021780 | Phosphatidylserine receptor |
| 212723_at | 2,28E-07 | 1,0489494 | 616,8445 | 1,6413085 | 992,3727 | PTDSR | AK021780 | Phosphatidylserine receptor |
| 212726_at | 0,000238 | 0,9750796 | 538,5111 | 1,2927582 | 700,35913 | PHF2 | AB014562 | PHD finger protein 2 |
| 212731_at | 2,27E-05 | 0,982225 | 246,77777 | 0,6429113 | 156,1318 | LOC157567 | U79297 | Hypothetical protein LOC157567 |
| 212737_at | 0,000459 | 1,0064312 | 1693,4741 | 1,4274476 | 2356,2861 | CSH2 | AL513583 | Chorionic somatomammotropin hormone 2 |
| 212740_at | 6,25E-10 | 0,88523966 | 533,8629 | 0,478632 | 286,7136 | PIK3R4 | BF740111 | Phosphoinositide-3-kinase, regulatory subunit 4, p150 |
| 212749_s_at | 6,92E-07 | 0,98310703 | 637,51855 | 0,6805843 | 441,68637 | RCHY1 | AL050144 | Ring finger and CHY zinc finger domain containing 1 |
| 212754_s_at | 4,63E-09 | 0,98624355 | 694,8554 | 0,691152 | 488,91364 | KIAA1040 | AI760249 | KIAA1040 protein |
| 212762_s_at | 5,65E-05 | 1,0264995 | 519,4296 | 1,5624069 | 777,6365 | TCF7L2 | AI703074 | Transcription factor 7-like 2 (T-cell specific, HMG-box) |
| 212766_s_at | 8,06E-07 | 1,0041761 | 606,92584 | 0,7433535 | 449,06815 | FLJ12671 | AW294587 | Hypothetical protein FLJ12671 |
| 212769_at | 6,90E-08 | 1,0267067 | 217,71112 | 1,5677612 Drosophila) | 335,93637 | TLE3 | AI567426 | Transducin-like enhancer of split 3 (E(sp1) homolog, |
| 212771_at | 1,40E-05 | 1,0036414 | 304,07776 | 0,73899025 | 227,95906 | C10orf38 | AU150943 | Chromosome 10 open reading frame 38 |
| 212781_at | 4,53E-05 | 0,98159504 | 476,90744 | 0,66993487 | 323,6 | RBBP6 | AK026954 | Retinoblastoma binding protein 6 |
| 212785_s_at | 1,57E-09 | 0,9688745 | 806,21484 | 0,7116147 | 591,1773 | HDCMA18P | AA160181 | HDCMA18P protein |
| 212789_at | 2,23E-06 | 0,9771698 | 316,237 | 0,65546286 | 212,35909 | KIAA0056 | AI796581 | KIAA0056 protein |
| 212792_at | 2,77E-08 | 0,9962697 | 284,62595 | 0,7121267 | 203,51819 | KIAA0877 | AB020684 | KIAA0877 protein |
| 212795_at | 1,24E-05 | 0,9490054 | 1558,341 | 0,6774037 | 1138,9136 | KIAA1033 | AL137753 | KIAA1033 protein |
| 212802_s_at | 2,29E-08 | 0,9914318 | 1019,54816 | 0,6938858 | 721,14087 | DKFZP434C 212 | AK023841 | DKFZP434C212 protein |
| 212804_s_at | 0,00378 | 0,95502144 | 286,4704 212 | 0,718398 | 218,25908 | DKFZP434C | AK023841 | DKFZP434C212 protein |
| 212807_s_at | 5,09E-07 | 0,9509592 | 340,51855 | 1,3361024 | 479,18634 | SORT1 | BE742268 | Sortilin 1 |
| 212813_at | 0,00164 | 1,0291963 | 436,27036 | 0,7281337 | 334,1091 | JAM3 | AA149644 | Junctional adhesion molecule 3 |
| 212815_at | 0,0098 | 0,9724228 | 503,65555 | 0,7611544 | 395,50906 | ASCC3 | AA156961 | DJ467N11.1 protein |
| 212827_at | 0,000146 | 0,9261723 | 5490,2817 | 0,56684107 | 3638,0728 | IGHM | X17115 | Immunoglobulin heavy constant mu |
| 212831_at | 8,90E-15 | 0,9526956 | 219,54074 | 1,8839686 | 423,65906 | EGFL5 | BF110421 | EGF-like-domain, multiple 5 |
| 212843_at | 7,81E-05 | 1,045128 | 309,84442 | 0,60256374 | 184,16364 | NCAM1 | AA126505 | Neural cell adhesion molecule 1 |
| 212846_at | 2,29E-08 | 0,98356044 | 1339,3372 | 0,68535453 | 940,6546 | KIAA0179 | D80001 | KIAA0179 |
| 212855_at | 1,79E-05 | 1,0210896 | 223,41483 | 0,5612732 | 131,79999 | KIAA0276 | D87466 | KIAA0276 protein |
| 212859_x_at | 0,00282 | 0,95550287 | 816,6667 | 1,2271444 | 1035,6772 | MT1E | BF217861 | Metallothionein 1 E (functional) |
| 212862_at | 3,16E-07 | 0,9995017 | 544,3185 | 1,377334 | 733,10004 | CDS2 | AL568982 | CDP-diacylglycerol synthase (phosphatidate cytidylyltransferase) 2 |
| 212867_at | 0,00178 | 1,0353245 | 936,91473 | 0,811702 | 740,3817 | NCOA2 | AI040324 | Nuclear receptor coactivator 2 |
| 212872_s_at | 3,41 E-05 | 1,0327958 | 278,47037 | 0,7762935 | 210,68634 | USP49 | AK023092 | Ubiquitin specific protease 49 |
| 212880_at | 3,04E-10 | 1,0160133 | 647,09625 | 0,7499308 | 479,82724 | WDR7 | AB011113 | WD repeat domain 7 |
| 212885_at | 1,26E-10 | 0,918747 | 485,0111 | 0,5222774 | 277,93637 | MPHOSPH1 0 | AL545921 | M-phase phosphoprotein 10 (U3 small nucleolar ribonucleoprotein) |
| 212887_at | 0,00129 | 0,9521389 | 567,5481 | 0,74381363 | 450,44095 | SEC23A | AI753659 | Sec23 homolog A (S. cerevisiae) |
| 212893_at | 8,52E-06 | 0,96080655 | 497,14444 | 0,7045695 | 362,8455 | ZZZ3 | AL080063 | Zinc finger, ZZ domain containing 3 |
| 212896_at | 1,17E-07 | 1,0172517 | 932,37775 | 0,7494114 | 680 | KIAA0052 | D29641 | KIAA0052 |
| 212898_at | 0,000317 | 0,92509365 | 420,54443 | 0,6071024 | 283,79544 | KIAA0406 | AB007866 | KIAA0406 gene product |
| 212900_at | 0,000348 | 1,028399 | 469,17776 | 0,7539185 | 345,0955 | SEC24A | BE645231 | SEC24 related gene family, member A (S. cerevisiae) |
| 212905_at | 0,000119 | 0,98582304 | 351,66666 | 0,6764546 | 234,53636 | CSTF2T | BF732638 | Cleavage stimulation factor, 3' pre-RNA, subunit 2, 64kDa, tau variant |
| 212911_at | 2,87E-05 | 0,97943777 | 162,92592 | 0,73288137 | 121,581825 | KIAA0962 | AB023179 | KIAA0962 protein |
| 212914_at | 0,00109 | 0,9509794 | 1064,2963 | 0,7429318 | 827,4091 | CBX7 | AV648364 | Chromobox homolog 7 |
| 212917_x_at | 4,35E-07 | 1,1234584 | 786,5148 | 0,63304925 IMAGE:4094588 | 447,18182 | FLJ22028 | BF219234 | 601882083F1 NIH_MGC_57 Homo sapiens cDNA clone 5', mRNA sequence. |
| 212918_at | 2,17E-1 | 0,9982272 | 933,3926 | 0,5013597 | 475,05908 | FLJ22028 | BF219234 | 601882083F1 NIH_MGC_57 Homo sapiens cDNA clone IMAGE:4094588 5', mRNA sequence. |
| 212919_at | 3,22E-05 | 0,99073476 | 1392,6295 | 0,7721397 | 1074,9501 | DCP2 | AV715578 | DCP2 decapping enzyme homolog (S. cerevisiae) |
| 212920_at | 1,40E-05 | 1,0148182 | 415,39258 | 0,7007325 Rnpc2 | 285,85907 | | AV682285 | Transcribed locus, weakly similar to XP_514612.1 similar to protein [Pan troglodytes] |
| 212927_at | 4,05E-05 | 1,0112636 | 347,84445 | 0,65859985 | 224,96362 | SMC5L1 | AB011166 | SMC5 structural maintenance of chromosomes 5-like 1 |
| 212930_at | 0,000153 | 1,0487802 | 253,26671 | 0,7074419 | 172,39998 | ATP2B1 | AW576457 | ATPase, Ca++transporting, plasma membrane 1 |
| 212943_at | 4,63E-09 | 1,0239887 | 1182,7778 | 0,5590012 | 657,3227 | KIAA0528 | AB011100 | KIAA0528 gene product |
| 212944_at | 8,68E-05 | 0,95921236 | 703,2704 | 0,5861465 | 435,40454 | MRPS6 | AK024896 | Mitochondrial ribosomal protein S6 |
| 212945_s_at | 2,07E-08 | 0,94094294 | 252,33334 | 0,43632987 | 128,4909 | MGA | BE502432 | MAX gene associated |
| 212948_at | 0,00224 | 1,0041696 | 515,02594 | 1,303901 | 680,7454 | CAMTA2 | AB020716 | Calmodulin binding transcription activator 2 |
| 212954_at | 0,000119 | 0,9979441 | 347,8259 | 0,58442235 | 230,61366 | DYRK4 | AF263541 | Dual-specificity tyrosine-(Y)-phosphorylation regulated |
| 212956_at | 6,30E-05 | 0,93058264 | 1233,3074 | 0,60437524 | 779,05 | KIAA0882 | AB020689 | KIAA0882 protein |
| 212959_s_at | 3,22E-05 | 1,0487839 | 1443,7777 | 0,759373 | 1027,9772 | MGC4170 | AK001821 | MGC4170 protein |
| 212966_at | 0,00192 | 0,9690242 | 248,84071 | 1,2463093 | 329,36362 | HIC2 | AB028943 | Hypermethylated in cancer 2 |
| 212974_at | 5,06E-05 | 1,0007315 | 309,1889 | 1,5034142 | 474,35916 | KIAA0870 | AB020677 | KIAA0870 protein |
| 212975_at | 1,05E-08 | 0,9502176 | 617,02966 | 1,4819899 | 957,7727 | KIAA0870 | AB020677 | KIAA0870 protein |
| 212981_s_at | 0,000196 | 1,04122 | 60,688877 | 0,64319676 IMAGE:4344389 | 37,590908 | | BF791738 | 602251923F1 NIH_MGC_84 Homo sapiens cDNA clone 5', mRNA sequence. |
| 212984_at | 1,59E-05 | 1,0305424 | 356,2852 | 0,63245153 IMAGE:3877146 | 219,80002 | | BE786164 | 601474273F1 NIH_MGC_68 Homo sapiens cDNA clone 5', mRNA sequence. |
| 212996_s_at | 0,000107 | 1,0258923 | 123,57408 | 0,70493144 | 90,55909 | C21orf108 | AF231919 | Chromosome 21 open reading frame 108 |
| 213000_at | 1,46E-06 | 0,9758886 | 556,9925 | 0,6102778 | 363,21365 | ZCWCC3; NXP2; ZCW5; KIAA0136 | AP000693 | Homo sapiens genomic DNA, chromosome 21q22.2, BAC clone:KB739011, CBR1-HLCS region. |
| 213002_at | 0,00292 | 0,9272051 | 373,4408 | 1,3843958 | 542,7319 | MARCKS | BF347326 | 602020601F1 NCl_CGAP_Brn67 Homo sapiens cDNA clone IMAGE:4155937 5', mRNA sequence. |
| 213012_at | 1,26E-06 | 1,0223143 | 130,73334 | 0,6262365 | 83,936356 | NEDD4 | D42055 | Neural precursor cell expressed, developmentally down-regulated 4 |
| 213019_at | 3,82E-05 | 0,99571973 | 535,17035 | 0,73045784 | 399,07727 | RANBP6 | AI123233 | RAN binding protein 6 |
| 213020_at | 0,000502 | 0,9177106 | 207,11852 | 0,62142754 | 138,18637 | GOSR1 | AI741876 | Golgi SNAP receptor complex member 1 |
| 213021_at | 0,00502 | 0,8916597 | 332,18515 | 0,7000336 | 255,4909 | GOSR1 | AI741876 | Golgi SNAP receptor complex member 1 |
| 213024_at | 0,00224 | 0,9762159 | 261,28143 | 0,75202775 | 202,38635 | TMF1 | BF593908 | nab48b03.x1 Soares_NSF_F8_9W_OT_PA_P_S1 Homo sapiens cDNA clone IMAGE:3268948 3', mRNA sequence. |
| 213025_at | 0,00436 | 1,0336548 | 613,4963 | 0,7819224 | 467,65912 | FLJ20274 | AL134904 | DKFZp762M0710_s1 762 (synonym: hmel2) Homo sapiens cDNA clone DKFZp762M0710 3', mRNA sequence. |
| 213027_at | 2,23E-06 | 1,0292541 | 615,78156 | 0,6868749 | 404,22275 | SSA2 | AU146655 | Sjogren syndrome antigen A2 (60kDa, ribonucleoprotein autoantigen SS-A/Ro) |
| 213038_at | 0,000715 | 0,95318955 | 636,9741 | 1,218505 | 790,3681 | FLJ90005 | AL031602 | |
| 213044_at | 5,71E-09 | 1,0147085 | 3048,9148 | 0,73149717 | 2225,909 | ROCK1 | N22548 | Rho-associated, coiled-coil containing protein kinase 1 |
| 213046_at | 0,0014 | 1,0243015 | 839,5407 | 1,3744249 | 1114,991 | PABPN1 | AI130920 | Poly(A) binding protein, nuclear 1 |
| 213064_at | 3,61E-05 | 1,0763015 | 471,19257 | 0,771161 | 335,20456 | EML5 | N64802 | Echinoderm microtubule associated protein like 5 |
| 213070_at | 3,04E-05 | 1,0240353 | 478,19635 | 0,7216217 | 336,69543 | PIK3C2A | AV682436 | Phosphoinositide-3-kinase, class 2, alpha polypeptide |
| 213072_at | 8,52E-06 | 0,93998957 | 154,67776 | 1,3956019 | 227,3682 | CYHR1 | AI928387 | Cysteine and histidine rich 1 |
| 213073_at | 2,02E-05 | 0,9949 | 371,4703 | 0,75493014 | 282,0409 | ZFYVE26 | AB002319 | Zinc finger, FYVE domain containing 26 |
| 213090_s_at | 9,67E-06 | 0,99941987 | 696,6778 | 0,749288 | 529,55005 | TAF4 | AI744029 | TAF4 RNA polymerase II, TATA box binding protein (TBP)-associated factor, 135kDa |
| 213092_x_at | 0,00178 | 1,0807812 | 152,34076 | 0,81287944 | 114,54091 | KIAA0974 | BF240590 | KIAA0974 |
| 213095_x_at | 0,00192 | 1,0225232 | 5684,705 | 1,3085418 | 7270,473 | AIF1 | AF299327 | Allograft inflammatory factor 1 |
| 213102_at | 8,68E-05 | 0,9956705 | 4076,2708 | 0,74246347 | 3146,95 | ACTR3 | Z78330 | ARP3 actin-related protein 3 homolog (yeast) |
| 213117_at | 1,75E-09 | 0,98077327 | 171,23701 | 0,52141964 | 92,30454 | KLHL9 | AW138594 | Kelch-like 9 (Drosophila) |
| 213123_at | 7,92E-10 | 0,9346371 | 377,4 | 0,517478 | 204,94545 | | BE222709 | hu51g06.x1 NCI_CGAP_Brn41 Homo sapiens cDNA clone IMAGE:3173626 3', mRNA sequence. |
| 213127_s_at | 4,44E-06 | 0,9383031 | 444,83337 | 0,6626125 homolog | 319,67728 | MED8 | BG230758 | Mediator of RNA polymerase II transcription, subunit 8 (yeast) |
| 213129_s_at | 1,40E-05 | 1,0295372 | 190,62965 | 0,75243086 | 141,19092 | IQWD1 | BE908931 | Glycine cleavage system protein H (aminomethyl carrier) |
| 213134_x_at | 8,24E-08 | 1,0279604 | 411,0111 | 2,0115662 | 846,1091 | BTG3 | AI765445 | BTG family, member 3 |
| 213138_at | 0,000549 | 0,99831015 | 383,11484 | 1,3196318 | 526,72723 | ARID5A | M62324 | AT rich interactive domain 5A (MRF1-like) |
| 213145_at | 2,28E-07 | 1,061268 | 1019,7151 | 0,7137759 | 674,8591 | FBXL14 | BF001666 | F-box and leucine-rich repeat protein 14 |
| 213146_at | 7,02E-05 | 0,94489884 | 261,6371 | 1,3732034 IMAGE:826781 | 380,36365 | KIAA0346 | AA521267 | aa75f03.s1 NCI_CGAP_GCB1 Homo sapiens cDNA clone 3', mRNAsequence. |
| 213149_at | 0,00085 | 1,0012918 | 190,05556 | 0,7306019 IMAGE:2773371 | 140,88637 | DLAT | AW299740 | xs53f02.x1 NCI_CGAP_Kid11 Homo sapiens cDNA clone 3', mRNA sequence. |
| 213152_s_at | 0,000382 | 1,0311029 | 592,0258 | 0,75184757 | 433,84543 | SRP46 | AI343248 | Splicing factor, arginine/serine-rich, 46kD |
| 213164_at | 5,78E-08 | 0,99646676 | 764,3629 | 0,6095916 | 467,1091 | MRPS6 | AI867198 | Mitochondrial ribosomal protein S6 |
| 213165_at | 4,35E-07 | 1,022479 | 995,14813 | 0,7560035 | 732,5136 | CAP350 | AI041204 | Centrosome-associated protein 350 |
| 213168_at | 1,57E-09 | 0,9560265 | 1258,0186 | 0,6237506 | 817,02264 | SP3 | AU145005 | Sp3 transcription factor |
| 213170_at | 0,000238 | 0,96375066 | 397,19254 | 0,76092565 | 310,71362 | GPX7 | AA406605 | Glutathione peroxidase 7 |
| 213178_s_at | 0,00352 | 1,0079881 | 511,5408 | 1,3074311 | 650,4864 | MAPK8IP3 | AB028989 | Mitogen-activated protein kinase 8 interacting protein 3 |
| 213188_s_at | 1,28E-08 | 1,0217041 | 519,83704 | 0,6194336 | 320,56363 | MINA | AL574514 | MYC induced nuclear antigen |
| 213189_at | 4,35E-07 | 0,97734207 | 316,6148 | 0,6345894 | 212,87274 | MINA | AL574514 | MYC induced nuclear antigen |
| 213203_at | 3,88E-06 | 1,0094187 | 771,9778 | 0,75367767 | 582,04553 | SNAPC5 | AI633709 | Small nuclear RNA activating complex, polypeptide 5, |
| 213227_at | 5,78E-08 | 0,94640446 | 327,67776 | 0,55823976 | 190,9773 | PGRMC2 | BE879873 | Progesterone receptor membrane component 2 |
| 213233_s_at | 0,00152 | 0,92695713 | 664,71484 | 0,73724097 | 527,3273 | KIAA1354 | AA460694 | zx69c04.s1 Soares_total_fetus_Nb2HF8_9w Homo sapiens cDNA clone IMAGE:796710 3', mRNA sequence. |
| 213238_at | 0,000382 | 0,97972035 | 461,62222 | 0,7557553 | 361,44543 | ATP10D | AI478147 | ATPase, Class V, type 10D |
| 213239_at | 0,000656 | 0,9806048 | 212,11113 | 0,69639623 | 157,15907 | C13orf24 | NM_006346 | Chromosome 13 open reading frame 24 |
| 213243_at | 1,09E-06 | 1,0115864 | 568,79266 | 0,77939427 | 436,7409 | COH1 | AI052003 | Cohen syndrome 1 |
| 213246_at | 4,82E-08 | 1,0256628 | 387,9815 | 0,6845375 | 263,27274 | C14orf109 | AI346504 | Chromosome 14 open reading frame 109 |
| 213262_at | 6,26E-10 | 1,0558366 | 274,68146 | 0,61811465 | 160,51817 | SACS | AI932370 | Spastic ataxia of Charlevoix-Saguenay (sacsin) |
| 213274_s_at | 8,68E-05 | 0,9559611 | 1077,0962 | 1,418528 IMAGE:3889595 | 1603,7 | CTSB | BE875786 | 601487244F1 NIH_MGC_69 Homo sapiens cDNA clone 5', mRNA sequence. |
| 213275_x_at | 0,000382 | 0,9643208 | 1861,1111 | 1,3208628 | 2540,6316 | CTSB | BE875786 | 601487244F1 NIH_MGC_69 Homo sapiens cDNA clone IMAGE:3889595 5', mRNA sequence. |
| 213278_at | 3,71E-07 | 0,9988217 | 354,08893 | 0,7454247 | 263,4591 | MTMR9 | AW014788 | Myotubularin related protein 9 |
| 213281_at | 5,06E-06 | 0,9813738 | 204,94444 | 1,8516866 | 384,85907 | JUN | BE327172 | hw08a05.x1 NCI_CGAP_Lu24 Homo sapiens cDNA clone IMAGE:3182288 3' similar to contains element MSR1 repetitive element ;, mRNA sequence. |
| 213291_s_at | 3.61E-05 | 0,95581186 | 829,7408 | 0,70739245 | 617,71356 | UBE3A | AA160522 | Ubiquitin protein ligase E3A (human papilloma virus E6-associated protein, Angelman syndrome) |
| 213297_at | 3,88E-06 | 1,0071743 | 181,79259 | 1,4807706 | 275,8227 | NOLA2 | AW131783 | Nucleolar protein family A, member 2 (H/ACA small nucleolar RNPs) |
| 213300_at | 4,28E-05 | 0,9527694 | 656,0148 | 1,2170879 | 848,8773 | KIAA0404 | AW168132 | KIAA0404 protein |
| 213302_at | 0,00134 | 0,9308516 | 199,44815 | 0,517926 | 127,58636 | PFAS | AL044326 | Phosphoribosylformylglycinamidine synthase (FGAR amidotransferase) |
| 213304_at | 4,53E-05 | 0,9494203 | 209,13704 | 0,6835223 | 149,71362 | KIAA0423 | AI823592 | KIAA0423 |
| 213305_s_at | 0,00152 | 1,0284983 | 794,4666 | 0,7991166 | 615,7636 | PPP2R5C | L42375 | Protein phosphatase 2, regulatory subunit B (B56), gamma isoform |
| 213307_at | 0,00807 | 0,9412623 | 106,848145 | 1,2382792 | 141,79547 | SHANK2 | AB028945 | SH3 and multiple ankyrin repeat domains 2 |
| 213330_s_at | 8,52E-06 | 1,0187513 | 541,263 | 0,7223168 | 381,18634 | STIP1 | BE886580 | Stress-induced-phosphoprotein 1 (Hsp70/Hsp90-organizing protein) |
| 213331_s_at | 0,00171 | 1,0095468 | 213,95555 | 0,7700275 | 161,48183 | NEK1 | AL050385 | NIMA (never in mitosis gene a)-related kinase 1 |
| 213344_s_at | 0,00577 | 0,92204994 | 202,4074 | 1,2160203 | 262,5591 | H2AFX | H51429 | H2A histone family, member X |
| 213357_at | 4,44E-06 | 1,006882 | 704,8926 | 0,7113409 | 507,85455 | GTF2H5 | AV701318 | General transcription factor IIH, polypeptide 5 |
| 213365_at | 9,65E-05 | 0,9730529 | 174,14816 | 0,7489857 | 135,45454 | KIAA1504 | N64622 | yz86f08.s1 Soares_multiple_sclerosis_2NbHMSP Homo sapiens cDNA clone IMAGE:289959 3' similar to PIR:A61209 A61209 hypertension-induced protein S-A- rat ;, mRNA sequence. |
| 213374_x_at | 2,77E-08 | 0,9469508 | 265,24814 | 0,56845665 | 164,85002 | HIBCH | AW000964 | 3-hydroxyisobutyryl-Coenzyme A hydrolase |
| 213386_at | 0,000925 | 0,7625475 | 131,84813 | 1,2016716 | 185,61365 | | AV726900 | MRNA full length insert cDNA clone EUROIMAGE 1585492 |
| 213388_at | 1,46E-06 | 0,896573 | 218,91112 | 0,45275414 | 114,92727 | | H15535 | ym27c01.s1 Soares infant brain 1 NIB Homo sapiens cDNA clone IMAGE:49385 3' similar to contains Alu repetitive element;contains MER37 repetitive element ;, mRNA |
| 213396_s_at | 0,000262 | 0,9627292 | 556,08154 | 1,2246522 | 698,16364 | AKAP10 | AA456929 | A kinase (PRKA) anchor protein 10 |
| 213405_at | 1,43E-10 | 1,0150517 | 593,74084 | 0,63407797 | 374,5409 | RAB22A | N95443 | RAB22A, member RAS oncogene family |
| 213410_at | 0,000238 | 1,0390512 | 535,0629 | 0,7182331 | 356,50906 | C10orf137 | AL050102 | Chromosome 10 open reading frame 137 |
| 213434_at | 8,68E-05 | 0,92497057 | 372,34076 | 1,3249239 | 511,88184 | EPIM | H95263 | Epimorphin |
| 213445_at | 0,000131 | 0,9560443 | 277,47037 | 1,3184187 | 383,13638 | ZC3HDC3 | D63484 | Zinc finger CCCH type domain containing 3 |
| 213460_x_at | 0,00755 | 0,8596764 | 1208,0002 | 0,680436 | 895,5681 | WBSCR20C | N29665 | Williams Beuren syndrome chromosome region 20C |
| 213471_at | 0,00861 | 0,9283411 | 172,64816 | 1,2031192 | 214,0318 | NPHP4 | AB014573 | Nephronophthisis 4 |
| 213483_at | 3,71 E-07 | 1,0579218 | 594,7148 | 0,70979685 | 390,89087 | KIAA0073 | AK025679 | KIAA0073 protein |
| 213494_s_at | 5,78E-08 | 0,94180125 | 229,65926 | 1,470428 | 357,43182 | YY1 | AA748649 | YY1 transcription factor |
| 213502_x_at | 1,24E-05 | 0,989173 | 2057,2258 | 0,58787936 (IgL-C16.1). | 1328,241 | IGLL3; 16.1 | X03529 | Human germline gene 16.1 for Ig lambda L-chain C region |
| 213517_at | 5,06E-05 | 1,0783137 | 342 | 1,5775989 | 494,3273 | PCBP2 | AW103422 | Poly(rC) binding protein 2 |
| 213524_s_at | 1,28E-08 | 1,0510328 | 549,4815 | 4,5385246 | 2068,4182 | G0S2 | NM_015714 | Putative lymphocyte G0/G1 switch gene |
| 213526_s_at | 0,00242 | 1,0460292 | 240,50743 | 0,81298894 | 190,34546 | F25965 | BF215644 | Protein F25965 |
| 213528_at | 3,75E-09 | 1,001661 | 354,01852 | 0,55875105 | 195,82727 | MGC9084 | AL035369 | Hypothetical protein MGC9084 |
| 213530_at | 7,80E-05 | 0,9798618 | 200,67407 | 0,6345776 | 134,04091 | RAB3GAP | AI040009 | RAB3 GTPase-activating protein |
| 213537_at | 0,00436 | 0,8444606 | 1019,60736 | 1,419278 | 1651,6091 | HLA-DPA1 | AI128225 | Major histocompatibility complex, class II, DP alpha 1 |
| 213538_at | 1,96E-09 | 1,0203385 | 1078,737 | 1,6247785 | 1732,2229 | SON | AI936458 | SON DNA binding protein |
| 213554_s_at | 0,000549 | 1,0340025 | 627,5631 | 1,3493855 | 828,48645 | H41 | BG257762 | Hypothetical protein H41 |
| 213593_s_at | 0,00807 | 0,98636734 | 469,61115 | 1,6025198 | 737,62726 | TRA2A | AW978896 | Transformer-2 alpha |
| 213596_at | 0,00304 | 0,90500814 | 251,73705 | 0,6877557 | 180,05453 | CASP4 | AL050391 | Caspase 4, apoptosis-related cysteine protease |
| 213598_at | 0,000382 | 1,0552104 | 876,6666 | 0,8176141 | 667,19995 | HSA9761 | W87688 | Putative dimethyladenosine transferase |
| 213606_s_at | 0,00807 | 1,1635255 | 190,90002 | 1,5860212 | 316,27728 | ARHGDIA | AI571798 | Rho GDP dissociation inhibitor (GDI) alpha |
| 213620_s_at | 8,06E-07 | 1,0464741 | 3595,3188 | 0,674587 | 2344,2683 | ICAM2 | AA126728 | Intercellular adhesion molecule 2 |
| 213626_at | 5,71 E-09 | 1,0155058 | 403,80365 | 0,6159872 | 243,79547 | CBR4 | AL049442 | Carbonic reductase 4 |
| 213629_x_at | 7,81 E-05 | 0,9864987 | 803,4518 | 1,240981 | 1012,8546 | MT1F | BF246115 | Metallothionein 1F (functional) |
| 213639_s_at | 2,87E-05 | 0,9640627 | 295,57776 | 0,66182506 | 207,71362 | ZNF500 | AB011129 | Zinc finger protein 500 |
| 213659_at | 5,09E-07 | 0,98334754 | 211,1963 | 0,6753969 | 145,47275 | ZNF75 | AA209420 | Zinc finger protein 75 (D8C6) |
| 213666_at | 0,000715 | 0,96018654 | 685,7741 | 1,2301786 | 878,33185 | 06.Sep | AK026589 | Septin 6 |
| 213677_s_at | 0,000131 | 0,9627871 | 672,5 | 0,75349873 | 523,84985 | PMS1 | BG434893 | PMS1 postmeiotic segregation increased 1 (S. cerevisiae) |
| 213686_at | 0,000502 | 0,97485983 | 241,11856 | 1,2519093 | 298,80908 | | AI186145 | Clone 24583 mRNA sequence |
| 213689_x_at | 5,78E-08 | 0,9914711 | 394,11108 | 0,62026316 | 243,85909 | LOC388650 | AL137958 | Hypothetical LOC388650 |
| 213694_at | 4,99E-11 | 1,048758 | 533,2185 | 0,50132734 | 253,77274 | RSBN1 | AW027347 | Round spermatid basic protein 1 |
| 213701_at | 3,75E-09 | 0,90065455 | 265,37408 | 0,34253052 | 110,29545 | Cep290 | AW299245 | Centrosome protein cep290 |
| 213716_s_at | 7,50E-06 | 1,065754 | 1134,985 | 1,9509507 | 2203,0322 | SECTM1 | BF939675 | Secreted and transmembrane 1 |
| 213737_x_at | 0,00468 | 1,0308632 | 2568,7185 | 0,8126861 | 2095,5999 | | AI620911 | Transcribed locus |
| 213743_at | 2,57E-06 | 0,9850183 | 370,47034 | 0,68288887 | 256,07275 | CCNT2 | BE674119 | Cyclin T2 |
| 213748_at | 0,00755 | 0,9302945 | 231,36667 | 1,2446634 | 297,3091 | TRIM66 | AW271713 | Tripartite motif-containing 66 |
| 213750_at | 8,52E-06 | 1,0058253 | 573,19995 | 0,7532689 | 430,90912 | | AA928506 | Full length insert cDNA YH77E09 |
| 213754_s_at | 2,02E-05 | 0,9994954 | 252,45555 | 0,72587436 | 177,99547 | PAIP1 | AW613203 | Poly(A) binding protein interacting protein 1 |
| 213761_at | 5,07E-06 | 0,95138174 | 428,11484 | 0,66778076 | 300,05002 | MDM1 | AW664850 | Mdm4, transformed 3T3 cell double minute 1, p53 binding protein (mouse) |
| 213773_x_at | 2,69E-07 | 0,9741166 | 1944,9075 | 0,7388642 | 1468,6637 | NSUN5 | AW248552 | NOL1/NOP2/Sun domain family, member 5 |
| 213786_at | 9,64E-05 | 1,0649607 | 188,64073 | 0,71008927 | 131,73636 | TAX1BP1 | AI935415 | Tax1 (human T-cell leukemia virus type I) binding protein 1 |
| 213804_at | 0,000656 | 0,9571783 | 294,51852 | 0,7618036 | 233,95457 | INPP5B | AI039084 | ox26h07.s1 Soares_tota_fetus_Nb2HF8_9w Homo sapiens cDNA clone IMAGE:1657501 3 similar to gb:M74161 75 KD INOSITOL-1,4,5-TRISPHOSPHATE 5-PHOSPHATASE PRECURSOR (HUMAN);, mRNA sequence. |
| 213811_x_at | 0,00352 | 0,99593705 | 891,6666 | 0,7912988 IMAGE:4525138 | 733,06366 | TCF3 | BG393795 | 602416645F2 NIH_MGC_92 Homo sapiens cDNA clone 5', mRNA sequence. |
| 213812_s_at | 0,00282 | 1,0056083 | 1608,1852 | 1,5289357 | 2432,0952 | CAMKK2 | AK024748 | Calcium/calmodulin-dependent protein kinase kinase 2, |
| 213822_s_at | 6,29E-05 | 1,0143613 | 249,12221 | 1,3629183 | 340,43185 | UBE3B | BE856776 | Ubiquitin protein ligase E3B |
| 213830_at | 0,00178 | 0,9912202 | 1655,0554 | 0,5269071 | 968,2682 | TRD@ | AW007751 | T-cell receptor rearranged delta-chain mRNA V-region (V-delta 3-J) |
| 213838_at | 0,000886 | 0,9625823 | 517,2963 | 0,71453714 | 375,88184 | RANBP9 | AA191426 | RAN binding protein 9 |
| 213848_at | 6,91 E-08 | 0,95597774 | 844,98517 | 0,67664593 | 592,1545 | DUSP7 | AI655015 | Dual specificity phosphatase 7 |
| 213859_x_at | 1,40E-05 | 0,9562733 | 158,1148 | 0,6055649 | 100,70454 | SMARCA5 | AI652586 | SWI/SNF related, matrix associated, actin dependent regulator of chromatin, subfamily a, member 5 |
| 213878_at | 7,02E-05 | 0,9796219 | 772,8777 | 0,77722836 | 612,4228 | RECQL | AI685944 | RecQ protein-like (DNA helicase Q1-like) |
| 213883_s_at | 0,0098 | 1,010958 | 733,0074 | 0,7845769 | 570,5091 | BBP | AA012917 | Beta-amyloid binding protein precursor |
| 213885_at | 0,00078 | 0,96265036 | 175,16295 | 1,2198501 | 221,43637 | TRIM3 | AA114843 | Tripartite motif-containing 3 |
| 213888_s_at | 8,06E-07 | 1,0615346 | 3897,9224 | 0,7818152 | 2845,309 | ADORA2BP | AL022398 | |
| 213891_s_at | 1,79E-05 | 1,0779516 | 439,93332 | 0,72923064 | 295,17273 | TCF4 | AI927067 | Transcription factor 4 |
| 213902_at | 9,67E-06 | 0,9817202 | 1142,4075 | 1,3559321 | 1582,4137 | ASAH1 | AI379338 | N-acylsphingosine amidohydrolase (acid ceramidase) 1 |
| 213986_s_at | 0,00707 | 1,10979 | 307,42963 | 1,9566058 | 527,9455 | C19orf6 | AI805266 | Chromosome 19 open reading frame 6 |
| 213988_s_at | 9,36E-07 | 1,0139408 | 2944,5295 | 1,6536024 | 4765,6367 | SAT | BE971383 | Spermidine/spermine N1-acetyltransferase |
| 214002_at | 1,09E-06 | 0,94067925 | 228,72223 | 1,475975 | 357,70456 | MYL6 | AA419227 | Myosin, light polypeptide 6, alkali, smooth muscle and non-muscle |
| 214047_s_at | 5,06E-05 | 0,96826327 | 915,8445 | 0,7626382 | 723,6454 | MBD4 | AI913365 | Methyl-CpG binding domain protein 4 |
| 214048_at | 8,68E-05 | 0,95316523 | 179,58887 | 0,5898581 | 114,736374 | MBD4 | AI913365 | Methyl-CpG binding domain protein 4 |
| 214052_x_at | 6,92E-07 | 1,0087472 | 155,93333 | 1,8119265 | 280,5273 | XTP2 | AW238632 | HBxAg transactivated protein 2 |
| 214057_at | 0,00707 | 0,9592379 | 287,42593 | 1,2392334 | 381,05457 | MCL1 | H71805 | Myeloid cell leukemia sequence 1 (BCL2-related) |
| 214060_at | 0,000216 | 0,96513474 | 363,54074 | 1,4546733 | 540,04095 | SSBP1 | BE220360 | Single-stranded DNA binding protein 1 |
| 214061_at | 1,03E-05 | 0,9511735 | 260,50742 | 0,6830317 | 187,29092 | MGC21654 | AI017564 | Unknown MGC21654 product |
| 214084_x_at | 9,65E-05 | 0,94705635 | 2372,0781 | 1,4669192 | 3820,186 | GTF2IP1 | AW072388 | General transcription factor II, i, pseudogene 1 |
| 214086_s_at | 9,81 E-08 | 1,0634019 | 153,11479 | 0,73938257 | 106,87728 | PARP2 | AK001980 | Poly (ADP-ribose) polymerase family, member 2 |
| 214107_x_at | 1,10E-05 | 0,99804944 | 536,9853 | 1,457508 | 780,48175 | FLJ11822 | AW340850 | Hypothetical protein FLJ11822 |
| 214113_s_at | 5,09E-07 | 1,0012974 | 857,56665 | 0,70595914 | 608,08185 | RBM8A | AI738479 | RNA binding motif protein 8A |
| 214118_x_at | 1,28E-08 | 0,99590427 | 792,73334 | 0,6410379 | 528,6683 | PCM1 | AI205598 | Pericentriolar material 1 |
| 0214129_at | 3,74E-14 | 1,0149299 | 440,14444 | 0,5854105 | 256,11816 | PDE4DIP | AI821791 | Phosphodiesterase 4D interacting protein (myomegalin) |
| 214130_s_at | 4,94E-10 | 1,0206536 | 104,11481 | 0,43654326 | 49,813637 | PDE4DIP | AI821791 | Phosphodiesterase 4D interacting protein (myomegalin) |
| 214157_at | 1,17E-07 | 1,0186731 | 163,58519 | 1,4492905 | 233,12274 | GNAS | AA401492 | GNAS complex locus |
| 214167_s_at | 1,59E-05 | 1,0941476 | 9064,938 | 0,8114753 | 6521,0864 | RPLPO | AA555113 | Ribosomal protein, large, P0 |
| 214179_s_at | 3,04E-10 | 1,0285573 | 476,67776 | 0,5781304 | 270,61362 | NFE2L1 | H93013 | Nuclear factor (erythroid-derived 2)-like 1 |
| 214185_at | 0,00271 | 0,90956074 | 157,31111 | 1,2112147 | 207,70453 | KHDRBS1 | AW592227 | KH domain containing, RNA binding, signal transduction associated 1 |
| 214193_s_at | 8,23E-08 | 0,88898677 | 99,95555 | 0,32457617 | 39,745453 | MGC29875 | AI770084 | Hypothetical protein MGC29875 |
| 214196_s_at | 0,00707 | 0,89034975 | 1105,163 | 1,2809106 | 1571,1863 | TPP1 | AA602532 | Tripeptidyl peptidase I |
| 214214_s_at | 1,59E-05 | 0,9850988 | 1041,3777 | 0,7246292 | 766,5045 | C1QBP | AU151801 | Complement component 1, q subcomponent binding protein |
| 214230_at | 6,24E-10 | 1,0526257 | 138,27777 | 1,8911138 LOC460146 | 245,6909 | CDC42 | R37664 | Transcribed locus, strongly similar to XP_530582.1 [Pan troglodytes] |
| 214259_s_at | 0,000348 | 0,99742335 | 975,97034 | 0,7892464 | 783,53186 | AKR7A2 | AW074911 | Aldo-keto reductase family 7, member A2 (aflatoxin aldehyde reductase) |
| 214281_s_at | 6,26E-10 | 0,9563463 | 654,32965 | 0,6489512 | 443,0818 | RCHY1 | AA524525 | Ring finger and CHY zinc finger domain containing 1 |
| 214291_at | 0,00919 | 0,9373777 | 496,84448 | 1,1789477 | 599,6455 | RPL17 | AA522618 | Ribosomal protein L17 |
| 214327_x_at | 0,00919 | 0,9540225 | 20206,588 | 0,7016934 | 14558,285 | TPT1 | AI888178 | Tumor protein, translationally-controlled 1 |
| 214335_at | 2,57E-06 | 0,910945 | 314,69626 | 1,2400827 | 424,5136 | RPL18 | AI669349 | Ribosomal protein L18 |
| 214366_s_at | 0,00919 | 0,93338794 | 674,13336 | 1,1933242 | 857,1863 | ALOX5 | AA995910 | Arachidonate 5-lipoxygenase |
| 214395_x_at | 0,000262 | 1,0533392 | 187,43704 | 1,504022 | 274,8091 | EEF1D | AI335509 | Eukaryotic translation elongation factor 1 delta (guanine nucleotide exchange protein) |
| 214421_x_at | 0,00304 | 0,9829765 | 135,00372 | 1,3547398 | 186,25908 | CYP2C9 | AV652420 | Cytochrome P450, family 2, subfamily C, polypeptide 9 |
| 214437_s_at | 0,00233 | 1,08784 | 344,84818 | 0,84535223 | 259,53638 | SHMT2 | NM_005412 | Serine hydroxymethyltransferase 2 (mitochondrial) |
| 214438_at | 3,88E-10 | 0,9463947 | 349,25925 | 1,6727349 | 622,07275 | HLX1 | M60721 | H2.0-like homeo box 1 (Drosophila) |
| 214440_at | 1,38E-07 | 0,928104 | 413,9407 | 0,53279376 | 228,66818 | NAT1 | NM_000662 | N-acetyltransferase 1 (arylamine N-acetyltransferase) |
| 214449_s_at | 0,00406 | 0,9675388 | 412,9445 | 1,2873758 | 577,0818 | RHOQ | NM_012249 | Ras homolog gene family, member Q |
| 214467_at | 1,94E-06 | 1,0225202 | 1503,4332 | 0,72565687 | 1075,4501 | GPR65 | NM_003608 | G protein-coupled receptor 65 |
| 214470_at | 0,00119 | 0,97644365 | 5062,9404 | 0,6572171 | 3434,2275 | KLRB1 | NM_002258 | Killer cell lectin-like receptor subfamily B, member 1 |
| 214496_x_at | 0,0014 | 0,95586026 | 334,78888 | 1,2221016 | 420,13635 | MYST4 | NM_012330 | MYST histone acetyltransferase (monocytic leukemia) 4 |
| 214501_s_at | 1,56E-08 | 0,9628967 | 2343,7852 | 1,326558 | 3198,7544 | H2AFY | AF044286 | H2A histone family, member Y |
| 214511_x_at | 7.01E-09 | 0,99116784 | 672,6 | 2,1655784 | 1567,2682 | LOC440607 | L03419 | Fc-gamma receptor I B2 |
| 214519_s_at | 3.61E-05 | 0,9787891 | 146,28517 | 0,7192368 | 107,3409 | RLN2 | NM_005059 | Relaxin 2 |
| 214567_s_at | 4,78E-05 | 0,9530352 | 603,60004 | 0,5084415 | 342,5318 | XCL2 | NM_003175 | Chemokine (C motif) ligand 2 |
| 214582_at | 9,67E-06 | 0,93533677 | 657,2185 | 0,6279252 | 440,95908 | PDE3B | NM_000753 | synonym: cGIPDE1; cyclic nucleotide phosphodiesterase; go_component: membrane [goid 0016020] [evidence IEA]; go_function: hydrolase activity [goid 0016787] [evidence IEA]; go_function: cGMP-inhibited cyclic-nucleotide phosphodiesterase activity [goid 0004 |
| 214617_at | 3,22E-05 | 1,0526886 | 11520,774 | 0,5882506 IMAGE:2140948 | 6979,2544 | PRF1 | AI445650 | tj08g03.x1 NCI_CGAP_Gas4 Homo sapiens cDNA clone 3' similar to contains MER30.t3 MER30 repetitive element ;, mRNA sequence. |
| 214662_at | 4,04E-05 | 0,9952242 | 218,82224 | 0,73040295 | 161,32274 | WDR43 | D26488 | WD repeat domain 43 |
| 214669_x_at | 7,50E-06 | 0,9705455 | 2949,4036 | 0,49041447 | 1899,9272 | IGKC | BG485135 | Anti-rabies virus immunoglobulin rearranged kappa chain V-region |
| 214670_at | 3,03E-09 | 0,97951186 | 590,4001 | 0,59998953 | 364,39548 | ZNF36 | AA653300 | ag65c10.s1 Gessler Wilms tumor Homo sapiens cDNA clone IMAGE:1127826 3' similar to contains Alu repetitive element;, mRNA sequence. |
| 214677_x_at | 4,53E-05 | 1,0062306 | 9875,494 | 0,46147978 | 5883,8677 | IGLC2 | X57812 | Immunoglobulin lambda constant 2 (Kern-Oz- marker) |
| 214686_at | 0,00327 | 0,9642991 | 1376,485 | 0,70070696 | 1016,16376 | ZNF266 | AA868898 | MRNA; cDNA DKFZp686N1450 (from clone |
| 214690_at | 0,0073 | 0,9876994 | 81,674065 | 0,58198565 | 55,16364 | TAF1B | AA004579 | TATA box binding protein (TBP)-associated factor, RNA polymerase I, B, 63kDa |
| 214696_at | 4,94E-12 | 1,010311 | 643,11115 DKFZp6860 | 2,8645132 | 1719,8136 | MGC14376; 06159 | AF070569 | Homo sapiens clone 24659 m RNA sequence. |
| 214714_at | 0,000161 | 1,0298222 | 1107,5592 | 1,4724731 | 1583,0546 | ZNF394 | AK022360 | Zinc finger protein 394 |
| 214721_x_at | 0,00468 | 1,0196598 | 188,73334 | 1,2876304 | 235,53635 | CDC42EP4 | AL162074 | CDC42 effector protein (Rho GTPase binding) 4 |
| 214731_at | 0,00109 | 1,0153439 | 272,9518 023 | 1,2753426 | 340,31818 | DKFZp547A | AB037854 | Hypothetical protein DKFZp547A023 |
| 214735_at | 4,02E-08 | 0,98375964 | 762,77045 | 0,56930655 | 441,57275 | PIP3-E | AW166711 | Phosphoinositide-binding protein PIP3-E |
| 214739_at | 5,07E-06 | 0,9692309 | 398,77777 | 0,6805908 | 284,6773 | LRCH3 | AI357539 | Leucine-rich repeats and calponin homology (CH) domain containing 3 |
| 214746_s_at | 2,57E-06 | 0,9358321 | 960,49634 | 1,3801876 | 1401,7773 | ZNF467 | BE549732 | Zinc finger protein 467 |
| 214751_at | 3,75E-09 | 1,0554068 | 171,92224 | 0,52900475 | 84,59546 | | BE541042 | 601064390F1 NIH_MGC_10 Homo sapiens cDNA clone IMAGE:3450599 5', mRNA sequence. |
| 214766_s_at | 0,000238 | 0,97717696 | 207,66296 | 0,7015078 | 149,53639 | ELYS | AL080144 | ELYS transcription factor-like protein TMBS62 |
| 214768_x_at | 0,00661 | 0,98825425 | 465,96667 | 0,7120667 | 329,78638 | IGKC | BG540628 | HRV Fab N8-VL |
| 214773_x_at | 0,00327 | 0,91652274 | 332,69626 | 0,680816 | 266,64093 | MGC3794 | AI983505 | Putative MAPK activating protein |
| 214787_at | 8,24E-08 | 1,0877703 | 138,92221 | 0,55965126 | 70,13636 | MYCPBP | BE268538 | C-myc promoter binding protein |
| 214805_at | 2,69E-07 | 1,0256273 | 1959,3113 | 1,8717942 | 3402,5408 | EIF4A1 | U79273 | Eukaryotic translation initiation factor 4A, isoform 1 |
| 214807_at | 0,0014 | 0,9938254 | 239,82964 | 1,4409401 DKFZp564O0862) | 341,6909 | | AI278204 | MRNA; cDNA DKFZp564O0862 (from clone |
| 214833_at | 0,00861 | 1,0345955 | 469,32227 | 0,78183067 | 346,43634 | KIAA0792 | AB007958 | KIAA0792 gene product |
| 214836_x_at | 4,44E-06 | 1,0482535 | 1661,7258 | 0,61028546 | 1019,04095 | IGKC | BG536224 | HRV Fab N8-VL |
| 214875_x_at | 0,000161 | 1,0363626 | 1868,0815 | 1,6398281 | 2995,1091 | APLP2 | AW001847 | Amyloid beta (A4) precursor-like protein 2 |
| 214881_s_at | 0,00178 | 0,9665703 | 231,93704 | 0,7395723 | 181,00455 | UBTF | X56687 | Upstream binding transcription factor, RNA polymerase I |
| 214924_s_at | 1,68E-06 | 1,0327363 | 775,1963 | 1,3152007 | 992,3318 | OIP106 | AK000754 | OGT(O-Glc-NAc transferase)-interacting protein 106 KDa |
| 214937_x_at | 4,99E-11 | 0,9836768 | 926,91846 | 0,5583698 | 526,55457 | PCM1 | AI924817 | Pericentriolar material 1 |
| 214943_s_at | 2,87E-05 | 1,0838441 | 115,30742 | 0,47878012 | 57,399998 | KIAA0117 | D38491 | KIAA0117 protein |
| 214948_s_at | 6,58E-06 | 0,96951 | 753,18884 | 0,7491129 | 589,4135 | | AL050136 | Similar to family with sequence similarity 9, member C |
| 214988_s_at | 1,72E-13 | 0,9785854 | 3505,9148 | 0,5711971 | 2067,2637 | SON | X63071 | SON DNA binding protein |
| 214995_s_at | 0,00452 | 0,9152152 | 636,4185 | 0,67183214 | 469,6091 | KIAA0907 | BF508948 | KIAA0907 protein |
| 215001_s_at | 0,000238 | 0,97268456 | 4029,619 | 1,2444512 | 5236,009 | GLUL | AL161952 | Glutamate-ammonia ligase (glutamine synthase) |
| 215023_s_at | 0,00706 | 0,9642956 | 194,1074 | 0,75014895 | 154,09999 | PEX1 | AC000064 | |
| 215029_at | 0,000119 | 1,0597421 | 228,25923 | 1,7022306 | 374,39087 | FLJ12666 | AL117451 | Hypothetical protein FLJ12666 |
| 215030_at | 1,46E-06 | 0,99348176 | 189,59628 | 0,6709757 | 130,07729 | GRSF1 | AK023187 | G-rich RNA sequence binding factor 1 |
| 215051_x_at | 0,000348 | 0,9749934 | 8001,3037 | 1,229583 | 10195,068 | AIF1 | BF213829 | Allograft inflammatory factor 1 |
| 215069_at | 1,40E-05 | 1,0017843 | 141,38518 | 0,7063478 | 100,91364 | NMT2 | AK025065 | N-myristoyltransferase 2 |
| 215075_s_at | 0,000119 | 1,0073142 | 899,4371 | 1,3013967 | 1150,7092 | GRB2 | L29511 | Growth factor receptor-bound protein 2 |
| 215082_at | 0,00109 | 0,9195313 | 229,65556 | 1,1497511 | 278,88635 | ELOVL5 | BF973387 | ELOVL family member 5, elongation of long chain fatty acids (FEN1/Elo2, SUR4/Elo3-like, yeast) |
| 215087_at | 1,92E-12 | 0,95661145 | 236,1074 132 | 1,8175579 | 441,41818 | DKFZP434H | AL109730 | Homo sapiens m RNA full length insert cDNA clone EUROIMAGE 68600. |
| 215090_x_at | 3,39E-06 | 0,98144287 | 764,6592 | 1,4394159 | 1108,4683 | FLJ11822 | AK021884 | Hypothetical protein FLJ11822 |
| 215096_s_at | 0,000715 | 1,0118791 | 2244,9407 | 0,7520578 | 1697,4728 | ESD | AU145746 | Esterase D/formylglutathione hydrolase |
| 215111_s_at | 0,00557 | 1,0480876 | 1498,8186 | 0,7536483 | 1140,0544 | TGFB1I4 | AK027071 | Transforming growth factor beta 1 induced transcript 4 |
| 215118_s_at | 0,000459 | 1,0840191 | 703,87775 | 0,5814518 | 406,17273 | MGC27165 | AW519168 | Hypothetical protein MGC27165 |
| 215121_x_at | 0,000715 | 0,98634666 | 9623,081 | 0,48235297 | 6273,741 | IGLC2 | AA680302 | Immunoglobulin lambda constant 2 (Kern-Oz- marker) |
| 215126_at | 0,0014 | 0,8560009 | 114,04815 | 1,3576014 | 179,04092 | | AL109716 | CDNA FLJ42949 fis, clone BRSTN2006583 |
| 215136_s_at | 5,09E-07 | 1,0321904 | 560,06665 | 0,74853736 | 406,02277 | EXOSC8 | AL050353 | Exosome component 8 |
| 215165_x_at | 0,000419 | 0,94752914 | 340,34445 | 0,6938728 | 252,01366 | UMPS | AL080099 | Uridine monophosphate synthetase (orotate phosphoribosyl transferase and orotidine-5'-decarboxylase) |
| 215171_s_at | 0,000216 | 0,9611954 | 989,8555 | 0,76336074 A | 796 | TIMM17A | AK023063 | Translocase of inner mitochondrial membrane 17 homolog (yeast) |
| 215176_x_at | 9,65E-05 | 1,0185648 | 1924,6481 | 0,52285767 | 1201,3545 | IGKC | AW404894 | HRV Fab 027-VL |
| 215178_x_at | 0,00378 | 1,0042922 | 469,66293 | 1,3096478 | 605,9318 | ASAHL | AV724215 | AV724215 HTB Homo sapiens cDNA clone HTBBOD125', mRNA sequence. |
| 215210_s_at | 9,07E-06 | 0,9881501 | 713,25195 | 1,4203297 | 1021,74554 | DLSTP | S72422 | Dihydrolipoamide S-succinyltransferase pseudogene (E2 component of 2-oxo-glutarate complex) |
| 215214_at | 0,000146 | 1,0720766 | 222,12222 | 0,620685 | 140,14091 | IGLC2 | H53689 | Immunoglobulin lambda constant 2 (Kern-Oz- marker) |
| 215223_s_at | 1,43E-10 | 1,0198047 | 1029,4706 | 1,8603048 | 1920,4546 | SOD2 | W46388 | Superoxide dismutase 2, mitochondrial |
| 215232_at | 0,00948 | 0,921242 | 164,94075 | 1,249115 fis, | 203,38637 | KIAA0672 | AK023797 | unnamed protein product; Homo sapiens cDNA FLJ13735 clone PLACE3000155, weakly similar to Homo sapiens mRNA for KIAA0672 protein. |
| 215245_x_at | 0,000459 | 0,9788776 | 1013,389 | 0,70893896 | 751,0273 | FMR1 | AA830884 | Fragile X mental retardation 1 |
| 215284_at | 1,78E-07 | 0,9974243 | 72,75556 | 1,7790264 | 126,186356 | | AF070575 | Homo sapiens clone 24407 mRNA sequence. |
| 215322_at | 4,82E-08 | 1,0271742 | 207,61115 | 1,9391592 | 371,50455 | | AL080190 | MRNA; cDNA DKFZp434A202 (from clone DKFZp434A202) |
| 215375_x_at | 3,61E-05 | 1,0122921 | 287,57407 | 1,5986769 | 438,02728 | | AK023938 | CDNA FLJ13876 fis, clone THYR01001401 |
| 215379_x_at | 0,000262 | 1,0907915 | 2435,1445 | 0,6510944 | 2274,6091 | IGLC2 | AV698647 | Immunoglobulin lambda constant 2 (Kern-Oz- marker) |
| 215399_s_at | 5,78E-06 | 1,0813117 | 1108,5112 | 1,4599053 IMAGE:2263518 | 1491,1909 | OS-9 | AI683900 | tw54e04.x1 NCI_CGAP_Ut1 Homo sapiens cDNA clone 3', mRNA sequence. |
| 215479_at | 0,00436 | 0,8680061 | 180,3963 | 1,3990432 | 291,62726 | SEMA6A | AK000787 | Sema domain, transmembrane domain (TM), and cytoplasmic domain, (semaphorin) 6A |
| 215498_s_at | 7,50E-06 | 0,8564015 | 2073,863 | 1,3633101 | 3102,9863 | MAP2K3 | AA780381 | Mitogen-activated protein kinase kinase 3 |
| 215501_s_at | 3,16E-07 | 0,9708801 | 179,6963 | 1,7741348 | 327,2591 | DUSP10 | AK022513 | Dual specificity phosphatase 10 |
| 215529_x_at | 0,00119 | 1,1146612 | 561,8668 | 0,65563655 | 346,5227 | DIP2 | AI590053 | tr75d05.x1 NCI_CGAP_Pan1 Homo sapiens cDNA clone IMAGE:2224137 3' similar to contains Alu repetitive element;, mRNA sequence. |
| 215596_s_at | 4,34E-07 | 0,9538798 | 632,44073 | 0,62551475 | 416,4318 | ZNF294 | AL163248 | |
| 215602_at | 0,00502 | 0,9964632 | 266,9296 | 0,68510914 | 184,98636 | FGD2 | AK024456 | FYVE, RhoGEF and PH domain containing 2 |
| 215605_at | 4,05E-05 | 0,9564072 | 384,7222 | 1,4051485 | 566,3818 | NCOA2 | AU145806 | Nuclear receptor coactivator 2 |
| 215691_x_at | 0,000549 | 1,0746408 | 1293,263 | 0,82836026 | 1005,2637 | C1orf41 | AV702994 | Chromosome 1 open reading frame 41 |
| 215706_x_at | 3,61 E-05 | 0,96373695 | 2651,015 | 1,4834204 | 4133,737 | ZYX | BC002323 | Zyxin |
| 215716_s_at | 4,44E-06 | 1,0776966 | 610,5443 | 0,67607623 | 382,69092 | ATP2B1 | L14561 | |
| 215718_s_at | 0,000924 | 1,167176 | 153,35925 | 0,8143754 | 102,61364 | PHF3 | AI949220 | wq20e12.x1 NCI_CGAP_Gas4 Homo sapiens cDNA clone IMAGE:2471854 3' similar to TR:Q92576 Q92576 MYELOBLAST KIAA0244 ;, mRNA sequence. |
| 215726_s_at | 6,64E-05 | 1,0003757 | 289,42596 | 0,75604904 | 223,49545 | CYB5; MCB5 | M22976 | Human cytochrome b5 mRNA, 3' end. |
| 215760_s_at | 0,00861 | 1,001656 | 123,64815 | 1,4617412 | 205,04546 | KIAA0963 | AC005390 | |
| 215806_x_at | 0,00129 | 0,96419215 | 3782,263 TCRGC2; | 0,5918464 | 2609,5679 | TRGC2; TRGC2(2X); TRGC2(3X) | M13231 | Similar to T-cell receptor gamma chain V region PT-gamma-1/2 precursor |
| 215854_at | 0,00339 | 0,99986386 | 109,899994 | 1,2603692 HEMBA1006665 3', | 137,48183 | | AU146050 | AU146050 HEMBA1 Homo sapiens cDNA clone mRNA sequence. |
| 215925_s_at | 7,81 E-05 | 0,9357681 | 279,0851 | 0,49758303 | 159,94545 | CD72 | AF283777 | CD72 antigen |
| 215946_x_at | 5,06E-05 | 0,9841579 | 1175,8036 | 0,53852904 | 764,5227 | LOC91316 | AL022324 | |
| 215967_s_at | 9,36E-07 | 1,0054356 | 718,07776 | 0,637413 | 465,22272 | LY9 | AL582804 | Lymphocyte antigen 9 |
| 215983_s_at | 3,39E-06 | 1,0050373 | 242,98149 | 0,72429293 | 179,4182 | D8S2298E | D83768 | Reproduction 8 |
| 216015_s_at | 5,71 E-09 | 0,91706645 | 171,87407 | 2,058952 | 408,47726 | CIAS1 | AK027194 | Cold autoinflammatory syndrome 1 |
| 216036_x_at | 0,00807 | 1,0030863 | 338,73706 | 1,3056636 | 445,11365 | WDTC1 | AK001734 | WD and tetratricopeptide repeats 1 |
| 216041_x_at | 1,59E-05 | 0,98339725 | 5903,711 | 1,4108908 | 8589,641 | GRN | AK023348 | Granulin |
| 216044_x_at | 1,96E-09 | 0,9942059 | 360,61484 | 0,5982763 | 220,55908 | LOC388650 | AK027146 | Hypothetical LOC388650 |
| 216060_s_at | 0,000549 | 1,0035262 | 279,53336 | 0,7409795 | 205,92271 | DAAM1 | AK021890 | Dishevelled associated activator of morphogenesis 1 |
| 216088_s_at | 3,61 E-05 | 1,0647526 | 529,07776 | 0,8371469 | 409,39542 | PSMA7 | AL078633 | |
| 216092_s_at | 0,00806 | 0,9696067 | 168,55556 | 1,2549044 | 209,07726 | SLC7A8 | AL365347 | Solute carrier family 7 (cationic amino acid transporter, y+ system), member 8 |
| 216095_x_at | 2,41E-05 | 1,031074 | 1144,3889 | 0,81832606 | 906,81824 | MTMR1 | AF057354 | Myotubularin related protein 1 |
| 216109_at | 0,00378 | 1,1687138 | 203,52965 | 1,8101804 | 278,7727 | THRAP2 | AK025348 | Thyroid hormone receptor associated protein 2 |
| 216121_at | 0,000348 | 0,72595716 | 63,81852 | 1,3127296 | 100,34545 | | AL080106 | Homo sapiens genomic DNA; cDNA DKFZp5660053 (from clone DKFZp5660053). |
| 216177_at | 1,28E-08 | 0,98663086 | 458,77777 | 0,72374594 | 335,35458 | RPL29 | AW582267 | QVO-ST0215-060100-083-c06 ST0215 Homo sapiens cDNA, mRNA sequence. |
| 216191_s_at | 0,000119 | 0,99770874 | 2215,8108 | 0,44997996 delta | 1090,8137 | TRA@ | X72501 | T-cell receptor rearranged delta-chain m RNA V-region (V-3-J) |
| 216199_s_at | 5,06E-05 | 1,0113894 | 740,75195 | 0,752084 | 528,8501 | MAP3K4 | AL109942 | |
| 216202_s_at | 1,09E-06 | 0,9645774 | 468,53702 | 1,4801098 | 729,08185 | SPTLC2 | U15555 | Serine palmitoyltransferase, long chain base subunit 2 |
| 216203_at | 5,78E-08 | 1,0326643 | 145,80742 | 1,6060009 | 228,09544 | SPTLC2 | U15555 | Serine palmitoyltransferase, long chain base subunit 2 |
| 216221_s_at | 2,48E-13 | 1,0065942 | 2818,026 | 0,6886588 | 1929,891 | PUM2 | D87078 | Vacuolar protein sorting 35 (yeast) |
| 216236_s_at | 1,05E-08 | 0,9468966 | 476,88522 | 2,2922516 | 1259,4092 | SLC2A14 | AL110298 | Solute carrier family 2 (facilitated glucose transporter), member 3 |
| 216243_s_at | 6,30E-05 | 1,0317049 | 382,46664 | 1,7666129 | 621,9728 | IL1RN | BE563442 | Interleukin 1 receptor antagonist |
| 216248_s_at | 7,81 E-05 | 1,1203134 | 301,97778 | 2,8961172 | 677,12274 | NR4A2 | S77154 | Nuclear receptor subfamily 4, group A, member 2 |
| 216260_at | 1,96E-09 | 0,8521213 | 55,892593 | 1,5585644 | 98,65453 | DICER1 | AK001827 | Dicer1, Dcr-1 homolog (Drosophila) |
| 216262_s_at | 5,06E-06 | 0,963439 | 408,76294 | 0,7201232 | 301,54092 | TGIF2 | AL050318 | |
| 216267_s_at | 0,000715 | 1,0077237 | 311,20374 | 0,78480786 | 244,99547 | PL6 | BF034906 | Placental protein 6 |
| 216321_s_at | 3,34E-08 | 0,9752822 | 1036,7593 | 0,6369695 (glucocorticoid | 687,2137 | NR3C1 | X03348 | Nuclear receptor subfamily 3, group C, member 1 receptor) |
| 216379_x_at | 7,81 E-05 | 1,0599711 | 797,5815 | 0,59662473 | 535,6455 | CD24 | AK000168 | Homo sapiens cDNA FLJ20161 fis, clone COL09252, highly similar to L33930 Homo sapiens CD24 signal transducer mRNA. |
| 216388_s_at | 0,00101 | 0,9398761 | 362,85925 | 1,1953529 BLT1; BLTR; | 471,60907 | LTB4R; P2Y7; GPR16; LTBR1; P2RY7; CMKRL1; LTB4R1 | U33448 | R2; Human putative G-protein-coupled receptor (GPR16) gene, complete cds. |
| 216409_at | 0,00406 | 0,9774024 | 198,60739 | 1,2547107 | 249,94093 | ACSL6 | AL390168 | Acyl-CoA synthetase long-chain family member 6 |
| 216484_x_at | 8,68E-05 | 0,9618236 | 1029,9221 | 1,221457 | 1310,6362 | HDGF | L24521 | Hepatoma-derived growth factor (high-mobility group protein 1-like) |
| 216565_x_at | 0,00807 | 1,0304474 | 1225,015 | 1,3084439 | 1615,9591 | | AL121994 | |
| 216570_x_at | 5,06E-05 | 1,00894 | 3807,752 | 0,78775966 | 2941,6047 | | AL096829 | |
| 216593_s_at | 5,65E-05 | 1,0101701 | 514,87787 | 0,71238726 | 369,91364 | PIGCP1 | AB000359 | pseudogene of PIGC; see also AB000360 putative; Homo sapiens PIGCP1 pseudogene. |
| 216602_s_at | 0,000925 | 0,9210709 | 300,76297 | 0,6894734 | 219,42728 | FARSL | AD000092 | |
| 216713_at | 9,65E-05 | 1,0298502 | 233,17035 | 0,56709105 | 132,29544 | CCM1 | AL049325 | Cerebral cavernous malformations 1 |
| 216733_s_at | 0,00661 | 0,8996496 | 108,87407 | 0,60402507 | 79,04092 | GATM | X86401 | Glycine amidinotransferase (L-arginine:glycine amidinotransferase) |
| 216804_s_at | 0,00755 | 0,98357034 | 219,16666 | 0,7047622 | 165,89546 | PDLIM5 | AK027217 | PDZ and LIM domain 5 |
| 216841_s_at | 0,0014 | 0,968647 | 332,22968 | 1,3002061 | 437,90002 | SOD2 | X15132 | Superoxide dismutase 2, mitochondrial |
| 216863_s_at | 0,000238 | 0,9477337 | 269,97775 | 0,70243853 | 195,44545 | KIAA0852 | AC004542 | |
| 216920_s_at | 0,00242 | 1,0030568 | 4020,5369 | 0,61813796 | 2824,1821 | TCRGC2 | M27331 | Similar to T-cell receptor gamma chain V region PT-gamma-1/2 precursor |
| 216944_s_at | 0,00468 | 1,0149624 | 295,72226 | 0,7085615 | 210,12271 | ITPR1 | U23850 | Inositol 1,4,5-triphosphate receptor, type 1 |
| 216950_s_at | 8,24E-08 | 1,0205495 | 677,95184 | 1,9871076 | 1358,2728 | FCGR1A | X14355 | Fc fragment of IgG, high affinity la, receptor for (CD64) |
| 216958_s_at | 0,00078 | 0,9769299 | 221,86667 | 0,73111546 | 169,55908 | IVD | AK022777 | Isovaleryl Coenzyme A dehydrogenase |
| 216984_x_at | 0,000146 | 1,025168 | 841,889 | 0,5098904 | 503,11816 | IGLC2 | D84143 | Immunoglobulin lambda constant 2 (Kern-Oz- marker) |
| 217019_at | 9,65E-05 | 0,9699749 | 162,32964 | 0,595161 | 98,568184 | | AL137162 | |
| 217020_at | 0,00192 | 0,8648112 | 153,45554 | 1,305212 | 211,11818 | RARB; HAP; RRB2; NR1B2 | X04014 | ORF; Homo sapiens DNA for HBV integration sites. |
| 217022_s_at | 0,000849 | 1,0176613 | 8300,245 | 0,24382864 | 4918,7866 | MGC27165 | S55735 | Hypothetical protein MGC27165 |
| 217042_at | 0,00158 | 1,0007732 | 150,27777 | 1,2702433 | 190,73181 | RDH11 | AL096716 | DKFZP564M1462 protein |
| 217043_s_at | 0,00436 | 1,0118062 | 308,0926 | 0,77846295 | 233,69547 | MFN1 | U95822 | Mitofusin 1 |
| 217092_x_at | 0,0014 | 0,95902735 | 2835,633 | 0,7657818 | 2243,2634 | | AL031589 | |
| 217106_x_at | 1,90E-05 | 1,0218182 | 947,7703 | 0,7940521 | 749,07275 | HSA9761 | AF091078 | Putative dimethyladenosine transferase |
| 217117_x_at | 0,000502 | 0,9856966 | 219,2111 | 1,3748243 | 301,24545 | MUC3B | AF007194 | Mucin 3A, intestinal |
| 217142_at | 0,000289 | 0,96367097 | 388,15927 | 0,59052235 | 237,50908 | | AL035687 | |
| 217143_s_at | 4,05E-05 | 1,0747929 | 2640,6333 | 0,54295003 | 1397,7999 | TRD@ | X06557 | T cell receptor delta locus |
| 217147_s_at | 1,11E-10 | 0,9198169 | 704,3741 | 0,4047943 | 316,9409 | TCRIM | AJ240085 | T cell receptor interacting molecule |
| 217164_at | 0,00078 | 1,0628088 | 150,04074 | 0,70832 | 92,38182 | | AK024108 | Homo sapiens cDNA FLJ14046 fis, clone HEMBA1006461. |
| 217165_x_at | 0,00755 | 0,9750395 | 648,7741 MGC32732 | 1,242724 | 848,35004 | MT1F; | M10943 | human metallothionein-If; Human metallothionein-If gene (hMT-If). |
| 217179_x_at | 0,00352 | 0,92833304 | 451,8037 | 0,578116 | 359,58636 | IGL@ | X79782 | Ig rearranged lambda-chain gene V-JI2/I3-region |
| 217192_s_at | 0,00618 | 0,9864514 | 318,2222 | 1,4015205 | 452,44092 | PRDM1 | AL022067 | |
| 217202_s_at | 0,00164 | 0,90519446 | 659,637 | 1,3943824 | 1021,9454 | | U08626 | Homo sapiens glutamine synthetase pseudogene, complete sequence. |
| 217207_s_at | 0,00406 | 0,97882867 | 248,37408 | 1,22981 | 306,96817 | BTNL3 | AK025267 | Butyrophilin-like 3 |
| 217208_s_at | 3,61E-05 | 0,9463579 | 148,10742 SAP97 | 1,364601 | 212,58635 | DLG1; hdlg; | AL121981 | match: proteins: Tr:Q62402 Sw:Q62696 Sw:Q12959; Human DNA sequence from clone RP5-1061C18 on chromosome 3p36.22-36.33 Contains the 5' part of the DLG1 gene encoding the discs protein (large Drosophila homolog 1, presynaptic protein SAP97) and a CpG island |
| 217221_x_at | 0,000161 | 0,9670886 | 625,81854 | 0,75895256 | 482,42728 | RBM10 | AL137421 | RNA binding motif protein 10 |
| 217227_x_at | 0,00378 | 1,0972255 | 284,1741 | 0,84450734 | 249,20454 | IGL@ | X93006 | Hepatitis B surface antigen antibody variable domain |
| 217234_s_at | 0,0078 | 0,8205104 | 832,32965 | 1,2456965 | 1252,4819 | VIL2 | AF199015 | Villin 2 (ezrin) |
| 217266_at | 1,10E-05 | 0,97829044 | 779,75555 | 0,7241206 | 573,3227 | | Z97353 | Human DNA sequence from clone RP1-90L6 on chromosome 22q11.21-11.23, complete sequence. |
| 217293_at | 5,94E-07 | 0,9954139 | 142,08147 | 1,4806055 | 205,01363 | | AF209975 | Homo sapiens tissue-type aorta m RNA sequence. |
| 217297_s_at | 0,00129 | 0,922476 | 560,5926 | 1,2977166 | 806,63184 | MYO9B | AF143684 | Myosin IXB |
| 217313_at | 7,46E-07 | 1,0368915 | 757,8444 | 0,74644923 | 545,5636 | | AC004692 | Homo sapiens PAC clone RP5-1107K12 from 7, complete sequence. |
| 217340_at | 0,00315 | 0,9418202 | 125,40741 | 0,64680964 | 87,32726 | | AL024509 | |
| 217370_x_at | 5,06E-06 | 0,9807983 | 475,85184 | 1,7189963 | 801,5454 | | S75762 | |
| 217379_at | 6,30E-05 | 0,97831565 | 5479,3 | 0,70302963 | 3919,764 | bA209A2.1 | AL121934 | match: proteins: O22431 O61231 O96647 P27635 P41805 P45633 P45634 P45635 P93847 Q08018 Q08200 Q08770 Q09127 Q09533 Q29195 Q39724 Q40649 Q40650 Q9ZVG7; Human DNA sequence from clone RP11-209A2 on chromosome 6 Contains a 60S ribosomal protein L10 (RPL10) ps |
| 217406_at | 0,00085 | 0,96303034 | 160,22224 | 1,3397498 | 233,56819 | | AL021937 | |
| 217408_at | 4,35E-07 | 1,0140785 | 672,9074 | 0,61092967 | 412,76367 | MRPS18B | AL050361 | Mitochondrial ribosomal protein S18B |
| 217412_at | 0,000365 | 0,98071694 | 194,22223 | 0,67667097 | 134,18636 | TRA@ | AE000659 | |
| 217473_x_at | 0,000262 | 0,9419185 | 457,48145 | 1,3791834 | 660,59094 | | AF229163 | |
| 217477_at | 0,00807 | 0,8635823 | 106,13705 | 1,1911613 | 138,25 | PIP5K1B | U78581 | Phosphatidylinositol-4-phosphate 5-kinase, type I, beta |
| 217480_x_at | 0,00755 | 0,97234654 | 896,04816 118; | 0,7454202 | 709,15 | IGKV1OR15- IGKVP2; IGKV1OR11 8 | M20812 | Ig kappa chain; Human kappa-immunoglobulin germline pseudogene (cos118) variable region (subgroup V kappa I). |
| 217499_x_at | 5,06E-05 | 0,92573357 | 481,7296 | 1,2196785 | 625,4955 | OR7E38P | AW874308 | Olfactory receptor, family 7, subfamily E, member 38 pseudogene |
| 217503_at | 1,64E-07 | 1,0283381 | 249,22961 | 1,5544555 | 365,79092 | STK17B | AA203487 | Serine/threonine kinase 17b (apoptosis-inducing) |
| 217508_s_at | 2,57E-06 | 0,95360476 | 150,76666 | 1,3210455 | 211,07726 | C18orf25 | BE783279 | Chromosome 18 open reading frame 25 |
| 217549_at | 0,000227 | 1,0663556 | 1186,0482 | 0,79164517 | 852,1636 | | AW574933 | Transcribed locus, weakly similar to NP_055301.1 neuronal thread protein AD7c-NTP [Homo sapiens] |
| 217604_at | 5,06E-05 | 0,9621723 | 129,37038 | 0,6792227 hypothetical | 93,63183 | | AI086530 | Transcribed locus, weakly similar to XP_510104.1 similar to protein FLJ25224 [Pan troglodytes] |
| 217609_at | 0,00152 | 1,0137349 | 149,88521 | 1,3015457 | 192,1409 | LOC283345 | BG420747 | RPL13-2 pseudogene |
| 217620_s_at | 0,00281 | 0,97225815 | 57,829628 | 1,3223858 | 78,3 | PIK3CB | AA805318 | Phosphoinositide-3-kinase, catalytic, beta polypeptide |
| 217627_at | 1,17E-07 | 0,99644244 | 234,55556 | 0,5481603 | 131,12271 | ZNF573 | BE515346 | Zinc finger protein 573 |
| 217637_at | 0,00242 | 0,8990816 | 130,16296 | 1,2522637 | 177,00912 | | R25692 | Full length insert cDNA clone ZE05A03 |
| 217655_at | 0,00661 | 1,0637617 | 331,25555 | 1,4622884 | 447,59546 | FXYD5 | BE552409 | FXYD domain containing ion transport regulator 5 |
| 217665_at | 3,62E-12 | 0,9983995 | 104,3852 | 0,47790083 | 52,42273 | | AA420614 | Transcribed locus, moderately similar to NP_000991.1 ribosomal protein L39 [Homo sapiens] |
| 217682_at | 0,000161 | 0,9349735 | 240,87407 | 1,2313021 | 306,8909 | PR00149 | AW503390 | PR00149 protein |
| 217722_s_at | 2,56E-05 | 1,0137718 | 907,6703 | 0,7375446 | 662,68176 | NEUGRIN | NM_016645 | Mesenchymal stem cell protein DSC92 |
| 217724_at | 8,24E-08 | 0,9841365 | 3783,4482 | 0,7532625 | 2906,0588 | PAI-RBP1 | AF131807 | PAI-1 mRNA-binding protein |
| 217728_at | 0,000216 | 0,9458565 | 10628,789 | 1,3082652 | 14696,685 | S100A6 | NM_014624 | S100 calcium binding protein A6 (calcyclin) |
| 217738_at | 1,96E-09 | 1,0062182 | 436,77405 | 1,7400141 | 803,65906 | PBEF1 | BF575514 | Pre-B-cell colony enhancing factor 1 |
| 217739_s_at | 7,03E-10 | 1,050485 | 844,48145 | 2,4338503 | 2078,891 | PBEF1 | NM_005746 | Pre-B-cell colony enhancing factor 1 |
| 217741_s_at | 6,92E-07 | 0,9773245 | 2407,8965 | 1,458744 | 3478,0415 | ZA20D2 | AW471220 | Zinc finger, A20 domain containing 2 |
| 217745_s_at | 0,000419 | 0,9971196 | 1057,1332 | 0,6970875 | 717,3409 | MAK3 | NM_025146 | Mak3 homolog (S. cerevisiae) |
| 217748_at | 0,000196 | 1,0374318 | 1413,8 | 1,3928033 | 1847,8638 | ADIPOR1 | NM_015999 | Adiponectin receptor 1 |
| 217764_s_at | 4,53E-05 | 0,98028195 | 2473,1482 | 1,2347339 | 3129,5186 | RAB31 | AF183421 | RAB31, member RAS oncogene family |
| 217775_s_at | 0,000146 | 0,9794919 | 647,4037 | 0,6840889 | 456,41815 | RDH11 | NM_016026 | DKFZP564M1462 protein |
| 217776_at | 2,56E-05 | 0,9778138 | 1070,3701 | 0,6543145 | 725,8409 | RDH11 | AF167438 | DKFZP564M1462 protein |
| 217783_s_at | 7,81E-05 | 1,1493008 | 3867,9336 | 1,5287544 | 5066,65 | YPEL5 | NM_016061 | Yippee-like 5 (Drosophila) |
| 217791_s_at | 0,000715 | 0,98857087 | 380,6889 | 0,7570929 | 296,8455 | ALDH18A1 | NM_002860 | Aldehyde dehydrogenase 18 family, member A1 |
| 217792_at | 6,30E-05 | 1,0136096 | 822,10004 | 0,76192385 | 606,78186 | SNX5 | NM_014426 | Sorting nexin 5 |
| 217796_s at | 0,00807 | 0,9689026 | 916,81854 | 1,2586408 | 1161,6865 | NPL4 | NM_017921 | Nuclear protein localization 4 |
| 217803_at | 0,000161 | 1,0167768 | 2173,9224 | 0,78929484 | 1696,0499 | GOLPH3 | NM_022130 | Golgi phosphoprotein 3 (coat-protein) |
| 217825_s_at | 1,43E-10 | 0,98595965 | 724,5889 | 1,4642017 | 1077,3046 | UBE2J1 | AF151039 | Ubiquitin-conjugating enzyme E2, J1 (UBC6 homolog, |
| 217832_at | 1,64E-07 | 0,98135215 | 1319,4703 | 0,6961887 | 938,14996 | SYNCRIP | NM_006372 | Synaptotagmin binding, cytoplasmic RNA interacting protein |
| 217834_s at | 8,06E-07 | 0,9464556 | 472,74814 | 0,71238637 | 354,74545 | SYNCRIP | NM_006372 | Synaptotagmin binding, cytoplasmic RNA interacting protein |
| 217835_x_at | 9,06E-12 | 1,0097309 | 4914,3115 | 1,3740202 | 6694,923 | C20orf24 | NM_018840 | Chromosome 20 open reading frame 24 |
| 217836_s_at | 0,000289 | 1,0211664 | 1313,5334 | 0,79052216 | 1027,5591 | YAP | NM_018253 | YY1 associated protein |
| 217842_at | 4,34E-07 | 0,9898253 | 272,35553 | 0,6604619 | 185,52272 | LUC7L2 | NM_016019 | LUC7-like 2 (S. cerevisiae) |
| 217873_at | 6,92E-07 | 0,9571733 | 2944,315 | 0,7212315 | 2205,3315 | CAB39 | NM_016289 | Calcium binding protein 39 |
| 217880_at | 0,000382 | 0,9011578 | 232,83331 | 0,58801126 | 150,96364 | CDC27 | AI203880 | Cell division cycle 27 |
| 217897_at | 0,00192 | 0,99057543 | 332,0741 | 1,2994534 | 437,72275 | FXYD6 | NM_022003 | FXYD domain containing ion transport regulator 6 |
| 217902_s_at | 4,04E-05 | 0,96989185 | 357,71112 | 0,7594825 | 279,36365 | HERC2 | NM_004667 | Hect domain and RLD 2 |
| 217903_at | 7,02E-05 | 0,9292682 | 449,9704 | 1,2117126 | 589,7727 | STRN4 | NM_013403 | Striatin, calmodulin binding protein 4 |
| 217907_at | 8,06E-07 | 0,9557917 | 1093,537 | 0,7269014 | 827,11365 | MRPL18 | NM_014161 | Mitochondrial ribosomal protein L18 |
| 217919_s_at | 5,65E-05 | 0,9484669 | 568,389 | 0,63837004 | 386,58636 | MRPL42 | BE782148 | Mitochondrial ribosomal protein L42 |
| 217930_s_at | 0,000119 | 1,0712181 | 331,24072 | 1,3947544 | 431,43634 | TOLLIP | NM_019009 | Toll interacting protein |
| 217933_s_at | 4,44E-06 | 0,97602487 | 2446,6372 | 1,5217562 | 3813,2275 | LAP3 | NM_015907 | Leucine aminopeptidase 3 |
| 217936_at | 0,00292 | 1,0358225 | 206,79257 | 0,8116962 | 163,75 | | AW044631 | wy78c11.x1 Soares_NSF_F8_9W_OT_PA_P_S1 Homo sapiens cDNA clone IMAGE:2554676 3', mRNA sequence. |
| 217941_s_at | 0,00242 | 1,0025761 | 1778,8853 | 0,79187053 | 1413,1271 | ERBB2IP | NM_018695 Erbb2 | interacting protein |
| 217942_at | 0,000419 | 0,9790204 | 1105,2223 | 0,78271884 | 881,0455 | MRPS35 | NM_021821 | Mitochondrial ribosomal protein S35 |
| 217945_at | 0,000145 | 1,0564424 | 1237,785 | 0,8046787 | 916,88635 | BTBD1 | NM_025238 | BTB (POZ) domain containing 1 |
| 217950_at | 0,00707 | 0,99466395 | 2277,837 | 0,72506607 | 1744,5317 | NOSIP | NM_015953 | Nitric oxide synthase interacting protein |
| 217952_x_at | 6,59E-06 | 0,98977727 | 785,04816 | 0,78197145 | 622,359 | PHF3 | AW189430 | PHD finger protein 3 |
| 217954_s_at | 0,000102 | 1,0284367 | 732,50006 | 0,8084661 | 579,44543 | PHF3 | NM_015153 | PHD finger protein 3 |
| 217957_at | 7,92E-10 | 0,99584687 | 659,75183 | 0,70159 | 458,32272 | GTL3 | NM_013242 | Likely ortholog of mouse gene trap locus 3 |
| 217959_s_at | 2,69E-07 | 0,9828341 | 822,73334 | 0,7313415 | 612,84534 | TRAPPC4 | NM_016146 | Trafficking protein particle complex 4 |
| 217963_s_at | 0,00129 | 0,9497207 | 2299,615 | 0,5692195 | 1455,3591 | NGFRAP1 | NM_014380 | Nerve growth factor receptor (TNFRSF16) associated |
| 217971_at | 3,71 E-07 | 0,96635014 | 812,3629 | 0,6748248 | 571,04083 | MAP2K1IP1 | NM_021970 1 | Mitogen-activated protein kinase kinase 1 interacting protein |
| 217976_s_at | 6,29E-05 | 1,0478601 | 516,29626 | 0,758036 | 376,85 | DNCLI1 | NM_016141 | Dynein, cytoplasmic, light intermediate polypeptide 1 |
| 217979_at | 4,52E-05 | 1,0068221 | 600,20013 | 0,62434655 | 376,01367 | TM4SF13 | NM_014399 | Transmembrane 4 superfamily member 13 |
| 217986_s_at | 0,00208 | 0,9974758 | 1127,8185 | 0,779101 | 909,6909 | BAZ1A | NM_013448 | Bromodomain adjacent to zinc finger domain, 1A |
| 217993_s_at | 2,56E-05 | 0,9703936 | 4991,218 | 0,7552807 | 3953,1274 | MAT2B | NM_013283 | Methionine adenosyltransferase II, beta |
| 217995_at | 3,39E-06 | 1,0053736 | 2217,1333 | 1,3232874 | 2937,3455 | SQRDL | NM_021199 | Sulfide quinone reductase-like (yeast) |
| 218003_s_at | 1,03E-05 | 1,0093561 | 953,1444 | 0,73228145 | 703,64545 | FKBP3 | NM_002013 | FK506 binding protein 3, 25kDa |
| 218005_at | 5,09E-07 | 0,99080616 | 1239,0889 | 0,70258677 | 885,3909 | ZNF22 | AA744771 | Zinc finger protein 22 (KOX 15) |
| 218011_at | 0,00327 | 1,0118833 | 1975,1406 | 0,80902344 | 1566,8091 | UBL5 | NM_024292 | Ubiquitin-like 5 |
| 218012_at | 0,00261 | 0,89204645 | 860,5223 | 1,2783773 | 1220,041 | TSPYL2 | NM_022117 | TSPY-like 2 |
| 218019_s_at | 3,88E-06 | 1,0021847 | 737,9371 | 0,73701817 | 537,7137 | C21orf97 | NM_021941 | |
| 218020_s_at | 8,06E-07 | 0,99196357 | 1053,9926 | 1,5517789 | 1634,2 | TEX27 | NM_021943 | Testis expressed sequence 27 |
| 218023_s_at | 8,59E-09 | 0,9998046 | 797,3037 | 1,4229535 | 1144,1998 | FAM53C | NM_016605 | Family with sequence similarity 53, member C |
| 218025_s_at | 7,00E-09 | 0,98096687 | 500,69995 | 0,6702465 | 338,02277 | PECI | NM_006117 | Peroxisomal D3,D2-enoyl-CoA isomerase |
| 218030_at | 0,00707 | 0,9003984 | 415,8148 | 1,18217 | 561,2454 | GIT1 | NM_014030 | G protein-coupled receptor kinase interactor 1 |
| 218033_s_at | 0,000107 | 1,0191894 | 225,5926 | 1,4498419 | 319,64542 | SNN | NM_003498 | Stannin |
| 218040_at | 0,00101 | 0,98380214 | 401,13702 | 0,67028344 | 274,34546 | FLJ10330 | NM_018061 | Sarcoma antigen NY-SAR-27 |
| 218041_x_at | 0,000262 | 1,049695 | 2513,7778 | 0,7570118 | 1825,8636 | SLC38A2 | NM_018573 | synonyms: ATA2, SAT2, SNAT2, PRO1068, KIAA1382; amino acid transporter 2; system A amino acid transporter; go_component: membrane [goid 0016020] [evidence IEA]; go_function: amino acid-polyamine transporter activity [goid 0005279] [evidence IEA]; go_proce |
| 218043_s_at | 0,0014 | 1,025358 | 208,80742 | 0,7293219 | 143,48183 | AZI2 | NM_022461 | 5-azacytidine induced 2 |
| 218047_at | 4,82E-08 | 1,0331581 | 1081,6482 | 0,7616307 | 794,30457 | OSBPL9 | NM_024586 | Oxysterol binding protein-like 9 |
| 218056_at | 3,75E-09 | 0,98774934 | 695,2778 | 0,6761118 | 479,8455 | BFAR | NM_016561 | Bifunctional apoptosis regulator |
| 218062_x_at | 4,63E-09 | 0,9947027 | 341,22223 | 1,5602909 | 533,84094 | CDC42EP4 | NM_012121 | CDC42 effector protein (Rho GTPase binding) 4 |
| 218073_s_at | 0,00755 | 1,0041173 | 158,18146 | 0,79414916 | 127,14544 | FLJ10407 | NM_018087 | Hypothetical protein FLJ10407 |
| 218085_at | 0,000262 | 0,96499676 | 423,1 | 0,7064823 | 320,85455 | SNF7DC2 | NM_015961 | synonyms: CGI-34, PNAS-2, C9orf83, HSPC177; chromosome 9 open reading frame 83; go_function: molecular_function unknown [goid 0005554] [evidence IEA]; Homo sapiens SNF7 domain containing 2 (SNF7DC2), |
| 218090_s_at | 0,00352 | 1,0075661 | 818,274 | 0,7890179 | 637,2364 | WDR11 | NM_018117 | WD repeat domain 11 |
| 218096_at | 7,50E-06 | 0,9983439 | 425,6592 | 0,7014919 (lysophosphatidic | 305,65002 | AGPAT5 | NM_018361 | 1-acylglycerol-3-phosphate O-acyltransferase 5 acid acyltransferase, epsilon) |
| 218098_at | 7,02E-05 | 0,99165493 | 933,9851 | 0,73028064 2 | 699,40906 | ARFGEF2 | NM_006420 | ADP-ribosylation factor guanine nucleotide-exchange factor (brefeldin A-inhibited) |
| 218099_at | 1,68E-06 | 1,0115997 | 341,06665 | 0,7409913 | 250,55452 | HT008 | NM_018469 | Uncharacterized hypothalamus protein HT008 |
| 218100_s_at | 4,05E-05 | 1,0004315 | 404,7111 | 0,7392698 | 299,7818 | ESRRBL1 | NM_018010 | Estrogen-related receptor beta like 1 |
| 218106_s_at | 0,000925 | 1,0177122 | 759,5408 | 0,80652153 | 611,9545 | MRPS10 | NM_018141 | Mitochondrial ribosomal protein S10 |
| 218120_s_at | 1,09E-06 | 1,0171092 | 854,2148 | 0,72966725 | 607,7864 | HMOX2 | D21243 | Heme oxygenase (decycling) 2 |
| 218123_at | 8,06E-07 | 1,0271767 | 581,62585 | 0,77947265 | 442,2818 | C21orf59 | NM_017835 | Chromosome 21 open reading frame 59 |
| 218128_at | 2,09E-10 | 0,9976327 | 132,12964 | 0,55594003 | 74,67273 | NFYB | AI804118 | Nuclear transcription factor Y, beta |
| 218129_s_at | 0,000107 | 0,97174495 | 179,28146 | 0,5548811 | 101,313644 | NFYB | NM_006166 | Nuclear transcription factor Y, beta |
| 218134_s_at | 2,57E-06 | 1,0147023 | 1365,6406 | 0,7410396 | 1000,76825 | RBM22 | NM_018047 | RNA binding motif protein 22 |
| 218135_at | 0,000238 | 1,0075308 | 410,563 | 0,6832153 | 291,0182 | PTX1 | NM_016570 | PTX1 protein |
| 218136_s_at | 2,29E-08 | 1,0105237 | 354,5037 | 1,5638276 | 543,30444 | MSCP | NM_018579 | Mitochondrial solute carrier protein |
| 218138_at | 2,69E-07 | 0,97351366 | 666,38513 | 0,5904019 | 428,74094 | MKKS | NM_018848 | McKusick-Kaufman syndrome |
| 218139_s_at | 5,02E-13 | 0,9435834 | 921,7222 | 0,48494416 | 479,81815 | C14orf108 | NM_018229 | Chromosome 14 open reading frame 108 |
| 218140_x_at | 9,67E-06 | 0,9589288 | 1107,911 | 0,70588905 | 812,7546 | SRPRB | NM_021203 | Signal recognition particle receptor, B subunit |
| 218146_at | 1,68E-06 | 1,0617833 | 744,88885 | 0,7594778 | 531,47266 | GLT8D1 | NM_018446 | Glycosyltransferase 8 domain containing 1 |
| 218147_s_at | 6,53E-11 | 1,0315653 | 330,9222 | 0,53652287 | 163,7682 | GLT8D1 | NM_018446 | Glycosyltransferase 8 domain containing 1 |
| 218152_at | 2,69E-07 | 0,9615607 | 534,88513 | 0,6673842 | 371,85455 | HMG20A | NM_018200 | High-mobility group 20A |
| 218156_s_at | 0,00618 | 0,91743845 | 202,27779 | 0,7275587 | 160,13635 | FLJ10534 | NM_018128 | Hypothetical protein FLJ10534 |
| 218160_at | 2,87E-05 | 0,9821461 | 820,23706 | 0,7808045 | 652,38184 | NDUFA8 | NM_014222 | NADH dehydrogenase (ubiquinone) 1 alpha subcomplex, 8, 19kDa |
| 218171_at | 4,05E-05 | 0,9590854 | 1719,0815 | 0,6979062 | 1238,1681 | VPS4B | AF195514 | Vacuolar protein sorting 4B (yeast) |
| 218172_s_at | 0,00109 | 1,0460507 | 453,97043 | 0,7428133 derlin-1; | 318,66818 | DERL1 | NM_018630 | synonyms: DER1, DER-1, MGC3067, PRO2577, FLJ13784; go_component: endoplasmic reticulum [goid 0005783] [evidence IEA]; go_component: integral to endoplasmic reticulum membrane [goid 0030176] [evidence IDA] [pmid 15215856]; go_function: protein bi |
| 218179_s_at | 4,53E-05 | 0,9583575 | 414,1963 | 0,6712045 | 293,96817 | FLJ12716 | NM_021942 | FLJ12716 protein |
| 218183_at | 2,77E-08 | 0,9868731 | 218,47778 | 0,54163486 | 124,61363 | C16orf5 | NM_013399 | Chromosome 16 open reading frame 5 |
| 218185_s_at | 7,81E-05 | 1,0367776 | 375,5556 | 0,66259515 | 238,12274 | ARMC1 | NM_018120 | Armadillo repeat containing 1 |
| 218194_at | 1,96E-09 | 0,9956896 | 1411,0076 | 0,66400737 144 | 933,7954 | DKFZP566E | NM_015523 | Small fragment nuclease |
| 218195_at | 1,10E-05 | 0,9416444 | 398,8555 | 0,6418424 | 274,7364 | C6orf211 | NM_024573 | Chromosome 6 open reading frame 211 |
| 218204_s_at | 6,53E-11 | 0,99971765 | 335,44446 | 0,57523423 | 191,07726 | FYCO1 | NM_024513 | FYVE and coiled-coil domain containing 1 |
| 218212_s_at | 4,43E-06 | 0,9473142 | 256,96295 | 0,6472228 | 173,25908 | MOCS2 | NM_004531 | Molybdenum cofactor synthesis 2 |
| 218217_at | 9,67E-06 | 1,017093 | 1702,8076 | 1,451865 | 2476,891 | SCPEP1 | NM_021626 | Serine carboxypeptidase 1 |
| 218221_at | 0,00706 | 1,0638648 | 208,14076 | 0,8133209 | 157,6909 | ARNT | AL042842 | Aryl hydrocarbon receptor nuclear translocator |
| 218224_at | 4,35E-07 | 0,9792594 | 836,07776 | 0,6672322 | 580,9818 | PNMA1 | NM_006029 | Paraneoplastic antigen MA1 |
| 218230_at | 2,02E-05 | 0,99029356 | 492,1037 | 0,6871125 | 354,58185 | ARFIP1 | AL044651 | ADP-ribosylation factor interacting protein 1 (arfaptin 1) |
| 218238_at | 0,0014 | 1,0468229 | 423,0852 | 0,8127047 | 331,9591 | GTPBP4 | NM_012341 | GTP binding protein 4 |
| 218241_at | 1,94E-07 | 0,9826714 | 789,81854 | 0,7722987 | 625,83636 | GOLGA5 | NM_005113 | Golgi autoantigen, golgin subfamily a, 5 |
| 218242_s_at | 1,22E-11 | 0,9636221 | 1122,2777 | 0,49397552 | 594,55 | SUV420H1 | NM_017635 | Suppressor of variegation 4-20 homolog 1 (Drosophila) |
| 218244_at | 7,92E-10 | 0,97101355 | 771,82965 | 0,55750954 | 451,79095 | NOL8 | NM_017948 | Nucleolar protein 8 |
| 218247_s_at | 0,00618 | 1,076295 | 687,45557 | 0,7610382 | 488,75452 | RKHD2 | NM_016626 | Ring finger and KH domain containing 2 |
| 218252_at | 1,46E-06 | 0,9918987 | 312,01852 | 0,6976928 | 217,3 | CKAP2 | NM_018204 | Cytoskeleton associated protein 2 |
| 218254_s_at | 3,22E-05 | 0,9244368 | 354,46295 | 0,64126945 | 249,41818 | SARA2 | NM_016103 | SAR1a gene homolog 2 (S. cerevisiae) |
| 218256_s_at | 3,75E-09 | 0,9594014 | 682,4592 | 0,565319 | 402,82724 | NUP54 | NM_017426 | Nucleoporin 54kDa |
| 218259_at | 2,75E-06 | 0,99406254 | 296,30368 | 0,7571921 | 227,06363 | MKL2 | NM_014048 | MKL/myocardin-like 2 |
| 218263_s_at | 0,000196 | 0,9861876 | 1466,4592 | 0,7001063 | 1021,93176 | LOC58486 | NM_021211 | Transposon-derived Buster1 transposase-like protein gene |
| 218269_at | 0,00119 | 0,99290234 | 580,8445 | 0,79358417 | 473,05908 | RNASE3L | NM_013235 | Nuclear RNase III Drosha |
| 218276_s_at | 3,16E-06 | 0,9624839 | 230,53703 | 0,6418864 | 156,28183 | SAV1 | NM_021818 | Salvador homolog 1 (Drosophila) |
| 218283_at | 2,95E-06 | 1,0337828 | 1280,3779 | 0,8176 | 1012,1227 | SS18L2 | NM_016305 | Synovial sarcoma translocation gene on chromosome 18- |
| 218285_s_at | 8,59E-09 | 1,027017 | 439,0741 | 0,6821998 | 297,1273 | DHRS6 | NM_020139 | Dehydrogenase/reductase (SDR family) member 6 |
| 218288_s_at | 1,26E-06 | 0,9958683 | 805,4777 | 0,7451742 | 610,41815 | MDS025 | NM_021825 | Hypothetical protein MDS025 |
| 218301_at | 2,56E-05 | 1,0002341 | 642,88153 | 1,2813292 | 813,56824 | RNPEPL1 | NM_018226 | Arginyl aminopeptidase (aminopeptidase B)-like 1 |
| 218303_x_at | 4,02E-08 | 1,0018717 | 1042,5482 | 0,64315015 | 682,3 | LOC51315 | NM_016618 | Hypothetical protein LOC51315 |
| 218304_s_at | 0,00164 | 0,958746 | 859,2 | 0,7160531 | 667,1181 | OSBPL11 | NM_022776 | Oxysterol binding protein-like 11 |
| 218306_s_at | 9,67E-06 | 0,969172 | 963 | 0,7519179 | 746,32733 | HERC1 | NM_003922 | Hect (homologous to the E6-AP (UBE3A) carboxyl terminus) domain and RCC1 (CHC1)-like domain (RLD) 1 |
| 218310_at | 0,000107 | 0,97157 | 544,5259 | 0,7410219 | 414,23636 | RABGEF1 | NM_014504 | RAB guanine nucleotide exchange factor (GEF) 1 |
| 218312_s_at | 0,00304 | 0,95985717 | 580,9741 | 0,74513835 | 451,22723 | FLJ12895 | NM_023926 | Hypothetical protein FLJ12895 |
| 218313_s_at | 2,69E-07 | 0,98106307 | 567,13336 | 0,66782707 | 394,15457 | GALNT7 | NM_017423 | UDP-N-acetyl-alpha-D-galactosamine:polypeptide N-acetylgalactosaminyltransferase 7 (GaINAc-T7) |
| 218319_at | 6,59E-06 | 1,0572376 | 930,00745 | 1,7065245 | 1554,941 | PELI1 | NM_020651 | Pellino homolog 1 (Drosophila) |
| 218329_at | 4,44E-06 | 1,0342838 | 508,92596 | 0,7371024 | 370,88638 | PRDM4 | NM_012406 | PR domain containing 4 |
| 218331_s_at | 0,000178 | 1,0083851 | 363,5778 | 0,71045804 | 261,98184 | FLJ20360 | NM_017782 | |
| 218335_x_at | 0,000715 | 0,9290054 | 1028,3519 | 1,2048588 | 1298,0863 | TNIP2 | NM_024309 | TNFAIP3 interacting protein 2 |
| 218339_at | 1,56E-06 | 1,0139375 | 537,4222 | 0,8110136 | 431,90002 | MRPL22 | NM_014180 | Mitochondrial ribosomal protein L22 |
| 218340_s_at | 0,00436 | 0,91749626 | 286,0185 | 0,6737358 | 208,86363 | FLJ10808 | NM_018227 | Hypothetical protein FLJ10808 |
| 218341_at | 0,00861 | 1,0177066 | 763,51855 | 0,7869377 | 579,3 | FLJ11838 | NM_024664 | Hypothetical protein FLJ11838 |
| 218356_at | 1,26E-06 | 0,9673171 | 546,3334 | 0,585145 | 343,09546 | FTSJ2 | NM_013393 | FtsJ homolog 2 (E. coli) |
| 218361_at | 1,96E-09 | 0,96744615 | 362,81482 | 0,30641183 | 152,4 | GOLPH3L | NM_018178 | Golgi phosphoprotein 3-like |
| 218372_at | 0,00152 | 0,89285207 | 223,20372 | 1,1859773 homolog | 289,4227 | MED9 | NM_018019 | Mediator of RNA polymerase II transcription, subunit 9 (yeast) |
| 218373_at | 9,81 E-08 | 0,90356535 | 557,1741 | 0,4565914 | 280,04544 | FTS | NM_022476 | Fused toes homolog (mouse) |
| 218375_at | 0,000348 | 0,9944543 | 450,0926 | 0,79355717 | 360,55905 | NUDT9 | NM_024047 | Nudix (nucleoside diphosphate linked moiety X)-type motif 9 |
| 218394_at | 0,00683 | 0,9339521 | 955,8038 | 1,2229142 | 1223,5408 | FLJ22386 | NM_024589 | Leucine zipper domain protein |
| 218395_at | 1,11E-10 | 0,99396116 | 528,1333 | 0,5479506 | 295,7818 | ACTR6 | NM_022496 | ARP6 actin-related protein 6 homolog (yeast) |
| 218400_at | 0,000419 | 1,0098797 | 983,974 | 1,5720792 | 1685,5227 | OAS3 | NM_006187 | 2'-5'-oligoadenylate synthetase 3, 100kDa |
| 218422_s_at | 4,05E-05 | 0,9616473 | 880,8482 | 0,5995936 | 551,6819 | C13orf10 | NM_022118 | Chromosome 13 open reading frame 10 |
| 218428_s_at | 0,000153 | 1,0468136 | 549,28516 | 0,8272703 | 438,19098 | REV1L | NM_016316 | REV1-like (yeast) |
| 218432_at | 5,78E-06 | 1,0033877 | 229,66298 | 0,6426601 | 150,22272 | FBXO3 | NM_012175 | F-box protein 3 |
| 218437_s_at | 0,000119 | 0,9625198 | 238,44073 | 0,72132516 | 179,80453 | LZTFL1 | NM_020347 | Leucine zipper transcription factor-like 1 |
| 218446_s_at | 1,01E-06 | 0,9882321 | 250,9 | 0,6532432 | 164,15454 | FAM18B | NM_016078 | Family with sequence similarity 18, member B |
| 218449_at | 9,65E-05 | 1,0571691 | 385,1 | 0,82388926 | 294,08636 | FLJ11200 | NM_018359 | Hypothetical protein FLJ11200 |
| 218452_at | 3,71E-07 | 0,97487724 | 302,05182 | 0,51243246 | 176,80455 | SMARCAL1 | NM_014140 | SWI/SNF related, matrix associated, actin dependent regulator of chromatin, subfamily a-like 1 |
| 218454_at | 5,06E-05 | 0,94636863 | 6254,6626 | 1,3339269 | 9035,496 | FLJ22662 | NM_024829 | Hypothetical protein FLJ22662 |
| 218461_at | 0,000502 | 0,955783 | 323,13327 | 0,69346297 | 245,2636 | MGC14560 | NM_016301 | Protein x 0004 |
| 218470_at | 0,000107 | 1,0061896 | 239,72224 | 0,66160876 | 152,04999 | CGI-04 | NM_015936 | CGI-04 protein |
| 218477_at | 5,06E-05 | 1,0002685 | 467,4555 | 0,6348725 | 309,21814 | TMEM14A | NM_014051 | Transmembrane protein 14A |
| 218486_at | 1,43E-10 | 0,99568963 | 781,15924 | 1,8113288 | 1394,0635 | KLF11 | AA149594 | Kruppel-like factor 11 |
| 218490_s_at | 4,35E-07 | 1,0480438 | 290,8629 | 0,6401477 | 174,8591 | ZNF302 | NM_018443 | Zinc finger protein 302 |
| 218491_s_at | 2,29E-08 | 1,0268456 | 897,237 | 0,7358455 | 638,9227 | THY28 | NM_014174 | Thymocyte protein thy28 |
| 218499_at | 0,000459 | 0,971953 | 1412,5146 | 0,7631653 | 1118,5001 | MST4 | NM_016542 | Mst3 and SOK1-related kinase |
| 218501_at | 0,00152 | 1,0536153 | 2122,8037 | 0,8290301 | 1699,0681 | ARHGEF3 | NM_019555 | Rho guanine nucleotide exchange factor (GEF) 3 |
| 218513_at | 2,23E-06 | 0,9797687 | 66,94074 | 0,55870086 | 39,922726 | FLJ11184 | NM_018352 | Hypothetical protein FLJ11184 |
| 218517_at | 1,79E-05 | 0,9735238 | 740,8518 | 0,7528801 | 573,0773 | PHF17 | NM_024900 | PHD finger protein 17 |
| 218518_at | 0,000131 | 0,9933108 | 807,2481 | 0,7923889 | 647,7 | C5orf5 | NM_016603 | Chromosome 5 open reading frame 5 |
| 218519_at | 3,39E-06 | 0,9826545 | 497,5963 | 0,64296985 | 330,18634 | SLC35A5 | NM_017945 | Solute carrier family 35, member A5 |
| 218521_s_at | 0,00304 | 0,94887966 | 116,05556 | 1,2106003 | 147,38182 | FLJ11011 | NM_018299 | Hypothetical protein FLJ11011 |
| 218530_at | 0,00252 | 1,0326017 | 722,9408 | 1,3183602 | 925,44086 | FHOD1 | NM_013241 | Formin homology 2 domain containing 1 |
| 218532_s_at | 0,000206 | 1,0065291 | 368,12964 | 0,6352976 | 228,46819 | FLJ20152 | NM_019000 | Hypothetical protein FLJ20152 |
| 218534_s_at | 1,28E-08 | 0,9585265 | 472,48523 | 0,53829473 | 268,84546 | AGGF1 | NM_018046 | Angiogenic factor with G patch and FHA domains 1 |
| 218535_s_at | 1,92E-12 | 0,9753645 | 364,0111 | 0,5406224 | 200,01363 | RIOK2 | NM_018343 | RIO kinase 2 (yeast) |
| 218536_at | 3,88E-06 | 0,98368466 | 374,837 | 0,739038 | 285,21362 | MRS2L | AF052167 | MRS2-like, magnesium homeostasis factor (S. cerevisiae) |
| 218538_s_at | 1,16E-08 | 1,0315046 | 223,84076 | 0,7039372 | 154,57272 | MRS2L | NM_020662 | MRS2-like, magnesium homeostasis factor (S. cerevisiae) |
| 218543_s_at | 0,00164 | 0,95769 | 1537,3889 | 1,23753 | 1994,3727 | ZC3HDC1 | NM_022750 | Zinc finger CCCH type domain containing 1 |
| 218545_at | 0,00178 | 1,0453684 | 517,4556 | 0,8143738 | 404,00455 | FLJ11088 | | NM_018318 GGAbindingpartner |
| 218565_at | 7,49E-06 | 0,95851403 | 285,2704 | 0,72124904 | 211,12726 | C9orf114 | BG223334 | Chromosome 9 open reading frame 114 |
| 218566_s_at | 0,000146 | 0,98947126 | 627,84436 | 0,6905083 | 431,52728 | CHORDC1 | NM_012124 | Cysteine and histidine-rich domain (CHORD)-containing, zinc binding protein 1 |
| 218571_s_at | 0,00304 | 0,9992192 | 1402,5447 | 0,77200186 | 1105,9681 | C14orf123 | NM_014169 | Chromosome 14 open reading frame 123 |
| 218572_at | 7,81E-05 | 1,0340436 | 730,0963 | 0,7630471 | 528,4682 | C14orf123 | NM_014169 | Chromosome 14 open reading frame 123 |
| 218573_at | 6,59E-06 | 0,96390384 | 329,55927 | 0,6165095 | 213,65454 | MAGEH1 | NM_014061 | Melanoma antigen family H, 1 |
| 218577_at | 4,35E-07 | 0,9944109 | 453,35184 | 0,6486588 | 298,6636 | FLJ20331 | NM_017768 | Hypothetical protein FLJ20331 |
| 218588_s_at | 0,000502 | 0,9526837 | 399,16293 | 0,6846431 | 281,93634 | C5orf3 | NM_018691 | Chromosome 5 open reading frame 3 |
| 218589_at | 3,41E-05 | 0,9147679 | 2135,5781 | 0,6278854 | 1459,9272 | P2RY5 | NM_005767 | Purinergic receptor P2Y, G-protein coupled, 5 |
| 218594_at | 0,000289 | 0,9631771 | 455,26663 | 0,766618 | 355,28635 | FLJ10359 | NM_018072 | |
| 218598_at | 3,33E-08 | 1,0405976 | 198,82224 | 0,6604601 | 125,004555 | RINT-1 | NM_021930 | Rad50-interacting protein 1 |
| 218599_at | 0,00352 | 0,988758 | 480,7815 | 1,2428278 | 593,1546 | REC8L1 | NM_005132 | REC8-like 1 (yeast) |
| 218604_at | 0,000262 | 0,9785935 | 703,9518 | 0,69723755 | 510,01364 | LEMD3 | NM_014319 | LEM domain containing 3 |
| 218605_at | 0,000549 | 0,9304571 | 459,9926 | 0,6908874 | 345,46817 | TFB2M | NM_022366 | Transcription factor B2, mitochondrial |
| 218606_at | 0,000317 | 0,9869746 | 3089,8293 | 1,2371904 | 3928,3044 | ZDHHC7 | NM_017740 | Zinc finger, DHHC domain containing 7 |
| 218611_at | 7,81E-05 | 0,99476737 | 2747,7815 | 1,2804885 | 3521,5178 | IER5 | NM_016545 | Immediate early response 5 |
| 218614_at | 1,96E-09 | 1,0337011 | 1729,9962 | 0,5246624 | 908,0863 | FLJ20696 | NM_018169 | Hypothetical protein FLJ20696 |
| 218615_s_at | 0,00192 | 0,89252526 | 242,49629 | 0,63895553 | 163,77274 | TMEM39A | NM_018266 | Transmembrane protein 39A |
| 218628_at | 0,00755 | 0,9849803 | 882,0186 | 0,77732307 | 715,3773 | CGI-116 | NM_016053 | CGI-116 protein |
| 218631_at | 6,91 E-08 | 0,9875142 | 331,62592 | 1,48909 | 501,42276 | AVPI1 | | NM_021732 Arginine vasopressin-induced 1 |
| 218647_s_at | 0,000656 | 1,0237594 | 362,63702 | 1,3081112 | 453,29092 | FLJ23476 | NM_024640 | Ischemia/reperfusion inducible protein |
| 218652_s_at | 0,00078 | 0,92768747 | 829,12585 | 0,7312211 | 638,2682 | GPI7 | NM_017733 | GPI7 protein |
| 218654_s_at | 1,38E-07 | 0,96912336 | 858,45184 | 0,72349143 | 629,60004 | MRPS33 | NM_016071 | Mitochondrial ribosomal protein S33 |
| 218660_at | 1,68E-06 | 0,96970904 | 914,76294 | 1,5769652 | 1517,7228 | DYSF | NM_003494 | Dysferlin, limb girdle muscular dystrophy 2B (autosomal recessive) |
| 218673_s_at | 1,26E-06 | 0,99791217 | 397,35187 | 1,3632134 | 543,1 | APG7L | NM_006395 | APG7 autophagy 7-like (S. cerevisiae) |
| 218674_at | 3,03E-09 | 1,0723492 | 125,78518 | 0,68994254 | 81,96362 | FLJ13611 | NM_024941 | Hypothetical protein FLJ13611 |
| 218681_s_at | 0,00178 | 0,9118686 | 524,8555 | 1,1873151 | 659,90906 | SDF2L1 | NM_022044 | Stromal cell-derived factor 2-like 1 |
| 218696_at | 0,00707 | 0,99522316 | 526,55554 | 0,7747527 | 420,03183 | EIF2AK3 | NM_004836 | Eukaryotic translation initiation factor 2-alpha kinase 3 |
| 218699_at | 0,000178 | 1,023551 | 1664,6444 | 0,7723219 | 1285,9319 | RAB7L1 | BG338251 | RAB7, member RAS oncogene family-like 1 |
| 218700_s_at | 0,00979 | 1,1113893 | 629,6926 | 0,80410415 | 415,8773 | RAB7L1 | BC002585 | RAB7, member RAS oncogene family-like 1 |
| 218708_at | 8,52E-11 | 1,0268936 | 648,2556 | 1,9878727 | 1287,5952 | NXT1 | NM_013248 | NTF2-like export factor 1 |
| 218710_at | 2,02E-05 | 0,9693937 | 464,8481 | 0,6462727 | 316,2182 | FLJ20272 | NM_017735 | Hypothetical protein FLJ20272 |
| 218713_at | 1,89E-08 | 1,0163862 | 425,61105 | 0,6363173 | 266,71817 | NARG2 | NM_024611 | NMDA receptor regulated 2 |
| 218716_x_at | 8,24E-08 | 1,008657 | 627,60376 | 0,7126551 | 437,99084 | MTO1 | NM_012123 | Mitochondrial translation optimization 1 homolog (S. |
| 218723_s_at | 0,00224 | 0,9727308 | 1203,1593 | 0,6235933 | 893,5727 | RGC32 | NM_014059 | Response gene to complement 32 |
| 218729_at | 1,27E-07 | 1,0182146 | 275,36673 | 0,59134936 | 169,73183 | LXN | NM_020169 | Latexin |
| 218732_at | 1,26E-06 | 1,0077609 | 454,3519 | 0,6977683 | 312,96817 | Bit1 | NM_016077 | Bcl-2 inhibitor of transcription |
| 218735_s_at | 2,28E-07 | 0,9954422 | 437,22592 | 0,65930504 | 288,11365 | ZNF544 | AA349848 | Zinc finger protein 544 |
| 218764_at | 0,0014 | 0,97256786 | 3179,263 | 0,72894096 | 2437,0774 | PRKCH | NM_024064 | synonyms: PKCL, PKC-L, PRKCL, MGC5363, MGC26269, nPKC-eta; go_function: ATP binding [goid 0005524] [evidence IEA]; go_function: kinase activity [goid 0016301] [evidence IEA]; go_function: transferase activity [goid 0016740] [evidence IEA]; go_function: di |
| 218768_at | 2,57E-06 | 1,0236125 | 795,95935 | 0,79971933 | 616,6182 | NUP107 | NM_020401 | Nucleoporin 107kDa |
| 218773_s_at | 0,00101 | 0,94673514 | 861,7148 | 1,2014673 | 1083,7227 | MSRB2 | NM_012228 | Methionine sulfoxide reductase B2 |
| 218791_s_at | 1,01E-06 | 0,9522281 | 182,26297 | 0,6775841 | 129,89091 | C15orf29 | NM_024713 | Chromosome 15 open reading frame 29 |
| 218805_at | 5,07E-06 | 0,86460465 | 3181,663 | 0,4415651 | 1531,4454 | GIMAP5 | NM_018384 | GTPase, IMAP family member 5 |
| 218810_at | 1,43E-10 | 0,96280605 | 326,2074 | 2,320047 | 808,52734 | FLJ23231 | NM_025079 | Hypothetical protein FLJ23231 |
| 218821_at | 3,61 E-05 | 0,9499098 | 557,65924 | 1,2563428 | 723,38635 | NPEPL1 | AL139349 | |
| 218827_s_at | 5,65E-05 | 1,0145049 | 392,9778 | 0,79594946 | 299,49088 | Cep192 | NM_018069 | Centrosomal protein 192 kDa |
| 218841_at | 0,00577 | 0,88143474 | 342,71112 | 0,642236 | 259,1227 | ASB8 | NM_024095 | Ankyrin repeat and SOCS box-containing 8 |
| 218846_at | 1,56E-08 | 1,0247135 | 568,2221 | 0,59771335 3, 130kDa | 322,92728 | CRSP3 | NM_004830 | Cofactor required for Sp1 transcriptional activation, subunit |
| 218854_at | 6,30E-05 | 0,9526541 | 555,49634 | 0,7191289 | 423,11813 | SART2 | NM_013352 | Squamous cell carcinoma antigen recognized by T cells 2 |
| 218866_s_at | 0,000419 | 0,9590695 | 434,4259 | 0,746757 | 341,12726 | POLR3K | NM_016310 | Polymerase (RNA) III (DNA directed) polypeptide K, 12.3 |
| 218870_at | 0,000161 | 0,9832318 | 2484,337 | 0,78537697 | 1972,4453 | ARHGAP15 | NM_018460 | Rho GTPase activating protein 15 |
| 218875_s_at | 1,38E-07 | 0,99563116 | 91,2889 | 0,52276814 | 47,386364 | FBXO5 | NM_012177 | F-box protein 5 |
| 218877_s_at | 0,0014 | 0,95442265 | 327,15182 | 0,646754 | 210,57272 | C6orf75 | NM_021820 | Chromosome 6 open reading frame 75 |
| 218878_s_at | 6,53E-11 | 0,9685876 | 502,85928 | 0,5910001 | 310,4227 | SIRT1 | NM_012238 | Sirtuin (silent mating type information regulation 2 homolog) 1 (S. cerevisiae) |
| 218880_at | 1,96E-09 | 0,9743116 | 263,59998 | 2,6947203 | 783,0863 | FOSL2 | N36408 | FOS-like antigen 2 |
| 218882_s_at | 5,27E-08 | 1,0230206 | 351,1667 | 0,6679148 | 226,14545 | WDR3 | NM_006784 | WD repeat domain 3 |
| 218889_at | 2,69E-07 | 1,0382 | 403,61856 | 0,6742788 | 264,6227 | C10orf117 | NM_022451 | Chromosome 10 open reading frame 117 |
| 218897_at | 0,000262 | 0,9097012 | 107,11111 | 0,56530327 | 69,12727 | MGC10993 | NM_030577 | Hypothetical protein MGC10993 |
| 218905_at | 1,24E-05 | 1,0216733 | 1138,2777 | 0,78571683 | 871,8 | FLJ20530 | NM_017864 | Hypothetical protein FLJ20530 |
| 218911_at | 2,28E-07 | 0,9857574 | 123,44446 | 0,6116681 | 76,13637 | YEATS4 | NM_006530 | YEATS domain containing 4 |
| 218918_at | 0,00755 | 0,9320035 | 560,7001 | 0,73823845 | 424,72726 | MAN1C1 | NM_020379 | Mannosidase, alpha, class 1C, member 1 |
| 218919_at | 0,000289 | 0,96778095 | 595,92224 | 0,75573564 | 466,8409 | FLJ14007 | NM_024699 | Hypothetical protein FLJ14007 |
| 218926_at | 0,000196 | 1,0245236 | 251,37407 | 0,77183515 | 190,5091 | MYNN | NM_018657 | Myoneurin |
| 218929_at | 2,27E-05 | 1,0318254 | 394,66293 | 0,66568345 | 252,85455 | CARF | NM_017632 | Collaborates/cooperates with ARF (alternate reading frame) protein |
| 218930_s_at | 0,00755 | 1,0583048 | 386,12595 | 0,7928157 | 296,9727 | FLJ11273 | NM_018374 | Hypothetical protein FLJ11273 |
| 218932_at | 3,75E-09 | 0,9832161 | 464,6778 | 0,6416786 | 304,54544 | FLJ20729 | NM_017953 | Hypothetical protein FLJ20729 |
| 218937_at | 0,000849 | 0,99810195 | 389,30374 | 0,7830728 | 306,77277 | ZNF434 | NM_017810 | Zinc finger protein 434 |
| 218946_at | 0,000178 | 1,0348358 | 594,3001 | 0,82583857 | 466,21365 | HIRIP5 | NM_015700 | HIRA interacting protein 5 |
| 218949_s_at | 2,02E-05 | 0,9561285 | 544,6666 | 0,6751861 | 387,8 | QRSL1 | NM_018292 | Glutaminyl-tRNA synthase (glutamine-hydrolyzing)-like 1 |
| 218962_s_at | 0,00661 | 0,9740904 | 380,2222 | 0,7305589 | 283,6 | FLJ13576 | NM_022484 | Hypothetical protein FLJ13576 |
| 218966_at | 0,00327 | 0,985726 | 257,1371 | 1,2441242 | 317,72726 | MYO5C | NM_018728 | Myosin VC |
| 218967_s_at | 3,39E-06 | 1,0136288 | 391,67783 | 0,6465977 | 242,22272 | PTER | NM_030664 | Phosphotriesterase related |
| 218968_s_at | 3,22E-05 | 0,9985129 | 405,8482 | 0,72395974 | 297,44547 | ZFP64 | NM_018197 | Zinc finger protein 64 homolog (mouse) |
| 218972_at | 5,08E-07 | 0,99002916 | 626,2111 | 1,3580703 | 847,6455 | TTC17 | NM_018259 | Tetratricopeptide repeat domain 17 |
| 218979_at | 1,25E-09 | 0,940026 | 304,41113 | 0,5279019 | 172,0818 | C9orf76 | NM_024945 | Chromosome 9 open reading frame 76 |
| 218981_at | 0,000779 | 0,9915642 | 124,974075 | 0,6170605 | 79,531815 | ACN9 | NM_020186 | ACN9 homolog (S. cerevisiae) |
| 218986_s_at | 0,00378 | 0,9922502 | 962,74066 | 0,6829183 | 776,19086 | FLJ20035 | NM_017631 | Hypothetical protein FLJ20035 |
| 218989_x_at | 5,09E-07 | 0,9872211 | 331,14072 | 0,63672066 | 215,96362 | SLC30A5 | NM_022902 | Solute carrier family 30 (zinc transporter), member 5 |
| 218999_at | 0,000119 | 0,9999457 | 590,4556 | 0,6729006 | 404,76367 | FLJ11000 | NM_018295 | Hypothetical protein FLJ11000 |
| 219001_s_at | 0,000196 | 0,9436992 | 358,3778 | 0,63145167 | 239,83636 | WDR32 | NM_024345 | WD repeat domain 32 |
| 219002_at | 5,94E-07 | 0,9728389 | 481,64816 | 0,6375113 | 314,78638 | FLJ21901 | NM_024622 | Hypothetical protein FLJ21901 |
| 219006_at | 1,35E-06 | 0,99312377 | 430,85187 | 0,69054276 | 309,2046 | C6orf66 | NM_014165 | Chromosome 6 open reading frame 66 |
| 219007_at | 0,0006 | 0,9018893 | 890,29626 | 0,6362017 | 628,1363 | NUP43 | NM_024647 | Nucleoporin 43kDa |
| 219008_at | 1,35E-06 | 1,018268 | 211,49997 | 0,68380296 | 147,00456 | FLJ21820 | NM_021925 | Hypothetical protein FLJ21820 |
| 219016_at | 5,65E-05 | 0,9667495 | 505,9037 | 1,2171992 | 634,2409 | UBOX5 | NM_021826 | U-box domain containing 5 |
| 219017_at | 0,000161 | 0,9584861 | 211,06667 | 0,7030689 | 155,48184 | ETNK1 | NM_018638 | Ethanolamine kinase 1 |
| 219022_at | 7,40E-05 | 1,027252 | 325,02963 | 0,7690018 | 246,28181 | FLJ12448 | NM_022895 | Hypothetical protein FLJ12448 |
| 219029_at | 2,02E-05 | 1,0118773 | 206,12224 | 0,6395692 | 132,98183 | FLJ21657 | NM_022483 | Hypothetical protein FLJ21657 |
| 219035_s_at | 8,51E-11 | 0,927082 | 1254,7445 | 0,5543259 | 741,9818 | RNF34 | NM_025126 | Ring finger protein 34 |
| 219037_at | 1,56E-09 | 0,9207796 | 158,1852 | 0,56170994 | 98,40453 | CGI-115 | NM_016052 | CGI-115 protein |
| 219041_s_at | 0,000289 | 0,9840079 | 1359,5371 | 0,72033066 | 985,9545 | REPIN1 | NM_014374 | Replication initiator 1 |
| 219045_at | 0,000161 | 1,016133 | 1123,0668 | 0,77216977 | 871,1682 | RHOF | NM_019034 | Ras homolog gene family, member F (in filopodia) |
| 219049_at | 1,59E-05 | 1,0176295 | 1048,4443 | 0,627961 | 648,4636 | ChGn | NM_018371 | Chondroitin beta1,4 N-acetylgalactosaminyltransferase |
| 219067_s_at | 0,000131 | 0,98062617 | 678,72595 | 0,7425174 | 516,6318 | C10orf86 | NM_017615 | Chromosome 10 open reading frame 86 |
| 219073_s_at | 6,92E-07 | 1,0496364 | 233,54813 | 0,5760296 | 130,42273 | OSBPL10 | NM_017784 | Oxysterol binding protein-like 10 |
| 219074_at | 2,75E-06 | 0,9738511 | 319,67776 | 0,67319643 | 222,86818 | TMEM34 | NM_018241 | Transmembrane protein 34 |
| 219081_at | 9,96E-10 | 0,9640317 | 445,26297 | 1,4157912 | 658,2683 | ANKHD1 | NM_024668 | Eukaryotic translation initiation factor 4E binding protein 3 |
| 219094_at | 0,00597 | 0,9778444 | 111,844444 | 1,3408996 | 152,92274 | ARMC8 | NM_014154 | Armadillo repeat containing 8 |
| 219112_at | 0,000332 | 0,67554355 | 588,69995 | 0,3055747 | 263,06818 | KIAA1961 | NM_016340 | KIAA1961 gene |
| 219123_at | 2,38E-10 | 0,91747516 | 301,7667 | 0,4531592 | 152,34091 | ZNF232 | NM_014519 | Zinc finger protein 232 |
| 219128_at | 3,80E-11 | 1,024067 | 399,0889 | 0,6265636 | 242,17728 | FLJ20558 | NM_017880 | Hypothetical protein FLJ20558 |
| 219130_at | 5,06E-05 | 0,9004874 | 445,1667 | 0,5785814 | 272,95453 | FLJ10287 | NM_019083 | Hypothetical protein FLJ10287 |
| 219132_at | 3,22E-05 | 1,0142435 | 534,863 | 1,3501899 | 698,0454 | PELI2 | NM_021255 | Pellino homolog 2 (Drosophila) |
| 219146_at | 6,30E-08 | 0,9369196 | 227,8963 | 0,57045287 | 136,92728 | FLJ22729 | NM_024683 | Hypothetical protein FLJ22729 |
| 219164_s_at | 0,00618 | 0,99585634 | 131,9778 | 0,77483046 | 105,25454 | C14orf103 | NM_018036 | Chromosome 14 open reading frame 103 |
| 219166_at | 0,000107 | 0,94167227 | 143,37778 | 0,5078526 | 78,08182 | C14orf104 | NM_018139 | Chromosome 14 open reading frame 104 |
| 219180_s_at | 9,67E-06 | 0,94091475 | 279,38516 | 1,3534406 | 400,8409 | PEX26 | AI817074 | Peroxisome biogenesis factor 26 |
| 219200_at | 5,94E-07 | 0,9962026 | 393,85556 | 0,6649153 | 265,13638 | MGC5297 | NM_024091 | Hypothetical protein MGC5297 |
| 219202_at | 0,000549 | 0,9813472 | 760,4667 | 1,2890427 | 1033,8635 | RHBDL6 | NM_024599 | Rhomboid, veinlet-like 6 (Drosophila) |
| 219209_at | 0,00661 | 1,0597669 | 350,14075 | 0,82942927 | 297,50458 | IFIH1 | NM_022168 | Interferon induced with helicase C domain 1 |
| 219217_at | 9,07E-06 | 1,0328091 | 369,28894 | 0,78928167 | 283,29544 | FLJ23441 | NM_024678 | Hypothetical protein FLJ23441 |
| 219228_at | 5,94E-07 | 0,93195546 | 535,3111 | 1,5032644 | 895,6908 | ZNF331 | NM_018555 | Zinc finger protein 331 |
| 219238_at | 0,000779 | 0,9248206 | 377,95557 | 0,65561515 | 278,39093 | PIGV | NM_017837 | Phosphatidylinositol glycan, class V |
| 219242_at | 9,65E-05 | 0,98925066 | 404,81482 | 0,66072196 | 292,12726 | Cep63 | NM_025180 | Centrosome protein Cep63 |
| 219243_at | 7,01E-09 | 0,895414 | 3725,8037 | 0,43257406 | 1911,0138 | GIMAP4 | NM_018326 | GTPase, IMAP family member 4 |
| 219244_s_at | 3,75E-09 | 0,97240984 | 491,70364 | 0,6865305 | 347,81363 | MRPL46 | NM_022163 | Mitochondrial ribosomal protein L46 |
| 219253_at | 0,000317 | 0,9839557 | 182,41481 | 0,6884328 | 128,53636 | FAM11B | NM_024121 | |
| 219256_s_at | 1,94E-06 | 0,9470439 | 717,5296 | 1,4483081 | 1121,2635 | SH3TC1 | NM_018986 | SH3 domain and tetratricopeptide repeats 1 |
| 219257_s_at | 7,02E-05 | 0,8536668 | 200,71483 | 1,3091704 | 285,1273 | SPHK1 | NM_021972 | Sphingosine kinase 1 |
| 219259_at | 2,87E-05 | 0,93832827 | 918,93713 | 1,4149414 | 1401,3862 | SEMA4A | NM_022367 | Sema domain, immunoglobulin domain (Ig), transmembrane domain (TM) and short cytoplasmic domain, (semaphorin) |
| 219276_x_at | 2,64E-12 | 1,0366637 | 404,40738 | 0,6132875 | 240,48636 | C9orf82 | NM_024828 | Chromosome 9 open reading frame 82 |
| 219279_at | 9,67E-06 | 1,018806 | 1070,7926 | 0,7332949 | 768,56366 | FLJ20220 | NM_017718 | |
| 219283_at | 0,00452 | 0,997278 | 401,44818 | 0,7576643 MGC19947, | 313,9409 | C1GALT1C1 | NM_014158 | synonyms: COSMC, C1GALT2, HSPC067, C1Gal-T2, c38h2-I1; core 1 beta3-galactosyltransferase-specific molecular chaperone; core 1 UDP-galactose:N-acetylgalactosamine-alpha-R beta 1,3-galactosyltransferase 2; go_function: transferase activity, trans |
| 219289_at | 0,00352 | 0,936991 | 378,4667 | 0,71081156 | 285,4864 | FLJ20718 | NM_017939 | Hypothetical protein FLJ20718 |
| 219290_x_at | 6,59E-06 | 0,984263 | 1171,174 | 0,6482516 | 790,6272 | DAPP1 | NM_014395 | Dual adaptor of phosphotyrosine and 3-phosphoinositides |
| 219294_at | 0,000138 | 0,95999724 | 69,922226 | 0,6724838 | 50,145454 | C6orf139 | NM_018132 | Chromosome 6 open reading frame 139 |
| 219299_at | 1,24E-05 | 0,9679422 | 435,1296 | 0,70738053 | 323,11362 | FLJ20772 | NM_017956 | Hypothetical protein FLJ20772 |
| 219303_at | 9,81E-08 | 0,9201285 | 315,04816 | 0,543528 | 180,56819 | C13orf7 | NM_024546 | Chromosome 13 open reading frame 7 |
| 219312_s_at | 0,000216 | 0,94720995 | 63,55925 | 1,5096083 | 96,54091 | ZBTB10 | NM_023929 | Zinc finger and BTB domain containing 10 |
| 219315_s_at | 2,87E-11 | 1,0072205 | 1167,4705 | 0,4863507 | 531,54095 | C16orf30 | NM_024600 | Chromosome 16 open reading frame 30 |
| 219322_s_at | 0,00048 | 0,9480221 | 267,55557 | 0,7476929 | 207,93181 | WDR8 | NM_017818 | WD repeat domain 8 |
| 219342_at | 2,56E-05 | 0,9450474 | 305,2889 | 0,63643545 | 208,85 | CAS1 | NM_022900 | O-acetyltransferase |
| 219345_at | 0,0014 | 0,9833337 | 196,74442 | 0,7859751 | 156,08183 | CGI-143 | NM_016074 | CGI-143 protein |
| 219348_at | 0,00755 | 1,0072852 | 290,57407 | 0,77654827 MDS032 | 231,84093 | MDS032 | NM_018467 | Uncharacterized hematopoietic stem/progenitor cells protein |
| 219351_at | 5,06E-05 | 1,0041485 | 690,24817 | 0,7619291 | 523,1455 | SEDL | NM_014563 | Spondyloepiphyseal dysplasia, late, pseudogene |
| 219353_at | 0,00114 | 0,96429324 | 148,62222 | 0,7100585 | 106,70909 | NHLRC2 | M_017687 | synonyms: FLJ20147, FLJ25621, FLJ33312, MGC45492, DKFZp779F115; 1200003G01Rik; Similar to RIKEN cDNA 1200003G01 gene; novel NHL repeat domain containing protein; go_function: electron transporter activity [goid 0005489] [evidence IEA]; go_process: electro |
| 219363_s_at | 2,64E-12 | 0,98114365 | 335,537 | 0,4985921 | 171,67271 | CGI-12 | NM_015942 | CGI-12 protein |
| 219368_at | 0,00085 | 0,9068224 | 152,73332 | 0,6545888 | 106,23183 | NAP1 L2 | NM_021963 | Nucleosome assembly protein 1-like 2 |
| 219375_at | 0,00105 | 1,0707889 | 587,263 | 0,7682304 | 408,5864 | CEPT1 | NM_006090 | Choline/ethanolamine phosphotransferase 1 |
| 219376_at | 0,00639 | 0,96128553 | 105,22593 | 0,694544 | 72,586365 | ZNF322A | NM_024639 | Zinc finger protein 322A |
| 219380_x_at | 0,00378 | 1,1575407 | 64,52223 | 0,4474002 | 26,481815 | POLH | NM_006502 | Polymerase (DNA directed), eta |
| 219382_at | 7,92E-10 | 1,0074176 | 419,66666 | 2,179734 | 835,4046 | SERTAD3 | NM_013368 | SERTA domain containing 3 |
| 219383_at | 7,99E-06 | 0,99019873 | 242,9111 | 0,5726739 | 144,88182 | FLJ14213 | NM_024841 | Hypothetical protein FLJ14213 |
| 219397_at | 0,000502 | 1,001925 | 859,94806 | 1,2564499 | 1093,9772 | FLJ13448 | NM_025147 | Hypothetical protein FLJ13448 |
| 219398_at | 0,000925 | 0,94587404 | 234,78146 | 1,3453934 | 324,70908 | CIDEC | NM_022094 | Cell death-inducing DFFA-like effector c |
| 219402_s_at | 0,00261 | 1,0388852 | 851,3815 | 1,3082402 | 1066,2544 | DERL1 | NM_024295 | Der1-like domain family, member 1 |
| 219403_s_at | 0,000178 | 0,9960235 | 537,6556 | 1,4324621 | 769,34094 | HPSE | NM_006665 | Heparanase |
| 219405_at | 2,38E-10 | 0,97388417 | 358,86295 | 0,5896461 | 221,18637 | TRIM68 | NM_018073 | Tripartite motif-containing 68 |
| 219421_at | 9,80E-08 | 1,063306 | 179,1 | 0,65844357 | 111,56818 | OSRF | NM_012382 | Osmosis responsive factor |
| 219433_at | 1,85E-10 | 1,0099219 | 179,03334 | 0,5331972 | 97,59545 | BCOR | NM_017745 | BCL6 co-repressor |
| 219434_at | 0,000146 | 1,0219393 | 743,08154 | 1,5026741 | 1086,2227 | TREM1 | NM_018643 | Triggering receptor expressed on myeloid cells 1 |
| 219435_at | 0,00178 | 0,8949493 | 433,35928 | 0,66199106 | 309,0954 | FLJ22170 | NM_025099 | Hypothetical protein FLJ22170 |
| 219439_at | 6,64E-05 | 1,0445169 | 242,20743 | 0,7620308 | 178,77272 beta-galactosyltransferase, | C1GALT1 | NM_020156 | Core 1 synthase, glycoprotein-N-acetylgalactosamine 3-1 |
| 219467_at | 1,38E-12 | 0,9696246 | 329,06296 | 0,48073408 | 163,72273 | FLJ20125 | NM_017676 | Hypothetical protein FLJ20125 |
| 219470_x_at | 0,00085 | 0,9413462 | 100,14073 | 1,3309954 | 138,65454 | CCNJ | NM_019084 | Cyclin J |
| 219471_at | 0,00618 | 0,94459337 | 555,4222 | 0,6792895 | 399,28635 | C13orf18 | NM_025113 | Chromosome 13 open reading frame 18 |
| 219484_at | 7,02E-05 | 0,94561213 | 239,72964 | 0,7226179 | 183,54546 | HCFC2 | NM_013320 | Host cell factor C2 |
| 219487_at | 4,82E-08 | 0,9795641 | 102,05926 | 0,5742432 | 58,84091 | FLJ23560 | NM_024685 | Hypothetical protein FLJ23560 |
| 219495_s_at | 1,50E-07 | 0,90123254 | 128,1148 | 0,45250025 | 69,309105 | ZNF180 | NM_013256 | Zinc finger protein 180 (HHZ168) |
| 219497_s_at | 0,00224 | 0,8999188 | 554,87036 | 0,65320295 | 399,5773 | BCL11A | NM_022893 | B-cell CLL/lymphoma 11A (zinc finger protein) |
| 219512_at | 0,00327 | 1,0405792 | 98,96667 | 0,8232007 | 78,240906 | C20orf172 | NM_024918 | Chromosome 20 open reading frame 172 |
| 219528_s_at | 0,00078 | 0,95751464 | 1805,6443 | 0,70822334 | 1357,5364 | BCL11B | NM_022898 | B-cell CLL/lymphoma 11B (zinc finger protein) |
| 219529_at | 0,00101 | 0,94827515 | 812,98505 | 0,5028177 | 461,5545 | CLIC3 | NM_004669 | Chloride intracellular channel 3 |
| 219557_s_at | 0,00378 | 0,9299185 | 141,84073 | 1,1687505 | 179,88635 | NRIP3 | NM_020645 | Nuclear receptor interacting protein 3 |
| 219571_s_at | 0,000262 | 0,9462323 | 559,7333 | 0,67446613 | 394,0227 | GIOT-3 | NM_016265 | |
| 219582_at | 0,00078 | 0,97598267 | 471,4555 | 1,3679671 | 644,83636 | OGFRL1 | NM_024576 | Opioid growth factor receptor-like 1 |
| 219593_at | 4,82E-08 | 0,99901974 | 1071,0852 | 1,7140145 | 1838,9045 | SLC15A3 | NM_016582 | Solute carrier family 15, member 3 |
| 219622_at | 9,06E-12 | 1,0252545 | 575,8 | 2,5236757 | 1481,7545 | RAB20 | NM_017817 | RAB20, member RAS oncogene family |
| 219624_at | 5,94E-07 | 1,0284599 | 164,0815 | 1,5634792 | 253,91364 | BAG4 | NM_004874 | BCL2-associated athanogene 4 |
| 219628_at | 0,00861 | 1,1834346 | 505,73703 | 0,91662806 | 389,11816 | WIG1 | NM_022470 | P53 target zinc finger protein |
| 219634_at | 1,38E-12 | 0,9097049 | 646,4259 | 1,7332628 | 1192,3999 | CHST11 | NM_018413 | Carbohydrate (chondroitin 4) sulfotransferase 11 |
| 219646_at | 1,56E-09 | 0,92531693 | 293,9037 | 1,4686323 | 455,2545 | FLJ20186 | NM_017702 | Hypothetical protein FLJ20186 |
| 219648_at | 0,000288 | 0,929885 | 161,45555 | 0,7067711 | 123,13182 | FLJ10116 | NM_018000 | Whn-dependent transcript 2 |
| 219657_s_at | 0,000238 | 0,9160193 | 443,2074 | 1,2337514 | 599,9637 | KLF3 | NM_016531 | Kruppel-like factor 3 (basic) |
| 219667_s_at | 4,63E-09 | 1,1355135 | 858,17035 | 0,45923316 | 364,38635 | BANK1 | NM_017935 | B-cell scaffold protein with ankyrin repeats 1 |
| 219672_at | 0,000161 | 0,979692 | 141,30368 | 1,8171775 | 264,7045 | ERAF | NM_016633 | Erythroid associated factor |
| 219676_at | 3,75E-09 | 0,9935974 | 186,69627 | 0,6092371 | 115,41818 | ZNF435 | NM_025231 | Zinc finger protein 435 |
| 219678_x_at | 3,22E-05 | 0,92858297 | 964,11475 | 0,70690966 | 718,09546 | DCLRE1C | NM_022487 | DNA cross-link repair 1C (PSO2 homolog, S. cerevisiae) |
| 219691_at | 1,93E-07 | 0,99694103 | 365,8259 | 0,42974433 | 177,28183 | SAMD9 | NM_017654 | Sterile alpha motif domain containing 9 |
| 219698_s_at | 0,000216 | 0,95881957 | 349,99258 | 0,7534088 | 271,88635 | METTL4 | NM_022840 | Methyltransferase like 4 |
| 219700_at | 0,00706 | 0,9479272 | 272,29633 | 0,71144694 | 213,13635 | PLXDC1 | NM_020405 | Plexin domain containing 1 |
| 219724_s_at | 1,59E-05 | 0,95262086 | 375,64447 | 0,63158613 | 245,68637 | KIAA0748 | NM_014796 | |
| 219734_at | 0,000206 | 0,9795533 | 658,0704 | 0,69616073 | 479,11813 | SIDT1 | NM_017699 | SID1 transmembrane family, member 1 |
| 219765_at | 5,78E-06 | 0,94787914 | 192,47778 | 0,57739174 | 119,64091 | FLJ12586 | NM_024620 | Hypothetical protein FLJ12586 |
| 219767_s_at | 2,02E-05 | 0,9938313 | 300,3593 | 0,7629027 | 229,18637 | CRYZL1 | NM_005111 | Crystallin, zeta (quinone reductase)-like 1 |
| 219774_at | 7,81E-05 | 1,0156864 | 194,98518 | 1,3517979 | 254,6 | FLJ10996 | NM_019044 | Hypothetical protein FLJ10996 |
| 219777_at | 2,52E-14 | 0,9815428 | 2209,0706 | 0,34466344 | 801,7909 | GIMAP6 | NM_024711 | GTPase, IMAP family member 6 |
| 219797_at | 0,000382 | 0,94749326 | 190,92221 | 1,3143735 | 263,32272 | MGC52110 | NM_012214 | Hypothetical protein MGC52110 |
| 219802_at | 0,00948 | 0,9927584 | 514,54443 | 0,7910836 | 411,74997 | FLJ22028 | NM_024854 | Hypothetical protein FLJ22028 |
| 219812_at | 3,34E-08 | 0,95679945 | 1404,0853 | 0,56918174 | 853,45905 | STAG3 | NM_024070 | Stromal antigen 3 |
| 219819_s_at | 1,17E-07 | 0,9466108 | 448,437 | 0,5620682 | 272,8909 | MRPS28 | NM_014018 | Mitochondrial ribosomal protein S28 |
| 219822_at | 0,000925 | 1,0370673 | 243,45184 | 0,71032065 | 178,13182 | MTRF1 | NM_004294 | Mitochondrial translational release factor 1 |
| 219843_at | 0,00661 | 1,0249857 | 156,27408 | 0,7944684 | 115,52273 | IPP | NM_005897 | Intracisternal A particle-promoted polypeptide |
| 219859_at | 0,00948 | 0,96632934 | 168,38148 | 1,2235607 domain) | 208,88635 | CLECSF9 | NM_014358 | C-type (calcium dependent, carbohydrate-recognition lectin, superfamily member 9 |
| 219870_at | 0,00129 | 0,9345948 | 371,6148 | 0,58804005 | 246,15 | ATF71P2 | NM_024997 | Activating transcription factor 7 interacting protein 2 |
| 219913_s_at | 1,43E-10 | 0,99256504 | 369,0852 | 0,48074198 | 174,01817 | CRNKL1 | NM_016652 | Crn, crooked neck-like 1 (Drosophila) |
| 219933_at | 6,59E-06 | 0,9607296 | 379,2074 | 0,70592 | 279,45908 | GLRX2 | NM_016066 | Glutaredoxin 2 |
| 219938_s_at | 3,38E-06 | 0,9461814 | 604,97406 | 1,3687038 | 900,94995 | PSTPIP2 | NM_024430 | Proline-serine-threonine phosphatase interacting protein 2 |
| 219940_s_at | 1,05E-08 | 1,0170548 | 1033,2073 | 0,7039952 | 720,4637 | FLJ11305 | NM_018386 | Hypothetical protein FLJ11305 |
| 219956_at | 0,00304 | 0,9795832 | 529,0333 | 1,2301977 | 668,69995 | GALNT6 | NM_00721 | UDP-N-acetyl-alpha-D-galactosamine:polypeptide N-acetylgalactosaminyltransferase 6 (GalNAc-T6) |
| 219972_s_at | 0,000125 | 1,0335917 | 139,44814 | 0,7294844 | 98,98637 | C14orf135 | NM_022495 | Chromosome 14 open reading frame 135 |
| 219979_s_at | 0,000187 | 1,0434484 | 279,21112 | 0,76705265 | 203,09093 | HSPC138 | NM_016401 | Hypothetical protein HSPC138 |
| 220001_at | 0,00152 | 0,86954665 | 586,99243 | 1,2951592 | 893,6409 | PADI4 | NM_012387 | Peptidyl arginine deiminase, type IV |
| 220019_s_at | 0,000502 | 0,98351973 | 208,52963 | 0,7729348 | 161,5409 | ZNF224 | NM_005774 | synonyms: BMZF2, BMZF-2, ZNF255; zinc finger 2, bone marrow; zinc finger protein 255; ZNF224 [amino acids 1-78]; Homo sapiens zinc finger protein 224; Zinc finger protein ZNF255; go_component: nucleus [goid 0005634] [evidence IEA]; go_function: DNA bindin |
| 220038_at | 0,000317 | 1,0503317 | 442,88516 | 0,71652305 | 300,9273 | SGKL | NM_013257 | Serum/glucocorticoid regulated kinase-like |
| 220046_s_at | 6,59E-06 | 1,0246 | 2468,352 | 1,4322948 | 3481,732 | CCNL1 | NM_020307 | Cyclin L1 |
| 220054_at | 0,00192 | 0,9557633 | 242,99998 | 0,6861997 | 170,90907 | IL23A | NM_016584 | Interleukin 23, alpha subunit p19 |
| 220059_at | 2,02E-05 | 1,0543619 | 388,58517 | 0,61031854 | 239,85 | BRDG1 | NM_012108 | BCR downstream signaling 1 |
| 220068_at | 2,27E-05 | 1,011242 | 401,9555 | 0,6273987 | 256,67273 | VPREB3 | NM_013378 | Pre-B lymphocyte gene 3 |
| 220088_at | 0,00352 | 0,9472319 | 1923,4442 | 1,3160728 | 2727,8816 | C5R1 | NM_001736 | Complement component 5 receptor 1 (C5a ligand) |
| 220104_at | 1,46E-06 | 1,0146683 | 284,31848 | 1,4882035 | 424,74548 | ZC3HAV1 | NM_020119 | Zinc finger CCCH type, antiviral 1 |
| 220140_s_at | 0,000196 | 0,9562741 | 444,09256 | 0,6912071 | 333,39545 | SNX11 | NM_013323 | Sorting nexin 11 |
| 220146_at | 0,000382 | 0,9737363 | 265,1926 | 0,5357102 | 166,74544 | TLR7 | NM_016562 | Toll-like receptor 7 |
| 220158_at | 0,00807 | 0,7948107 | 122,71111 | 1,1197835 | 163,1591 | LGALS14 | NM_020129 | Lectin, galactoside-binding, soluble, 14 |
| 220162_s_at | 0,00406 | 0,9595879 | 257,28888 | 1,3374873 | 336,31815 | CARD9 | NM_022352 | |
| 220169_at | 9,64E-05 | 1,0548816 | 167,95186 | 0,6250302 | 102,6909 | FLJ23235 | NM_024943 | Hypothetical protein FLJ23235 |
| 220175_s_at | 0,00352 | 0,9842291 | 459,75192 | 0,7391539 | 350 | CBWD1 | NM_020667 | COBW-like protein; Homo sapiens COBW domain containing 1 (CBWD1), mRNA. |
| 220206_at | 4,44E-06 | 0,96224433 | 185,21854 | 0,6845847 | 129,12727 | ZMYM1 | NM_024772 | Zinc finger, MYM domain containing 1 |
| 220235_s_at | 1,26E-06 | 0,9519366 | 371,55927 | 0,49143127 | 188,18181 | RIF1 | NM_018372 | Receptor-interacting factor 1 |
| 220239_at | 5,07E-06 | 0,99340415 | 209,91853 | 1,3936286 | 290,4 | KLHL7 | NM_018846 | Kelch-like 7 (Drosophila) |
| 220242_x_at | 0,00806 | 1,0012758 | 142,40741 | 0,7556662 | 105,40909 | FLJ10891 | NM_018260 | Hypothetical protein FLJ10891 |
| 220244_at | 0,00216 | 0,9592909 | 167,83704 | 1,3066884 | 231,7091 | LOH3CR2A | NM_013343 | Loss of heterozygosity, 3, chromosomal region 2, gene A |
| 220252_x_at | 2,75E-06 | 0,95412946 | 224,83702 | 0,5409334 | 133,00453 | CXorf21 | NM_025159 | Chromosome X open reading frame 21 |
| 220255_at | 1,25E-06 | 0,9701917 | 172,2926 | 0,57608384 | 107,100006 | FANCE | NM_021922 | Fanconi anemia, complementation group E |
| 220288_at | 0,000348 | 0,916019 | 116,7037 | 1,3080934 | 164,73636 | MYO15A | NM_016239 | Myosin XVA |
| 220299_at | 0,000145 | 0,9391208 | 111,59259 | 1,3446218 | 154,55 | SPATA6 | NM_019073 | Spermatogenesis associated 6 |
| 220306_at | 6,92E-07 | 1,0511514 | 418,95554 | 2,1918344 | 854,1909 | FAM46C | NM_017709 | Family with sequence similarity 46, member C |
| 220352_x_at | 0,00502 | 0,9879039 | 409,07037 | 0,7602571 | 312,13177 | MGC4278 | NM_024305 | |
| 220375_s_at | 0,00365 | 0,95471275 | 235,94072 | 1,2529087 | 319,56818 | | NM_024752 | |
| 220391_at | 1,89E-08 | 1,0067061 | 71,07407 | 0,4950268 | 37 | ZBTB3 | | NM_024784 Zinc finger and BTB domain containing 3 |
| 220418_at | 1,94E-06 | 1,0014696 | 389,88516 | 0,5124175 | 196,52275 | UBASH3A | NM_018961 | Ubiquitin associated and SH3 domain containing, A |
| 220439_at | 0,000289 | 1,0091834 | 229,92964 | 1,3472735 | 308,06818 | FLJ11700 | NM_024892 | |
| 220446_s_at | 9,65E-05 | 0,95718277 | 174,23705 | 1,278049 | 232,3273 | CHST4 | NM_005769 | Carbohydrate (N-acetylglucosamine 6-O) sulfotransferase 4 |
| 220577_at | 0,000145 | 0,9547075 | 827,2518 | 0,6653034 | 606,15906 | FLJ13373 | NM_025006 | |
| 220643_s_at | 8,58E-09 | 0,96347755 | 102,892586 | 0,5582666 | 58,113636 | FAIM | NM_018147 | Fas apoptotic inhibitory molecule |
| 220646_s_at | 1,10E-05 | 0,95359355 | 2610,8777 | 0,46396527 | 1336,3546 | KLRF1 | NM_016523 | Killer cell lectin-like receptor subfamily F, member 1 |
| 220647_s_at | 1,90E-05 | 1,0075157 | 437,95926 | 0,7901082 | 342,3818 | E2IG2 | NM_016565 | E2IG2 protein |
| 220671_at | 0,00577 | 0,9216473 | 198,67409 | 1,1875453 | 242,3682 | CCRN4L | NM_012118 | CCR4 carbon catabolite repression 4-like (S. cerevisiae) |
| 220702_at | 0,00468 | 1,0966281 | 173,43332 | 1,602644 | 231,5091 | PRO2037 | NM_018616 | |
| 220712_at | 5,02E-13 | 1,0427876 | 372,82968 | 2,1352518 | 772,2863 | | NM_024984 | |
| 220748_s_at | 1,09E-06 | 0,9827987 | 476,1852 | 1,6090846 | 772,65 | ZNF580 | NM_016202 | Zinc finger protein 580 |
| 220760_x_at | 5,65E-05 | 1,009885 | 613,0297 | 0,7795251 | 474,97272 | FLJ14345 | NM_024733 | Hypothetical protein FLJ14345 |
| 220761_s_at | 4,63E-09 | 0,95253533 | 1208,9703 | 0,59263295 | 739,6773 | RPS2 | NM_016281 | TAO kinase 3 |
| 220933_s_at | 1,59E-05 | 0,96909165 | 932,91113 | 0,67612803 PAPD6 | 646,2545 | ZCCHC6; | NM_024617 | synonym: PAPD6; PAP associated domain containing 6; go_component: nucleus [goid 0005634] [evidence IEA]; go_function: zinc ion binding [goid 0008270] [evidence IEA]; go_function: nucleic acid binding [goid 0003676] [evidence IEA]; go_function: nucleotidyl |
| 220939_s_at | 1,59E-05 | 0,96541727 | 1891,111 | 0,6680539 | 1349,6454 | DPP8 | NM_017743 | Dipeptidylpeptidase 8 |
| 220942_x_at | 2,95E-06 | 0,9977583 | 1614,4888 | 0,78985053 | 1278,55 | E2IG5 | NM_014367 | Growth and transformation-dependent protein |
| 220944_at | 0,00152 | 0,8773982 | 183,00368 | 1,3375987 | 258,02728 | PGLYRP4 | NM_020393 | Peptidoglycan recognition protein 4 |
| 220953_s_at | 6,90E-08 | 1,0494795 | 231,23334 | 1,4393587 | 316,78635 | PIP3AP | NM_019061 | Phosphatidylinositol-3-phosphate associated protein |
| 220987_s_at | 0,000216 | 0,9894628 | 1060,2853 | 1,2412504 | 1328,6001 | SNARK | NM_030952 | Likely ortholog of rat SNF1/AMP-activated protein kinase |
| 220992_s_at | 2,87E-11 | 0,9002243 | 198,51851 | 0,37388432 | 83,87272 | C1orf25 | NM_030934 | Chromosome 1 open reading frame 25 |
| 220999_s_at | 0,00352 | 0,9671588 | 1359,8074 | 0,75725824 [goid | 1065,0863 | CYFIP2 | NM_030778 | synonyms: PIR121, PRO1331; go_component: extracellular 0005576] [evidence IEA]; go_function: chemokine activity [goid 0008009] [evidence IEA]; go_process: immune response [goid 0006955] [evidence IEA]; Homo sapiens cytoplasmic FMR1 interacting prote |
| 221004_s_at | 0,000216 | 0,93837446 | 385,98148 | 0,6499519 | 293,54544 | ITM2C | NM_030926 | Integral membrane protein 2C |
| 221011_s_at | 0,000715 | 0,98070395 | 2494,4185 | 0,6865168 | 1746,3817 | LBH | NM_030915 | Likely ortholog of mouse limb-bud and heart gene |
| 221014_s_at | 1,24E-05 | 1,0127474 | 291,09262 | 0,5820222 | 172,73637 | RAB33B | NM_031296 | RAB33B, member RAS oncogene family |
| 221020_s_at | 1,24E-05 | 1,018848 | 478,36664 | 0,7352112 | 353,73636 | MFTC | NM_030780 | Mitochondrial folate transporter/carrier |
| 221027_s_at | 0,00661 | 0,99204427 | 153,80742 | 0,71427935 | 116,05909 | PLA2G12A | NM_030821 | Phospholipase A2, group XIIA |
| 221081_s_at | 6,71E-12 | 1,0520518 | 1881,7148 | 0,4871785 | 854,6454 | FLJ22457 | NM_024901 | Hypothetical protein FLJ22457 |
| 221193_s_at | 4,93E-12 | 0,94683623 | 326,8259 | 0,50601643 | 173,49544 | ZCCHC10 | NM_017665 | Zinc finger, CCHC domain containing 10 |
| 221208_s_at | 2,95E-06 | 1,0547234 | 187,94444 | 0,63679385 | 115,61364 | FLJ23342 | NM_024631 | Hypothetical protein FLJ23342 |
| 221211_s_at | 0,00085 | 1,0132638 | 973,77783 | 0,60756016 | 625,14105 | C21orf7 | NM_020152 | Chromosome 21 open reading frame 7 |
| 221213_s_at | 0,00261 | 0,91706425 | 145,41853 | 0,63042855 | 102,98182 | SUHW4 | NM_017661 | Suppressor of hairy wing homolog 4 (Drosophila) |
| 221230_s_at | 0,00119 | 1,0392396 | 1368,674 | 0,81248313 | 1070,141 | ARID4B | NM_016374 | AT rich interactive domain 4B (RBP1- like) |
| 221234_s_at | 1,24E-05 | 1,0342406 | 539,026 | 0,5870765 | 310,91364 | BACH2 | NM_021813 | BTB and CNC homology 1, basic leucine zipper transcription factor 2 |
| 221253_s_at | 0,000348 | 1,035757 | 1441,9109 | 0,7921406 | 1241,8682 | TXNDC5 | NM_030810 | Thioredoxin domain containing 5 |
| 221257_x_at | 0,00755 | 0,9567934 | 650,3778 | 0,74495447 | 482,98636 | FBXO38 | NM_030793 | F-box protein 38 |
| 221277_s_at | 2,71E-05 | 0,9674321 | 305,15927 | 0,68379694 | 217,32724 | PUS3 | NM_031307 | Pseudouridylate synthase 3 |
| 221290_s_at | 0,00261 | 1,0146319 | 121,19257 | 0,7457884 | 89,39091 | MUM1 | NM_016473 | synonyms: MUM-1, HSPC211, FLJ14868, FLJ22283; go_component: cellular_component unknown [goid 0008372] [evidence ND]; go_function: molecular_function unknown [goid 0005554] [evidence ND]; go_process: biological_process unknown [goid 0000004] [evidence ND]; |
| 221311_x_at | 5,34E-05 | 0,968548 | 253,84447 | 0,72814 | 188,75908 | DJ122O8.2 | NM_020466 | Hypothetical protein dJ122O8.2 |
| 221432_s_at | 0,00406 | 0,98672056 | 628,47046 | 1,2387022 | 796,0454 | SLC25A28 | NM_031212 | Solute carrier family 25, member 28 |
| 221443_x_at | 0,00617 | 0,90513766 | 133,14816 | 1,1601841 | 169,86816 | PRLH | NM_015893 | Prolactin releasing hormone |
| 221475_s_at | 6,30E-05 | 0,9399626 | 15977,292 | 0,71678185 | 11995,382 | RPL15 | NM_002948 | Ribosomal protein L15 |
| 221477_s_at | 0,000925 | 0,98989797 | 1058,663 | 1,3368893 | 1480,7455 | | BF575213 | HepG2 3' region Mbol cDNA, clone hmd2a08m3. |
| 221493_at | 0,0006 | 1,0176287 | 1372,6368 | 0,7806686 | 1057,5317 | TSPYL1 | AL136629 | TSPY-like 1 |
| 221505_at | 0,00755 | 1,0979352 | 2093,5188 | 0,87283087 | 1644,3456 | ANP32E | AW612574 | Acidic (leucine-rich) nuclear phosphoprotein 32 family, member E |
| 221514_at | 8,99E-08 | 0,97571313 | 339,7111 | 0,69334817 (yeast) | 240,73184 | UTP14A | BC001149 | UTP14, U3 small nucleolar ribonucleoprotein, homolog A |
| 221517_s_at | 0,00618 | 1,0234634 | 423,7148 | 0,7557618 | 323,41818 | CRSP6 | AF105421 | Cofactor required for Sp1 transcriptional activation, subunit 6,77kDa |
| 221522_at | 0,000459 | 0,98642975 | 594,0481 | 0,7692124 | 459,50458 | ANKRD27 | AL136784 | Ankyrin repeat domain 27 (VPS9 domain) |
| 221523_s_at | 0,000178 | 0,9998179 | 194,59262 | 1,3221358 | 263,8909 | RAGD | AL138717 | |
| 221531_at | 6,59E-06 | 1,025461 | 498,1482 | 0,75789756 | 369,26364 | REC14 | AF309553 | Recombination protein REC14 |
| 221534_at | 0,00261 | 0,9465874 | 801,0704 | 0,7569526 | 638,52734 | Bles03 | AF073483 | Basophilic leukemia expressed protein BLES03 |
| 221539_at | 0,00378 | 0,98761785 | 582,99634 | 1,2538207 | 749,02734 | EIF4EBP1 | AB044548 | Eukaryotic translation initiation factor 4E binding protein 1 |
| 221541_at | 0,000119 | 0,9446591 | 1272,2407 | 1,4340246 | 1918,7502 | DKFZP434B 044 | AL136861 | Hypothetical protein DKFZp434B044 |
| 221556_at | 2,87E-05 | 0,9153534 | 344,37778 | 0,6053534 | 225,53183 | CDC14B | AU145941 | CDC14 cell division cycle 14 homolog B (S. cerevisiae) |
| 221558_s_at | 9,65E-05 | 0,9536261 | 3078,4336 | 0,641966 | 2111,1504 | LEF1 | AF288571 | Lymphoid enhancer-binding factor 1 |
| 221559_s_at | 2,87E-05 | 1,0023057 | 359,32224 | 0,6444981 | 221,78636 | MIS12 | BC000229 | MIS12 homolog (yeast) |
| 221563_at | 1,17E-07 | 0,9876433 | 347,24442 | 1,6281149 | 575,4818 | DUSP10 | N36770 | Dual specificity phosphatase 10 |
| 221565_s_at | 5,65E-05 | 1,0335852 | 1140,7555 | 0,72200096 | 786,3819 | FAM26B | BC000039 | Family with sequence similarity 26, member B |
| 221568_s_at | 0,000715 | 1,0005845 | 504,02966 | 0,679941 | 345,0909 | LIN7C | AF090900 | Lin-7 homolog C (C. elegans) |
| 221571_at | 5,06E-06 | 0,9881042 | 321,3593 | 0,6815376 | 222,72272 | TRAF3 | AI721219 | as68b11.x1 Barstead colon HPLRB7 Homo sapiens cDNA clone IMAGE:2333853 3', mRNA sequence. |
| 221589_s_at | 4,63E-09 | 0,9380031 | 259,90744 | 0,61934716 | 173,62273 | ALDH6A1 | AF130089 | Aldehyde dehydrogenase 6 family, member A1 |
| 221593_s_at | 0,000289 | 1,0166669 | 833,7704 | 0,801035 | 660,2546 | RPL31 | BC001663 | Ribosomal protein L31 |
| 221596_s_at | 0,00485 | 0,90348154 | 251,19632 0523 | 0,6224942 | 163,32729 | DKFZP564O | AL136619 | Hypothetical protein DKFZp564O0523 |
| 221601_s_at | 9,81 E-08 | 0,9757202 | 5364,0703 | 0,6053985 | 3368,041 | TOSO | AI084226 | Regulator of Fas-induced apoptosis |
| 221602_s_at | 3,71 E-07 | 1,0303556 | 2716,8376 | 0,6382332 | 1705,4501 | TOSO | AF057557 | Regulator of Fas-induced apoptosis |
| 221616_s_at | 0,000113 | 1,0353316 | 170,53337 | 1,3239576 (TBP)-associated | 220,20001 | TAF9L | AF077053 | TAF9-like RNA polymerase II, TATA box binding protein factor, 31 kDa |
| 221619_s_at | 2,27E-05 | 0,9146181 | 4648,3735 | 1,1704072 | 5890,0635 | MTCH1 | AF189289 | Mitochondrial carrier homolog 1 (C. elegans) |
| 221622_s_at | 9,36E-07 | 0,98625755 | 1540,5371 | 0,7687172 | 1188,3772 | HT007 | AF246240 | Uncharacterized hypothalamus protein HT007 |
| 221626_at | 1,09E-06 | 0,9498119 | 173,15553 | 0,586838 | 111,40454 | ZNF506 | AL136548 | Zinc finger protein 506 |
| 221637_s_at | 2,69E-07 | 1,0112654 | 628,4 | 0,7291386 | 454,58182 | MGC2477 | BC001434 | Hypothetical protein MGC2477 |
| 221645_s_at | 0,000238 | 1,0612996 | 311,85556 | 0,6726328 | 196,09091 | ZNF83 | M27877 | Zinc finger protein 83 (HPF1) |
| 221648_s_at | 0,000227 | 0,973139 | 325,69263 | 0,7129281 | 234,38182 | PNAS-4 | AK025651 | CGI-146 protein |
| 221651_x_at | 3,88E-06 | 1,0553 | 14163,74 | 0,58122855 | 8642,6045 | IGKC | BC005332 | Immunoglobulin kappa constant |
| 221652_s_at | 9,97E-10 | 1,0174454 | 426,56668 | 0,6356352 | 266,71362 | C12orf11 | AF274950 | Chromosome 12 open reading frame 11 |
| 221654_s_at | 1,09E-06 | 1,0219268 | 2260,3967 | 1,3743308 | 3049,9368 | USP3 | AF077040 | Ubiquitin specific protease 3 |
| 221671_x_at | 2,23E-06 | 1,0026699 | 14210,46 | 0,52689755 | 8358,081 | IGKC | M63438 | Immunoglobulin kappa constant |
| 221675_s_at | 0,000119 | 0,97786856 | 1071,9888 | 1,2458723 | 1360,9545 | CHPT1 | AF195624 | Choline phosphotransferase 1 |
| 221688_s_at | 3,75E-09 | 1,0331894 | 1206,6777 | 0,7123414 | 820,17267 | C15orf12 | AL136913 | Chromosome 15 open reading frame 12 |
| 221691_x_at | 1,10E-05 | 1,0515859 | 4384,5215 | 0,7185542 | 2937,927 | NPM1 | AB042278 | Nucleophosmin (nucleolar phosphoprotein B23, numatrin) |
| 221695_s_at | 0,00216 | 1,0430433 | 231,2074 | 1,4076165 | 303,73636 | MAP3K2 | AF239798 | Mitogen-activated protein kinase kinase kinase 2 |
| 221727_at | 5,06E-06 | 0,9856307 | 583,16675 | 1,4583181 | 875 | PC4 | BF209507 | Activated RNA polymerase II transcription cofactor 4 |
| 221736_at | 0,000178 | 1,0347087 | 749,2408 | 0,7998747 | 574,86365 | KIAA1219 | BG236163 | KIAA1219 protein |
| 221740_x_at | 0,00224 | 0,9819438 | 231,16296 | 0,75680995 | 176,8409 | KIAA0563 | BG249885 | 602319218F1 NIH_MGC_89 Homo sapiens cDNA clone IMAGE:4414397 5', mRNA sequence. |
| 221744_at | 1,24E-05 | 1,1727748 | 590,0074 | 0,76890016 | 367,5091 | HAN11 | AK026008 | WD-repeat protein |
| 221749_at | 0,0014 | 0,9614034 | 776,6703 | 0,73419714 | 586,7999 | YTHDF3 | AU157915 | YTH domain family, member 3 |
| 221751_at | 0,000289 | 0,9763038 | 930,29254 | 0,7090651 | 691,0364 | PANK3 | AL565516 | Pantothenate kinase 3 |
| 221753_at | 0,00618 | 0,91695577 | 551,0407 | 1,1536682 | 698,0091 | SSH1 | AB037719 | Slingshot homolog 1 (Drosophila) |
| 221755_at | 3,34E-08 | 0,99736 | 687,9111 | 1,5357786 | 1068,4546 | EHBP1L1 | BG334196 | EH domain binding protein 1-like 1 |
| 221763_at | 0,00224 | 0,99819094 | 711,89636 | 0,7485859 | 552,0545 | JMJD1C | AI694023 | Jumonji domain containing 1C |
| 221768_at | 0,000107 | 1,060998 | 616,9112 | 1,5191739 binding | 898,1227 | SFPQ | AV705803 | Splicing factor proline/glutamine rich (polypyrimidine tract protein associated) |
| 221771_s_at | 2,28E-07 | 0,9972157 | 1034,2482 | 0,7024498 | 734,49097 | HSMPP8 | BC003542 | M-phase phosphoprotein, mpp8 |
| 221778_at | 2,87E-05 | 1,006926 | 842,85187 | 1,3624738 | 1122,9093 | KIAA1718 | BE217882 | KIAA1718 protein |
| 221782_at | 0,000153 | 0,95238835 | 280,95926 | 0,7065126 | 209,96362 | DNAJC10 | AL137648 | DnaJ (Hsp40) homolog, subfamily C, member 10 |
| 221786_at | 2,14E-05 | 0,9775093 | 358,57776 | 0,70201707 | 252,54546 | PHF10 | AF055030 | PHD finger protein 10 |
| 221787_at | 1,09E-06 | 0,98143244 | 418,34076 | 0,7129803 | 301,8364 | PHF10 | AF055030 | PHD finger protein 10 |
| 221790_s_at | 8,24E-08 | 1,0109086 | 1217,311 | 0,56670934 | 707,9546 | ARH | AL545035 | LDL receptor adaptor protein |
| 221802_s_at | 2,02E-05 | 1,0470706 | 438,2741 | 1,5970887 | 625,40454 | KIAA1598 | AU157109 | KIAA1598 |
| 221803_s_at | 1,56E-08 | 0,9699217 | 386,7037 | 0,5823607 | 239,05453 | NRBF2 | AA883074 | Nuclear receptor binding factor 2 |
| 221842_s_at | 0,00178 | 0,981551 | 610,4371 | 0,73621905 IMAGE:3935546 | 459,56363 | ZNF131 | BE972394 | 601652210F1 NIH_MGC_82 Homo sapiens cDNA clone 5', mRNA sequence. |
| 221891_x_at | 1,46E-06 | 1,0948119 | 9795,333 | 0,75093204 | 6495,5005 | HSPA8 | AA704004 | Heat shock 70kDa protein 8 |
| 221897_at | 0,000146 | 0,9792422 | 630,45557 | 0,76605695 | 488,9546 | TRIM52 | AA205660 | LOC441121 |
| 221899_at | 5,06E-05 | 0,9233552 | 1454,0924 | 0,56323165 | 855,67267 | PFAAP5 | AI809961 | Phosphonoformate immuno-associated protein 5 |
| 221918_at | 0,00224 | 1,0764846 | 892,3 | 0,7688209 | 653,9137 | PCTK2 | AI742210 | wg39c02.x1 Soares_NSF_F8_9W_OT_PA_P_S1 Homo sapiens cDNA clone IMAGE:2367458 3', mRNA sequence. |
| 221919_at | 1,59E-05 | 0,9923398 | 248,45184 | 1,5285025 | 381,5409 | HNRPA1 | AW450929 | Heterogeneous nuclear ribonucleoprotein A1 |
| 221920_s_at | 3,88E-06 | 1,0342841 | 302,18146 | 1,8200713 | 542,13635 | MSCP | BE677761 | Mitochondrial solute carrier protein |
| 221931_s_at | 2,77E-08 | 0,9939618 | 505,5297 | 0,67579955 | 344,39093 | SEH1L | AV701173 | SEH1-like (S. cerevisiae) |
| 221935_s_at | 0,0078 | 0,9440627 | 93,977776 MGC34132 | 0,6779349 | 68,6 | AER61; | AK023140 | unnamed protein product; Homo sapiens cDNA FLJ13078 fis, clone NT2RP3002002. |
| 221937_at | 7,01E-09 | 0,9781909 | 1981,9928 | 0,6302172 | 1286,8999 | | AI472320 | CDNA FLJ34482 fis, clone HLUNG2004067 |
| 221943_x_at | 0,000303 | 1,0035553 | 826,2408 | 1,5006375 | 1245,8318 | RPL38 | AW303136 | Ribosomal protein L38 |
| 221957_at | 2,56E-05 | 0,94551903 | 434,2259 | 1,227751 | 561,4727 | PDK3 | BF939522 | Pyruvate dehydrogenase kinase, isoenzyme 3 |
| 221960_s_at | 3,39E-06 | 0,8517507 | 135,90741 | 1,5125351 | 220,60909 | RAB2 | AI189609 | RAB2, member RAS oncogene family |
| 221970_s_at | 5,65E-05 | 0,9382445 | 932,4185 0724 | 0,6748253 | 653,2319 | DKFZP586L | AU158148 | DKFZP586L0724 protein |
| 221985_at | 0,000317 | 0,9458284 | 306,70737 | 1,2121673 | 393,16367 | DRE1 | AW006750 | DRE1 protein |
| 221989_at | 1,28E-08 | 1,0123909 | 1191,5741 | 1,6061486 | 1856,032 | RPL10 | AW057781 | Ribosomal protein L10 |
| 222014_x_at | 2,51E-08 | 0,95190233 | 491,22968 | 0,6207768 | 319,75452 | MTO1 | AI249752 | Mitochondrial translation optimization 1 homolog (S. |
| 222018_at | 2,27E-05 | 1,0078844 | 433,86295 | 1,4389414 | 611,08636 | NACA | AI992187 | Nascent-polypeptide-associated complex alpha polypeptide |
| 222026_at | 0,0014 | 0,98716044 | 205,98888 | 1,2644788 IMAGE:3651572 | 259,17725 | | BF437591 | 7p74e11.x1 NCI_CGAP_Pr28 Homo sapiens cDNA clone 3' similar to contains element MSR1 repetitive element ;, mRNA sequence. |
| 222028_at | 8,67E-07 | 0,9305903 | 372,42218 | 0,6569972 | 260,73642 | ZNF45 | AI967981 | Zinc finger protein 45 |
| 222030_at | 4,01E-07 | 0,95607823 | 409,9296 | 1,576265 | 658,0045 | SIVA | AW024335 | CD27-binding (Siva) protein |
| 222038_s_at | 0,00105 | 0,93789804 | 65,07777 | 1,3436261 | 91,58182 | CGI-48 | AA993099 | CGI-48 protein |
| 222044_at | 1,11E-10 | 1,0159819 | 481,12595 | 2,0559561 | 933,25 | C20orf67 | AI199589 | Chromosome 20 open reading frame 67 |
| 222045_s_at | 3,04E-10 | 0,9223972 | 253,40369 | 1,5403495 | 414,42276 | C20orf67 | AI199589 | Chromosome 20 open reading frame 67 |
| 222046_at | 4,82E-08 | 1,0051119 | 217,09999 | 1,5886351 | 345,0409 | ARS2 | AI523895 | Arsenate resistance protein ARS2 |
| 222071_s_at | 0,00105 | 0,98327035 | 119,3 | 0,6379061 | 79,85454 | SLCO4C1 | BE552428 | Solute carrier organic anion transporter family, member 4C1 |
| 222077_s_at | 3,88E-06 | 1,0041872 | 193,60002 | 0,7113162 | 140,06819 | RACGAP1 | AU153848 | Rac GTPase activating protein 1 |
| 222082_at | 1,17E-05 | 0,8990702 | 145,1074 | 1,3773907 | 227,40001 | ZBTB7 | AI568395 | Zinc finger and BTB domain containing 7 |
| 222088_s_at | 3,04E-10 | 0,95695716 | 751,52594 | 2,1664824 | 1827,4729 | SLC2A14 | AA778684 | Solute carrier family 2 (facilitated glucose transporter), member 3 |
| 222099_s_at | 5,65E-05 | 0,97157663 | 909,1666 | 1,2394127 | 1135,1272 | C19orf13 | AW593859 | Chromosome 19 open reading frame 13 |
| 222103_at | 8,52E-11 | 1,0240723 | 765,0073 | 0,5417846 | 396,48636 | ATF1 | AI434345 | Activating transcription factor 1 |
| 222104_x_at | 0,000146 | 1,2239609 | 652,75183 | 0,79502565 | 398,79544 | GTF2H3 | AI569458 | General transcription factor IIH, polypeptide 3, 34kDa |
| 222108_at | 4,74E-06 | 0,9263751 | 594,33325 | 0,6139259 | 388,74997 | | AC004010 | Homo sapiens BAC clone GS1-99H8 from 12, complete sequence. |
| 222111_at | 0,00502 | 1,1565683 | 258,58145 | 0,77581686 | 165,02272 | KIAA1164 | AU145293 | Hypothetical protein KIAA1164 |
| 222113_s_at | 0,000419 | 0,95878255 | 232,32593 | 1,2617686 | 299,5545 | EPS15L1 | AV710549 | Epidermal growth factor receptor pathway substrate 15-like |
| 222115_x_at | 0,00618 | 0,9840996 | 206,80742 | 1,4537923 | 318,57272 | N-PAC | BC003693 | Cytokine-like nuclear factor n-pac |
| 222119_s_at | 0,000146 | 1,0458122 | 633,07776 | 0,8347668 | 499,43185 | FBXO11 | AL117620 | F-box protein 11 |
| 222122_s_at | 0,000348 | 0,95044327 | 611,1223 | 0,71631163 | 456,95456 | THOC2 | BG403671 | THO complex 2 |
| 222128_at | 3,41E-05 | 0,94725496 | 135,27405 MYSB; | 0,6107796 | 90,29091 | CACNB2; CACNLB2 | U80764 | Human EST clone 122887 mariner transposon Hsmar1 sequence. |
| 222140_s_at | 0,000317 | 1,0216029 | 329,15924 | 0,7748265 | 248,57726 | GPR89 | AK021758 | G protein-coupled receptor 89 |
| 222142_at | 0,000549 | 0,99462366 | 107,340744 | 1,4803754 | 164,57272 | CYLD | AK024212 | Cylindromatosis (turban tumor syndrome) |
| 222146_s_at | 2,56E-05 | 1,0090226 | 296,6 | 0,52052224 | 180,32274 | TCF4 | AK026674 | Transcription factor 4 |
| 222151_s_at | 0,00164 | 1,0183072 | 212,32225 | 0,8124914 | 167,78181 | Cep63 | AK023738 | Centrosome protein Cep63 |
| 222182_s_at | 0,000196 | 1,0018847 | 1065,8221 | 0,726081 | 758,19543 | CNOT2 | BG105204 | CCR4-NOT transcription complex, subunit 2 |
| 222186_at | 0,000131 | 1,085655 | 202,83333 | 1,637941 | 287,98633 | ZA20D3 | AL109684 | Zinc finger, A20 domain containing 3 |
| 222201_s_at | 1,57E-09 | 1,0120226 | 577,4074 | 0,5298148 | 315,61365 | CASP8AP2 | AB037736 | CASP8 associated protein 2 |
| 222204_s_at | 4,02E-08 | 0,95523936 | 318,95184 | 0,55763996 | 183,65909 | RRN3 | AL110238 | RRN3 RNA polymerase I transcription factor homolog |
| 222218_s_at | 0,000317 | 1,021603 | 2114,1965 | 1,3419237 | 2795,2454 | PILRA | AJ400843 | Paired immunoglobin-like type 2 receptor alpha |
| 222233_s_at | 0,000549 | 0,9489504 | 371,95557 | 0,6325771 | 253,71819 | DCLRE1C | AK022922 | DNA cross-link repair 1C (PSO2 homolog, S. cerevisiae) |
| 222239_s_at | 2,41 E-05 | 1,0346745 | 248,6259 | 0,6897221 | 167,72273 | DDX26 | AL117626 | DEAD/H (Asp-Glu-Ala-Asp/His) box polypeptide 26 |
| 222243_s_at | 1,46E-06 | 1,0162294 | 391,05188 | 0,7296069 | 278,68637 | TOB2 | AB051450 | Transducer of ERBB2, 2 |
| 222273_at | 2,69E-07 | 0,9910566 | 475,79254 | 0,58523375 KIAA1503 | 278,91818 | | AI419423 | Transcribed locus, weakly similar to XP_209041.2 protein [Homo sapiens] |
| 222286_at | 0,00178 | 0,959035 | 116,888885 | 0,72545815 | 89,21363 | PSIP1 | R43279 | PC4 and SFRS1 interacting protein 1 |
| 222297_x_at | 0,000289 | 1,0412589 | 582,3963 | 0,7793954 | 430,34998 | RPL18 | AV738806 | AV738806 CB Homo sapiens cDNA clone CBCAWB04 5', mRNA sequence. |
| 222303_at | 0,00502 | 1,142527 | 369,0852 | 1,7023728 | 552,25 | ETS2 | AV700891 | V-ets erythroblastosis virus E26 oncogene homolog 2 |
| 222309_at | 6,92E-07 | 1,0553595 | 131,3926 | 2,0497143 | 241,13637 | C6orf62 | AW972292 | Chromosome 6 open reading frame 62 |
| 222311_s_at | 0,00304 | 1,0818208 | 317,41483 | 1,385767 | 404,5364 | SFRS15 | AA648521 | Splicing factor, arginine/serine-rich 15 |
| 222317_at | 6,90E-08 | 1,0552909 | 176,86665 | 0,5327637 | 88,16818 | PDE3B | AA888858 | Phosphodiesterase 3B, cGMP-inhibited |
| 222343_at | 0,000145 | 0,88264066 | 64,103714 | 1,4338737 | 96,95911 | BCL2L11 | AA629050 | BCL2-like 11 (apoptosis facilitator) |
| 222366_at | 0,0014 | 1,0574574 | 297,85184 | 1,5193888 | 422,09998 | ADNP | W86781 | Activity-dependent neuroprotector |
| 266_s_at | 0,000779 | 0,9261026 | 173,15556 | 0,40023723 | 96,98637 | CD24 | L33930 | CD24 antigen (small cell lung carcinoma cluster 4 antigen) |
| 31799_at | 1,40E-05 | 0,9885121 | 84,744446 | 0,61822176 | 53,968185 | COPB2 | AF070618 | Coatomer protein complex, subunit beta 2 (beta prime) |
| 31861_at | 0,000925 | 0,8906598 | 702,13336 | 1,2040175 | 919,4091 | IGHMBP2 | L14754 | Immunoglobulin mu binding protein 2 |
| 32042_at | 5,06E-05 | 0,98632926 | 137,0778 | 0,75145483 | 105,281815 | COVA1 | S72904 | Cytosolic ovarian carcinoma antigen 1 |
| 32091_at | 3,71 E-07 | 1,0165236 | 371,36298 | 0,6921957 | 251,93637 | KIAA0446 | AB007915 | Start codon is not identified; Homo sapiens mRNA for KIAA0446 protein, partial cds. |
| 32137_at | 0,00134 | 0,925959 | 252,86296 | 1,2184486 | 321,70908 | JAG2 | AF029778 | Jagged 2 |
| 32540_at | 0,00806 | 0,8963047 | 116,87037 | 1,2138094 | 155,65456 | PPP3CC | AI762547 | Protein phosphatase 3 (formerly 2B), catalytic subunit, gamma isoform (calcineurin A gamma) |
| 32723_at | 4,37E-10 | 0,9798111 | 133,11479 | 0,49866307 | 69,74091 | CSTF1 | L02547 | Cleavage stimulation factor, 3' pre-RNA, subunit 1, 50kDa |
| 32837_at | 3,61 E-05 | 1,0146043 | 697,0259 | 1,3135179 (lysophosphatidic | 922,9364 | AGPAT2 | U56418 | 1-acylglycerol-3-phosphate O-acyltransferase 2 acid acyltransferase, beta) |
| 33323_r_at | 0,000549 | 0,8549321 | 358,4333 | 1,2808573 | 522,38635 | SFN | X57348 | H.sapiens mRNA (clone 9112). |
| 33760_at | 0,000549 | 1,0325748 | 286,45926 | 0,7983345 | 221,15002 | PEX14 | AB017546 | Peroxisomal biogenesis factor 14 |
| 34031_i_at | 3,42E-07 | 1,0280862 | 465,94446 | 0,59738356 | 272,78638 | CCM1 | U90268 | Cerebral cavernous malformations 1 |
| 34764_at | 1,56E-08 | 1,0289186 | 195,89261 | 0,731719 | 140,2682 | LARS2 | D21851 | Leucyl-tRNAsynthetase2,mitochondrial |
| 35201_at | 2,02E-05 | 0,9100278 | 1194,1704 | 1,3932315 | 1725,1501 | HNRPL | X16135 | Heterogeneous nuclear ribonucleoprotein L |
| 35617_at | 0,000925 | 1,0143932 | 354,13702 | 1,2750244 | 442,80905 | MAPK7 | U29725 | Mitogen-activated protein kinase 7 |
| 35626_at | 0,000107 | 0,9381477 | 1314,8334 | 1,2748448 | 1760,468 | SGSH | U30894 | N-sulfoglucosamine sulfohydrolase (sulfamidase) |
| 35820_at | 0,00101 | 1,0627797 | 821,1074 | 1,5718155 | 1259,1499 | GM2A | X62078 | |
| 35974_at | 6,58E-06 | 1,0324898 | 842,9963 | 0,7788173 | 636,85 | LRMP | U10485 | Lymphoid-restricted membrane protein |
| 36004_at | 4,05E-05 | 0,93682575 | 752,58887 | 1,2713679 | 1010,2591 | IKBKG | AF074382 | Inhibitor of kappa light polypeptide gene enhancer in B-cells, kinase gamma |
| 36564_at | 3,39E-06 | 0,89286673 | 685,7147 | 1,4414549 | 1097,5046 | IBRDC3 | W27419 | IBR domain containing 3 |
| 36711_at | 5,94E-07 | 1,1337453 | 209,50737 | 2,6679885 | 513,77734 | MAFF | AL021977 | |
| 36829_at | 3,54E-13 | 1,0339923 | 643,3815 | 3,106158 | 2030,7456 | PER1 | AF022991 | Period homolog 1 (Drosophila) |
| 36994_at | 8,06E-07 | 0,97171074 | 4872,36 ATPL; | 1,3406873 | 6660,845 | ATP6V0C; VATL; Vma3; ATP6C; ATP6L | M62762 | Human vacuolar H+ ATPase proton channel subunit mRNA, complete cds. |
| 37028_at | 2,57E-06 | 1,0102799 | 890,3 | 1,882551 | 1616,3818 | PPP1R15A | U83981 | Protein phosphatase 1, regulatory (inhibitor) subunit 15A |
| 37462_i_at | 0,00224 | 0,9579688 | 876,2889 PRP11; | 1,2050297 | 1073,4818 | SF3A2; SAP62; SF3a66 | L21990 | Human spliceosomal protein (SAP 62) gene, complete cds. |
| 37802_r_at | 0,00577 | 1,0623983 | 47,24814 | 0,7731897 | 34,427277 | KIAA1164 | AL049226 | Hypothetical protein KIAA1164 |
| 37943_at | 9,67E-06 | 0,9914892 | 282,0741 | 0,673818 | 196,97728 | ZFYVE26 | AB002319 | Zinc finger, FYVE domain containing 26 |
| 38037_at | 3,75E-09 | 0,9657956 | 149,9 | 3,8807583 | 582,7955 | HBEGF | M60278 | Heparin-binding EGF-like growth factor |
| 38069_at | 0,00224 | 0,9706154 | 1043,7925 | 1,2572064 | 1365,8727 | CLCN7 | Z67743 | Chloride channel 7 |
| 38241_at | 7,50E-06 | 0,9475194 | 999,5519 | 0,7080407 | 756,50006 | BTN3A3 | U90548 | Butyrophilin, subfamily 3, member A3 |
| 38290_at | 2,23E-06 | 0,95528924 | 589,3481 | 0,7381735 | 447,62274 | RGS14 | AF037195 | Regulator of G-protein signalling 14 |
| 38487_at | 3,22E-05 | 0,81727993 | 1355,9702 | 1,4123603 | 2224,686 | STAB1 | D87433 | Stabilin 1 |
| 38671_at | 1,40E-05 | 0,9254972 | 959,87775 | 1,2750006 | 1293,7772 | PLXND1 | AB014520 | Plexin D1 |
| 38710_at | 1,46E-06 | 0,9059503 | 1533,7261 | 1,3329948 | 2175,6272 | OTUB1 | AL096714 | OTU domain, ubiquitin aldehyde binding 1 |
| 39248_at | 0,00352 | 0,9984269 | 1643,8851 | 0,6784146 | 1113,0591 | AQP3 | N74607 | Aquaporin 3 |
| 39318_at | 0,000459 | 0,94540113 | 1156,9296 | 0,5654542 | 769,42725 | TCL1A | X82240 | T-cell leukemia/lymphoma 1A |
| 396_f_at | 0,00119 | 0,90082 | 1120,7186 | 1,3361112 | 1585,659 | EPOR | X97671 | H.sapiens mRNA for erythropoietin receptor. |
| 39729_at | 0,00282 | 1,0017112 | 1415,3851 | 0,72545505 | 1020,32733 | PRDX2; PRP; TSA; NKEFB; PRXII; TDPX1; MGC4104 | L19185 | Human natural killer cell enhancing factor (NKEFB) mRNA, complete cds. |
| 40016_g_at | 0,00421 | 1,0054731 | 376,3629 | 0,79581267 | 297,36816 | MAST4 | AB002301 | Microtubule associated serine/threonine kinase family member 4 |
| 40020_at | 8,51E-06 | 0,9719538 | 169,31851 | 1,2731009 (flamingo | 219,85457 | CELSR3 | AB011536 | Cadherin, EGF LAG seven-pass G-type receptor 3 homolog, Drosophila) |
| 40465_at | 1,94E-06 | 0,9315456 | 525,97406 | 0,6687986 | 382,43182 | DDX23 | AF026402 | DEAD (Asp-Glu-Ala-Asp) box polypeptide 23 |
| 40472_at | 1,26E-06 | 0,9439229 | 317,88885 | 0,5382518 | 201,55002 | LOC254531 | AF007155 | PLSC domain containing protein |
| 41113_at | 0,00392 | 0,91013646 | 245,27405 | 0,71600145 | 185,45 | ZNF500 | AI871396 | Zinc finger protein 500 |
| 41329_at | 2,95E-06 | 1,0021822 | 287,16666 | 0,6492427 | 186,84549 | PACE-1 | AI458463 | Ezrin-binding partner PACE-1 |
| 41386_i_at | 4,63E-09 | 0,9057915 | 1309,5963 | 1,4398783 | 2016,0092 | JMJD3 | AB002344 | Jumonji domain containing 3 |
| 41644_at | 1,94E-06 | 0,969307 | 363,5037 | 1,4529239 | 542,63635 | SASH1 | AB018333 | SAM and SH3 domain containing 1 |
| 41657_at | 0,00538 | 0,9095198 | 601,68896 | 1,1456872 | 739,1092 | STK11 | AF035625 | Serine/threonine kinase 11 (Peutz-Jeghers syndrome) |
| 44669_at | 2,23E-06 | 1,0146705 | 298,18152 | 0,7454862 | 220,39545 | | N31716 | Homo sapiens, clone IMAGE:3626627, mRNA |
| 44673_at | 0,00468 | 0,88836783 | 258,11853 | 1,7213346 | 716,80005 | SN | N53555 | Sialoadhesin |
| 44790_s_at | 0,000382 | 1,1248852 | 281,4593 | 0,7323341 | 185,70909 | C13orf18 | AI129310 | Chromosome 13 open reading frame 18 |
| 44822_s_at | 0,000131 | 0,9735904 | 228,90741 | 1,2319577 | 286,55457 | KIAA1193 | AW003889 | KIAA1193 |
| 45288_at | 4,15E-06 | 0,9094963 | 176,82591 | 0,5498452 | 109,431816 | ABHD6 | AA209239 | Abhydrolase domain containing 6 |
| 45749_at | 0,00261 | 0,9357578 | 1279,0481 | 1,2002382 | 1587,8318 | FLJ13725 | AA400206 | Hypothetical protein FLJ13725 |
| 46167_at | 2,77E-08 | 0,9748918 | 327,47778 | 0,6578149 Homo | 223,33635 | TTC4 | W22690 | 71 G4 Human retina cDNA Tsp509I-cleaved sublibrary sapiens cDNA not directional, mRNA sequence. |
| 46947_at | 5,65E-05 | 0,9847704 | 182,59999 | 1,3733356 | 253,17271 | GNL3L | T87245 | Guanine nucleotide binding protein-like 3 (nucleolar)-like |
| 48659_at | 0,000196 | 0,8938024 | 969,7296 | 1,2835926 | 1321,2091 | FLJ12438 | W60802 | Hypothetical protein FLJ12438 |
| 51146_at | 6,52E-11 | 0,9579665 | 153,58519 | 0,45127985 | 78,86819 | PIGV | AA203365 | Phosphatidylinositol glycan, class V |
| 52164_at | 1,56E-06 | 0,96211964 | 431,87778 | 0,72299343 | 322,53635 | C11orf24 | AA065185 | Chromosome 11 open reading frame 24 |
| 52285_f_at | 3,71 E-07 | 1,0507921 | 65,94445 | 0,5242322 | 34,945454 | C18orf9 | AW002970 | Chromosome 18 open reading frame 9 |
| 52731_at | 9,64E-05 | 0,9473489 | 204,55927 | 0,65741754 | 148,80908 | FLJ20294 | AI359466 | Hypothetical protein FLJ20294 |
| 53076_at | 0,000886 | 0,9803358 | 268,14075 | 0,7743524 (galactosyltransferase | 213,81818 | B4GALT7 | AI040029 | Xylosylprotein beta 1,4-galactosyltransferase, polypeptide 7 I) |
| 53912_at | 0,000549 | 1,0143963 | 904,12964 | 0,7223777 | 665,2136 | SNX11 | AI668643 | Sorting nexin 11 |
| 53968_at | 3,22E-05 | 0,99715143 | 498,27774 | 0,78929347 | 396,2 | KIAA1698 | AI869988 | KIAA1698 protein |
| 54037_at | 3,22E-05 | 0,9041169 | 88,103714 | 0,5534061 | 54,459095 | HPS4 | AL041451 | Hermansky-Pudlak syndrome 4 |
| 54051_at | 0,000138 | 0,9936506 | 79,60741 | 0,62519354 | 54,66818 | PKNOX1 | H59033 | yr40e10.r1 Soares fetal liver spleen 1 NFLS Homo sapiens cDNA clone IMAGE:207786 5', mRNA sequence. |
| 55692_at | 3,61E-05 | 0,9291582 | 786,6666 | 0,71173763 | 605,9318 | ELMO2 | W22924 | Engulfment and cell motility 2 (ced-12 homolog, C. elegans) |
| 56829_at | 9,67E-06 | 0,89788795 | 791,08514 | 1,2099435 | 1038,7726 | T1 | H61826 | Tularik gene 1 |
| 57082_at | 5,09E-07 | 1,0138378 | 2061,9114 | 0,5908414 | 1162,0272 | ARH | AA169780 | LDL receptor adaptor protein |
| 57516_at | 2,08E-06 | 0,99507403 | 148,73334 | 0,63180614 | 92,64089 | MGC13138 | AA746290 | Hypothetical protein MGC13138 |
| 57715_at | 5,78E-06 | 0,9882365 | 952,94073 | 0,6872404 | 663,709 | FAM26B | W72694 | Family with sequence similarity 26, member B |
| 59644_at | 0,000382 | 1,0269815 | 190,02596 | 0,69630796 | 134,97275 | BIKE | AI735391 | at10e09.x1 Barstead aorta HPLRB6 Homo sapiens cDNA clone IMAGE:2354728 3', mRNA sequence. |
| 59705_at | 0,0078 | 0,92690104 | 55,829636 | 1,1883523 | 70,43637 | SCLY | AA911739 | Selenocysteine lyase |
| 59999_at | 6,17E-06 | 1,0019906 | 345,5852 | 0,76727766 | 267,36816 | HIF1AN | W37897 | Hypoxia-inducible factor 1, alpha subunit inhibitor |
| 60084_at | 0,000227 | 1,0176169 | 112,37777 | 1,4834237 | 168,97273 | CYLD | AI453099 | Cylindromatosis (turban tumor syndrome) |
| 63009_at | 1,17E-07 | 1,0000279 | 286,8778 | 0,72121334 | 209,62727 | FLJ10539 | AI188402 | Hypothetical protein FLJ10539 |
| 64064_at | 2,02E-05 | 0,88774127 | 3031,4746 | 0,5005084 | 1602,8455 | GIMAP5 | AI435089 | GTPase, IMAP family member 5 |
| 64418_at | 1,22E-11 | 0,990528 | 1122,4553 | 0,6046487 | 691,2363 | | AI472320 | CDNA FLJ34482 fis, clone HLUNG2004067 |
| 64440_at | 2,27E-05 | 0,97642195 | 371,22964 | 1,4696413 | 547,75006 | IL17RC | AI560217 | Interleukin 17 receptor C |
| 64486_at | 0,00109 | 1,0370022 | 1388,615 | 1,3896616 | 1820,9911 | CORO1B | AI341234 | Coronin, actin binding protein, 1B |
| 64488_at | 0,00282 | 0,9857845 | 152,01114 | 1,322756 | 209,84544 | | AW003091 | CDNA FLJ38849 fis, clone MESAN2008936 |
| 65472_at | 6,92E-07 | 0,9969866 | 181,47035 | 0,61919105 | 115,795456 | | AI161338 | qb80a04.x1 Soares_fetal_heart_NbHH19W Homo sapiens cDNA clone IMAGE:1706382 3' similar to TR:O21123 O21123 CYTOCHROME OXIDASE I ;, mRNA sequence. |
| 65718_at | 0,000289 | 0,8184987 | 98,3 | 1,2997878 | 139,86363 | GPR124 | AI655903 | G protein-coupled receptor 124 |
| 74694_s_at | 5,07E-06 | 0,99987096 | 567,0518 | 0,7612715 | 429,68637 | FRA | AA907940 | oI24e02.s1 Soares_NFL_T_GBC_S1 Homo sapiens cDNA clone IMAGE:1524410 3' similar to contains TAR1.t1 TAR1 TAR1 repetitive element ;, mRNA sequence. |
| 78047_s_at | 2,69E-07 | 0,98559433 | 1114,474 | 1,3488567 | 1521,132 | MMP24 | AW001777 | Hypothetical LOC400843 |
| 89948_at | 1,11E-10 | 1,0150225 | 408,03333 | 1,7546769 | 697,4954 | C20orf67 | AI743331 | Chromosome 20 open reading frame 67 |
| 90265_at | 0,00129 | 1,024836 | 1419,8408 | 1,2831619 | 1751,6455 | CENTA1 | AW050627 | Centaurin, alpha 1 |
| 91682_at | 2,02E-05 | 0,95979387 | 196,32222 | 1,4538498 | 292,0318 | EXOSC4 | AI571298 | Exosome component 4 |
| 91703_at | 1,26E-06 | 1,0031043 | 842,51483 | 1,5317441 | 1301,409 | EHBP1L1 | AA149545 | EH domain binding protein 1-like 1 |
| 91816_f_at | 0,00806 | 0,82593465 | 87,144455 | 1,2501318 | 123,31818 | | C18318 | C18318 Human placenta cDNA (TFujiwara) Homo sapiens cDNA clone GEN-560E03 5', mRNA sequence. |
| AFFX-BioDn-3₋ | 0,00861 | 1,064509 | 8256,042 | 0,8094354 | 6043,745 | | | |
| AFFX-M27830₋ | 0,00152 | 1,0390761 | 457,73337 | 2,206056 | 1031,9727 | | M27830 | Human 28S ribosomal RNA gene, complete cds. |
| AFFX-r2-Hs28S | 8,68E-05 | 1,4356257 | 889,98157 | 6,0785093 | 2286,3362 | | M11167 | Human 28S ribosomal RNA gene. |

**Table 11: Expression levels of a set of transcripts discriminating liver transplant recipients under treatment with immunosuppressive drugs from healthy volunteers**

| | | Test Set | | HV I | Liver | Crossvalidation Set | | HV I | Liver | | |
|---|---|---|---|---|---|---|---|---|---|---|---|
| Affymetrix ID | Strengt h | p-value | Normalize d Fold Change | Raw Value | Raw Value | p-value | Normaliz ed Fold Change | Raw Value | Raw Value | Symbol | Product |
| 214129_at | 28,2 | 2,16E-05 | 0,591 | 425,789 | 263,414 | 3,74E-14 | 0,577 | 440,144 | 256,118 | PDE4DIP | Phosphodiesterase 4D interacting protein (myomegalin) |
| 207338_s_at | 25,92 | 0,000352 | 0,52 | 309,589 | 160,076 | 1,72E-13 | 0,419 | 398,719 | 165,855 | ZNF200 | Zinc finger protein 200 |
| 212831_at | 25,92 | 0,000702 | 1,648 | 234,156 | 368,376 | 8,90E-15 | 1,978 | 219,541 | 423,659 | EGFL5 | EGF-like-domain, multiple 5 |
| 219315_s_at | 25,92 | 0,00683 | 0,687 | 877,744 | 630,21 | 2,87E-11 | 0,483 | 1167,471 | 531,541 | C16orf30 | Chromosome 16 open reading frame 30 |
| 219777_at | 25,92 | 0,000166 | 0,45 | 1618,667 | 793,867 | 2,52E-14 | 0,351 | 2209,071 | | 801,791 GIMAP6 | GTPase, IMAP family member 6 |
| 36829_at | 25,92 | 9,70E-05 | 2,347 | 811,722 | 1887,057 | 3,54E-13 | 3,004 | 643,382 | 2030,746 | PER1 | Period homolog 1 (Drosophila) |
| 203449_s_at | 25,53 | 1,55E-05 | 0,685 | 360,333 | 248,286 | 1,38E-12 | 0,588 | 408,478 | 244,236 | TERF1 | Telomeric repeat binding factor (NIMA-interacting) 1 |
| 210240_s_at | 25,53 | 7,75E-06 | 1,826 | 704,889 | 1332,271 | 2,64E-12 | 2,032 | 686,452 | 1395,341 | CDKN2D | Cyclin-dependent kinase inhibitor 2D (p19, inhibits CDK4) |
| | | | | | | | | | | | |
| 201773_at | 25,15 | 1,55E-05 | 0,648 | 1015,145 | 661,971 | 1,72E-13 | 0,552 | 1207,355 | 678,109 | ADNP | Activity-dependent neuroprotector |
| 203427_at | 25,15 | 0,000561 | 0,52 | 476,278 | 260,948 | 3,75E-14 | 0,375 | 656,893 | 252,891 | ASF1A | ASF1 anti-silencing function 1 homolog A (S. cerevisiae) |
| 201170_s_at | 23,8 | 1,07E-06 | 2,264 | 1433,289 | 3371,681 | 1,92E-12 | 2,066 | 1476,485 | 3059,977 | BHLHB2 | Basic helix-loop-helix domain containing, class B, 2 |
| 201778_s_at | 23,8 | 0,000166 | 0,79 | 3176,367 | 2531,305 | 1,11E-10 | 0,779 | 3350 | 2600,982 | KIAA0494 | KIAA0494 gene product |
| 205214_at | 23,8 | 1,65E-06 | 3,151 | 1074,8 | 3087,491 | 1,72E-13 | 2,861 | 1109,474 | 2899,036 | STK17B | Serine/threonine kinase 17b (apoptosis-inducing) |
| 209662_at | 23,8 | 4,06E-05 | 0,406 | 353,533 | 160,762 | 2,96E-13 | 0,359 | 458,226 | 165,436 | CETN3 | Centrin, EF-hand protein, 3 (CDC31 homolog, yeast) |
| 214988_s_at | 23,8 | 5,47E-05 | 0,685 | 2867,478 | 2018,609 | 1,72E-13 | 0,584 | 3505,915 | 2067,264 | SON | SON DNA binding protein |
| 221193_s_at | 23,8 | 0,0127 | 0,77 | 234,511 | 178,405 | 4,93E-12 | 0,534 | 326,826 | 173,495 | ZCCHC10 | Zinc finger, CCHC domain containing 10 |
| 202983_at | 23,23 | 2,98E-05 | 0,544 | 409,089 | 227,819 | 9,93E-13 | 0,499 | 503,989 | 254,05 | SMARCA3 | SWI/SNF related, matrix associated, actin dependent regulator of chromatin, subfamily a, member 3 |
| 203037_s_at | 23,23 | 0,000702 | 0,713 | 2134,911 | 1561,053 | 1,72E-13 | 0,542 | 2511,645 | 1357,386 | MTSS1 | Metastasis suppressor 1 |
| 204181_s_at | 23,23 | 0,0049 | 1,403 | 315,744 | 447,476 | 4,99E-11 | 1,63 | 285,156 | | 464,027 ZNF297B | Zinc finger protein 297B |
| 206082_at | 23,23 | 0,000127 | 0,485 | 632,278 | 322,695 | 4,99E-11 | 0,465 | 645,615 | 301,382 | HCP5 | HLA complex P5 |
| 209566_at | 23,23 | 0,00197 | 0,682 | 233,933 | 163,019 | 8,16E-14 | 0,539 | 316,285 | 175,591 | INSIG2 | Insulin induced gene 2 |
| 218810_at | 23,23 | 7,75E-06 | 2,026 | 366,678 | 733,686 | 1,43E-10 | 2,41 | 326,207 | 808,527 | FLJ23231 | Hypothetical protein FLJ23231 |
| 202861_at | 22,74 | 5,47E-05 | 3,708 | 500,578 | 1594,857 | 1,22E-11 | 3,673 | 453,863 | 1669,3 | PER1 | Period homolog 1 (Drosophila) |
| 202912_at | 22,74 | 7,75E-06 | 2,544 | 337,389 | 826,052 | 4,93E-12 | 2,833 | 285,719 | 838,264 | ADM | Adrenomedullin |
| 205089_at | 22,74 | 5,38E-06 | 0,625 | 291,3 | 184,81 | 6,71 E-12 | 0,674 | 294,007 | 196,136 | ZNF7 | Zinc finger protein 7 (KOX 4, clone HF.16) |
| 205407_at | 22,74 | 2,98E-05 | 0,563 | 155,767 | 90,267 | 2,87E-11 | 0,46 | 228,619 | 102,982 | RECK | Reversion-inducing-cysteine-rich protein with kazal motifs |
| | | | | | | | | | | | |
| 209304_x_at | 22,74 | 0,000166 | 1,617 | 1121,978 | 1849,291 | 5,02E-13 | 2,214 | 902,352 | 1994,6 | GADD45B | Growth arrest and DNA-damage-inducible, beta |
| 217835_x_at | 22,74 | 0,000352 | 1,234 | 5461,278 | 6757,242 | 9,06E-12 | 1,361 | 4914,312 | 6694,923 | C20orf24 | Chromosome 20 open reading frame 24 |
| 202225_at | 22,55 | 0,00108 | 0,724 | 307,189 | 227,067 | 7,07E-13 | 0,664 | 337,611 | 223,555 | CRK | V-crk sarcoma virus CT10 oncogene homolog (avian) |
| 202521_at | 22,55 | 2,16E-05 | 0,73 | 1454,378 | 1069,414 | 3,54E-13 | 0,729 | 1463,023 | 1067,477 | CTCF | CCCTC-binding factor (zinc finger protein) |
| 202710_at | 22,55 | 2,16E-05 | 0,495 | 309,033 | 162,719 | 1,38E-12 | 0,444 | 392,678 | 170,014 | BET1 | BET1 homolog (S. cerevisiae) |
| 211758_x_at | 22,55 | 0,000215 | 0,694 | 1151,856 | 822,033 | 1,85E-10 | 0,632 | 1325,563 | 849,3 | TXNDC9 | Thioredoxin domain containing 9 |
| 211924_s_at | 22,55 | 0,000127 | 2,352 | 553,978 | 1423,048 | 3,88E-10 | 2,568 | 494,593 | 1382,168 | PLAUR | Plasminogen activator, urokinase receptor |
| 214696_at | 22,55 | 2,48E-06 | 2,959 | 595,856 | 1719,462 | 4,94E-12 | 2,835 | 643,111 | 1719,814 | MGC14376; | DKFZp686O06159 Homo sapiens clone 24659 mRNA sequence. |
| 222045_s_at | 22,55 | 1,10E-05 | 1,709 | 256,622 | 449,5 | 3,04E-10 | 1,67 | 253,404 | 414,423 | C20orf67 | Chromosome 20 open reading frame 67 |
| 201054_at | 21,92 | 0,000702 | 0,787 | 1100,5 | 862,5 | 1,85E-10 | 0,634 | 1389,27 | 868,05 | HNRPA0 | Heterogeneous nuclear ribonucleoprotein A0 |
| 201362_at | 21,92 | 9,70E-05 | 0,7 | 886,378 | 633,81 | 3,04E-10 | 0,687 | 954,767 | | 650,886 IVNS1ABP | Influenza virus NS1A binding protein |
| 203574_at | 21,92 | 7,75E-06 | 3,165 | 855,378 | 2659,586 | 2,17E-11 | 2,827 | 887,411 | 2425,164 | NFIL3 | Nuclear factor, interleukin 3 regulated |
| 216221_s_at | 21,92 | 7,32E-05 | 0,739 | 2464,267 | 1860,562 | 2,48E-13 | 0,684 | 2818,026 | 1929,891 | PUM2 | Vacuolar protein sorting 35 (yeast) |
| 219467_at | 21,92 | 5,38E-06 | 0,479 | 296,4 | 149,51 | 1,38E-12 | 0,496 | 329,063 | 163,723 FLJ20125 | | Hypothetical protein FLJ20125 |
| 220712_at | 21,92 | 1,55E-05 | 1,997 | 397,2 | 806,381 | 5,02E-13 | 2,048 | 372,83 | 772,286 | | |
| 200719_at | 21,18 | 0,0058 | 1,47 | 572,611 | 849,924 | 7,08E-13 | 1,626 | 515,119 | 835,568 SKP1A | | |
| 201142_at | 21,18 | 6,90E-07 | 0,665 | 780,933 | 524,795 | 2,29E-08 | 0,69 | 771,404 | 538,564 EIF2S1 | | Eukaryotic translation initiation factor 2, subunit 1 alpha, 35kDa |
| 202638_s_at | 21,18 | 0,0527 | 1,292 | 426,4 | 587,814 | 1,63E-11 | 1,897 | 293,674 | 551,95 ICAM1 | | Intercellular adhesion molecule 1 (CD54), human rhinovirus receptor |
| 202741_at | 21,18 | 0,000873 | 0,594 | 3197,489 | 2047,8 | 6,71E-12 | 0,478 | 4130,097 | 2009,955 PRKACB | | Protein kinase, cAMP-dependent, catalytic, beta |
| 203008_x_at | 21,18 | 0,000702 | 0,689 | 952,489 | 681,124 | 3,88E-10 | 0,614 | 1121,3 | 698,677 TXNDC9 | | Thioredoxin domain containing 9 |
| 204908_s_at | 21,18 | 9,70E-05 | 2,537 | 552,178 | 1528,624 | 2,17E-11 | 2,869 | 479,544 | 1482,263 BCL3 | | B-cell CLL/lymphoma 3 |
| 206130_s_at | 21,18 | 0,12 | 1,47 | 739,556 | 1168,305 | 4,35E-07 | 2,019 | 501,57 | 1078,423 ASGR2 | | Asialoglycoprotein receptor 2 |
| 209022_at | 21,18 | 0,00288 | 0,849 | 2378,156 | 2030,571 | 3,80E-11 | 0,722 | 2826,137 | 2049,077 STAG2 | | Stromal antigen 2 |
| 210180_s_at | 21,18 | 0,000702 | 1,678 | 312,267 | 529,905 | 1,43E-10 | 2,042 | 273,956 | 566,982 SFRS10 | | Splicing factor, arginine/serine-rich 10 (transformer 2 homolog, Drosophila) |
| 214438_at | 21,18 | 0,00683 | 1,409 | 388,533 | 571,386 | 3,88E-10 | 1,767 | 349,259 | 622,073 HLX1 | | H2.0-like homeo box 1 (Drosophila) |
| 218285_s_at | 21,18 | 0,00108 | 0,741 | 413,689 | 312,343 | 8,59E-09 | 0,664 | 439,074 | 297,127 DHRS6 | | Dehydrogenase/reductase (SDR family) member 6 |
| 201832_s_at | 20,7 | 5,47E-05 | 0,685 | 1438,978 | 1006,952 | 2,64E-12 | 0,663 | 1650,693 | 1096,282 VDP | | Vesicle docking protein p115 |
| 202060_at | 20,7 | 0,000702 | 0,644 | 1081,3 | 725,005 | 1,63E-11 | 0,572 | 1264,474 | 737,555 SH2BP1 | | SH2 domain binding protein 1 (tetratricopeptide repeat containing) |
| 203138_at | 20,7 | 0,0418 | 0,814 | 543,189 | 445,214 | 1,92E-12 | 0,59 | 717,474 | 424,9 HAT1 | | Histone acetyltransferase 1 |
| 203752_s_at | 20,7 | 2,48E-06 | 1,5 | 7904,689 | 11833,362 | 4,94E-10 | 1,442 | 7781,989 | 11121,354 JUND | | Jun D proto-oncogene |
| 209471_s_at | 20,7 | 0,0731 | 0,883 | 789,978 | 706,795 | 2,38E-10 | 0,675 | 1081,226 | 712,118 FNTA | | Farnesyltransferase, CAAX box, alpha |
| 209545_s_at | 20,7 | 0,00197 | 1,595 | 999,067 | 1661,305 | 1,38E-12 | 1,758 | 862,674 | 1572,155 RIPK2 | | Receptor-interacting serine-threonine kinase 2 |
| 211998_at | 20,7 | 1,10E-05 | 1,706 | 3085,678 | 5131,79 | 4,94E-10 | 1,9 | 2782,197 | 5213,937 H3F3B | | H3 histone, family 3B (H3.3B) |
| 212313_at | 20,7 | 7,32E-05 | 0,644 | 1047,522 | 692,367 | 1,43E-10 | 0,637 | 1131,078 | 707,836 MGC29816 | | Hypothetical protein MGC29816 |
| 218139_s_at | 20,7 | 0,00239 | 0,634 | 744,744 | 486,133 | 5,02E-13 | 0,514 | 921,722 | 479,818 C14orf108 | | Chromosome 14 open reading frame 108 |
| 219634_at | 20,7 | 3,68E-06 | 1,686 | 698,089 | 1180,772 | 1,38E-12 | 1,905 | 646,426 | 1192,4 CHST11 | | Carbohydrate (chondroitin 4) sulfotransferase 11 |
| 201158_at | 20,33 | 7,32E-05 | 0,242 | 231,556 | 90,967 | 4,99E-11 | 0,221 | 270,437 | 77,277 NMT1 | | N-myristoyltransferase 1 |
| 204370_at | 20,33 | 5,47E-05 | 1,89 | 635,211 | 1214,352 | 2,64E-12 | 1,823 | 593,433 | 1070,805 HEAB | | ATP/GTP-binding protein |
| 213188_s_at | 20,33 | 3,68E-06 | 0,692 | 440,2 | 309,21 | 1,28E-08 | 0,606 | 519,837 | 320,564 MINA | | MYC induced nuclear antigen |

Preferably the present invention is defined as in the following embodiments:
1. A method of identifying a subject with melanoma comprising:
   determining a dataset that comprises the level of expression of one or more melanoma expression vectors; and
   displaying each of the melanoma expression vectors with a separate identifier.
2. The method of embodiment 1, wherein the one or more melanoma expression vectors comprise three or more genes selected from Table 2, Table 8, Table 9, Table 12 or a combination thereof.
3. The method of embodiment 1, wherein the six or more genes disposed on a microarray, the genes selected from: RNA-binding region (RNP 1, RRM) containing 1; retinoblastoma binding protein 6; GrpE-like 1, mitochondrial (*E. coli*); pellino homolog 1 (Drosophila); ring finger protein 10; hypothetical protein LOC90637; Enhancer of polycomb homolog 1 (Drosophila); Full length insert cDNA clone ZB81B12; PTK9 protein tyrosine kinase 9; cell division cycle associated 4; period homolog 1 (Drosophila); zinc finger protein 237; TCDD-inducible poly(ADP-ribose) polymerase; cyclin L1; TSPY-like 2; glutamate receptor, ionotropic, N-methyl D-aspartate-like 1A; KIAA0863 protein; v-maf musculoaponeurotic fibrosarcoma oncogene homolog F (avian); PRO0149 protein; protein tyrosine phosphatase type IVA, member 1; serine/threonine kinase receptor associated protein; purine-rich element binding protein B; phosphodiesterase 4B, cAMP-specific (phosphodiesterase E4 dunce homolog, Drosophila); estrogen receptor binding protein; stress 70 protein chaperone, microsome-associated, 60kDa; BTG family, member 3; transferrin receptor (p90, CD71); hypothetical protein FLJ20436; phosphodiesterase 4D, cAMP-specific (phosphodiesterase E3 dunce homolog, Drosophila); KIAA0063 gene product; Vesicle transport through interaction with t-SNAREs homolog 1A (yeast); LOC440309; SNF1-like kinase; dual specificity phosphatase 8; RNA-binding region (RNP1, RRM) containing 2; dynein light chain 2; membrane associated DNA binding protein; serine/threonine kinase 17b (apoptosis-inducing); tumor necrosis factor, alpha-induced protein 3; heterogeneous nuclear ribonucleoprotein C (C1/C2); Phosphodiesterase 4D, cAMP-specific (phosphodiesterase E3 dunce homolog, Drosophila); kelch-like 15 (Drosophila); KIAA0863 protein; activating transcription factor 4 (tax-responsive enhancer element B67); villin 2 (ezrin); small nuclear ribonucleoprotein polypeptide A'; zinc finger protein 198; cAMP responsive element modulator; splicing factor 3a, subunit 1, 120kDa; CD83 antigen (activated B lymphocytes, immunoglobulin superfamily); DnaJ (Hsp40) homolog, subfamily B, member 6; chromosome 20 open reading frame 111; ethanolamine kinase 1; chromosome 4 open reading frame 15; ring finger protein 139; ribonuclease H1; jun D proto-oncogene; S-phase kinase-associated protein 1A (p19A); Methyl CpG binding protein 2 (Rett syndrome); heterogeneous nuclear ribonucleoprotein H3 (2H9); hypothetical protein DKFZp586I1420; nucleosome assembly protein 1-like 5; Ras-GTPase activating protein SH3 domain-binding protein 2; Zinc finger protein 331; Cullin 1; Shwachman-Bodian-Diamond syndrome; sterol-C5-desaturase (ERG3 delta-5-desaturase homolog, fungal)-like; phosphatidylinositol glycan, class A (paroxysmal nocturnal hemoglobinuria); isopentenyl-diphosphate delta isomerase; chromosome 20 open reading frame 67; v-maf musculoaponeurotic fibrosarcoma oncogene homolog K (avian); Wiskott-Aldrich syndrome protein interacting protein; kelch-like 7 (Drosophila); tumor necrosis factor, alpha-induced protein 3; hypothetical LOC388796; armadillo repeat containing 8; Membrane associated DNA binding protein; zinc finger protein 331; cAMP responsive element modulator; putative translation initiation factor; DnaJ (Hsp40) homolog, subfamily B, member 6; nuclear receptor subfamily 4, group A, member 2; cylindromatosis (turban tumor syndrome); nuclear receptor subfamily 4, group A, member 2; 6-pyruvoyltetrahydropterin synthase; activator of S phase kinase; activated RNA polymerase II transcription cofactor 4 (related to Activated RNA polymerase II transcriptional coactivator p15 (Positive cofactor 4) (PC4) (p14)); Hypothetical LOC388796; junction-mediating and regulatory protein; Hypothetical gene CG018; putative translation initiation factor; dual specificity phosphatase 5; Transducin-like enhancer of split 1 (E(sp1) homolog, Drosophila); mitochondrial carrier protein; protein tyrosine phosphatase type IVA, member 1; zinc finger protein 331; CDNA clone IMAGE:30332316, partial cds; interleukin enhancer binding factor 3, 90kDa; Homo sapiens, clone IMAGE:4753714, mRNA; SON DNA binding protein; AP1 gamma subunit binding protein 1; zinc finger protein 394; eukaryotic translation initiation factor 5; Activating transcription factor 7 interacting protein 2; hypothetical protein LOC285831; actin related protein 2/3 complex, subunit 5-like; hypothetical protein LOC144438; putative translation initiation factor; H3 histone, family 3B (H3.3B); kelch repeat and BTB (POZ) domain containing 2; CDNA FLJ40725 fis, clone TKIDN 1000001, highly similar to Translocase of inner mitochondrial membrane 23; cylindromatosis (turban tumor syndrome); phosphodiesterase 4D, cAMP-specific (phosphodiesterase E3 dunce homolog, Drosophila); heat shock 70kDa protein 14; HSPC128 protein; RNA (guanine-7-) methyltransferase; iduronate 2-sulfatase (Hunter syndrome); hypothetical protein MGC29814; Casein kinase 1, epsilon; protein phosphatase 1, regulatory (inhibitor) subunit 16B; isopentenyl-diphosphate delta isomerase; RAD23 homolog B (S. cerevisiae); Phosphodiesterase 4D, cAMP-specific (phosphodiesterase E3 dunce homolog, Drosophila); zinc finger protein 295; hypothetical protein FLJ13149; and BTG family, member 3 and combinations thereof.
4. The method of embodiment 1, wherein the six or more genes disposed on a microarray, the genes selected from: WARS; IFI53; IFP53; GAMMA-2; FAM46C; FLJ20202; H3F3B; H3.3B; FOXK2; ILF; ILF1; ILF-1; DUSP5; HVH3; ARF6; DKFZp762C186; BRD2; NAT; RNF3; FSRG1; RING3; D6S113E; KIAA9001; RORA; ROR1; ROR2; ROR3; RZRA; NR1F1; DKFZp762C186; DNAJB1; SUI1; CXCR4; HM89; LAP3; NPYR; WHIM; LESTR; NPY3R; HSY3RR; NPYY3R; D2S201E; GRINL1A; CTSB; TR1P-Br2; PDE4B; DPDE4; PDEIVB; PMAIP1; APR; NOXA; BTG2; PC3; TIS21; ASAHL; SON; SUI1; A121; ISO1; HERPUD1; SUP; Mif1; KIAA0025; DUSP2; PAC1; PAC-1; RNF139; RCA1; TRC8; HRCA1; MGC31961; TNFAIP3; A20; TNFA1P2; ARS2; HNRPL; hnRNP-L; P/OKcl.14; C20orf67; C20orf111; HSPC207; dJ1183I21.1; ZNF331; RITA; ZNF361; ZNF463; C20orf67; IER5; SBBI48; ; SUI1; JUN; AP1; CD69; TOB1; H3F3B; H3.3B; FOLR1; TNFAIP3; TCF8; BZP; ZEB; ZEB1; AREB6; ZFHEP; NIL-2A; ZFHX1A; NIL-2-A; DUSP10; MKP5; MKP-5; GGTLA4; MGC50550; dJ831C21.2; PMAIP1; ZC3HAV1; ZAP; FLB6421; ZC3HDC2; FLJ13288; MGC48898; DSIPI; DIP; GILZ; hDIP; TSC-22R; MCL1; TM; EAT; MCL1L; MCL1S; MGC1839; SH3TC1; FLJ20356; CIAS1; FCU; MWS; FCAS; NALP3; C1orf7; PYPAF1; AII/AVP; AGTAVPRL; SLC15A3; PHT2; PTR3; hPTR3; PTDSR; PSR; PTDSR1; KIAA0585; BHLHB2; DEC1; STRA13; Stra14; HMGE; KIAA0063; NR4A2; NOT; RNR1; HZF-3; NURR1; TINUR; NR4A2; NOT; RNR1; HZF-3; NURR1; TINUR; PTS; PTPS; HEAB; CLP1; hClp1; AREG; SDGF; CRDGF; MGC13647; EDG4; LPA2; EDG-4; LPAR2; CREM; ICER; MGC17881; MGC41893; CD83; BL11; HB15; ZNF394; FLJ12298 and combinations thereof.
5. The method of embodiment 1, wherein the one or more melanoma expression vectors comprise six or more genes over-expressed, under-expressed or combinations thereof selected from Table 12.
6. The method of embodiment 1, wherein the melanoma expression vectors comprises genes related to platelets, platelet glycoproteins, platelet-derived immune mediators, MHC/ribosomal proteins, MHC class I molecules, Beta 2-microglobulin, ribosomal proteins, hemoglobin genes or combinations thereof.
7. The method of embodiment 1, wherein the melanoma expression vectors comprises genes related to interferon-inducible genes, signaling molecules, kinases, RAS family members or combinations thereof.
8. The method of embodiment 1, wherein the level of expression of one or more melanoma expression vectors comprise the mRNA expression level, protein expression level or both mRNA expression level and protein expression level.
9. The method of embodiment 1, wherein the level of expression comprises a mRNA expression level and is quantitated by a method selected from the group consisting of polymerase chain reaction, real time polymerase chain reaction, reverse transcriptase polymerase chain reaction, hybridization, probe hybridization and gene expression array.
10. The method of embodiment 1 further comprising the step of detecting one or more polymorphisms in the one or more melanoma expression vectors.
11. The method of embodiment 1, wherein the level of expression is determined using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing.
12. A computer implemented method for determining the phenotype of a sample comprising:
   storing a plurality of sample probe intensities obtained from a gene microarray contacted with genes expressed by lymphocytes from a patient into a dataset;
   diagnosing the presence of melanoma in the patient based upon the plurality of sample probe intensities; and
   calculating a linear correlation coefficient between the plurality of sample probe intensities and a reference probe intensities; and
   accepting the tentative phenotype as the phenotype of the sample if the linear correlation coefficient is greater than a threshold value.
13. The computer implemented method of embodiment 12, wherein the method is stored onto a computer readable medium comprising computer-executable instructions for performing the method for determining the phenotype of a sample comprising:
   obtaining a plurality of sample probe intensities;
   diagnosing melanoma based upon the sample probe intensities for two or more metastatic melanoma expression vectors selected from one or more the genes listed in Tables 9 to 14; and
   calculating a linear correlation coefficient between the sample probe intensities and a reference probe intensity; and
   accepting the tentative phenotype as the phenotype of the sample if the linear correlation coefficient is greater than a threshold value.
14. A method of identifying a subject with immunosuppression associated with transplants comprising:
   calculating one or more immunosuppression expression vectors, each vector comprising the calculated expression of a subset of genes associated with immunosuppression; and
   displaying each of the melanoma expression vectors with a separate identifier.
15. The method of embodiment 14, wherein the six or more genes associated with immunosuppression are selected from Table 10, Table 11, Table 13 or a combination thereof.
16. The method of embodiment 14, wherein the level of expression of one or more immunosuppression associated expression vectors comprise the mRNA expression level, protein expression level or both mRNA expression level and protein expression level.
17. The method of embodiment 14, wherein the level of expression comprises a mRNA expression level and is quantitated by a method selected from the group consisting of polymerase chain reaction, real time polymerase chain reaction, reverse transcriptase polymerase chain reaction, hybridization, probe hybridization, and gene expression array.
18. The method of embodiment 14, further comprising the step of detecting one or more polymorphisms in the one or more immunosuppression associated expression vectors.
19. The method of embodiment 14, wherein the level of expression is determined using at least one technique selected from the group consisting of polymerase chain reaction, heteroduplex analysis, single stand conformational polymorphism analysis, ligase chain reaction, comparative genome hybridization, southern blotting, northern blotting, western blotting, enzyme-linked immunosorbent assay, fluorescent resonance energy-transfer and sequencing.
20. The method of embodiment 14, wherein the one or more immunosuppression associated expression vectors are derived from a leukocyte.
21. A computer implemented method for determining the propensity for immunosuppression in a sample from a patient suspected of having immunosuppression comprising:
   obtaining a dataset that comprises a plurality of sample probe intensities;
   calculating linear correlation coefficient between the sample probe intensities and reference probe intensities;
   diagnosing immunosuppression based upon the sample probe intensities; and
   accepting the tentative phenotype as the phenotype of the sample if the linear correlation coefficient is greater than a threshold value.
22. A microarray for identifying a human subject with melanoma comprising:
   disposing six or more genes on a substrate selected from the group consisting of six or more genes selected from Table 2, Table 8, Table 9, Table 12 or a combination thereof, wherein the genes are selected to include genes that are used to obtain a dataset of gene expression data that is used to calculate the presence of melanoma in a patient based on two or more positive indicators in a gene expression vector dataset.
23. A microarray for identifying a human subject predisposed to immunosuppression comprising:
   disposing six or more genes on a substrate selected from the group consisting of six or more genes selected from Table 10, Table 11, Table 13 or a combination thereof wherein the genes are selected to include genes that are used to obtain a dataset of gene expression data that is used to calculate the presence of immunosuppression in a patient based on two or more positive indicators in a gene expression vector dataset.
24. A method for displaying transcriptome vector data comprising:
   separating one or more genes into one or more modules to visually display an aggregate gene expression vector value for each of the modules; and
   displaying the aggregate gene expression vector value for overexpression, underexpression or equal expression of the aggregate gene expression vector value in each module.
25. The method of embodiment 23, wherein overexpression is identified with a first identifier and underexpression is identified with a second identifier.
26. The method of embodiment 23, wherein overexpression is identified with a first identifier and underexpression is identified with a second identifier, wherein the first identifier is a first color and the second identifier is a second color, wherein first and second identifiers are superimposed to provide a combined color.

### References

Agostini, L., Martinon, F., Bums, K., McDermott, M. F., Hawkins, P. N., and Tschopp, J. (2004). NALP3 forms an IL-lbeta-processing inflammasome with increased activity in Muckle-Wells autoinflammatory disorder. Immunity 20, 319-325.
Barrett, W. L., First, M. R., Aron, B. S., and Penn, I. (1993). Clinical course of malignancies in renal transplant recipients. Cancer 72, 2186-2189.
Berrebi, D., Bruscoli, S., Cohen, N., Foussat, A., Migliorati, G., Bouchet-Delbos, L., Maillot, M. C., Portier, A., Couderc, J., Galanaud, P., et al. (2003). Synthesis of glucocorticoid-induced leucine zipper (GILZ) by macrophages: an anti-inflammatory and immunosuppressive mechanism shared by glucocorticoids and IL-10. Blood 101, 729-738.
Bordea, C., Wojnarowska, F., Millard, P. R., Doll, H., Welsh, K., and Morris, P. J. (2004). Skin cancers in renal-transplant recipients occur more frequently than previously recognized in a temperate climate. Transplantation 77, 574-579.
Carroll, R. P., Ramsay, H. M., Fryer, A. A., Hawley, C. M., Nicol, D. L., and Harden, P. N. (2003). Incidence and prediction of nonmelanoma skin cancer post-renal transplantation: a prospective study in Queensland, Australia. Am J Kidney Dis 41, 676-683.
Chaussabel, D., and Sher, A. (2002). Mining microarray expression data by literature profiling. Genome Biol 3, RESEARCH0055.
Choi, B. M., Pae, H. O., Jeong, Y. R., Kim, Y. M., and Chung, H. T. (2005). Critical role of heme oxygenase-1 in Foxp3-mediated immune suppression. Biochem Biophys Res Commun 327, 1066-1071.
Corradetti, M. N., Inoki, K., and Guan, K. L. (2005). The stress-inducted proteins RTP801 and RTP801L are negative regulators of the mammalian target of rapamycin pathway. J Biol Chem 280, 9769-9772.
D'Adamio, F., Zollo, O., Moraca, R., Ayroldi, E., Bruscoli, S., Bartoli, A., Cannarile, L., Migliorati, G., and Riccardi, C. (1997). A new dexamethasone-induced gene of the leucine zipper family protects T lymphocytes from TCR/CD3-activated cell death. Immunity 7, 803-812.
Gabrilovich, D. (2004). Mechanisms and functional significance of tumour-induced dendritic-cell defects. Nat Rev Immunol 4, 941-952.
Gerlini, G., Romagnoli, P., and Pimpinelli, N. (2005). Skin cancer and immunosuppression. Crit Rev Oncol Hematol 56, 127-136.
Jachimczak, P., Apfel, R., Bosserhoff, A. K., Fabel, K., Hau, P., Tschertner, I., Wise, P., Schlingensiepen, K. H., Schuler-Thurner, B., and Bogdahn, U. (2005). Inhibition of immunosuppressive effects of melanoma-inhibiting activity (MIA) by antisense techniques. Int J Cancer 113, 88-92.
Kovanen, P. E., Rosenwald, A., Fu, J., Hurt, E. M., Lam, L. T., Giltnane, J. M., Wright, G., Staudt, L. M., and Leonard, W. J. (2003). Analysis of gamma c-family cytokine target genes. Identification of dual-specificity phosphatase 5 (DUSP5) as a regulator of mitogen-activated protein kinase activity in interleukin-2 signaling. J Biol Chem 278, 5205-5213.
Lee, J. H., Torisu-Itakara, H., Cochran, A. J., Kadison, A., Huynh, Y., Morton, D. L., and Essner, R. (2005a). Quantitative analysis of melanoma-induced cytokine-mediated immunosuppression in melanoma sentinel nodes. Clin Cancer Res 11, 107-112.
Lee, Y. R., Yang, I. H., Lee, Y. H., Im, S. A., Song, S., Li, H., Han, K., Kim, K., Eo, S. K., and Lee, C. K. (2005b). Cyclosporin A and tacrolimus, but not rapamycin, inhibit MHC-restricted antigen presentation pathways in dendritic cells. Blood.
Liyanage, U. K., Moore, T. T., Joo, H. G., Tanaka, Y., Herrmann, V., Doherty, G., Drebin, J. A., Strasberg, S. M., Eberlein, T. J., Goedegebuure, P. S., and Linehan, D. C. (2002). Prevalence of regulatory T cells is increased in peripheral blood and tumor microenvironment of patients with pancreas or breast adenocarcinoma. J Immunol 169, 2756-2761.
Monti, P., Leone, B. E., Zerbi, A., Balzano, G., Cainarca, S., Sordi, V., Pontillo, M., Mercalli, A., Di Carlo, V., Allavena, P., and Piemonti, L. (2004). Tumor-derived MUC1 mucins interact with differentiating monocytes and induce IL-10highIL-12low regulatory dendritic cell. J Immunol 172, 7341-7349.
Powell, J. D., Lerner, C. G., Ewoldt, G. R., and Schwartz, R. H. (1999). The -180 site of the IL-2 promoter is the target of CREB/CREM binding in T cell anergy. J Immunol 163, 6631-6639.
Puente Navazo, M. D., Valmori, D., and Ruegg, C. (2001). The alternatively spliced domain TnFnIII A1A2 of the extracellular matrix protein tenascin-C suppresses activation-induced T lymphocyte proliferation and cytokine production. J Immunol 167, 6431-6440.
Seimiya, M., Wada, A., Kawamura, K., Sakamoto, A., Ohkubo, Y., Okada, S., Hatano, M., Tokuhisa, T., Watanabe, T., Saisho, H., et al. (2004). Impaired lymphocyte development and function in Clast5/Stra13/DEC1-transgenic mice. Eur J Immunol 34, 1322-1332.
Soares, M. P., Lin, Y., Anrather, J., Csizmadia, E., Takigami, K., Sato, K., Grey, S. T., Colvin, R. B., Choi, A. M., Poss, K. D., and Bach, F. H. (1998). Expression of heme oxygenase-1 can determine cardiac xenograft survival. Nat Med 4, 1073-1077.
Theodosiou, A., Smith, A., Gillieron, C., Arkinstall, S., and Ashworth, A. (1999). MKP5, a new member of the MAP kinase phosphatase family, which selectively dephosphorylates stress-activated kinases. Oncogene 18, 6981-6988.
Viguier, M., Lemaitre, F., Verola, O., Cho, M. S., Gorochov, G., Dubertret, L., Bachelez, H., Kourilsky, P., and Ferradini, L. (2004). Foxp3 expressing CD4+CD25(high) regulatory T cells are overrepresented in human metastatic melanoma lymph nodes and inhibit the function of infiltrating T cells. J Immunol 173, 1444-1453.
Winoto, A., and Littman, D. R. (2002). Nuclear hormone receptors in T lymphocytes. Cell 109 Suppl, S57-66.
Woltman, A. M., van der Kooij, S. W., Coffer, P. J., Offringa, R., Daha, M. R., and van Kooten, C. (2003). Rapamycin specifically interferes with GM-CSF signaling in human dendritic cells, leading to apoptosis via increased p27KIP1 expression. Blood 101, 1439-1445.
Xu, X., Su, B., Barndt, R. J., Chen, H., Xin, H., Yan, G., Chen, L., Cheng, D., Heitman, J., Zhuang, Y., et al. (2002). FKBP12 is the only FK506 binding protein mediating T-cell inhibition by the immunosuppressant FK506. Transplantation 73, 1835-1838.

## Claims

1. A method of identifying a subject with melanoma comprising:
determining two or more melanoma vectors of expression, wherein the two or more melanoma vectors of expression comprise the expression level of six or more genes over-expressed, under-expressed or combinations thereof selected from of Table 10 or 11; and
displaying each of the melanoma expression vectors with a separate identifier.

2. The method of claim 1, further comprising the step of detecting one or more polymorphisms in the six or more genes.

3. The method of claim 1 further comprising a method for displaying melanoma transcriptome vector data comprising:
separating one or more genes into one or more modules to visually display an aggregate gene expression vector value for each of the modules; and
displaying the aggregate gene expression vector value for overexpression, underexpression or equal expression of the aggregate gene expression vector value in each module.

4. The method of any one of claims 1 to 3, wherein the six or more genes comprise genes related to platelets, platelet glycoproteins, platelet-derived immune mediators, MHC/ribosomal proteins, MHC class I molecules, Beta 2-microglobulin, ribosomal proteins, hemoglobin genes or combinations thereof and/or genes related to interferon-inducible genes, signaling molecules, kinases, RAS family members or combinations thereof.

5. The method of any one of claims 1 to 4, wherein the expression level comprises mRNA expression level, protein expression level or both mRNA expression level and protein expression level.

6. The method of any one of claims 1 to 5, wherein the expression level-comprises a mRNA expression level and is quantitated by a method selected from the group consisting of polymerase chain reaction, real time polymerase chain reaction, reverse transcriptase polymerase chain reaction, hybridization, probe hybridization and gene expression array.

7. The method of any one of claims 1 to 6, wherein the expression level is determined using at least one technique selected from the group consisting of polymerase chain reaction, heteroduplex analysis, single stand conformational polymorphism analysis, ligase chain reaction, comparative genome hybridization, Southern blotting, Northern blotting, Western blotting, enzyme-linked immunosorbent assay, fluorescent resonance energy-transfer and sequencing.

8. The method of claim 3, wherein overexpression is identified with a first identifier and underexpression is identified with a second identifier.

9. The method of claim 3, wherein overexpression is identified with a first identifier and underexpression is identified with a second identifier, wherein the first identifier is a first color and the second identifier is a second color, wherein first and second identifiers are superimposed to provide a combined color.

10. A computer readable medium comprising computer-executable instructions for performing the method of claim 1.
